# EUROPEAN PATENT SPECIFICATION

(11) **EP 2 320 947 B1**
(45) Date of publication and mention of the grant of the patent: **03.06.2015**
(21) Application number: 09795269.1
(22) Date of filing: 10.07.2009
(51) Int. Cl.: C07H 15/18, A61K 31/7034, A61K 39/39, A61K 31/70, A61P 35/00, A61P 31/16, A61P 31/04, A61K 39/00

(54) **ALPHA-GALACTOSYL CERAMIDE ANALOGS AND THEIR USE AS IMMUNOTHERAPIES, ADJUVANTS, AND INTIVIRAL, ANTIBACTERIAL, AND ANTICANCER AGENTS**
ALPHA-GALACTOSYL-CERAMID-ANALOGA UND IHRE VERWENDUNG ALS IMMUNTHERAPIE, HILFSSTOFFE UND ANTIVIRALE, ANTIBAKTERIELLE SOWIE KREBSMITTEL
ANALOGUES D'ALPHA-GALACTOSYLCÉRAMIDE, ET LEUR UTILISATION EN IMMUNOTHÉRAPIES, EN TANT QU'ADJUDANTS, ET EN TANT QU'AGENTS ANTIVIRAUX, ANTIBACTÉRIENS ET ANTICANCÉREUX

(30) Priority: 11.07.2008 US 218082
(43) Date of publication of application: 18.05.2011
(73) Proprietor: Academia Sinica, Taipei 115 (TW)
(72) Inventor: WONG, Chi-Huey, La Jolla, CA 92037 (US); YU, Alice, La Jolla, CA 92037 (US); CHANG, Ya-Jen, Boston, MA 02115 (US); LIN, Kun-Hsien, Zhonghe Dist. New Taipei City 235 (TW); CHENG, Edmond Y.S., Taipei (TW); JAN, Jia-Tsrong, Sindian, Taipei (TW); LIN, Yi-Ling, Xin-Dian, New Taipei City 231 (TW); HUNG, Jung-Tung, Da'an Dist., Taipei City 106 (TW)
(74) Representative: Roques, Sarah Elizabeth
(86) International application number: PCT/US2009/050328
(87) International publication number: WO 2010/006315

(56) References cited:
- WO-A1-2008/128207
- WO-A2-2006/026389
- WO-A2-2007/035717
- US-A1- 2007 238 871

## Description

### FIELD OF THE DISCLOSURE

The present disclosure relates to alpha-galactosyl ceramide (α-GalCer) analogs, and their use as immunotherapies, adjuvants, and antiviral, antibacterial, and anticancer agents.

### BACKGROUND

Natural killer T cells (NKTs) represent a subset of T lymphocytes with unique properties, including reactivity for natural or synthetic glycolipids presented by CD1d and expression of an invariant T cell antigen receptor (TCR) alpha chain. NKTs are different from functionally differentiated conventional αβ T cells in that they share properties of both natural killer cells and T cells are can rapidly produce both T_{H}1-type and T_{H}2-type responses upon stimulation with their ligands (innate immunity). The activation of NKTs paradoxically can lead either to suppression or stimulation of immune responses. For example, the production of T_{H}1 cytokines is thought to promote cellular immunity with antitumor, antiviral/antibacterial, and adjuvant activities, whereas T_{H}2 cytokine production is thought to subdue autoimmune diseases and promote antibody production. Because NKTs play a regulatory role in the immune system, they are attractive targets for immunotherapy.

Examples of α-Galcer having such properties are disclosed in US 2007/0238871, WO 2006/026389 or WO 2007/035717.

### SUMMARY OF THE DISCLOSURE

The present invention provides a compound represented by the structure of formula 1: 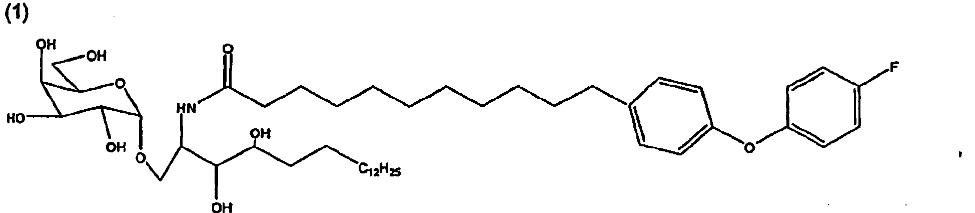 or a pharmaceutically acceptable salt thereof; for use in medicine.

The present invention also provides a method of synthesizing a compound represented by the structure of formula 1: 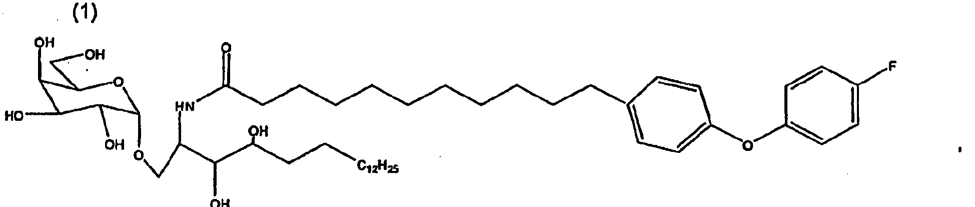 comprising:
selectively protecting 2,3-dihydroxy groups of D-lyxose by 2-methoxypropene in the presence of acid to give an actonide intermediate;
   subjecting a primary hydroxyl group of the actonide intermediate to trityl chloride and base condition to give a trityl ether intermediate;
   reacting the trityl ether intermediate with C₁₃H₂₇PPh₃-Br by Wittig olefination in the presence of lithium hexamethyldisilazide (LHMDS), whereby an alkene intermediate with the *E*/*Z* ratio of 2:1 by ¹H NMR spectrometry characterization is yielded;
   hydrogenating the alkene intermediate to give an alkane;
   activating a hydroxy group of the alkane by triflate anhydride and 2,6-lutidine to give a triflate intermediate;
   reacting the triflate intermediate with tetramethylguanidinium azide (TMGA) by S_{N}2 reaction to give an azido compound with inverted configuration;
   removing a trityl group of the azido compound by using triflouroacetic acid (TFA) to give phytosphingosine;
   effecting glycosylation of doner-galactose derivative and acceptor phytosphingosine using trifluoromethanesulfonic anhydride (Tf₂O) and dimethyl sulfide (Me₂S) as promoters to give a key intermediate having an azido group;
   reducing the azido group of the key intermediate using a Staudinger reaction to give an amine intermediate; and
   coupling the amine intermediate with fatty acid using EDC and HBTU.

The present invention also provides a compound of formula 1: 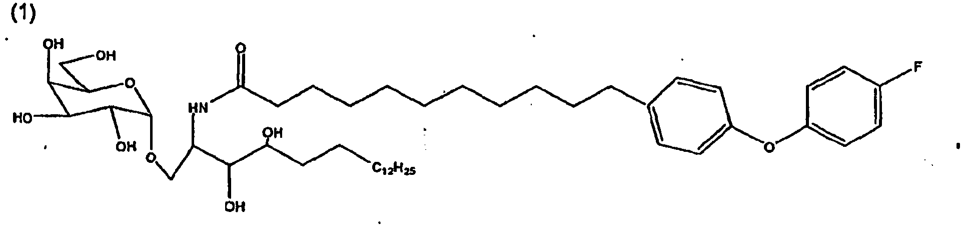 or a pharmaceutically acceptable salt thereof, for use in the treatment or prophylaxis of a viral or bacterial infection.

The present invention also provides a compound of formula 1: 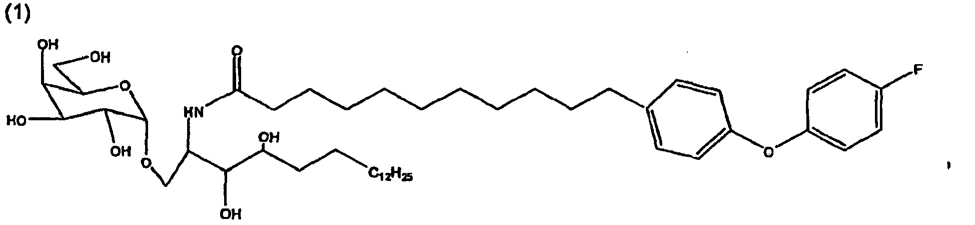 or a pharmaceutically acceptable salt thereof, for use in the treatment of cancer.

In one exemplary implementation, the compound of the invention is for use in a method in which DC development may be stimulated via the use of granulocyte-macrophage colony-stimulating-factor (GM-CSF), or in another exemplary implementation, interleukin (IL)-3, which may, in another exemplary implementation, enhance DC survival.

As described herein, the DCs may express myeloid markers, such as, for example, CD11c or an IL-3 receptor-α (IL-3Rα) chain (CD123). In another exemplary implementation, the compound of the invention is for use in a method in which the DCs may produce type I interferons (IFNs). Also as described herein, the DCs express costimulatory molecules. In another exemplary implementation, the compound of the invention is for use in a method in which the DCs may express additional adhesion molecules, which may, in one implementation, serve as additional costimulatory molecules, or as described herein, serve to target the DCs to particular sites *in vivo,* as described further hereinbelow.

As described herein, the compound of the invention is for use in a method in which the dendritic cells may express CD83, an endocytic receptor to increase uptake of the autoantigen such as DEC-205/CD205 in one implementation, or DC-LAMP (CD208) cell surface markers, or, in another implementation, varying levels of the antigen presenting MHC class I and II products, or in another implementation, accessory (adhesion and costimulatory) molecules including CD40, CD54, CD58 or CD86, or any combination thereof. In another implementation, the compound of the invention is for use in a method in which the dendritic cells may express varying levels of CD115, CD14, CD68 or CD32.

As described herein, the dendritic cells may be mature dendritic cells. The term "mature dendritic cells" may refer to a population of dendritic cells with diminished CD115, CD14, CD68 or CD32 expression, or a population of cells with enhanced CD86 expression, or a combination thereof. Mature dendritic cells may exhibit increased expression of one or more of p55, CD83, CD40 or CD86 or a combination thereof. In an implementation, the compound of the invention is for use in a method in which the dendritic cells will express the DEC-205 receptor on their surface. In another implementation, the compound of the invention is for use in a method in which maturation of the DCs may be accomplished via, for example, CD40 ligation, CpG oligodeoxyribonucleotide addition, ligation of the IL-1, TNFα or TOLL like receptor ligand, bacterial lipoglycan or polysaccharide addition or activation of an intracellular pathway such as TRAF-6 or NF-κB.

In one exemplary implementation, the compound of the invention is for use in a method in which inducing DC maturation may be in combination with endocytic receptor delivery of a preselected antigen. In one implementation, the compound of the invention is for use in a method in which endocytic receptor delivery of antigen may be via the use of the DEC-205 receptor.

In one exemplary implementation, the compound of the invention is for use in a method in which the maturation status of the dendritic may be confirmed, for example, by detecting either one or more of 1) an increase expression of one or more of p55, CD83, CD40 or CD86 antigens; 2) loss of CD115, CD14, CD32 or CD6B antigen; or 3) reversion to a macrophage phenotype characterized by increased adhesion and loss of veils following the removal of cytokines which promote maturation of PBMCs to the immature dendritic cells, by methods well known in the art, such as, for example, immunohistochemistry, FACS analysis, and others.

In one implementation, the compound of the invention is for use in a method in which NKT expansion varies in response to a presenting antigen. In one implementation, the compound of the invention is for use in a method in which an α-GalCer analog of this disclosure is supplied in the culture simultaneously with dendritic cell contact with the NKTs. In another implementation, the compound of the invention is for use in a method in which dendritic cells, which have already processed antigen are contacted with the NKTs.

The term "contacting a target cell" may refer herein to both direct and indirect exposure of cell to the indicated item. In one implementation, the compound of the invention is for use in a method in which contact of NKTs with an α-GalCer analog of this disclosure, a cytokines, growth factor, dendritic cell, or combination thereof, is direct or indirect. In one implementation, the compound of the invention is for use in a method in which contacting a cell may comprise direct injection of the cell through any means well known in the art, such as microinjection. It is also envisioned, in another implementation, that the compound of the invention is for use in a method in which supply to the cell is indirect, such as via provision in a culture medium that surrounds the cell, or administration to a subject, via any route well known in the art, and as described hereinbelow.

Methods for priming dendritic cells with antigen are well known to one skilled in the art, and may be effected, as described for example Hsu et al., Nature Med. 2:52-58 (1996); or Steinman et al. International application PCT/US93/03141.

In one implementation, the compound of the invention is for use in a method in which the α-GalCer analog is administered to a subject, and, in another implementation, the compound of the invention is for use in a method in which α-GalCer is targeted to the dendritic cell, wherein uptake occurs *in vivo,* for methods as described hereinbelow.

In one Implementation, the compound of the invention is for use in a method in which α-GalCer analog uptake and processing can occur within 24 hours, or in another implementation, the compound of the invention is for use in a method in which longer periods of time may be necessary, such as, for example, up to and including 4 days or, in another implementation, the compound of the invention is for use in a method in which shorter periods of time may be necessary, such as, for example, about 1-2 hour periods.

In another implementation, the compound of the invention is for use in a method in which the NKTs expanded by the dendritic cells are autologous, syngeneic or allogeneic, with respect to the dendritic cells.

As described herein, the NKTs can be used to modulate an immune response, In a disease-specific manner. It is to be understood that any immune response, wherein it is desired to enhance cytokine production, or elicit a particular cytokine profile, including interferon-γ, interleukin-2 and/or interleukin-4, the NKT cells may be thus utilized.

As described herein, the methods may further comprise the step of culturing previously isolated, NKTs with additional dendritic cells, and an α -GalCer analog of the present disclosure, for a period of time resulting in further NKT expansion, cytokine production, or a combination thereof.

Also described herein is a method for delaying onset, reducing incidence or suppressing a disease in a subject, comprising the steps of contacting in a culture NKTs with dendritic cells and an α -GalCer analog of the present disclosure, for a period of time resulting in NKT expansion, cytokine production or a combination thereof, and administering NKTs thus obtained to the subject, wherein the NKTs delay onset, reduce incidence or suppress a disease in the subject, thereby delaying onset, reducing incidence or suppressing a disease in the subject.

In one exemplary implementation, the compound of the invention is for use in a method in which cells for administration to a subject in this disclosure may be provided in a composition. These compositions may be administered parenterally or intravenously. The compositions for administration may be sterile solutions, or aqueous or non-aqueous suspensions or emulsions. The compositions may comprise propylene glycol, polyethylene glycol, injectable organic esters, for example ethyl oleate, or cyclodextrins. The compositions may also comprise wetting, emulsifying and/or dispersing agents. The compositions may also comprise sterile water or any other sterile injectable medium. The compositions may comprise adjuvants, which are well known to a person skilled in the art (for example, vitamin C, antioxidant agents, etc.) for some of the methods as described herein, wherein stimulation of an immune response is desired, as described further hereinbelow.

In one exemplary implementation, the disclosure provides a compound represented by the structure of formula 1:

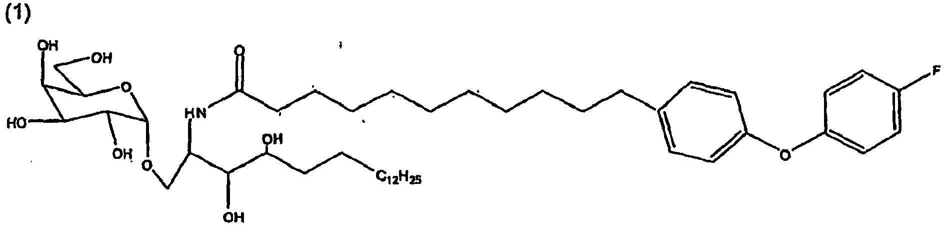

Also described herein is a compound represented by the structure of formula 2:

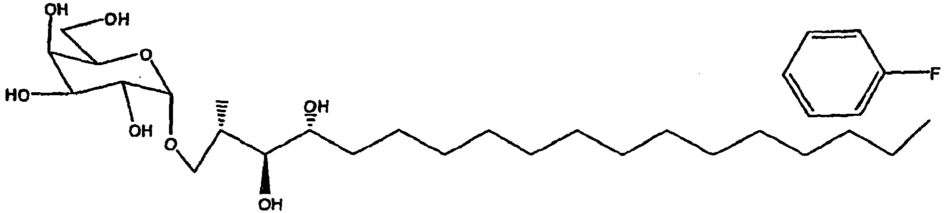

Also described herein is a compound represented by the structure of formula 3:

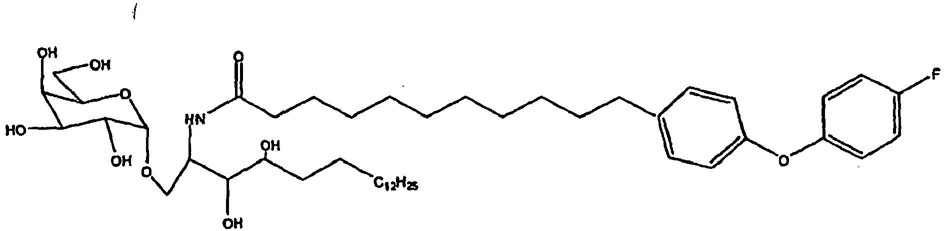

In one implementation, the compound of the invention is for use in a method in which the α -GalCer analogs, cells, vaccines or compositions of this disclosure may be administered to a subject via injection. In one implementation, injection may be via any means known in the art, and may include, for example, intra-lymphoidal, or SubQ injection.

In one implementation, the compound of the invention is for use in a method in which the α -GalCer analogs of the present disclosure are delivered to dendritic cells *in vivo* in the steady state, which, in another implementation, leads to expansion of disease ameliorating NKTs. Analog delivery in the steady state can be accomplished, as described in Bonifaz, et al. (2002) Journal of Experimental Medicine 196: 1627-1638; Manavalan et al. (2003) Transpl Immunol. 11: 245-58.

In another exemplary implementation, the compound of the invention is for use in a method in which select types of dendritic cells *in vivo* function to prime the NKTs.

Also described herein is a method for modulating an immune response, 8 which is an inappropriate or undesirable response. The immune response may be marked by a cytokine profile which is deleterious to the host.

In one exemplary implementation, the compound of the invention is for use in a method in which the NKTs of this disclosure may be administered to a recipient contemporaneously with treatment for a particular disease, such as, for example, contemporaneous with standard anti-cancer therapy, to serve as adjunct treatment for a given cancer. In another implementation, the compound of the invention is for use in a method in which the NKTs of this disclosure may be administered prior to the administration of the other treatment.

Also described herein is a method for modulating an immune response, which is directed to infection with a pathogen, and the immune response is not protective to the subject.

In another exemplary implementation, the compound of the invention is for use in a method in which the immune response results in a cytokine profile, which is not beneficial to the host. In one implementation, the compound of the invention is for use in a method in which the cytokine profile exacerbates disease. In one implementation, the compound of the invention is for use in a method in which a T_{H}2 response is initiated when a T_{H}1 response is beneficial to the host, such as for example, in lepromatous leprosy. In another implementation, the compound of the invention is for use in a method in which a T_{H}1 response is initiated, and persists in the subject, such as for example, responses to the egg antigen is schistosomiasis.

Also described herein is a method of activating a cytokine response in a subject whereby an effective amount of a compound or a salt or a mixture is administered, wherein the subject has an adaptive immune system that includes a population of cells, the population including at least one lymphocyte and at least one antigen-presenting cell, and wherein the compound is represented by the structure of formula 1: 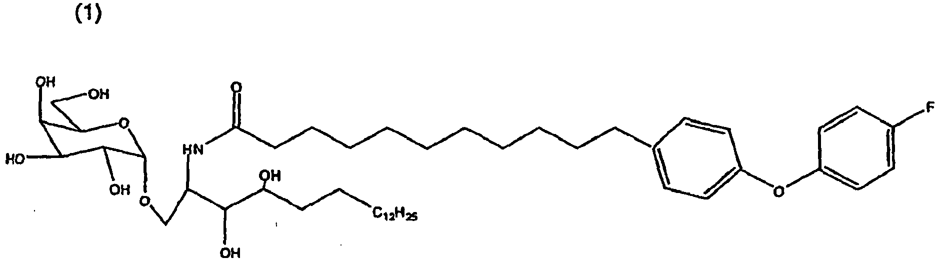 or a pharmaceutically acceptable salt thereof;
forming a complex between the compound and the antigen-presenting cell, wherein the formation of the complex results in the activation of a receptor on the lymphocyte; and
activating the lymphocyte to produce the cytokine response.

The at least one lymphocyte may be a T lymphocyte and in some cases the T lymphocyte may be a Natural Killer T cell. In some instances the Natural Killer T cell is an invariant Natural Killer T cell.

The at least one antigen-presenting cell may be a dendritic cell. In some instances the dendritic cell is an immature or a mature dendritic cell.

Administering the compound may be accomplished by subcutaneous administration, intravenous administration, intranasal administration or intramuscular administration.

The compound may form a complex with a CD1 molecule on the antigen-presenting cell. In some instances the CD1 molecule is a CD1d molecule. In some instances the receptor on the T lymphocyte is a T cell receptor. In some instances stimulating at least one other lymphocyte to produce the cytokine response, in some instances the at least one other lymphocyte is a T helper cell.

The cytokine response may be a T_{H}1-type cytokine response which produces T_{H}1 cytokines which may also be selected from the group consisting of IFN-γ, IL-1β, IL-2, IL-3, IL-8, IL-12, IL-15, TNF-α, GM-CSF, RANTES, MIP-1α and MCP-1.

Also as described herein, the cytokine response is a T_{H}2-type cytokine response which produces T_{H}2 cytokines which may also be selected from the group consisting of IL-4, IL-6, IL-8, IL-10, IL-13, RANTES, MIP-1α and MCP-1

In some exemplary implementations the disclosure provides a vaccine comprising an effective amount of a compound represented by the structure of formula 1: 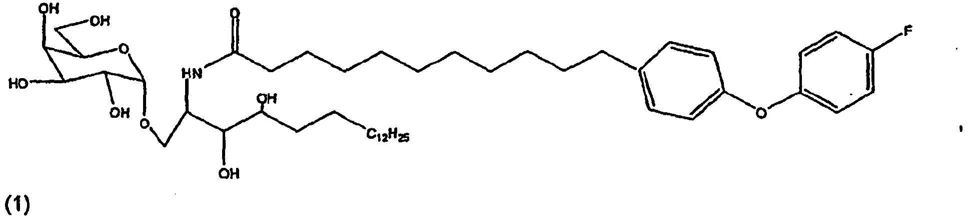 or a pharmaceutically acceptable salt thereof; and a vaccine agent.

In some instances the vaccine agent is selected from the group consisting of a killed microorganism, a live attenuated virus microorganism, a toxoid and a fragment of an inactivated or attenuated microorganism. In some instances the microorganism is a bacteria or a fungi. In some instances the toxoid is a tetanus or a diphtheria. In some instances the vaccine agent is capable of eliciting an immune response in a subject that is administered the vaccine. In some instances the compound acts as an immunologic adjuvant and is capable of modifying or augmenting the immune response elicited by the vaccine agent by stimulating the immune system which results in the subject responding to the vaccine more vigorously than without the compound.

In some exemplary implementations the disclosure provides a compound represented by the structure of formula 1: 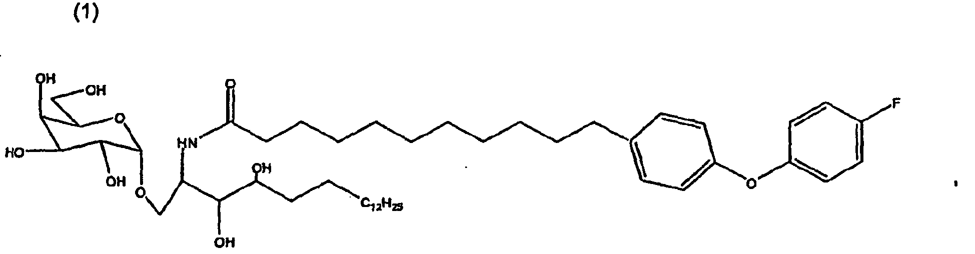 or a pharmaceutically acceptable salt thereof, for use in an anti-tumor immunotherapy.

As described herein, the administration is based on at least one of cancer, an elevated risk for cancer or precancerous precursors. In some aspects of the method the administration of the compound elicits a response in at least one of tumor and cancer cells. In some instances the response elicited is a slowing down in a growth of the tumor. In some instances the response elicited is a reduction in a size of the tumor.

As described herein, the administration of the compound is to effect an adaptive immune system that includes a population of cells, the population including at least one lymphocyte and wherein the response elicited is an expansion of the population of cells in the adaptive immune system.

As described herein, the expansion of the population of cells in the adaptive immune system includes an expansion in a number of T cells, CD8 T cells, NK cells or NKT cells. In some instances the method includes providing a cancer vaccine to which the compound is added to. The cancer may be selected from the group consisting of lung caner, breast cancer, hepatoma, leukemia, solid tumor and carcinoma.

The admistration may be based on an infectious disease resulting from the presence of pathogenic microbial agents. The pathogenic microbial agents may be selected from the group consisting of viruses, bacteria, fungi, protozoa, multicellular parasites and aberrant proteins. The pathogenic microbial agent may be a virus. The virus may be selected from the group consisting of Retroviridae, Picornaviridae, Calciviridae, Togaviridae, Flaviridae, Coronaviridae, Rhabdoviridae, Filoviridae, Paramyxoviridae, Orthomyxoviridae, Bungaviridae, Arena viridae, Reoviridae, Bimaviridae, Hepadnaviridae, Parvoviridae, Papovaviridae, Adenoviridae, Herpesviridae, Poxviridae and Iridoviridae. The pathogenic microbial agent may be a bacteria. The bacteria may be selected from the group consisting of Helicobacter pylon, Borellia burgdorferi, Legionella pneumophilia, Klebsiella Pneumoniae, Mycobacteria sps, Staphylococcus aureus, Neisseria gonorrhoeae, Neisseria meningitidis, Listenia monocytogenes, Streptococcus pyogenes, Streptococcus agatactiae, Streptococcus, Streptococcus faecalis, Streptococcus bovis, Streptococcus pneumoniae, pathogenic Campylobactersp., Enterococcus sp., Chiamidia sp., Haemophilus influenzae, Bacillus antracis, corynebacterium diphtheriae, corynebacterium sp., Erysipelothrix rhusiopathiae, Clostridium perfringers, Clostridium tetani, Enterobacter aerogenes, Kiebsiella pneumoniae, Pasturella multocida, Bacteroides sp., Fusobacterium nucleatum, Streptobacillus moniliformis, Treponema pallidium, Treponema pertenue, Leptospira, Actinomyces israelli, Sphingomonas capsulata and Francisella tularensis.

The administration of the compound to a subject may result in an enhanced bacterial clearance as compared to a subject not administered the compound. The administration of the compound may result in the killing of the microbial agent. The administration of the compound may result in the microbial agent not being able to grow.

### BRIEF DESCRIPTION OF THE FIGURES

The patent or application contains at least one drawing executed in color. Copies of this patent or patent application publication with color drawings will be provided by the Office upon request and payment of the necessary fee. α-GalCer analogs C1-C33 described herein do not form part of the invention, but α-GalCer analog C34 does form part of the invention.
Figure 1(A-B) are schematic illustrations showing Natural Killer T cell (NKT) function. Figure 1A shows a general scheme. Figure 1B shows how alpha-galactosyl ceramide (α-GalCer) and α-GalCer analogs are capable of binding to CD1d and stimulating a rapid T_{H}1 and T_{H}2 cytokine response.
Figure 2 shows the chemical structures of α-GalCer (C1) and various α-GalCer glycolipids (also referred to as analogs) including: glycolipids of bacterial origin (C3, C3 and C14), glycolipids modified with sulfonation (C4, C5 and C9), phenyl-alkyl chain glycolipids (C6-C8, C10-C11, C15-C16, C18-C34 (C34 being of the present invention), 7DW8-5 (aka, C8-5) and 7DW8-6 (aka, C8-6)) and phytosphingosine truncated glycolipids (C12, C13 and C17).
Figure 3 shows synthetic schemes for C12 and C13 α-GalCer analogs.
Figure 4 shows IL-2 cytokine secretion levels (pg/ml) by murine 1.2 hybridomas treated with α-GalCer or the indicated α-GalCer analogs.
Figure 5(A-C) show the "fold of increase" of (A) IFN-γ and IL-4, (B) IL-2 and IL-6, and (C) IL-12 and IL-10 cytokine production, normalized to DMSO control, by human CD161*/CD3* NKTs treated with α-GalCer or the indicated α-GalCer analogs and co-cultured with autologous immature CD14* DCs. Left side panels indicate a T_{H}1-type response and right side panels indicate a T_{H}2-type response.
Figure 6(A-B) show the (A) purity of human CD 161⁺CD3⁺ NKTs and (B) the "fold of increase" of the ratio of IFN-γ/IL-4 cytokine production, normalized to control (DMSO), derived from the data shown in Figure 5.
Figure 7 is a table showing the folds of increase over basal cytokine concentration in the supernatants of human NKTs from Figures 5 and 6 treated with α-GalCer or the indicated α-GalCer analogs.
Figure 8(A-F) shows the "fold of increase" of (A) IFN-γ, (B) IL-4, (C) the ratio of IFN-γ/IL-4, (D) IL-2, (E) IL-12 and (F) IL-6 cytokine production, normalized to control (DMSO), by naïve human NKTs treated with α-GalCer or the indicated α-GalCer analogs and co-cultured with autologous immature DCs.
Figure 9 shows the fold changes in the total number of iNKTs in response to the indicated α-GalCer analogs.
Figure 10(A-E) shows IFN-γ cytokine production by (A) naïve iNKTs co-cultured with autologous dendritic cells, (B) naïve iNKTs co-cultured with HeLa-CD1d cells, (C) α-GalCer-pulsed iNKTs co-cultured with HeLa-CD1d cells and (D) α-GalCer analog C11-pulsed iNKTs co-cultured with HeLa-CD1d cells, normalized to vehicle control (DMSO), treated with α-GalCer or the indicated α-GalCer analogs. (E) shows different basal levels of IFN-γ cytokine production in human naïve iNKTs, α-GalCer-pulsed iNKTs and α-GalCer analog C11-pulsed iNKTs.
Figure 11(A-C) shows (A) IFN-γ cytokine secretion levels (pg/ml), (B) IL-4 cytokine secretion levels (pg/ml) and (C) ratio of IFN-γ/IL-4 by human naïve iNKTs treated with α-GalCer or the indicated α-GalCer analogs.
Figure 12 is a table indicating the folds of increase over basal serum concentrations in the supernatants of human NKTs from Figure 10 treated with α-GalCer or the indicated α-GalCer analogs.
Figure 13 shows representative flow cytometry data for the expansion of human CD56⁺ cells (NK/NKT mixtures) cultured with autologous immature CD14⁺ dendritic cells and pulsed with α-GalCer or the indicated α-GalCer analogs. The percentage of CD161⁺/Vα24TCR⁺ cells in the NK/NKT mixtures is shown.
Figure 14 shows the total number of iNKTs (10³) found in the NK/NKT mixtures from Figure 13.
Figure 15 (A-B) show representative flow cytometry data for the expansion of human CD56⁺ cells (NK/NKT mixtures) cultured with autologous immature CD14⁺ dendritic cells pulsed with α-GalCer or the indicated α-GalCer analogs. (A) shows representative flow cytometry data of the percentage of CD161⁺/Vα24TCR⁺ cells in the NK/NKT mixtures and (B) shows the fold of increase in the total number of iNKTs found in the NK/NKT mixtures.
Figure 16 shows the expression levels, as Mean Fluorescence Intensity (MFI), of surface proteins CD40, CD80, CD86, and CD83, as well as the MHC class II cell surface receptor HLA-DR, on dendritic cells (DCs) after immature human DCs were incubated with α-GalCer or the indicated α-GalCer analogs.
Figure 17(A-B) shows how the α-GalCer analog C13 promotes maturation of human monocyte-derived DCs. (A) shows histograms for CD40, CD80, CD83, CD86, and HLA-DR expression in DCs in response to C13. (B) shows the morphology of DCs incubated with C13 for 48 hours.
Figure 18 shows a schematic illustration of the iNKT cell receptor signaling pathways.
Figure 19(A-E) demonstrates how α-GalCer analogs promote CD1d-dependent T cell receptor (TCR) activation of human NKTs. (A) shows expression of CD1d in HeLa cells transfected with CD1d (HeLa-CD1d). (B) shows the intracellular levels of phospho-CD3ε. (C) shows the intracellular levels of phospho-ERK1/2. (D) shows the intracellular levels of phospho-Syk. (E) shows the intracellular levels of phospho-CREB.

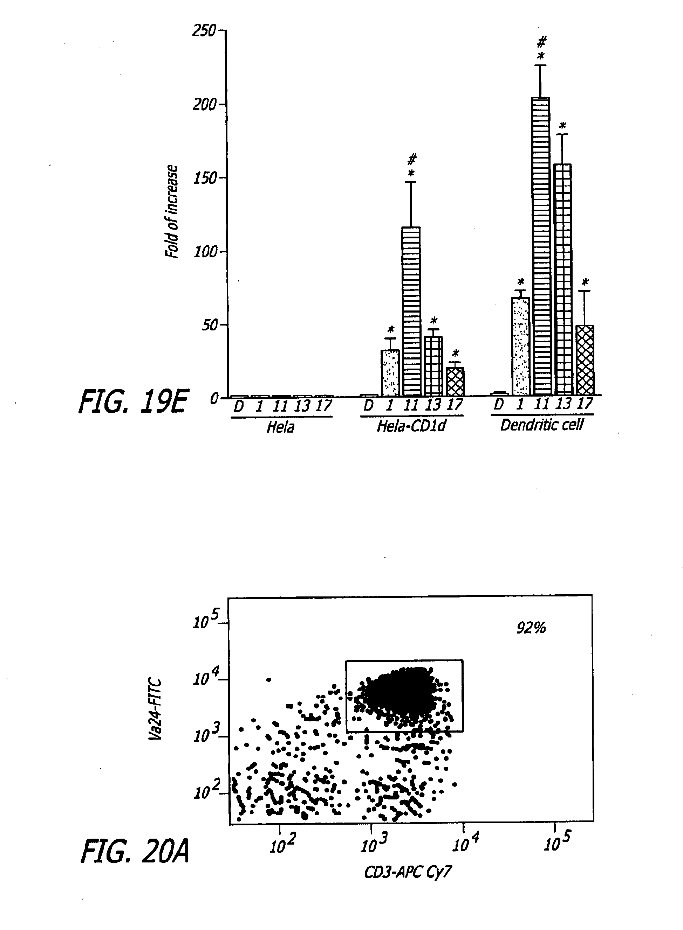

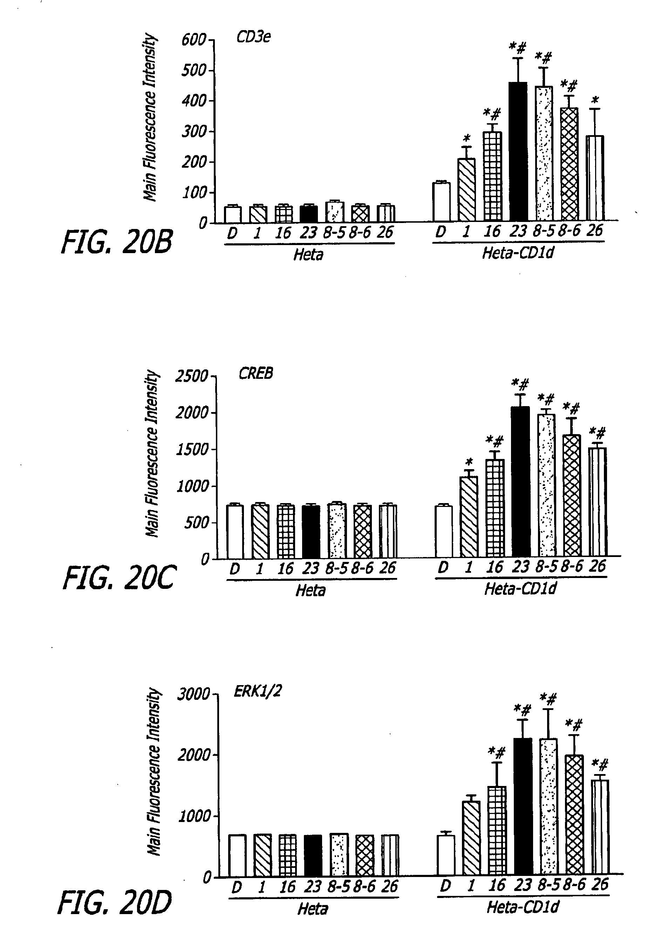

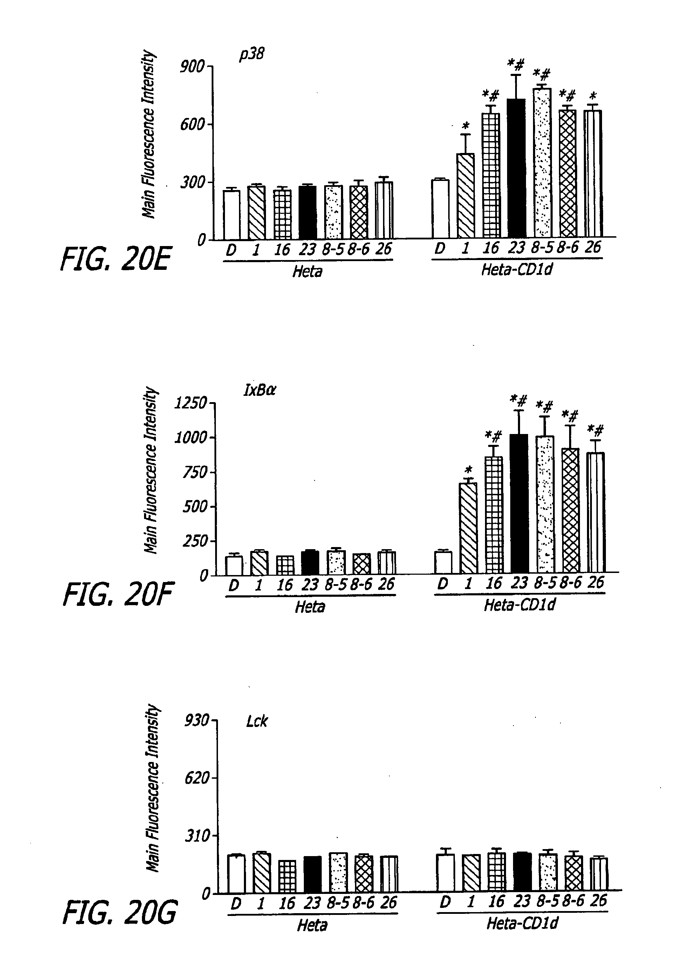

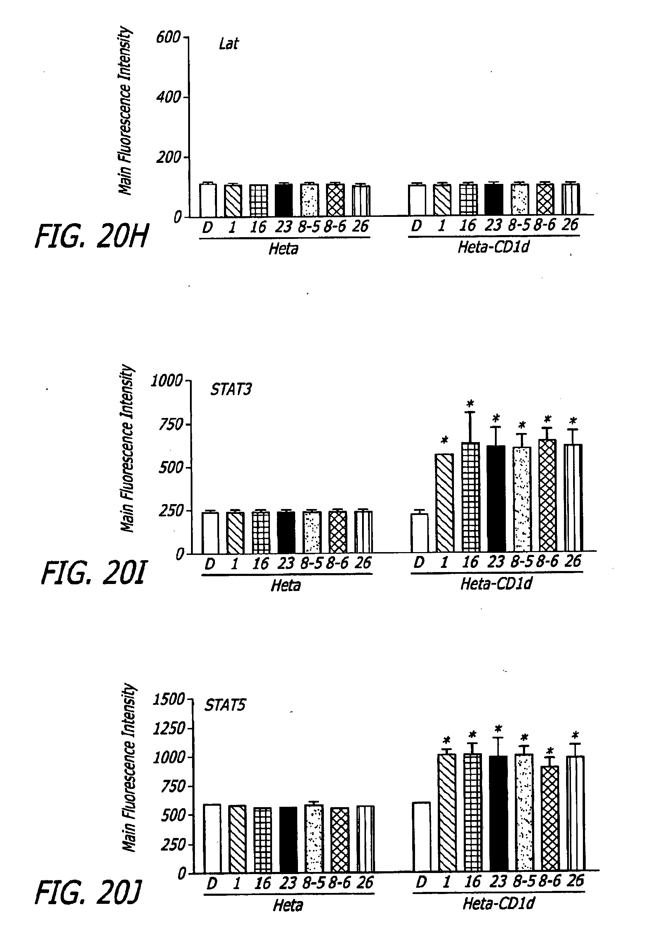

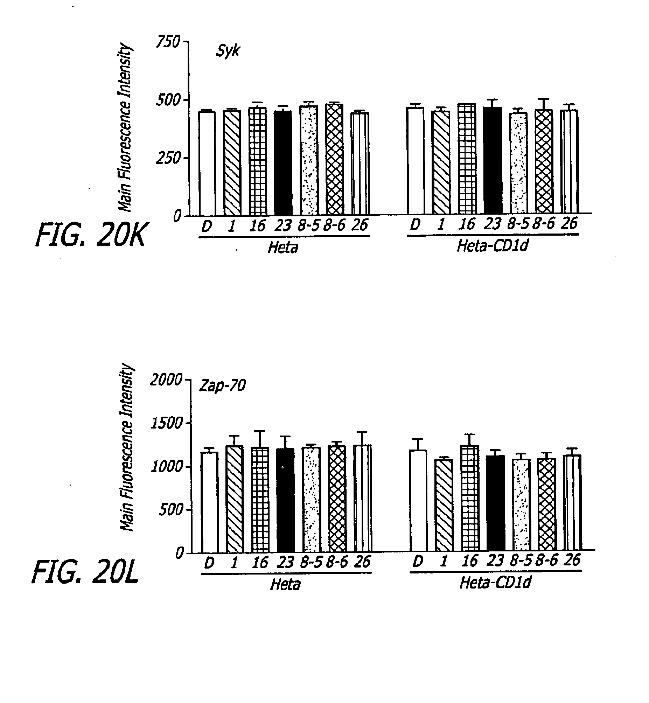

Figure 20(A-L) demonstrates how α-GalCer analogs promote CD1d-dependent T cell receptor (TCR) activation of naïve human iNKTs (Vα24⁺). (A) shows the determination of isolated naive human Vα24⁺ T cells by flow cytometry. (B-L) shows activation of TCR on iNKTs. HeLa or HeLa-CD1d cells were loaded with α-GalCer or α-GalCer analogs C16, C23, 7DW8-5, 7DW8-6 or C26, and then added to naïve Vα24⁺ T cells. The intracellular levels of the following phosphorylated proteins were measured and expressed as Median Fluorescence Intensity, and normalized to the amount of total input protein: (B) phospho-CD3ε (phosphotyrosine), (C) phospho-CREB (Ser-133), (D) phospho-ERK1 /2 (Thr-185/Tyr-187), (E) phospho-p38 (Thr-180/Tyr-182), (F) phospho-IκBα (Ser32), (G) phospho-Lck, (H) phospho-Lat, (I) phospho-STAT3 (Ser727), (J) phospho- STAT5 A/B (Tyr 694/699), (K) phospho-Syk (Phospho-tyrosine) and (L) phospho-Zap-70 (Phospho-tyrosine). *, p < 0.05, compared with DMSO control and #, p < 0.05, compared with α-GalCer.
Figure 21(A-C) shows how the α-GalCer analogs induced greater cell expansion and display higher capacity to bind CD1d-restricted NKTs and T cells. Spleens from BALB/c mice were harvested 72 hour after intraveneous (IV) injection of 0.1 µg /mouse of vehicle, α-GalCer or the indicated α-GalCer analogs. (A) percentage of mouse NKTs or (B) T cells were determined. (C) shows different binding affinities of α-GalCer and the indicated α-GalCer analogs to CD1d-restricted NKTs and T cells.

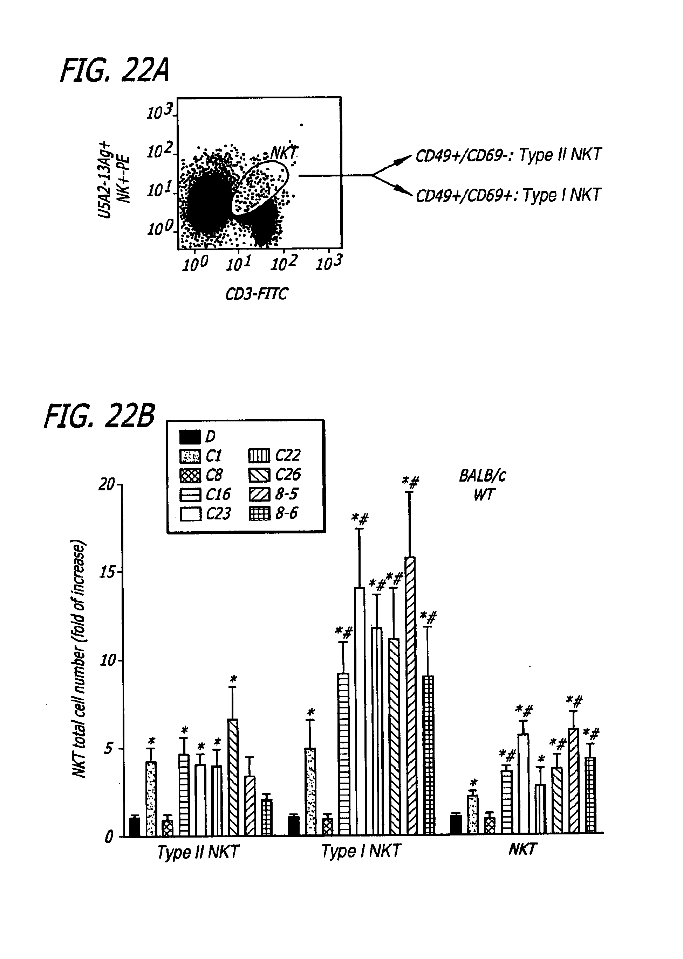

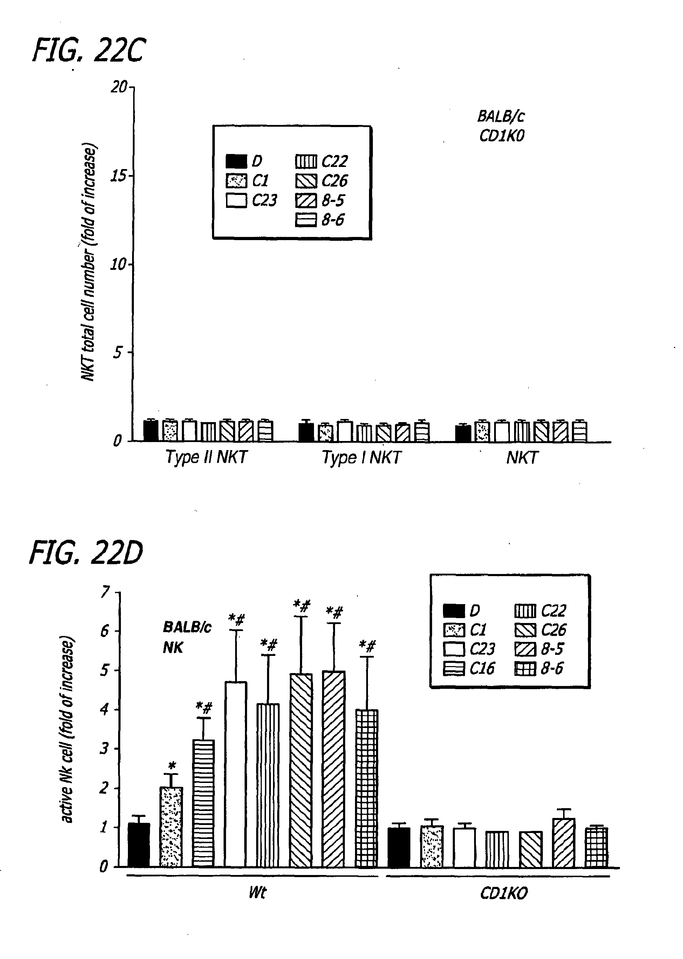

Figure 22(A-D) show the CD1d-dependent expansion of two NKTs subsets and NK activation in response to the α-GalCer analogs. (A-C) show the CD1d-dependent expansion of two NKTs subsets. Spleens from BALB/c wild type (WT) or CD1 KO mice were harvested 72 hours post-injection of α-GalCer or the indicated α-GalCer analogs. Total numbers of NKTs, and its two subtypes, designated as Type I NKT and Type II NKT in (B) WT or (C) CD1 KO mice in response were assessed by FACS. (D) CD1d dependent-activation of NKs. The expansion of total number of NKs in WT *(left panel)* or CD1 KO *(right panel)* mice in response were assessed by FACS.
Figure 23(A-C) show mouse serum levels (pg/ml) of various cytokines (A) IFN-γ, (B) IL-4, and (C) the ratio of IFN-γ/IL-4 after intraveneous (IV) injection with vehicle, α-GalCer or the indicated α-GalCer analogs at 0, 2, 18, 36, 48, 72 h post-injection and normalized to DMSO control.
Figure 24(A-C) show mouse serum levels (pg/ml) of various cytokines/chemokines A) IFN-γ, (B) IL-4, and (C) the ratio of IFN-γ/[L-4 at 2 and 18 h after IV injection with vehicle, α-GalCer or the indicated α-GalCer analogs.
Figure 25 is a table with the results (in folds of increase over basal cytokine concentration) in the supernatants of BALB/c mice injected IV with α-GalCer or the indicated α-GalCer analogs. All cytokines /chemokines peaked at 2 hours after injection, except those marked with a * peaked at 18 hours.
Figure 26 (A-H) show (A) the total number of nucleated cells and the spleen size, (B) the population of innate immune cells, including mature dendritic cells, (C) activated NKs, (D) activated NKTs, (E) active B cells, (F) active CD8⁺ T cells, (G) active CD4⁺ T cells and (H) the ratio of CD8⁺/CD4⁺ T cells, all normalized with DMSO, in response to the IV injection of vehicle, α-GalCer or the α-GalCer analogs from Figure 23.
Figure 27 (A-C) show mouse serum levels of various cytokines (A) IFN-γ, (B) IL-4, and (C) the ratio of IFN-γ/IL-4 after subcutaneous (SubQ) injection with vehicle, α-GalCer or the indicated α-GalCer analogs at 0, 2, 18, 36, 48, 72 h post-injection and normalized to DMSO control.
Figure 28(A-H) show (A) the total number of nucleated cells and the spleen size, (B) the population of innate immune cells, including mature dendritic cells, (C) activated NKs, (D) activated NKTs, (E) active B cells, (F) active CD8⁺ T cells, (G) active CD4⁺ T cells and (H) the ratio of CD8⁺/CD4⁺ T cells, all normalized with DMSO, in response to the SubQ injection of vehicle, α-GalCer or the α-GalCer analogs from Figure 27.
Figure 29(A-C) show mouse serum levels of various cytokines (A) IFN-γ, (B) IL-4, and (C) the ratio of IFN-γ/IL-4 after intramuscular (IM) injection with vehicle, α-GalCer or the indicated α-GalCer analogs at 0, 2, 18, 36, 48, 72 h post-injection and normalized to DMSO control.
Figure 30(A-H) show (A) the total number of nucleated cells and the spleen size, (B) the population of innate immune cells, including mature dendritic cells, (C) activated NKs, (D) activated NKTs, (E) active B cells, (F) active CD8⁺ T cells, (G) active CD4⁺ T cells and (H) the ratio of CD8⁺ /CD4⁺ T cells, all normalized with DMSO, in response to the IM injection of vehicle, α-GalCer or the α-GalCer analogs from Figure 29.

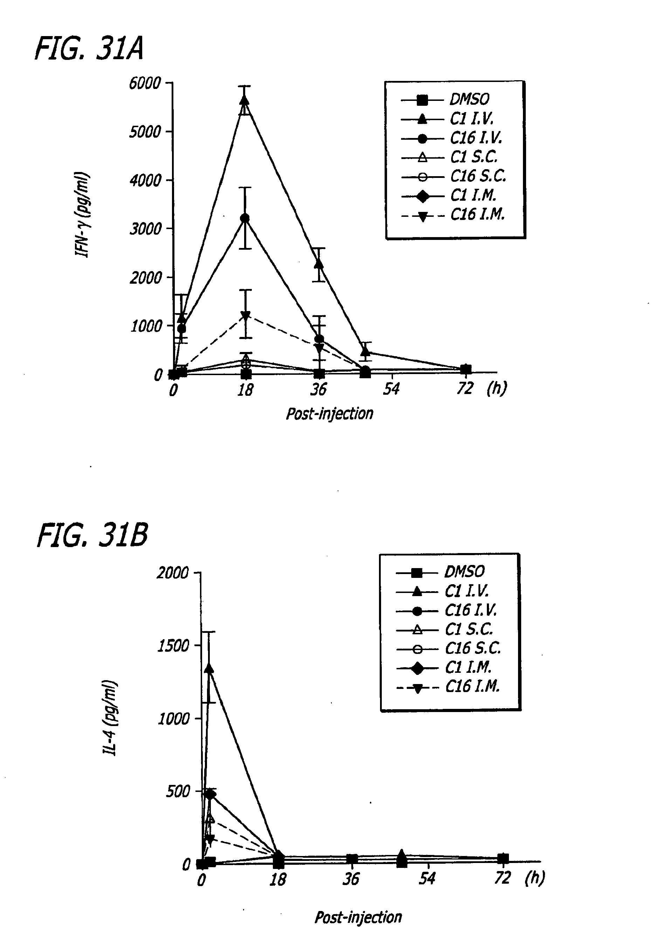

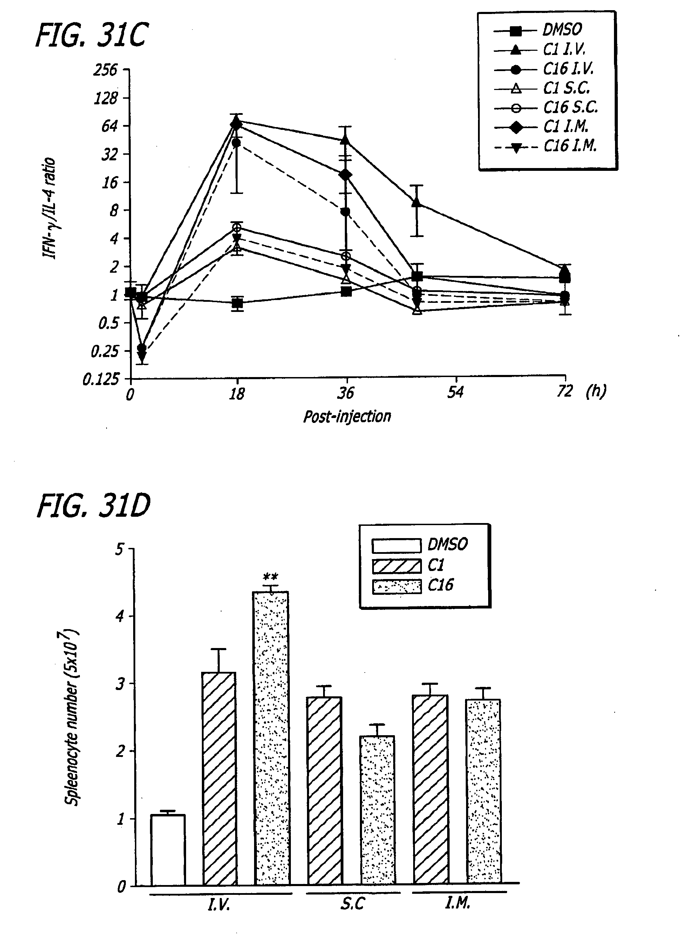

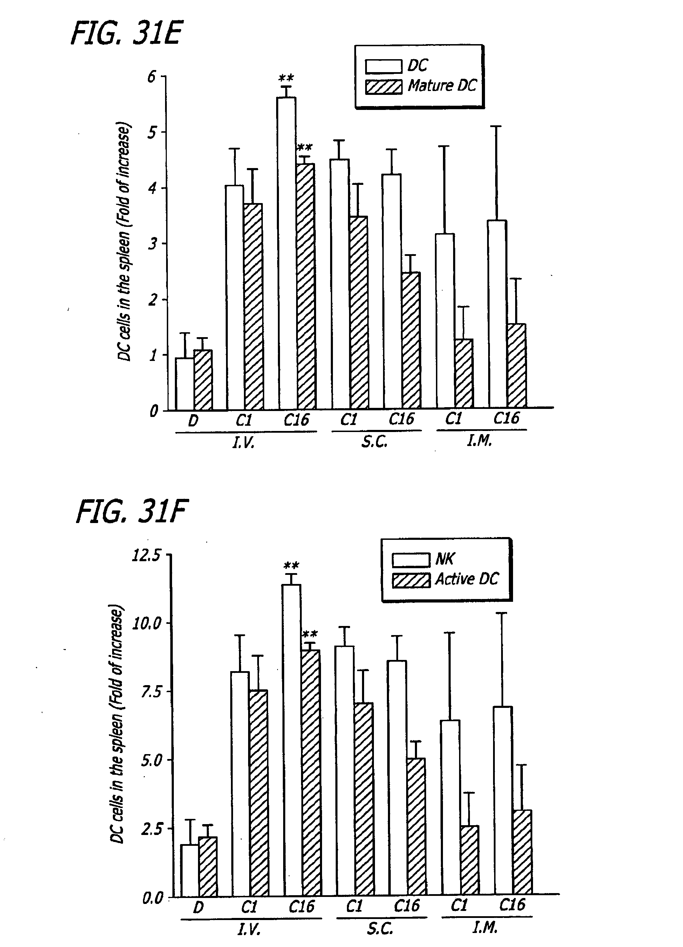

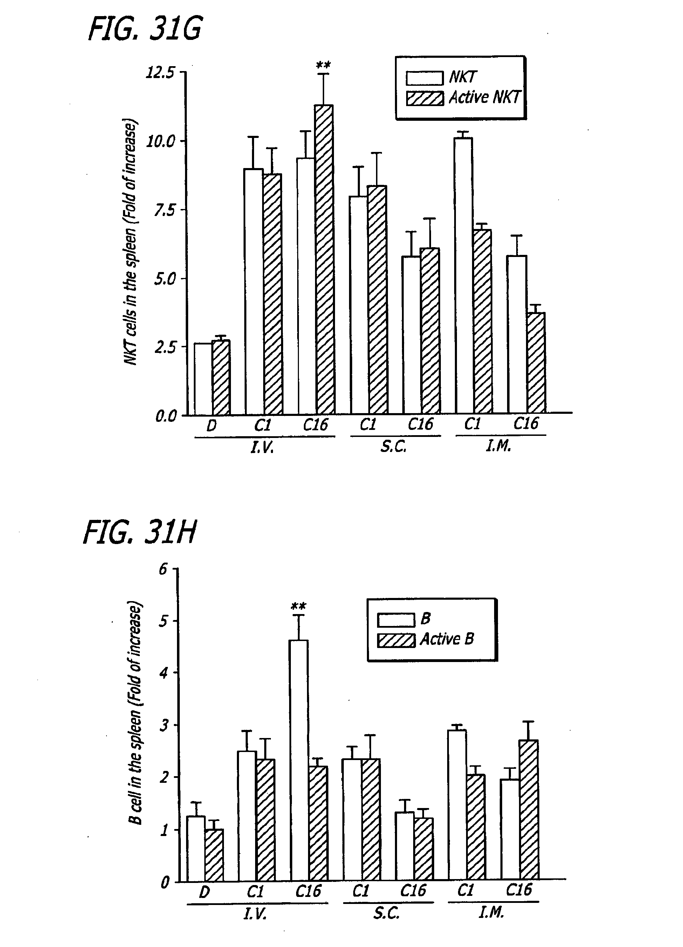

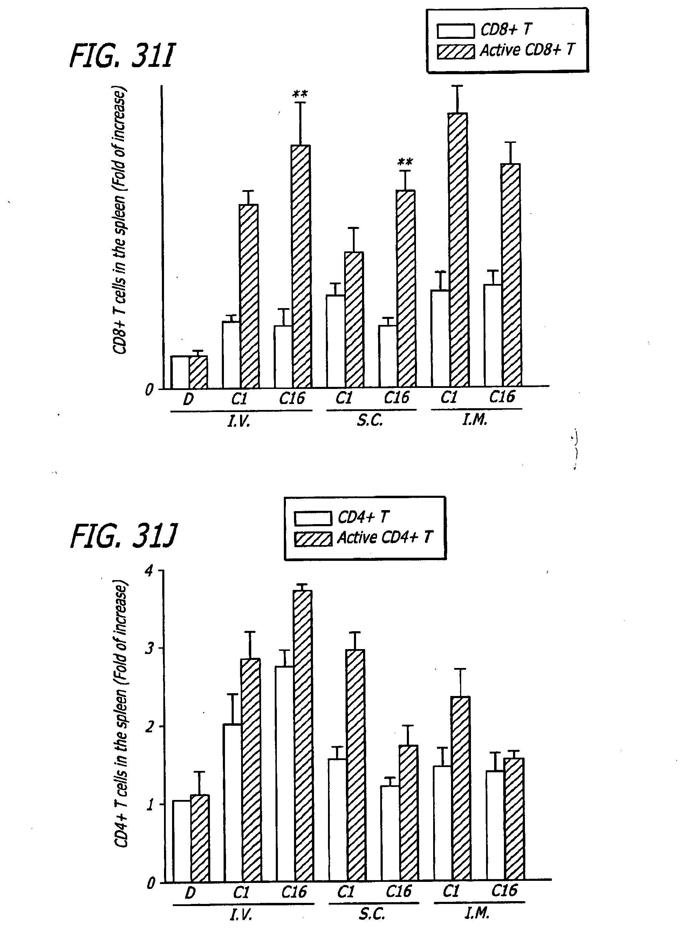

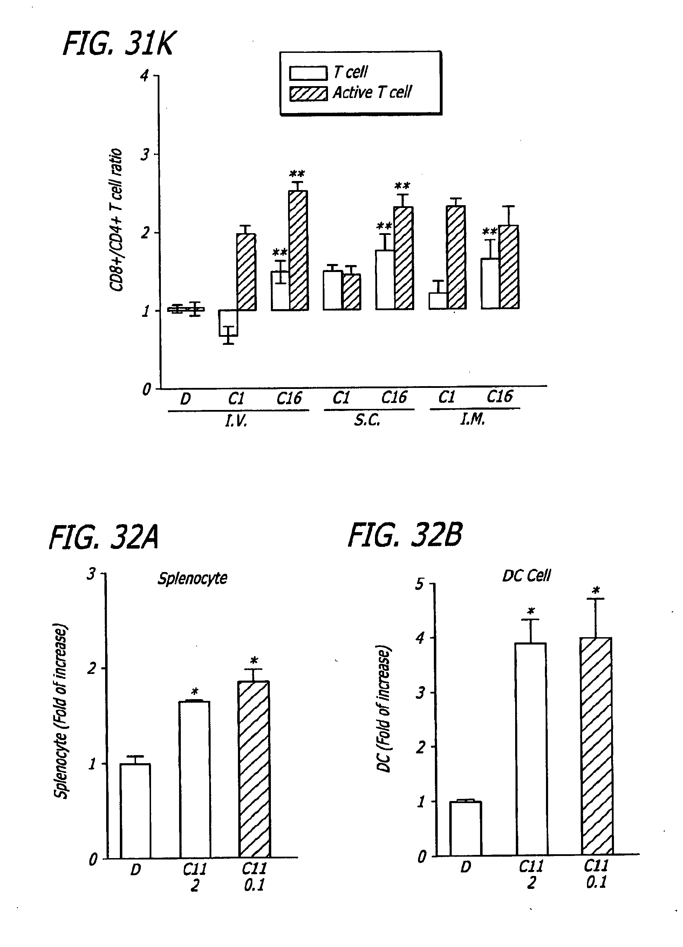

Figure 31 (A-K) show the effects of route of administration (IV, SubQ or IM) of vehicle, α-GalCer or the indicated α-GalCer analogs on cytokine kinetics and splenocytes expansion/activation. (A) shows mouse serum levels (pg/ml) of IFN-γ. (B) shows mouse serum levels (pg/ml) of IL-4. (C) shows the ratio of IFN-γ/IL-4 (log 10). (D) shows the total number of mouse nucleated cells (splenocytes). (E) shows the population of innate immune cells, including mature dendritic cells in the spleen. (F) shows the population of activated NKs in the spleen. (G) shows the population of activated NKTs in the spleen. (H) shows the population of active B cells in the spleen. (I) shows the population of active CD8⁺ T cells in the spleen. (J) shows the population of active CD4⁺ T cells in the spleen. (K) shows the ratio of CD8⁺/CD4⁺ T cells. All analysis was performed by normalizing to vehicle.

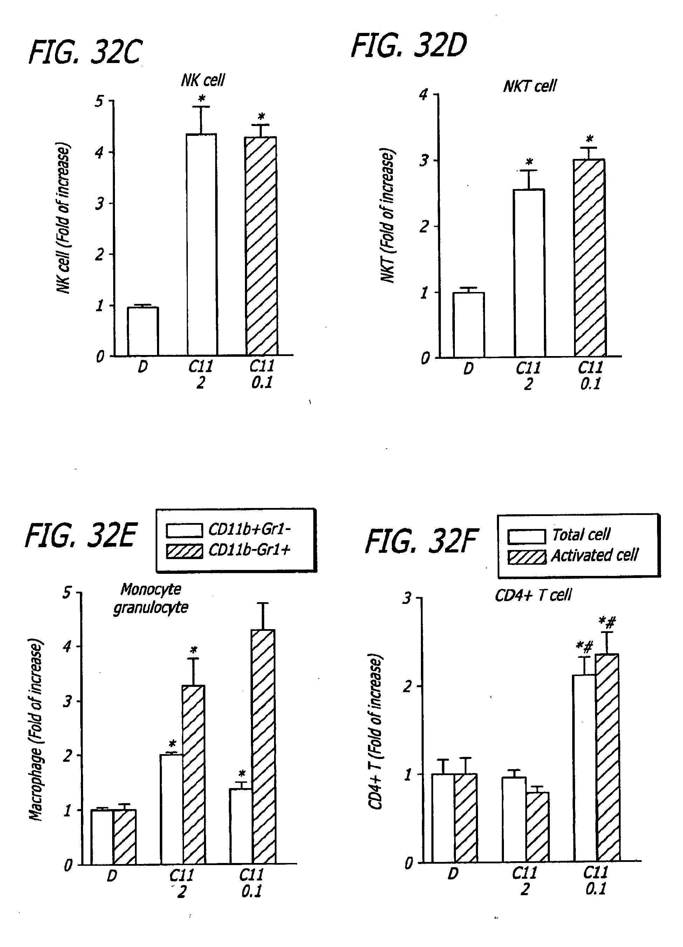

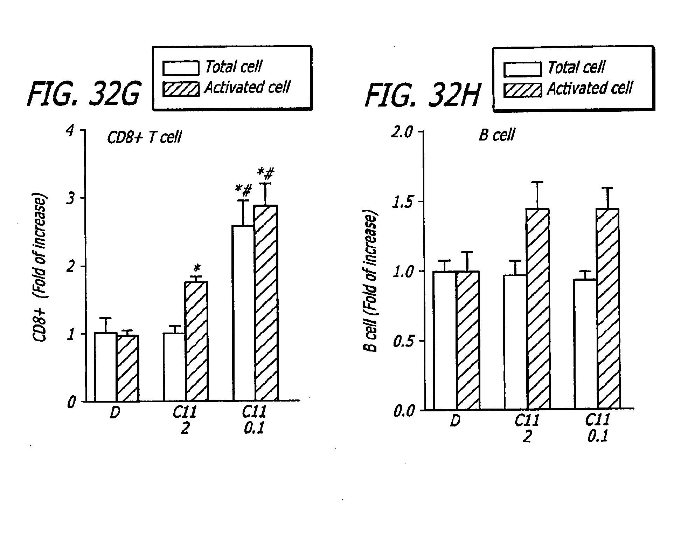

Figure 32(A-H) show the dose-response of splenocytes expansion/activation in response to the IV administration of the α-GalCer analog C11 or vehicle. (A) shows the total number of mouse nucleated cells (splenocytes). (B) shows the population of innate immune cells, including mature dendritic cells, in the spleen. (C) shows the population of activated NKs in the spleen. (D) shows the population of activated NKTs in the spleen. (E) shows the population of monocyte granulocyte cells in the spleen. (F) shows the population of active CD4⁺ T cells in the spleen. (G) shows the population of active CD8⁺ T cells in the spleen. (H) shows the population of active B cells in the spleen. All analysis was performed by normalizing to vehicle.
Figure 33(A-D) shows mouse serum levels of various cytokines (B) IFN-γ, (C) IL-4, and (D) the ratio of IFN-γ/IL-4 after IV injection with (A) vehicle, α-GalCer or various α-GalCer analogs at 0, 12, 24, 36, 48, 72 h post-injection and normalized to vehicle control.
Figure 34 is a table with the results (in folds of increase over basal cytokine concentration) in the supernatants of BALB/c mice injected IV with α-GalCer or the indicated α-GalCer analogs from Figure 33. All cytokines /chemokines peaked at 2 hours after injection, except those marked with a * peaked at 18 hours.
Figure 35 (A-G) show serum levels (pg/ml) of various cytokines/chemokines at 2 and 18 h after IV injection of vehicle, α-GalCer or the indicated α-GalCer analogs to wild type BALB/c (wt) and CD1d KO BALB/c (CD1KO) mice. (A) IFN-γ. (B) IL-4 (C) IFN-γ/IL-4 ratio (log 10). (D) IL-10. (E) IL-12p70. (F) KC. (G) MCP-1.

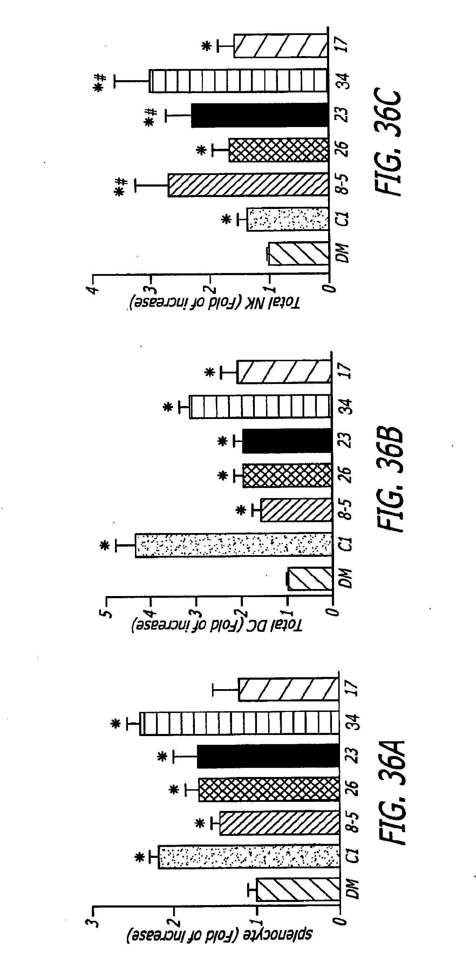

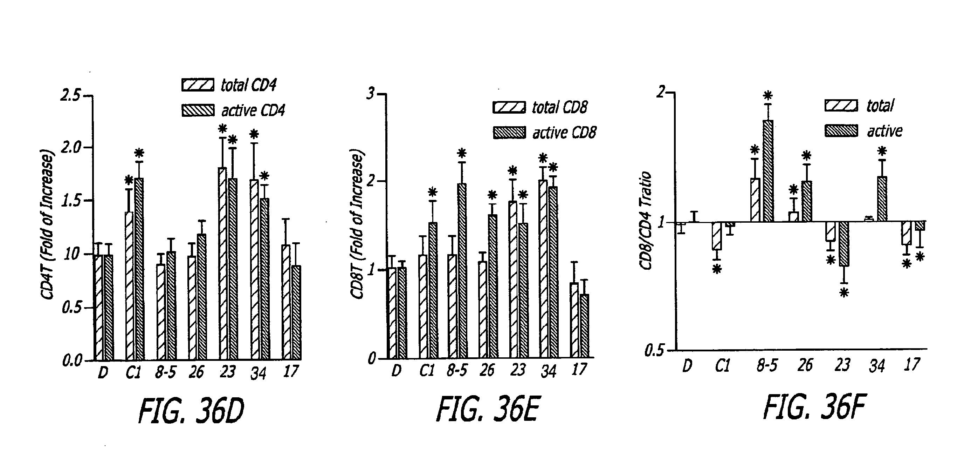

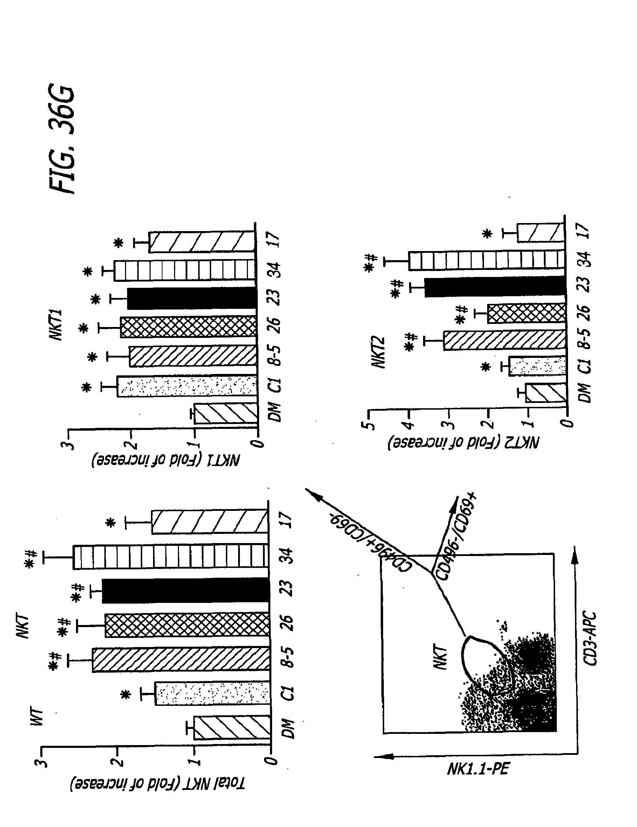

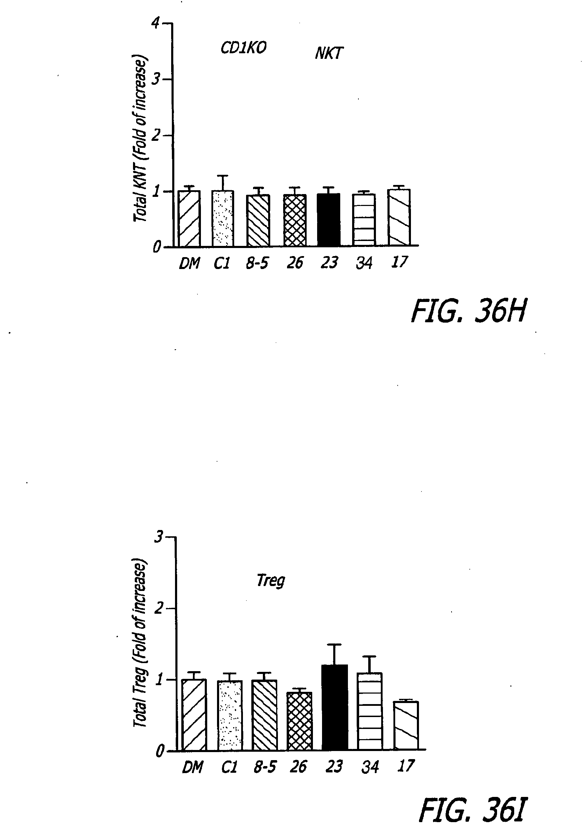

Figure 36(A-I) shows the expansion/activation of splenocytes in C57BL/6 mice after IV injection of vehicle, α-GalCer or the indicated α-GalCer analogs, and (G-I) shows the CD1d-dependent activation of two NKTs subsets (C57BL/6 wild type (Wt) and CD1 KO mice and after IV injection of vehicle, α-GalCer or the indicated α-GalCer analogs. (A) shows the total number of C57BL/6 mouse nucleated cells (splenocytes). (B) shows the population of mature dendritic cells. (C) shows the population of activated NKs. (D) shows the population of active CD4⁺ T cells. (E) shows the population of active CD8⁺T cells. (F) shows the ratio of CD8⁺/CD4⁺ T cells normalized with DMSO. (G) shows determination of NKT cells in Wt mice by flow cytometry (*lower*-*left panel),* total number of NKTs *(upper-left panel),* and its two subtypes including Type **II** NKT *(upper-right panel)* and Type I NKT *(lower-right panel).* (H) shows the total number of NKTs in CD1 KO mice. (I) shows the total number of Treg cells in Wt mice. All analysis was performed by normalizing to vehicle.
Figure 37(A-B) show how α-GalCer analogs can prolong survival of mice bearing lung cancer. C57BL/6 mice were inoculated IV with mouse lung cancer cells (TC-1), and then treated with control, α-GalCer or the indicated α-GalCer analog twice per week for four weeks.
   (A) shows the results from the testing of Group I α-GalCer analogs. (B) shows the results from the testing of Group II α-GalCer analogs. (C) shows the results from the testing of Group III α-GalCer analogs. (D) shows the results from the testing of Group IV α-GalCer analogs. Shown are the Kaplan Meier survival curves (left *panels)* and changes in body weight *(right panels)* of mice bearing lung cancer. The control is the mouse without tumor inoculation.

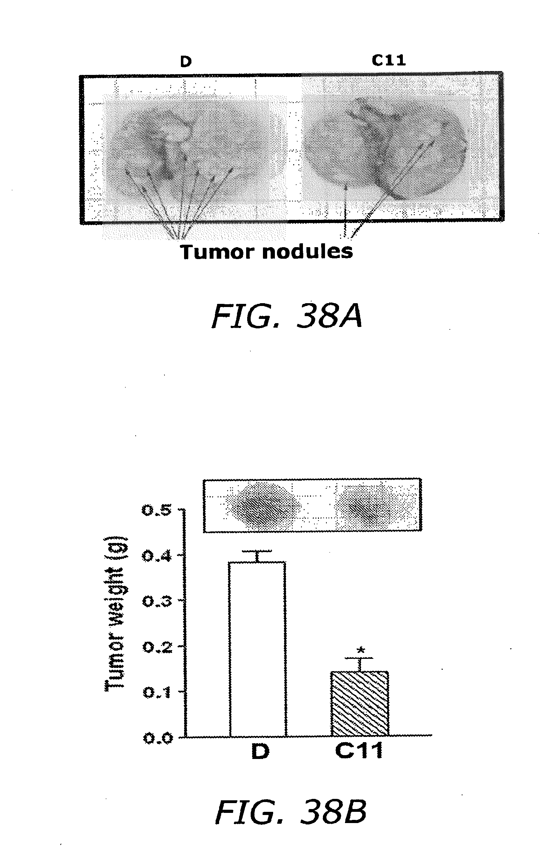

Figure 38(A-B) show tumor nodules and sizes (A) on a surface of lungs of mice treated with α-GalCer analog C11 or control, and sacrificed on day 16 after tumor inoculation with TC-1 cells and (B) in subcutaneous tumors of mice treated with α-GalCer analog C11 or control, and sacrificed on day 16 after SubQ tumor inoculation with mouse breast cancer cells (4T-1).

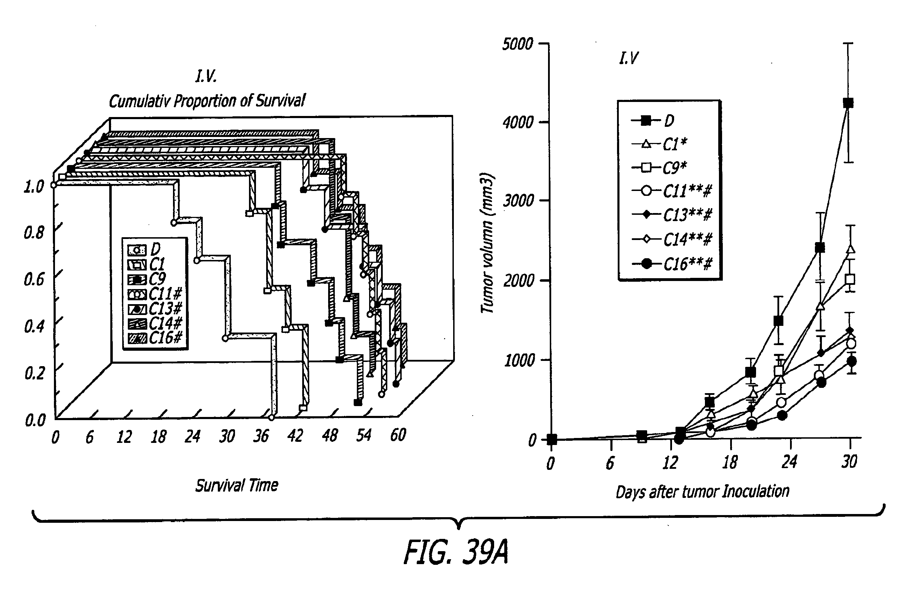

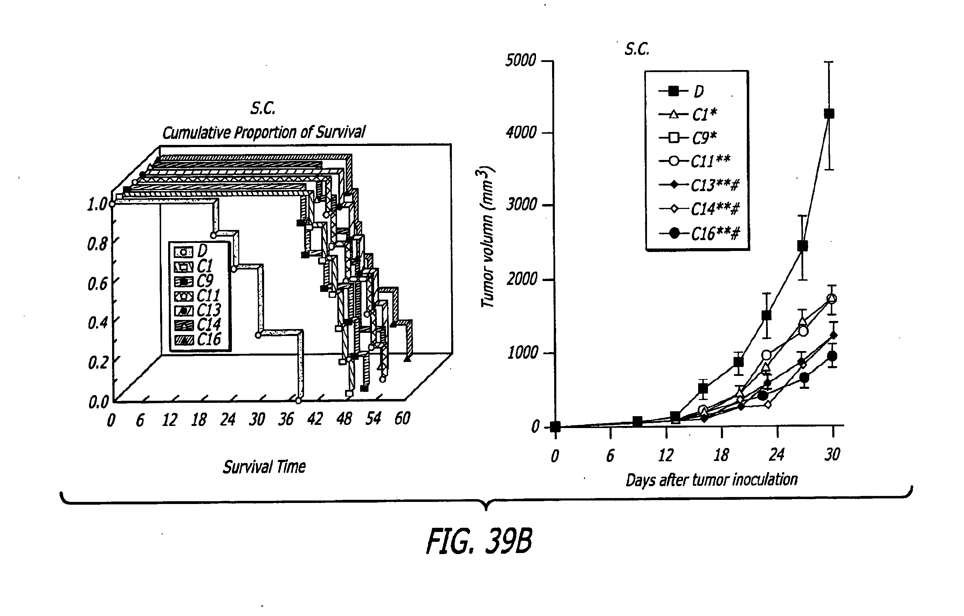

Figure 39(A-B) shows Kaplan Meier survival curves *(left panel)* and tumor growth *(right panel)* of mice subcutaneously inoculated with mouse breast cancer cells 4T-1, and treated with control, α-GalCer or the indicated α-GalCer analog three days after inoculation, and twice per week for four weeks by (A) IV injection or (B) SubQ injection.

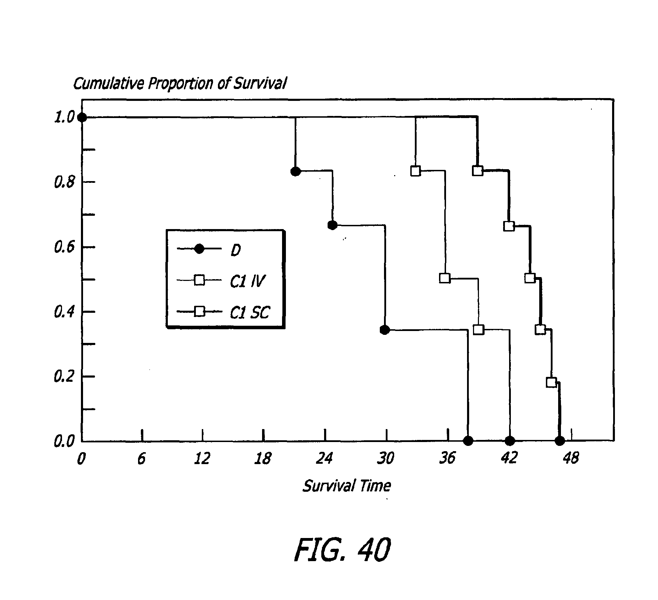

Figure 40 shows Kaplan Meier survival curves of mice bearing breast cancer and treated by either IV or SubQ injection with α-GalCer (C1). SubQ delivery of C1 is more effective than IV delivery in prolonging the survival of mice bearing breast cancer.

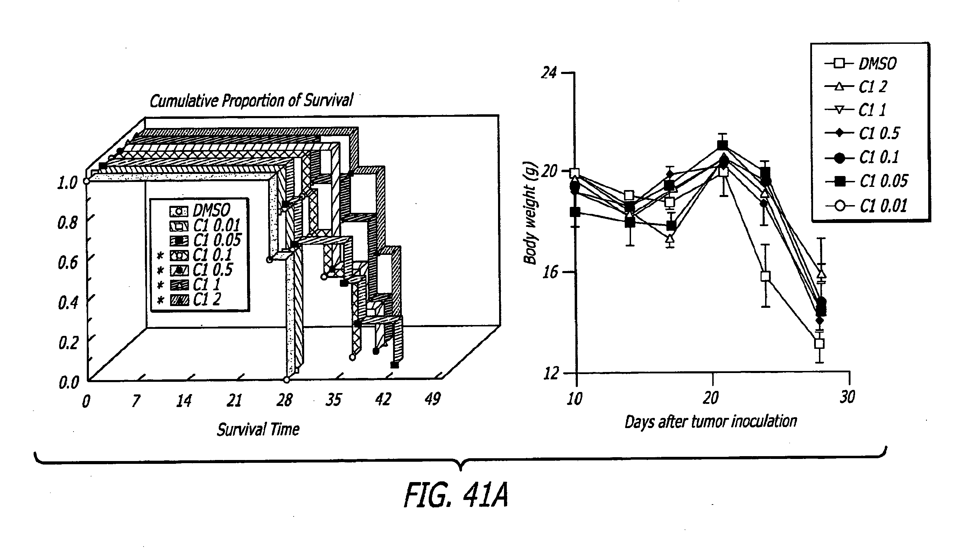

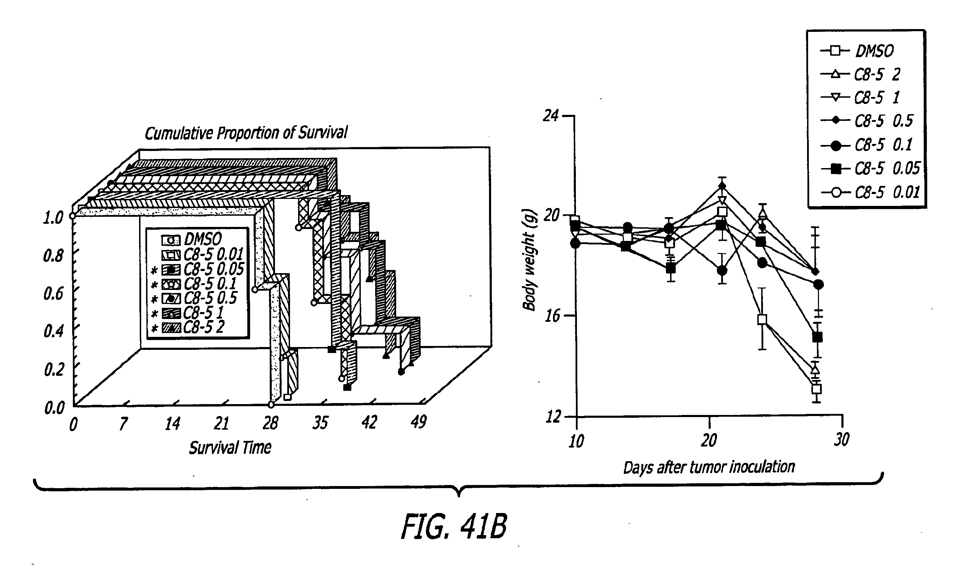

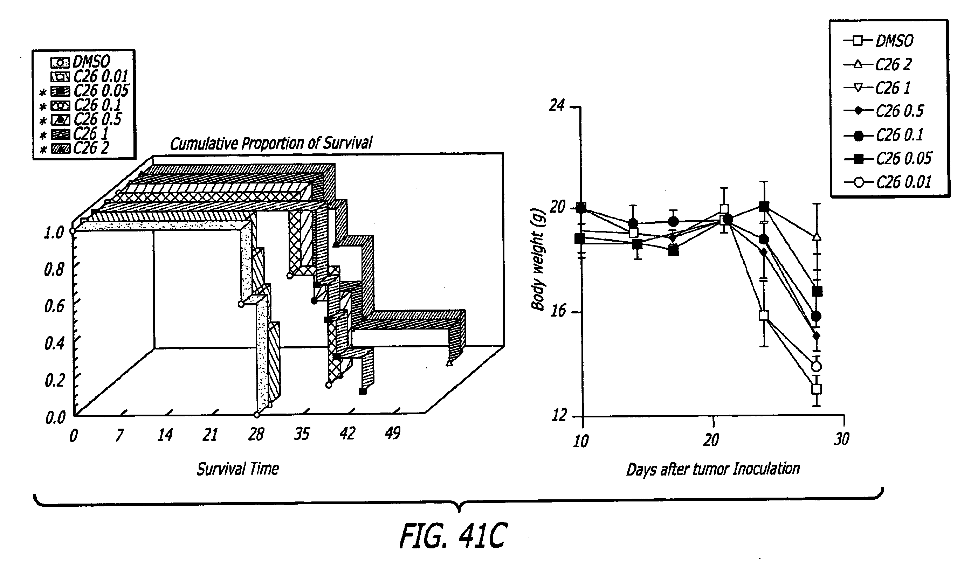

Figure 41 (A-C) show optimization of therapeutic anticancer protocols of α-GalCer analogs by dosage of administration. Changes in body weight *(right panel)* and Kaplan Meier survival curves *(Left panel)* of C57BL/6 mice after IV inoculation with mouse lung cancer cells (TC-1), and then treated with α-GalCer or α-GalCer analogs 7DW8-5 or C26 at various dosages twice per week or once per week for four weeks. (A) α-GalCer. (B) α-GalCer analog 7DW8-5. (C) α-GalCer analogs C26.

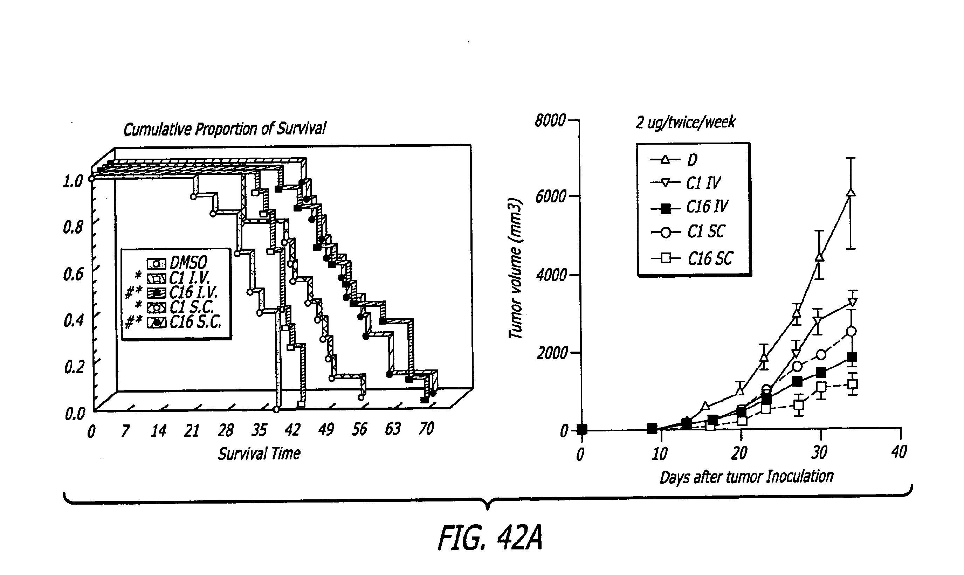

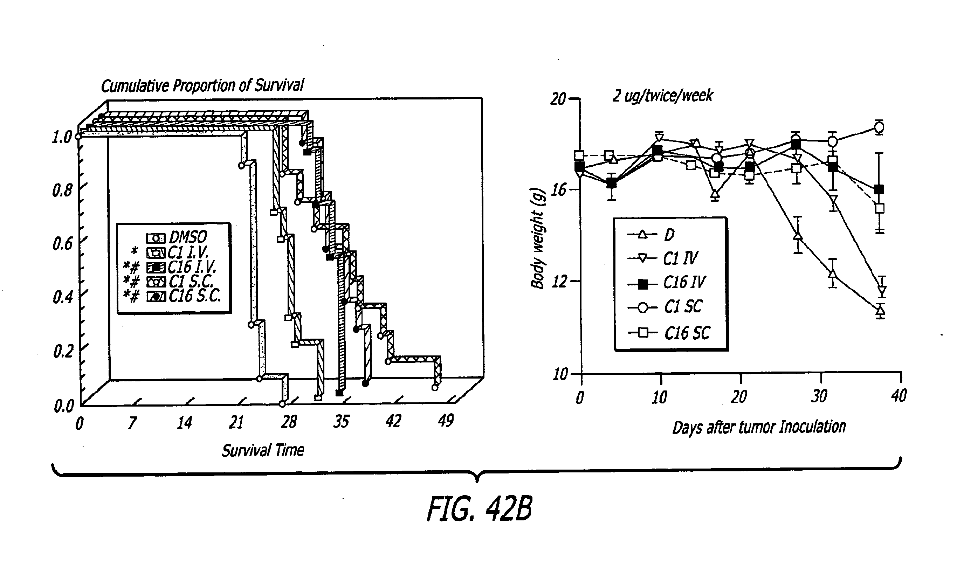

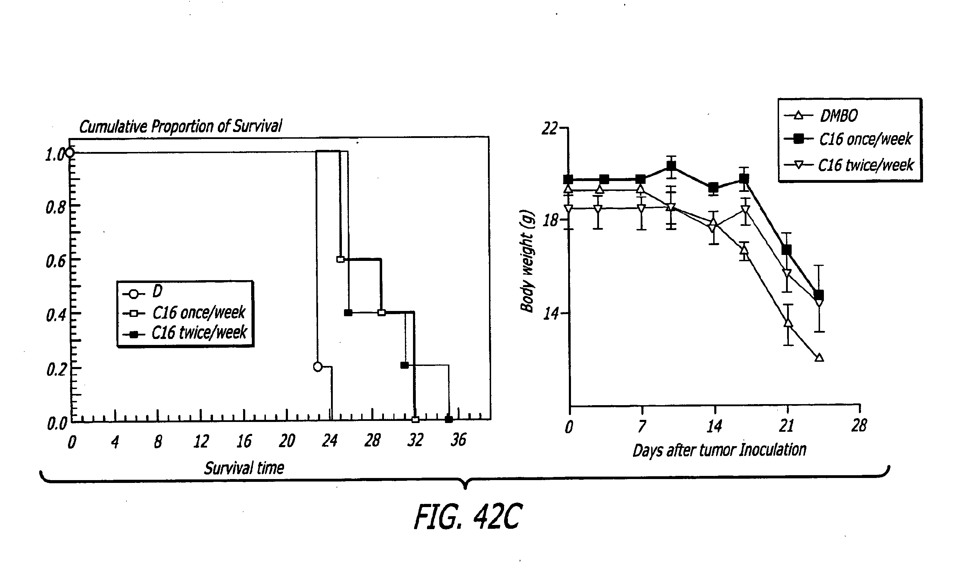

Figure 42(A-C) show optimization of therapeutic anticancer protocols of α-GalCer analogs by varying routes and frequency. (A) shows the tumor volume (mm³) *(right panel)* and Kaplan Meier survival curves *(left panel)* of BALB/c mice after SubQ inoculation with mouse breast cancer cells, 4T-1, and then treated three days after inoculation with vehicle, α -GalCer or the indicated α-GalCer analogs twice per week for four weeks by the IV or SubQ route. (B) shows changes in body weight *(right panel)* and Kaplan Meier survival curves *(left panel)* of C57BL/6 mice after IV inoculation with mouse lung cancer cells, TC-1, and then treated three days after inoculation with vehicle, α-GalCer or the indicated α-GalCer analogs twice per week for four weeks by the IV or SubQ route. (C) shows the impacts of frequency of administration on body weight *(right panel)* and Kaplan Meier survival curves (left *panel)* of C57BL/6 mice after IV inoculation with mouse lung cancer cells, TC-1, and then treated with vehicle or α-GalCer analog C16 twice per week or once per week for four weeks by the IV route.

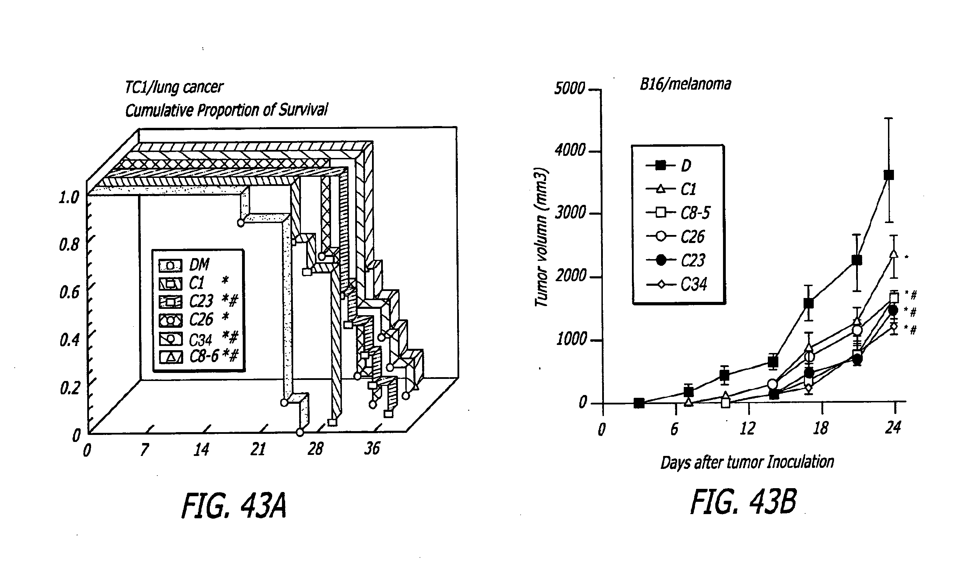

Figure 43(A-B) show the evaluation of the anticancer efficacy of various α-GalCer analogs. C57BL/6 mice were IV inoculated with mouse lung cancer cells, TC-1, or SubQ inoculated with mouse melanoma, B16 cells, and then treated with vehicle, α-GalCer or the indicated α-GalCer analogs once per week for four weeks. (A) shows the Kaplan Meier survival curves. (B) shows the tumor volume (mm³) growth curves.

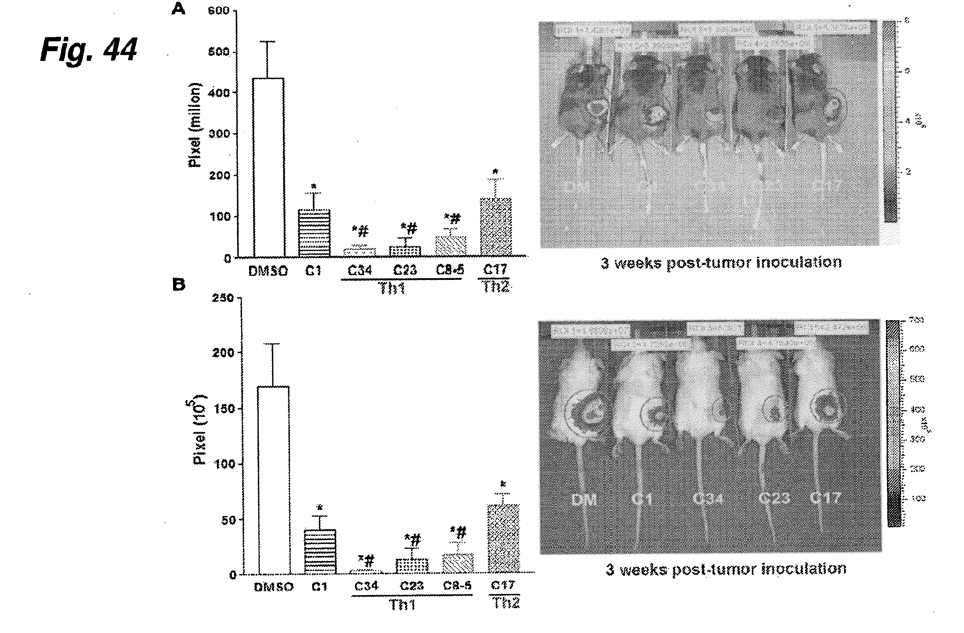

Figure 44(A-B) show the real time assessment of tumor growth in (A) C57BL/6 mice after SQ inoculation with lung cancer cells (TC-1-GRP-Luciferase) or (B) breast cancer cells (4T-1-GFP-Luciferase), and then treated with vehicle, α-GalCer or the indicated α-GalCer analogs once per week for four weeks.

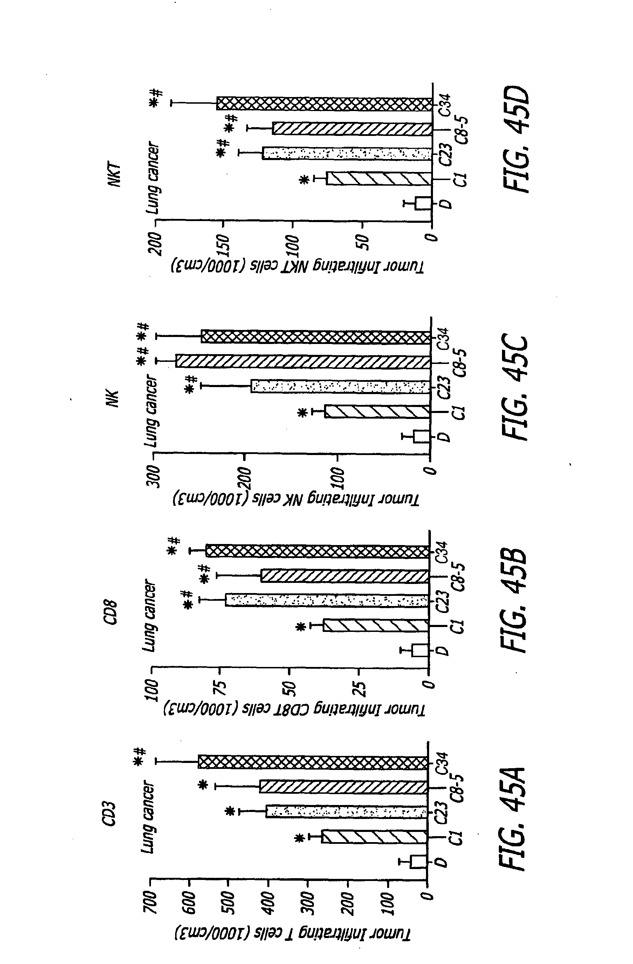

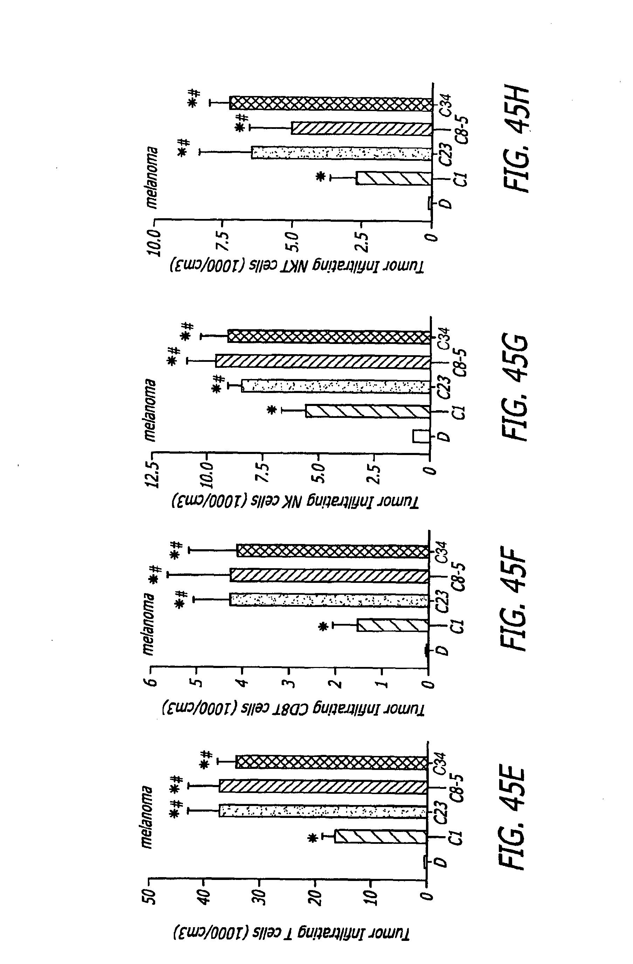

Figure 45(A-H) show T_{H}1-biased α-GalCer analogs elicit more tumor infiltrating lymphocytes in lung and melanoma tumors. (A-D) show tumor infiltrating lymphocytes in lung cancer cells (TC-1). C57BL/6 mice were treated with vehicle, α-GalCer or α-GalCer analogs C23, C8-5 or C34 (C34 being of the present invention) at 0.1 µg /mouse once per week for three weeks. (A) shows the population of CD3⁺ cells. (B) shows the population of CD8 T cells. (C) shows the population of NK cells. (D) shows the population of NKTs. All analysis was performed by normalizing to vehicle. (E-H) show tumor infiltrating lymphocytes in melanoma cells. C57BL/6 mice were treated with vehicle, α-GalCer or α-GalCer analogs C23, C8-5 or C34 at 0.1 µg/mouse once per week for three weeks. (E) shows the population of CD3⁺ cells. (F) shows the population of CD8 T cells. (G) shows the population of NKs. (H) shows the population of NKTs. All analysis was performed by normalizing to vehicle.

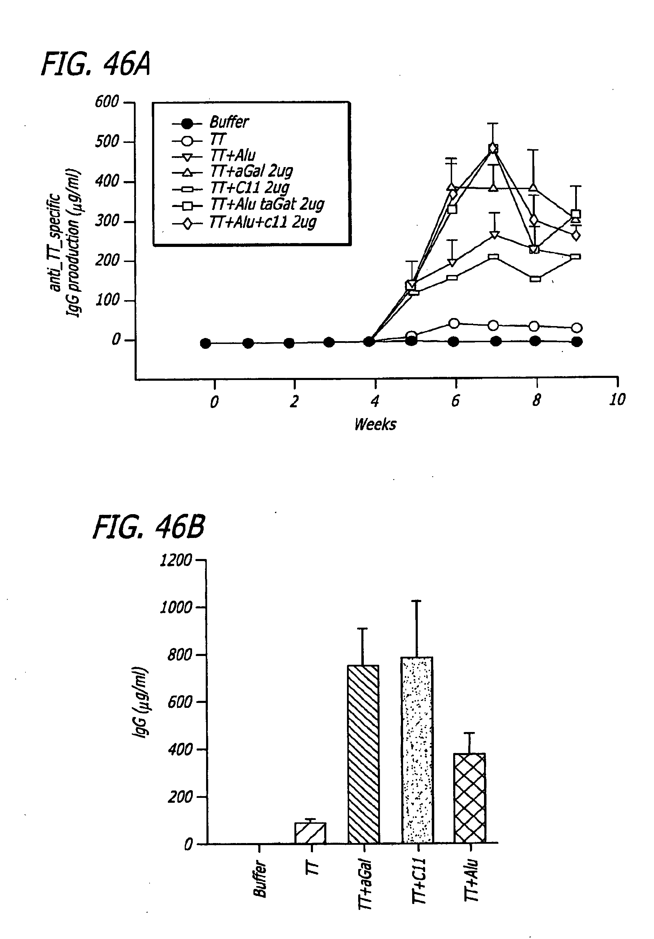

Figure 46(A-B) show adjuvant effects of alum, α-GalCer and α-GalCer analog C11 on antibody response to tetanus toxoid (TT) -protein vaccine. (A) mice were vaccinated TT without or with conventional adjuvant alum, α-GalCer or α-GalCer analog C11 on day 0 (first vaccination) and day 28 (4 weeks-second vaccination). Serum was harvested weekly for determination of anti-TT-specific antibodies. (B) shows the effects of conventional adjuvant alum, α-GalCer and α-GalCer analog C11 on delayed antigen boost 20 weeks after the second vaccination.

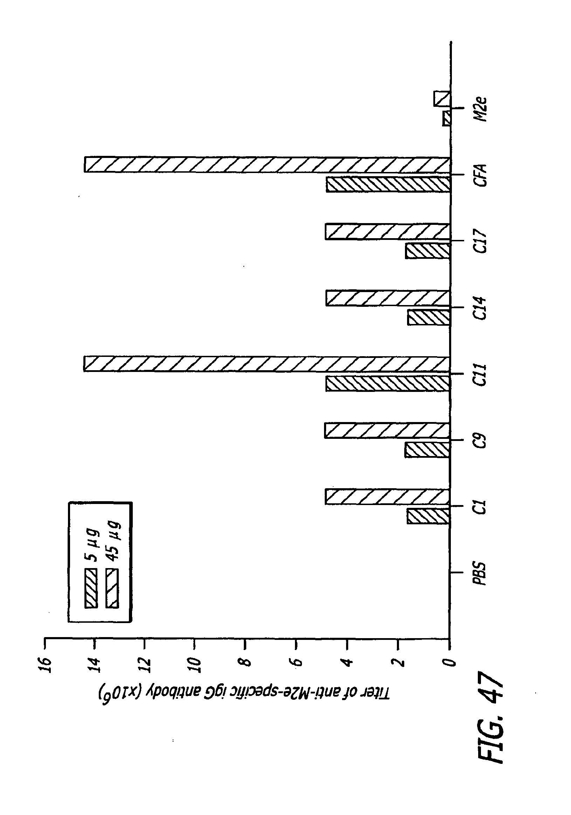

Figure 47 shows adjuvant effects of conventional adjuvant alum, α-GalCer and various α-GalCer analogs on peptide containing extracellular domain of M2 (M2e) protein of H1N1 virus strain, two weeks after a third immunization. BALB/c mice were vaccinated with 5 or 45 µg of M2e peptide with or without α-GalCer and various α-GalCer analogs on week 0, 3 and 6.

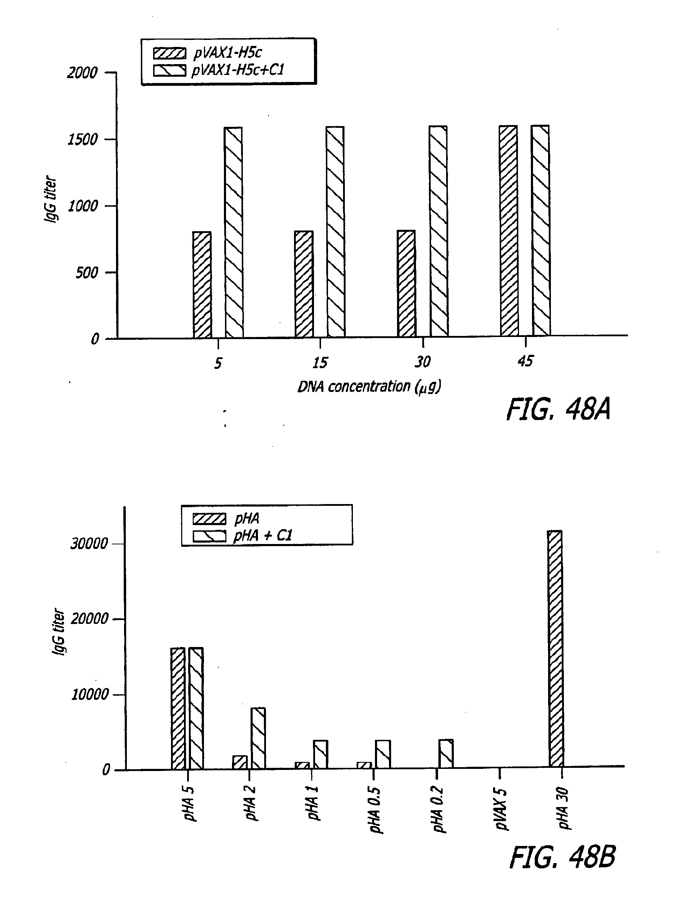

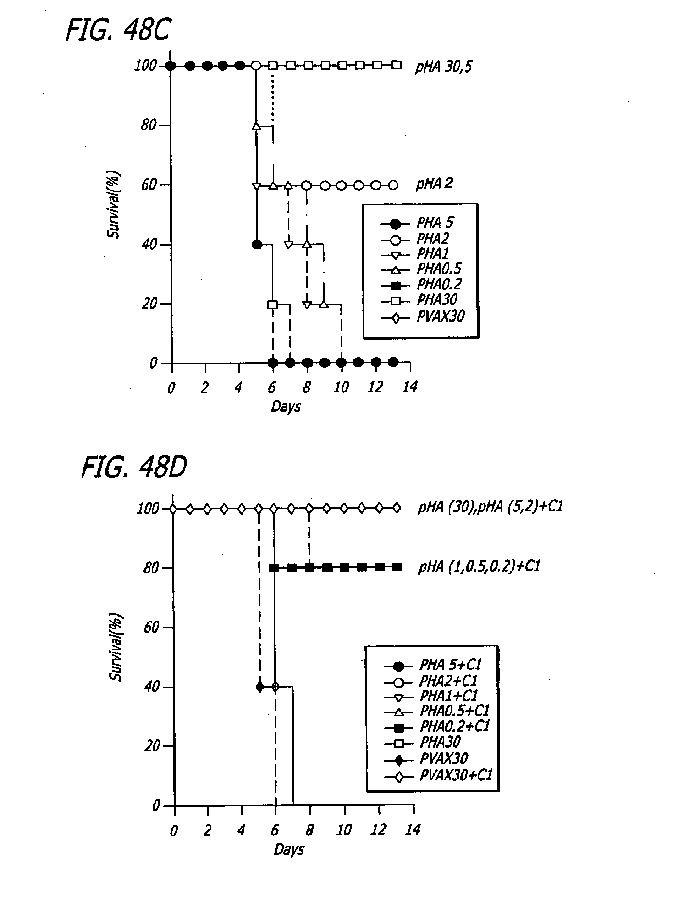

Figure 48(A-C) shows adjuvant effects of α-GalCer (C1) on mice immunized with pHA, a DNA plasmid containing consensus sequence of full length H5 of avian influenza viruses. (A) mice were immunized with between 5 and 45 µg of pHA without or with C1 on week 0 and 3. (B) mice were immunized with low doses of pHA vaccine without or with C1. (C) shows protection against viral challenge with 20 LD₅₀ of Vietnam reassortant influenza strain NIBRG-14 two weeks after H5 DNA vaccine without or with C1.

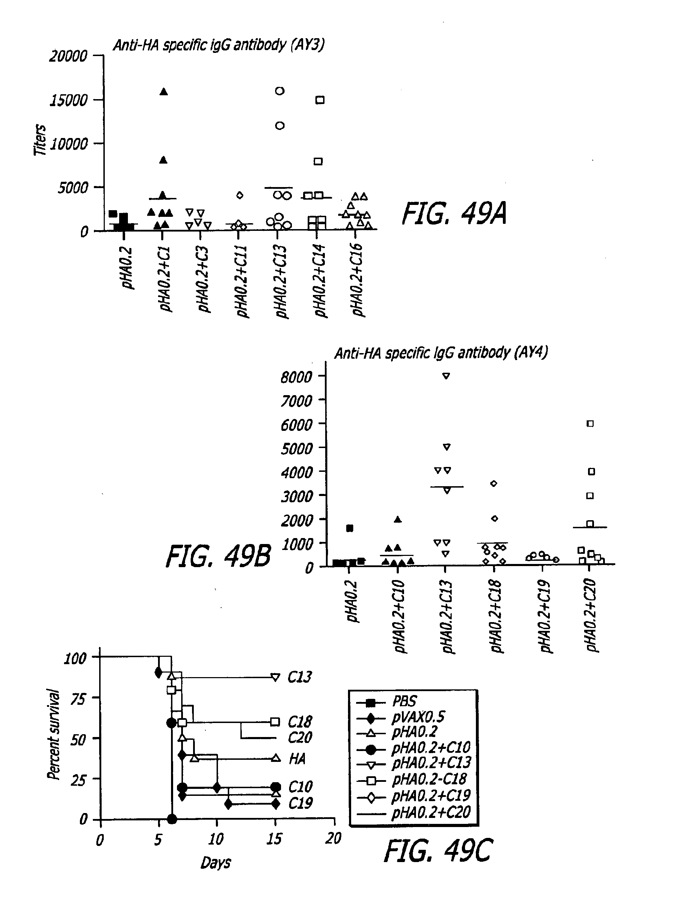

Figure 49(A-C) show induction of anti-HA-specific IgG antibody after mice were immunized with pHA with or without C1 or the indicated α-GalCer analogs. (A) shows titers of anti-HA specific IgG antibody (AY3) in mice following immunization with 0.2 µg pHA. (B) shows titers of anti-HA specific IgG antibody (AY4) In mice following immunization with 0.2 µg pHA. (C) shows percent mouse survival following viral challenge.

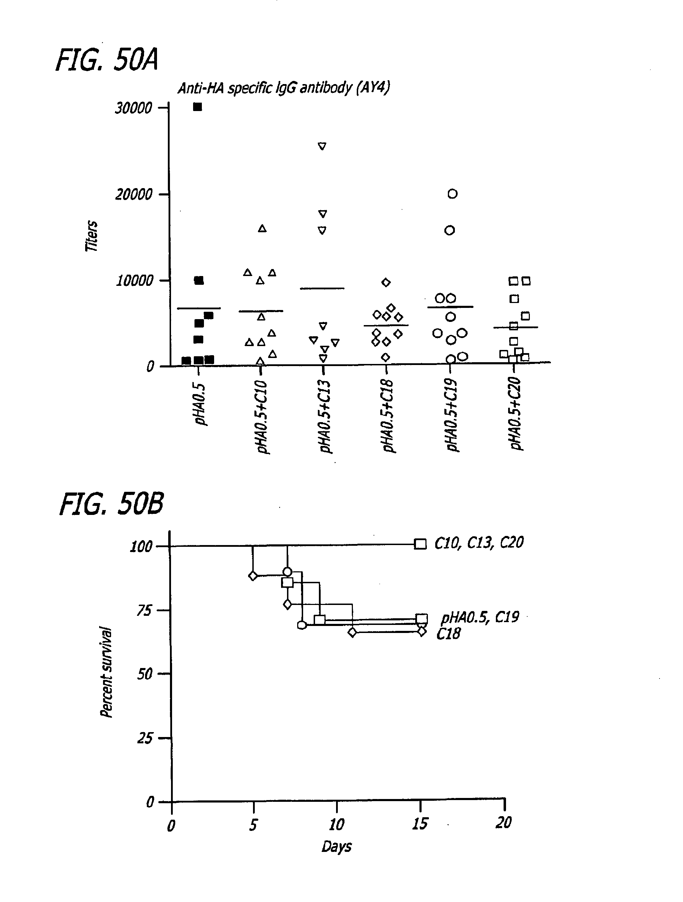

Figure 50(A-B) show induction of anti-HA-specific IgG antibody after mice were immunized with pHA with or without C1 or the indicated α-GalCer analogs. (A) shows titers of anti-HA specific IgG antibody (AY4) following immunization with 0.5 µg pHA and the indicated α-GalCer analogs. (B) shows percent survival following viral challenge.

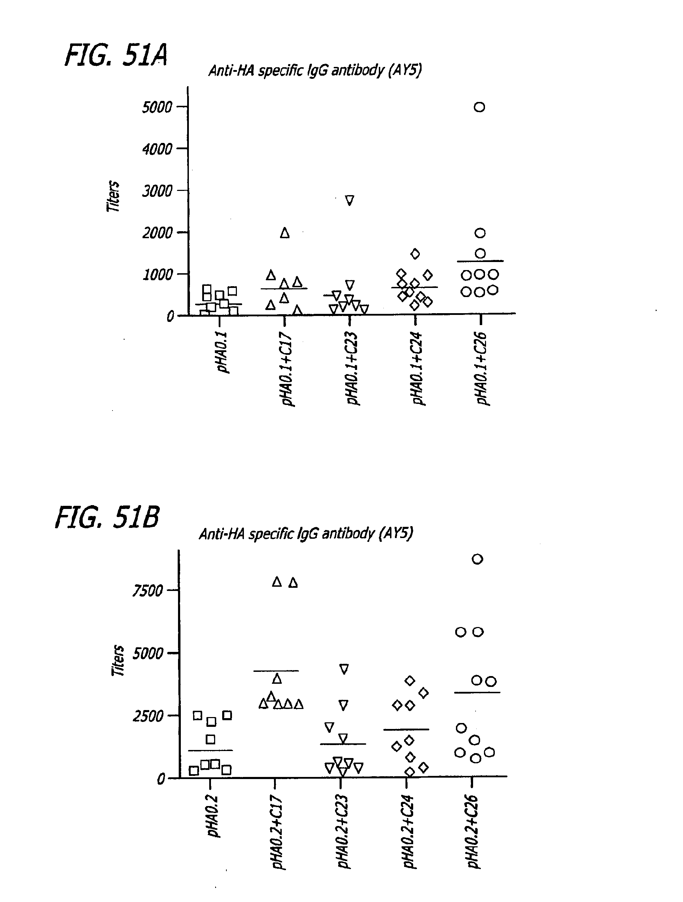

Figure 51(A-B) show mouse titer of anti-HA specific IgG antibody (AY5) following immunization with either (A) 0.1 µg pHA or (B) 0.2 µg pHA and the indicated α-GalCer analogs.

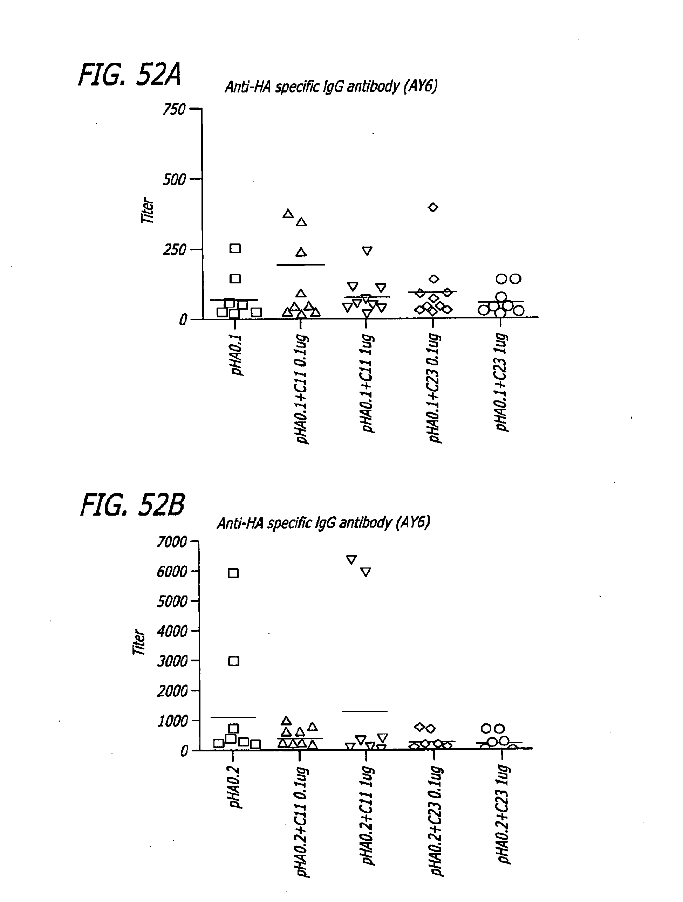

Figure 52(A-B) show mouse titer of anti-HA specific IgG antibody (AY6) following immunization with either (A) 0.1 µg pHA or (B) 0.2 µg pHA and the indicated α-GalCer analogs at 0.1 µg or 1 µg.

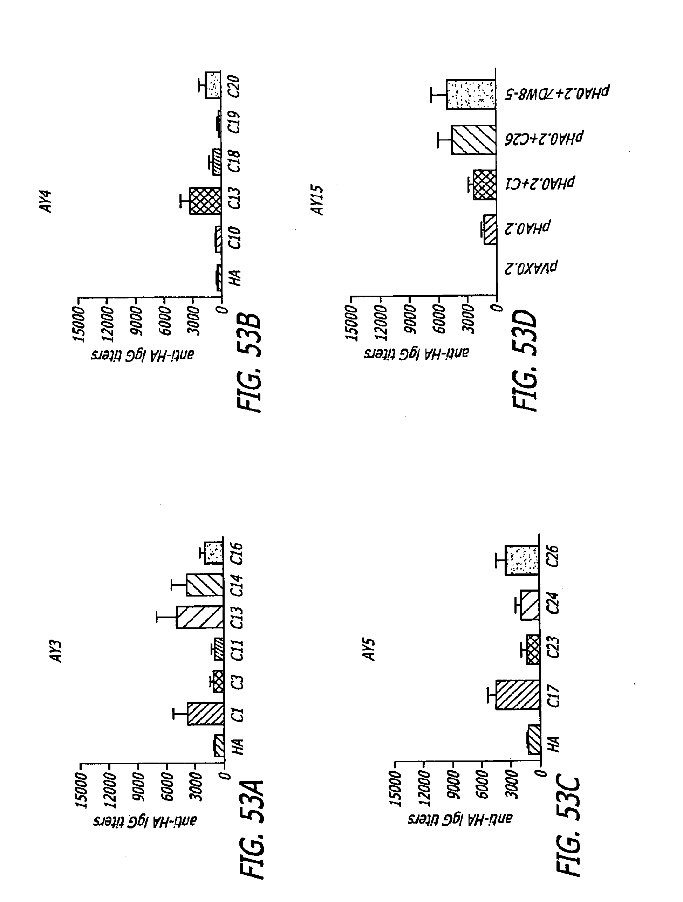

Figure 53 (A-D) show the induction of anti-HA-specific IgG antibody by α-GalCer or the indicated α-GalCer analogs. BALB/c mice were vaccinated by electrotransfer in muscle with α-GalCer or the indicated α-GalCer analogs with pHAc and boosted once with the same formulation 4 weeks later. Blood samples were collected at 2 weeks after the second vaccination and tested for anti-HAc-specific IgG antibody titers by ELISA. (A) shows titers of anti-HA specific IgG antibody (AY3). (B) shows titers of anti-HA specific IgG antibody (AY4). (C) titers of anti-HA specific IgG antibody (AY5). (D) shows titers of anti-HA specific IgG antibody (AY16).

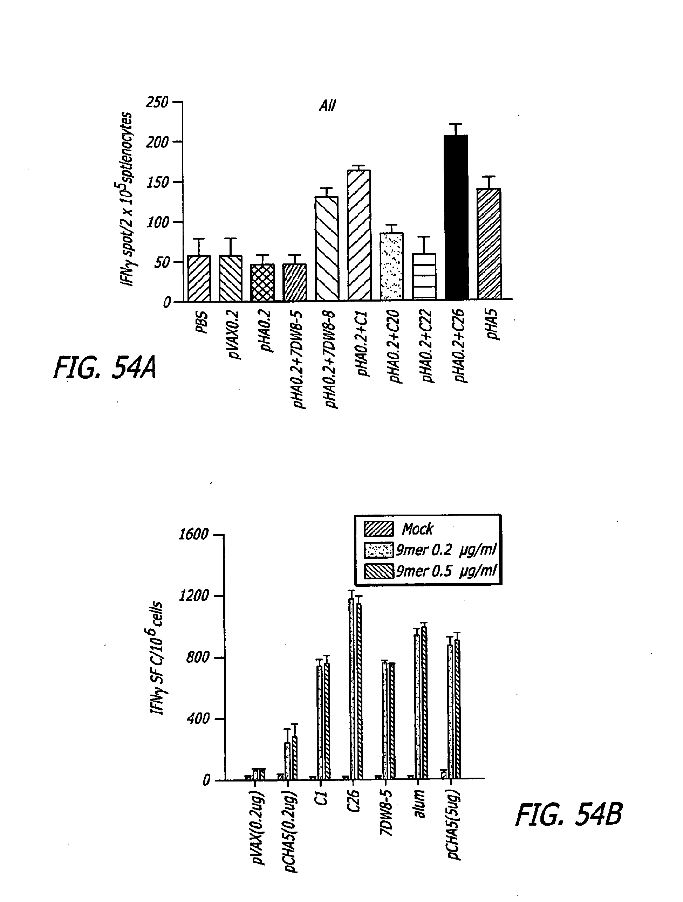

Figure 54(A-B) show (A) HA-specific IFN-γ producing cells and (B) HA-specific peptide response cells. BALB/c mice were vaccinated by electrotransfer in muscle with pHAc and α-GalCer or the indicated α-GalCer analogs and boosted once with the same formulation three weeks later. Splenocytes were cultured with HA-specific peptide (9-mer) and spots were determined after 1 day.

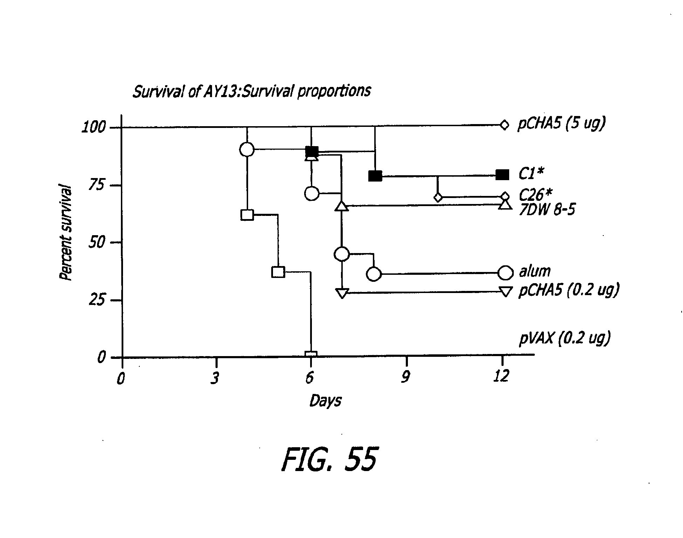

Figure 55 shows protection against viral challenge. BALB/c mice were vaccinated by electrotransfer in muscle with pHAc and α-GalCer or the indicated α-GalCer analogs and boosted once with the same formulation three weeks later. Mice were challenged with 200 LD₅₀ NIBRG-14 viruses at two weeks after the second vaccination and mice survival was monitored.

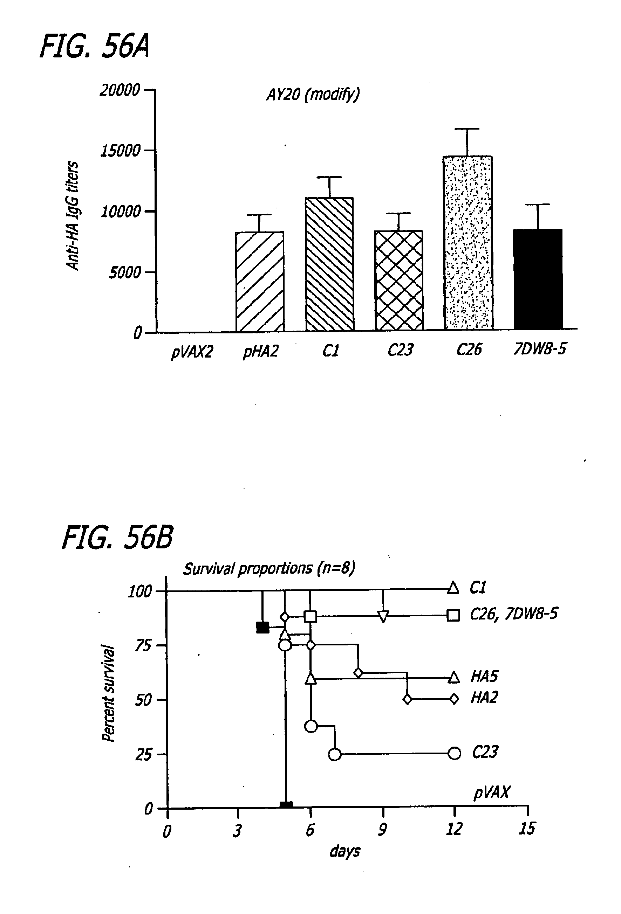

Figure 56 (A-B) show the effect of single dose vaccination. BALB/c mice were vaccinated by electrotransfer in muscle with pHAc (2 µg) and α-GalCer or the indicated α-GalCer analogs (2 µg). (A) Blood samples were collected three weeks later and tested for anti-HAc-specific IgG antibody titers. (B) Mice were challenged with 200 LD₅₀ NIBRG-14 viruses at three weeks after prime and survival was monitored.

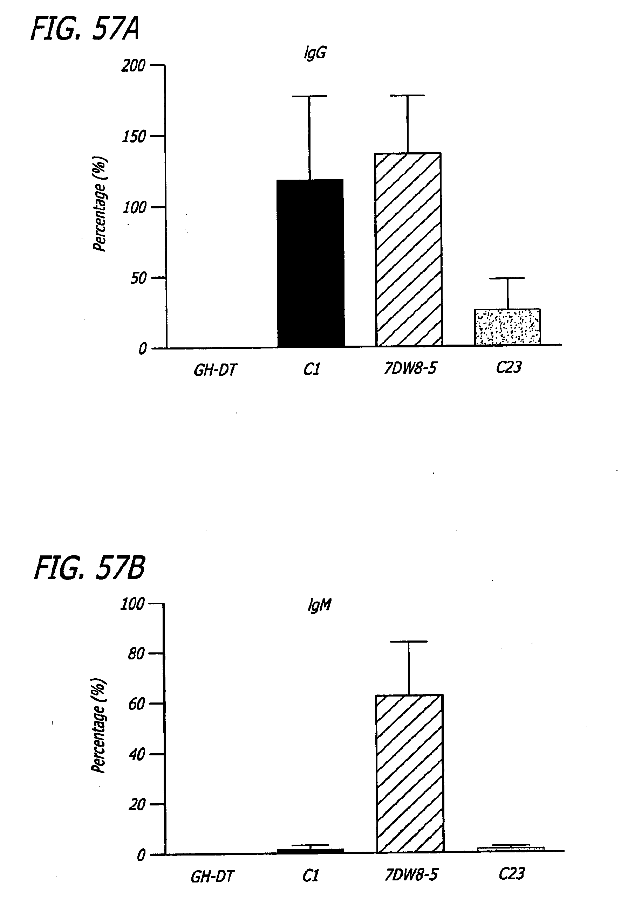

Figure 57 (A-B) show adjuvant effects of α-GalCer or the indicated α-GalCer analogs on carbohydrate antigens. BALB/c mice were vaccinated by IM injection with α-GalCer or the indicated α-GalCer analogs and mixed with globo H-DT and boosted twice within a two week interval. Blood samples were collected two weeks after a third vaccination and tested for (A) anti-globo H-specific IgG antibody and (B) anti-globo H-specific IgM antibody production.

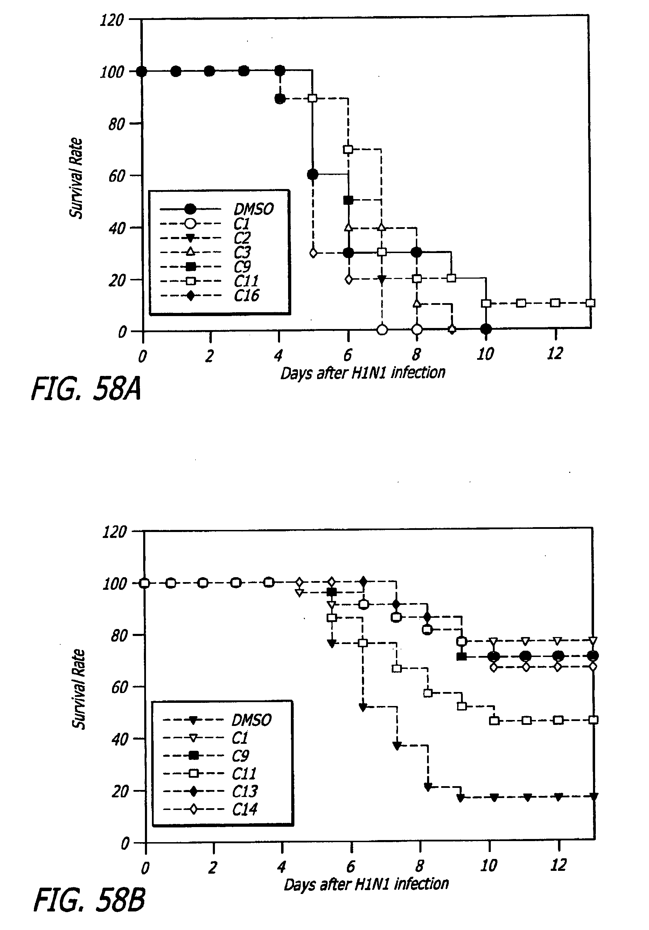

Figure 58(A-B) shows survival rate when BALB/c mice were treated with α-GalCer or the indicated α-GalCer analogs via intraperitoneal (IP) route (A) starting at 30 min after FLU-A virus serotype H1N1 (WSN) virus challenge and (B) starting 2 weeks prior to H1N1 virus challenge.

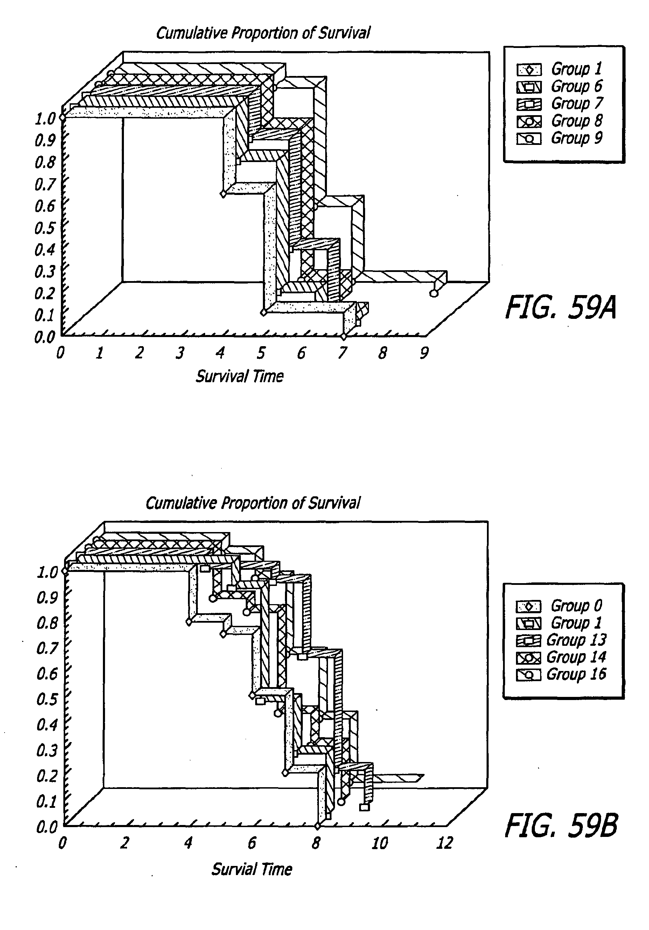

Figure 59 (A-B) shows cumulative proportion of survival of BALB/c mice infected with H1N1 (WSN) and treated with α-GalCer or the indicated α-GalCer analogs (A) starting at 2 weeks prior to virus challenge with a high dose of H1N1 (WSN) virus and (B) via intranasal route.

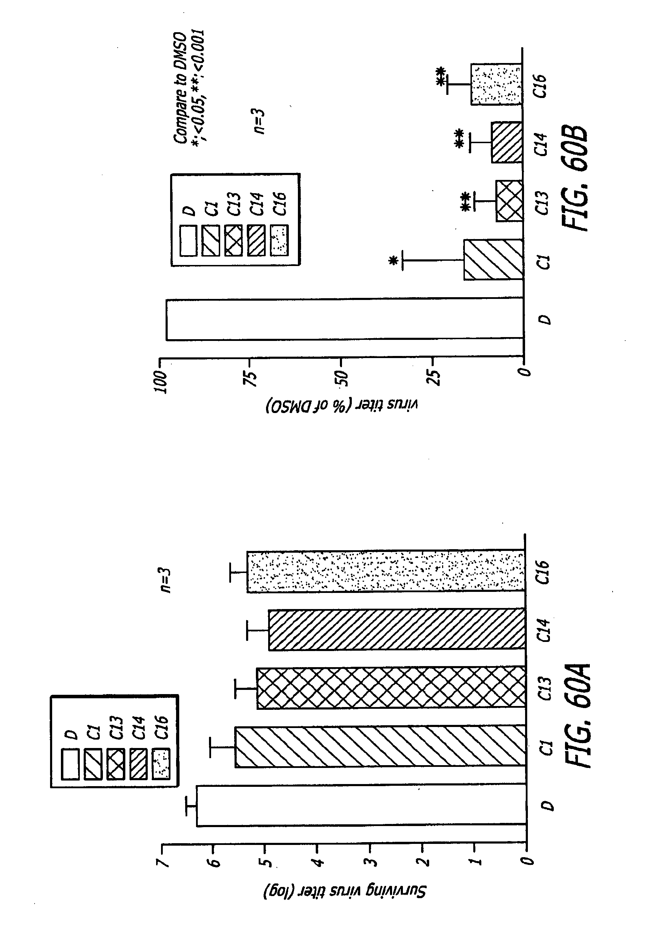

Figure 60(A-B) show the cytopathetic effect (CPE) of Madin-Darby canine kidney (MDCK) cells *in vitro.* MDCK cells were pretreated with vehicle, α-GalCer or one of the α-GalCer analogs C13, C14 or C16 at 10 µg /ml for four hours, followed by infection with FLU-A virus serotype H1N1 (WSN) at 10TCID5O. (A) shows the survival virus titer (log10) after treatment of glycolipids *in vitro* and (B) shows the virus titer in MDCK cells at 48 hours post-infection.

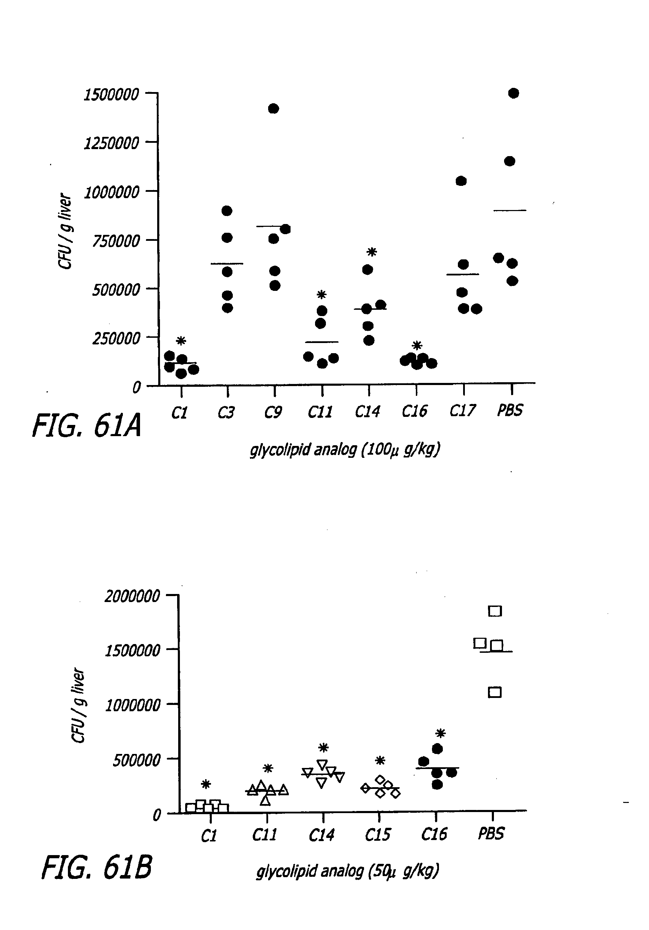

Figure 61 (A-B) show antibacterial efficacies of α-GalCer or the indicated α-GalCer analogs treated at (A) 100 µg /kg or (B) 50 µg /kg in mice infected with Sphingomonas capsulata.

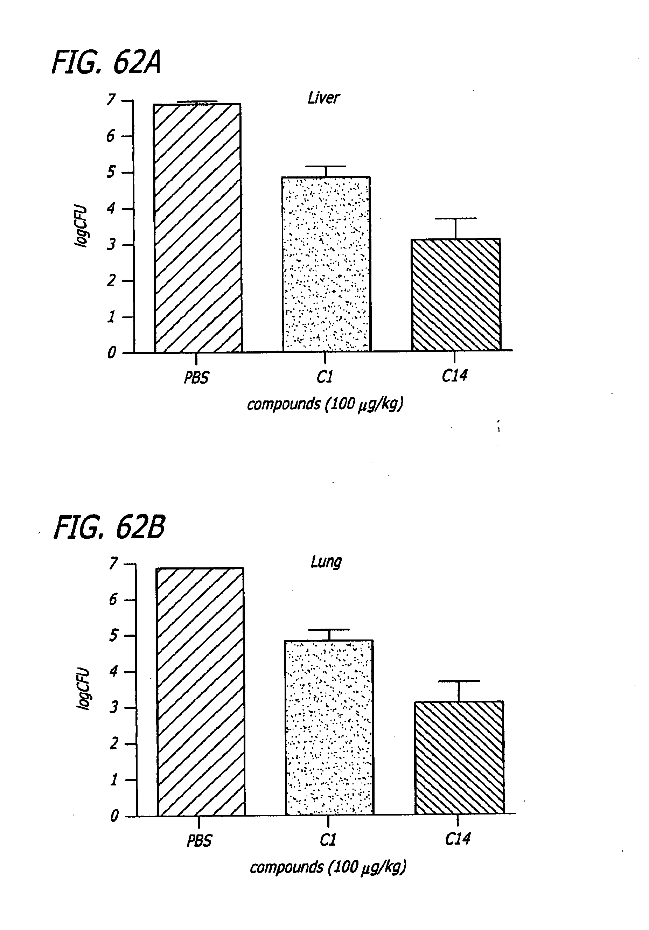

Figure 62 (A-B) show the antibacterial efficacy of α-GalCer or the indicated α-GalCer analogs in mice infected with Klebsiella pneumoniae. C1 and C14 can significantly reduce the bacterial loads in (A) mouse lung and (B) liver after injection.

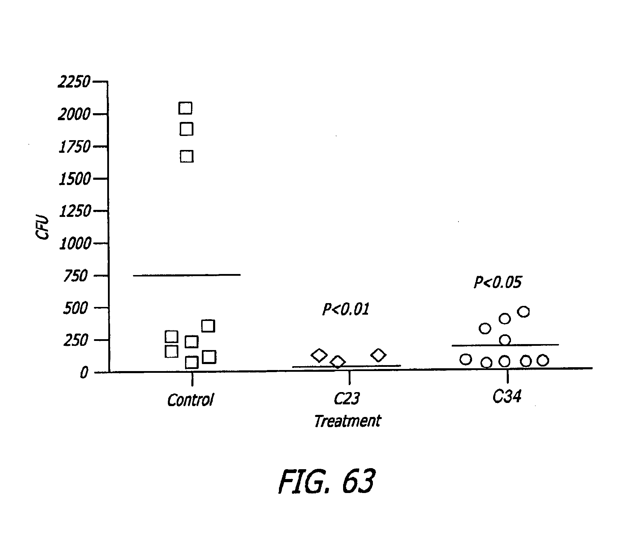

Figure 63 shows that the CFU numbers (in lungs) of the groups treated with C23 and C34 (C34 being of the present invention) at 50 µg/kg, are significant in comparison to the untreated group.

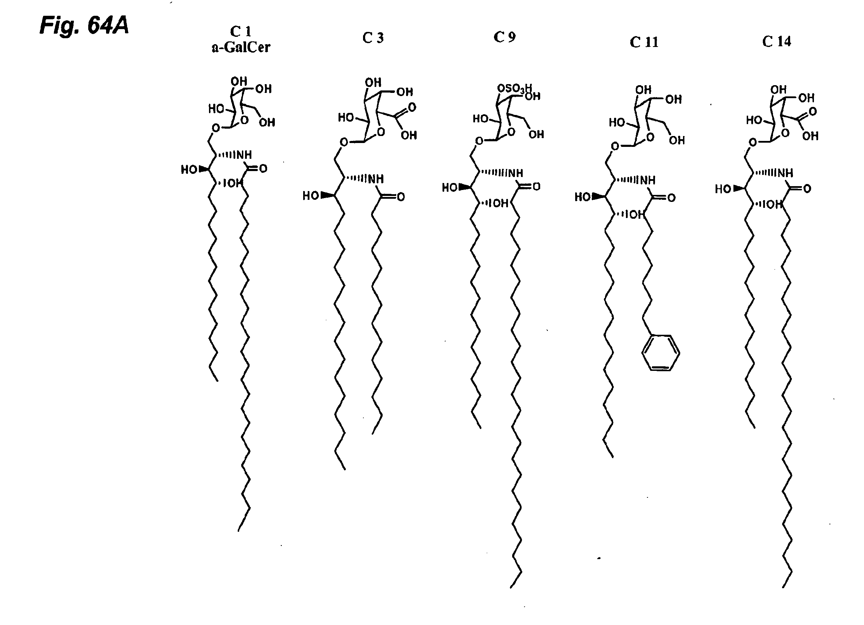

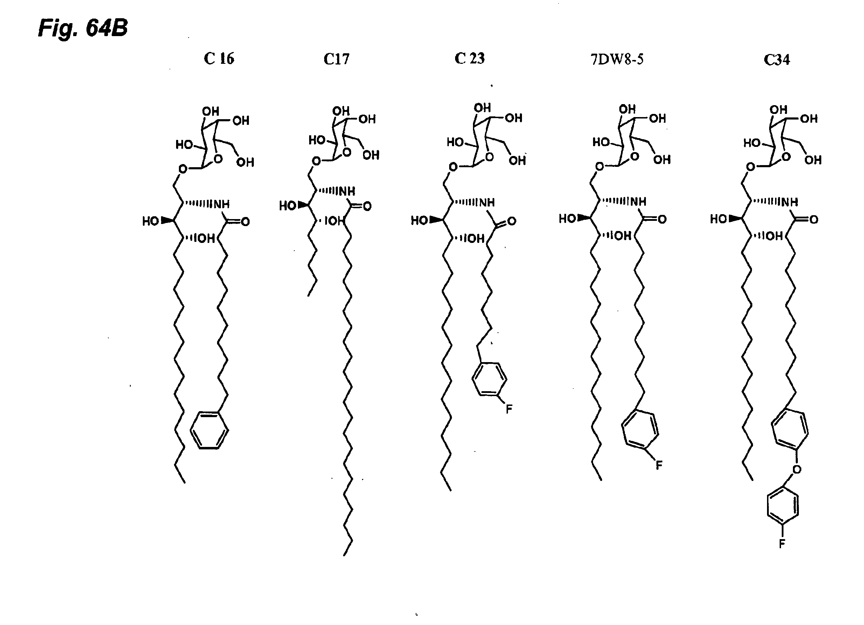

Figure 64 (A-B) show the chemical structures of α-GalCer (C1) and various α-GalCer glycolipids (also referred to as analogs) including: C3, C9, C11, C14, C16, C17, C23, 7DW8-5 (aka, C8-5), and C34 (C34 being of the present invention).

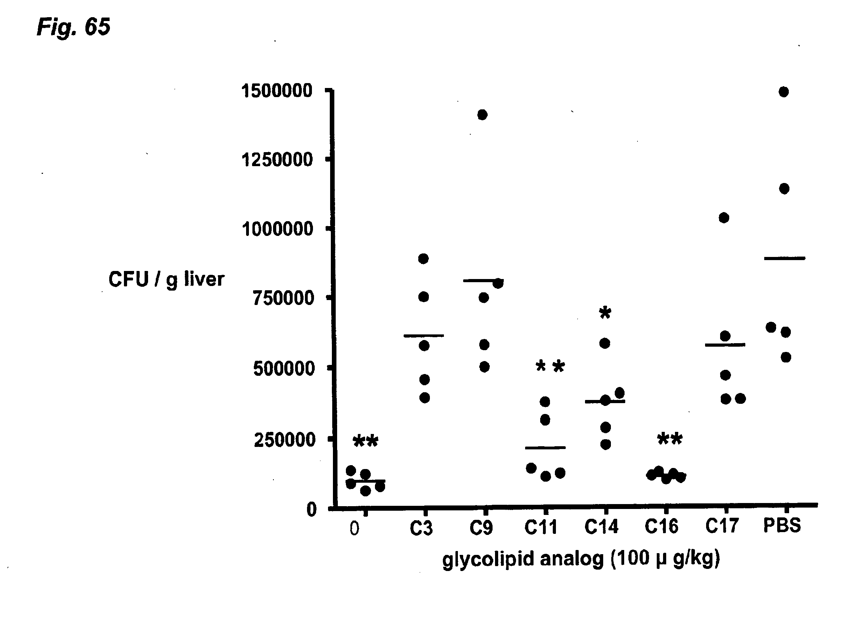

Figure 65 shows the antibacterial efficacies of glycolipids treated at 100 µg /kg in mice infected with *Sphingomorias capsulata.* The CFU values in the livers of mice in each treatment group measured at 24 hr after infection are represented with a dot. The mean CFU values of each treatment group are indicated with short horizontal lines. Statistic analysis using ANOVA found that the groups treated with C1, C11, C14, and C16 resulted in significantly reduced bacterial load in livers. Groups with significant difference from the PBS control are marked with stars: "*" for P<0.05, "*" for P<0.01.

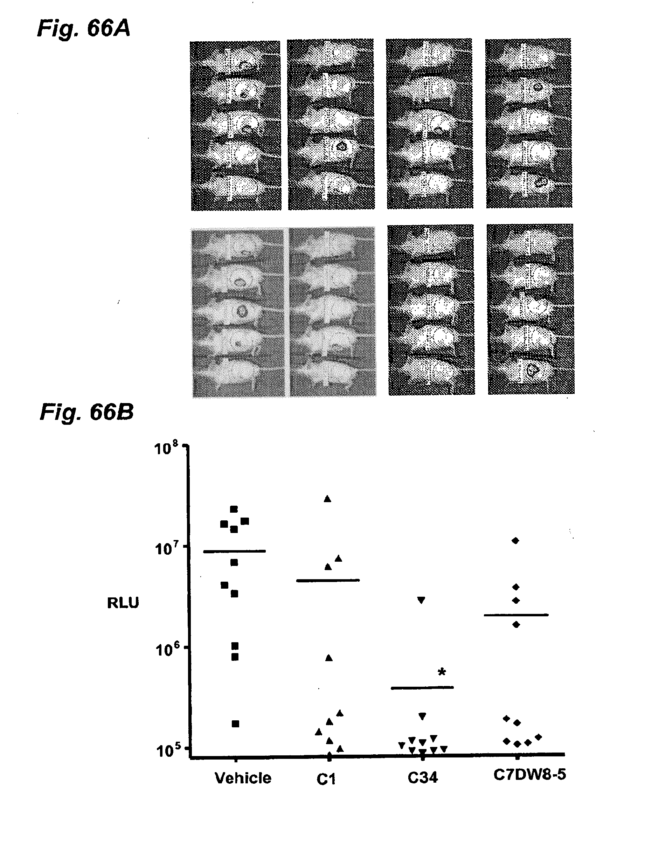

Figure 66 (A-B) show the antibacterial efficacies of selected glycolipids in the *Staphylococcus aureus* thigh infection model. The antibacterial efficacies of glycolipids at 150 µg /kg administered by IP injections at 3 hr post-infection were evaluated by image analyses at 48 hour after infection with luminescence S aureus Xen29. (A) Images of mice in different treatments were taken at 48 hr post-infection. (B) The relative luminescence (RLU) values of each mouse measured at 48 hr post-infection were plotted as individual symbols, and the average values of each treatment group are shown with horizontal lines. *: p<0.05.

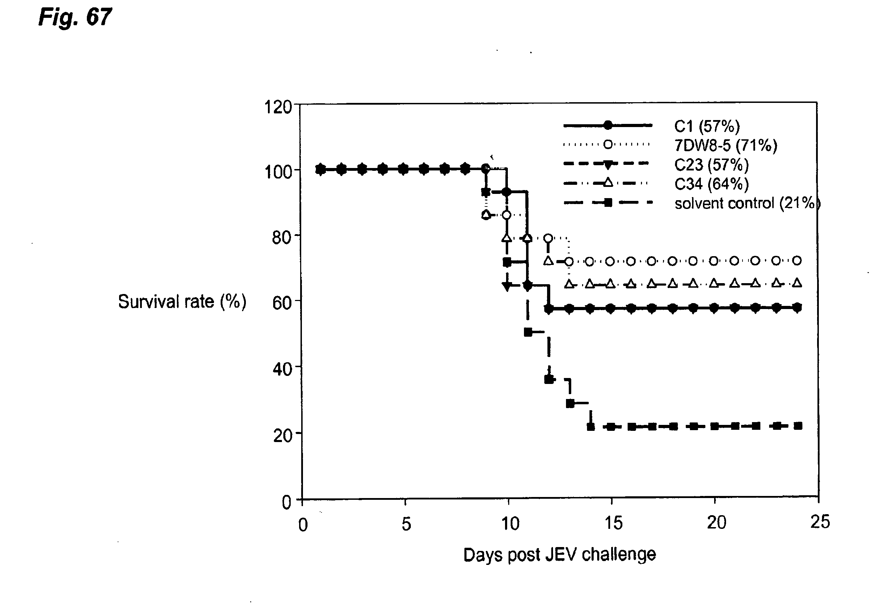

Figure 67 shows glycolipid enhancement of host protection against JEV infection. Seven-week-old wild-type C57BL/6 mice were intraperitoneally (IP) injected with 1 µg of compound C1, 7DW8-5, C23, C34, or solvent for each group one day before virus challenge. The mice were IP infected with 5x10⁵ PFU of JEV (RP-9 strain) and simultaneously injected intracerebrally with 30 µg PBS. One day after virus infection, mice were IP boosted with 1 µg of C1, 7DW8-5, C23, C34 or solvent. The mice were monitored daily for 25 days and the percentage of survival is shown. Fourteen mice were included in each experimental group. Survival curve comparisons were performed using Prism software statistical analysis with the log rank test and P < 0.05 as compared to solvent control is indicated with "*".

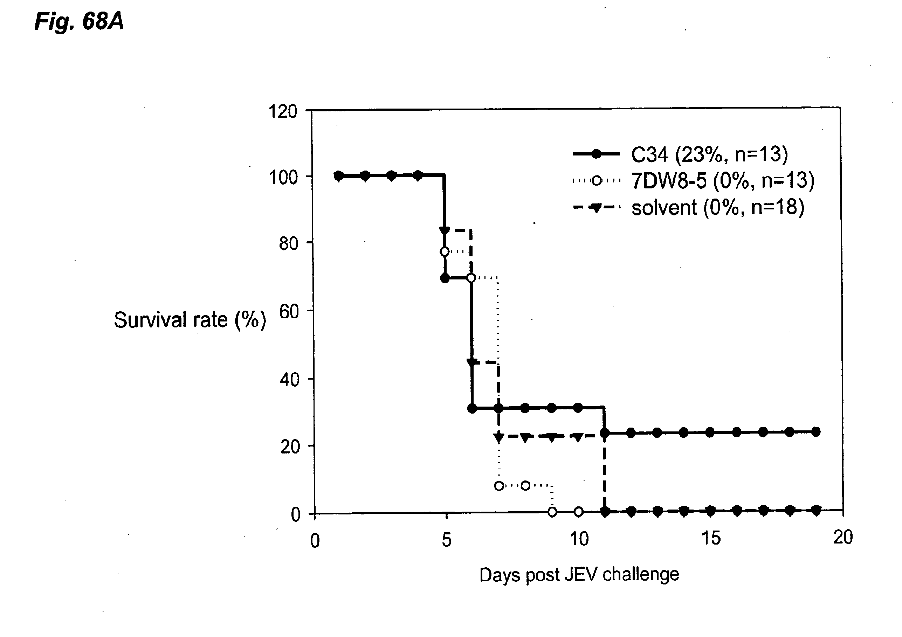

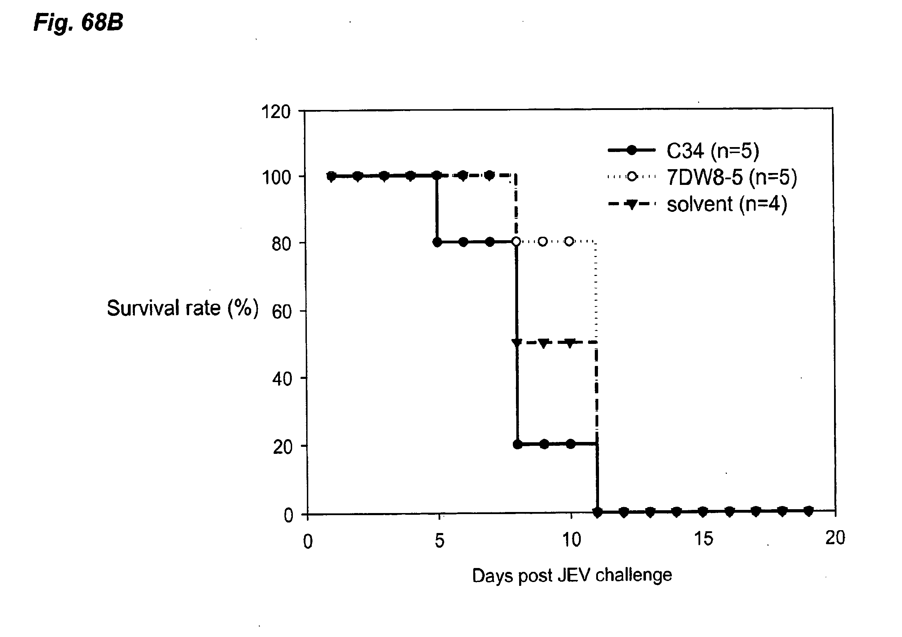

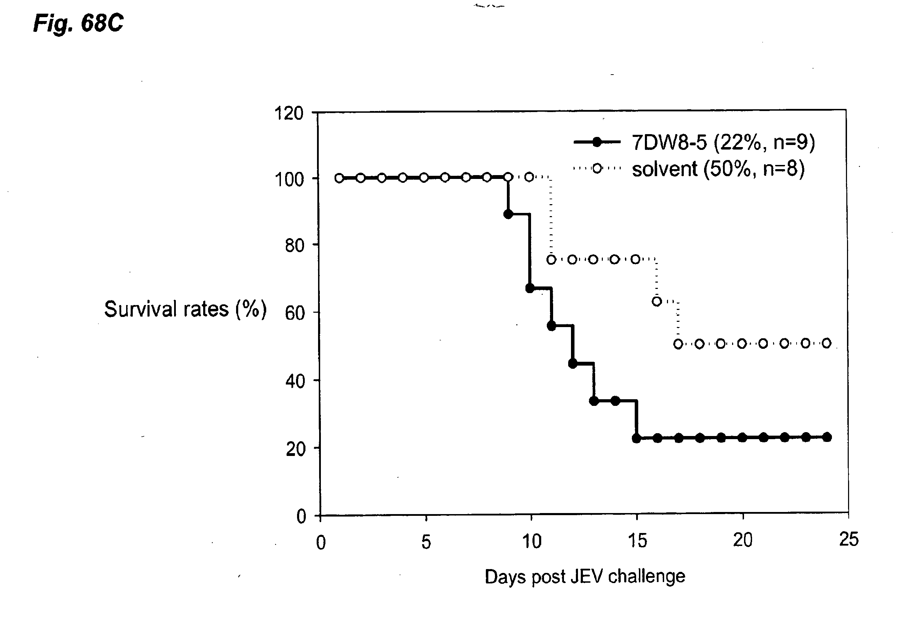

Figure 68 (A-C) show glycolipids' reliance on the host innate and adapted immunity components to trigger protection against JEV infection. Seven-week-old immunodeficient mice lacking of Stat-1 (A), Immunoglobulin µ-chain (B), or CD8α-chain (C) were administrated with C34, 7DW8-5, or solvent as described in Fig. 67 using a two-dose protocol. The Stat-1 knockout mice were challenged with 0.1 PFU of JEV (RP-9 strain) intraperitoneally. The 1µg -chain knockout and CD8α-chain knockout mice were challenged as described in Fig. 67. The mice were monitored daily for 25 days. The percentage survival is shown and the number of mice in each experimental group is shown in the legend to each panel.

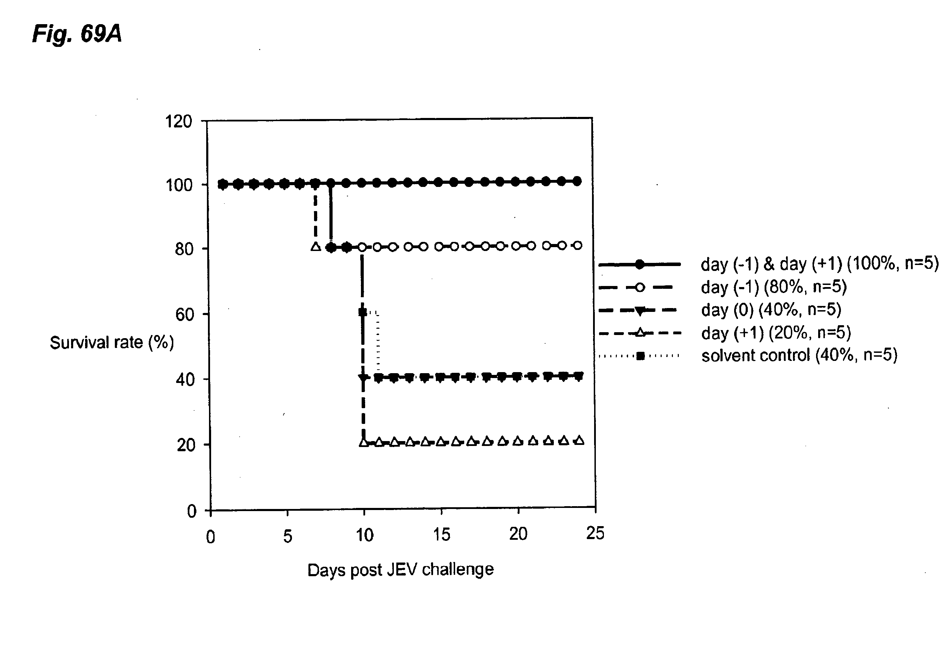

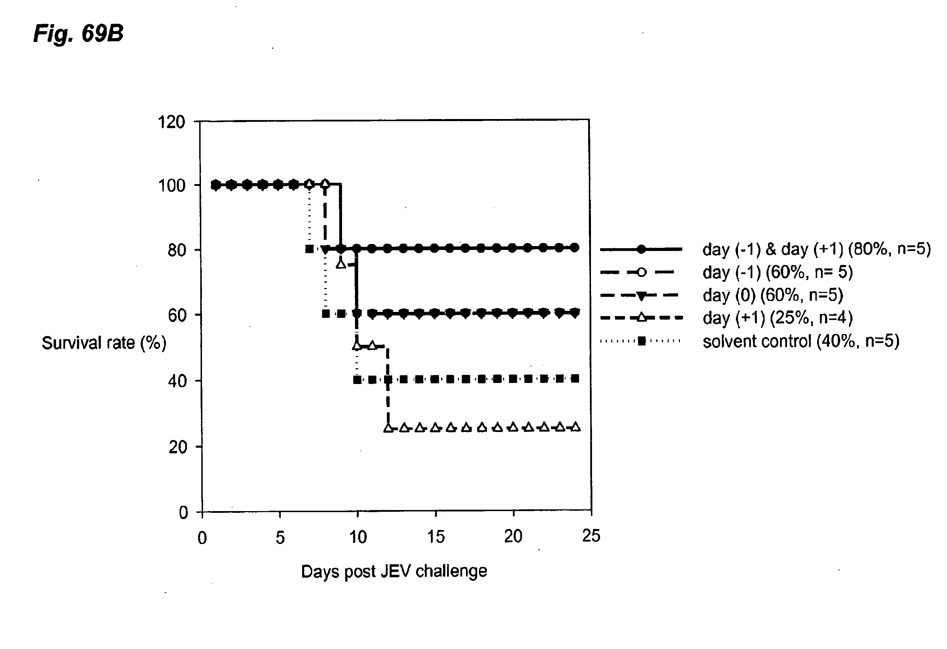

Figure 69 (A-B) show results of a two-dose protocol, one day before and one day after JEV infection, giving the best protective effect. Glycolipid C34 (A) or 7DW8-5 (B) was administrated to 7-week-old wild-type C57BL/6 mice by a two-dose protocol as described in Fig. 67 [day (-1) & day (+1)] or only once one day before [day (-1)], at the same day [day (0)], or one day after [day (+1)] viral Infection. The mice were challenged and monitored as described in Fig. 67. The percentage survival is shown and the number of mice in each experimental group is shown in the legend to each panel.

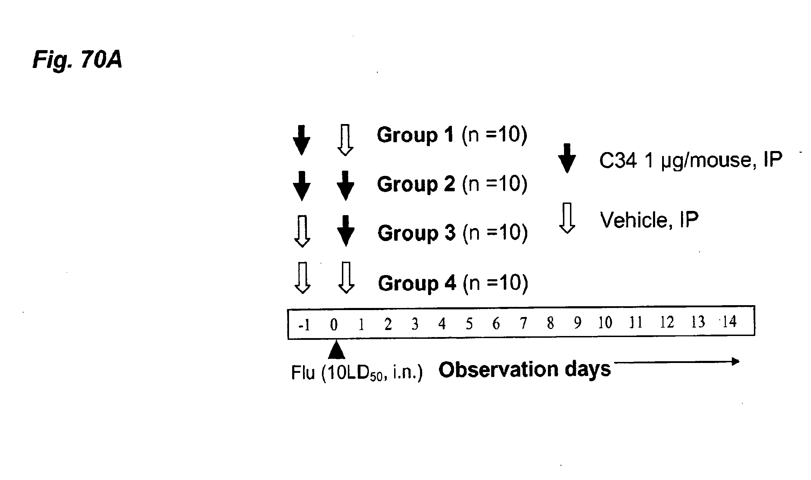

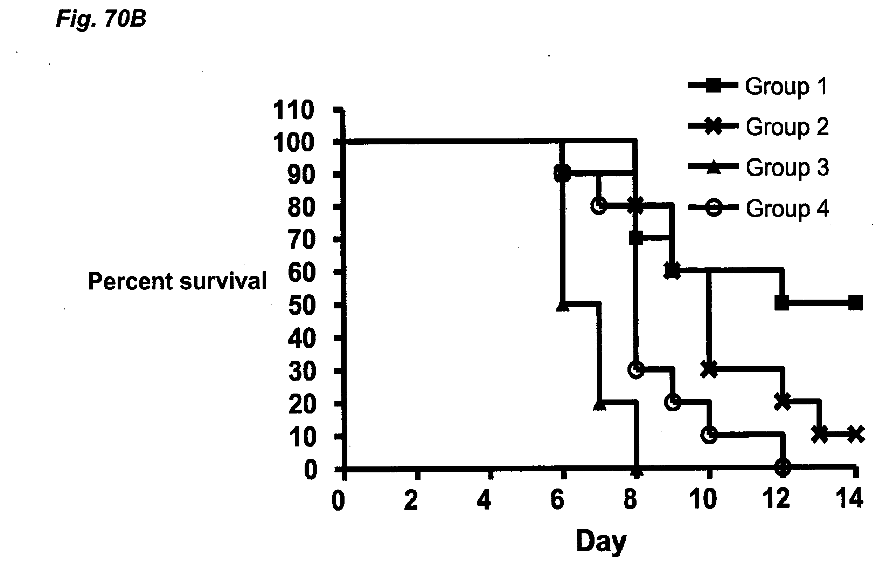

Figure 70 (A-B) show a survival profile of influenza infected mice receiving C34 at different times and doses. Blab/C mice were infected with ten LD₅₀ influenza virus by intra-nasal route. Mice were grouped into four groups receiving two IP injections at both day -1 and day +1 relative to the influenza infection. The study protocol with contents of the IP injection (1 µg/mouse C34 or the PBS vehicle) and the injection times of different treatment groups are described in (A). The survival curves of different treatment groups are shown (B).

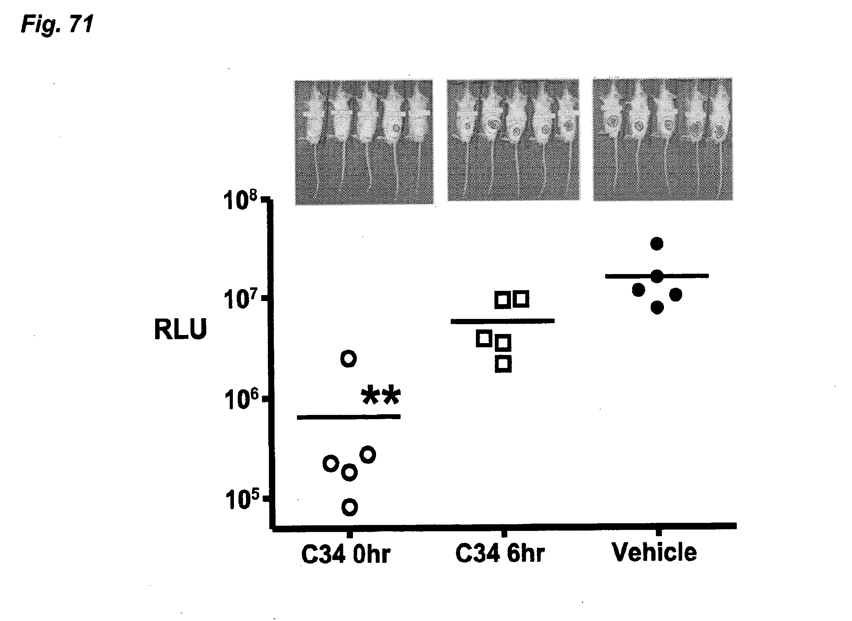

Figure 71 shows effects of C34 administration time on infection clearance of the murine thigh-wound infection model. Mice infected with luminescence S. *aureus* at left thighs were grouped into vehicle control group and two groups that received C34 at 0, and 6 hours after infection. Mice were imaged at 48 hour post infection. The images of the luminescence bacterial infection and the relative luminescence units are shown similar to Figure 66. "**" for significance with p<0.01.

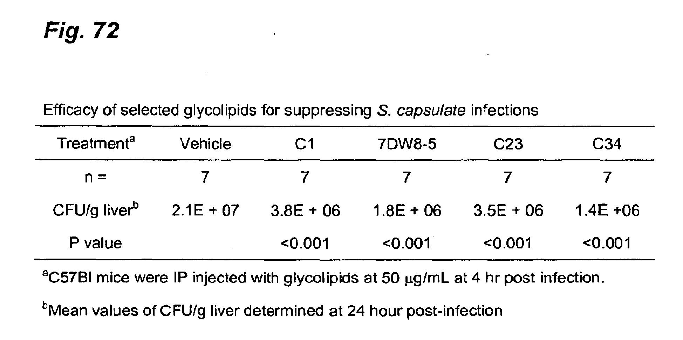

Figure 72 is a table showing the efficacy of selected glycolipids for suppressing S. *capsulate* infections.

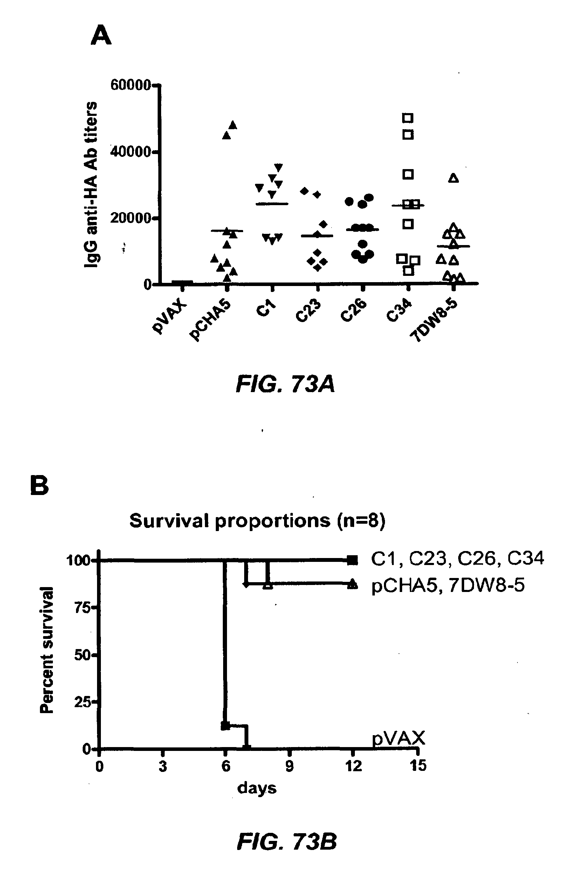

Figures 73(A-B) show immunoprotection against avian influenza virus infection by intramuscular injection of two dosages of pCHA5 +/- glycolipid vaccine. Sera were collected and tested for HA-specific antibody titers by ELISA (A) and mice were challenged with 200 LD₅₀ of NIBRG-14 viruses and monitored for survival (B).

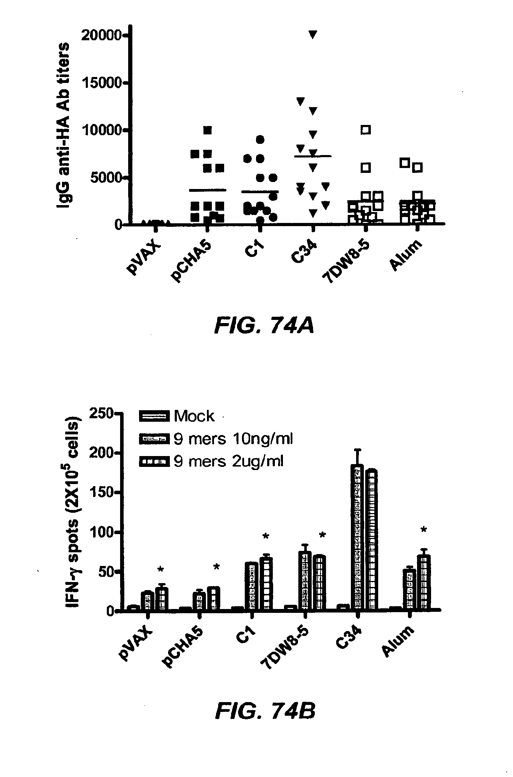

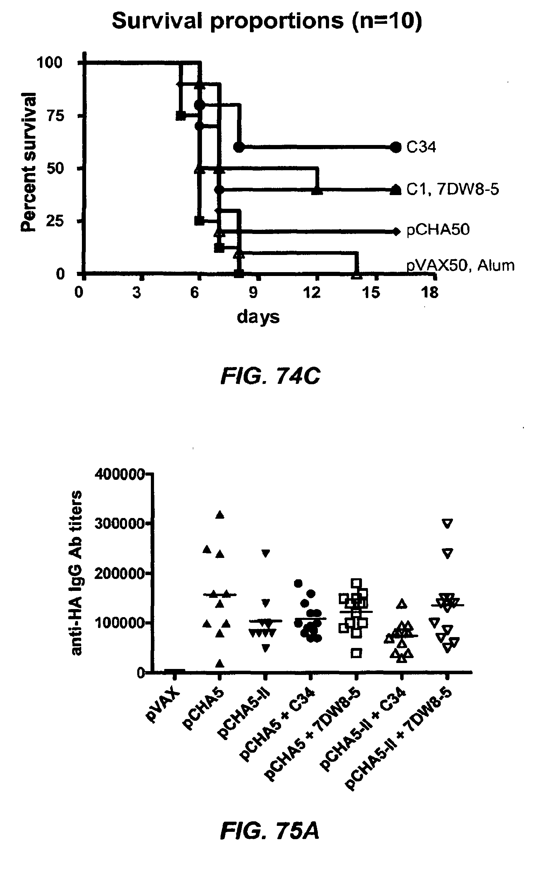

Figures 74(A-C) show immune responses and mice survival after single IM dose of pCHA5 with and without glycolipids as adjuvants. Three weeks after vaccination with pCHA5 (50 µg) +/- glycolipids (2 µg) or alum through IM route, sera were collected and tested HA-specific antibody titers by ELISA (A). At the same time, mice were sacrificed and splenocytes were harvested for ELISPOT assay (B). Another group of mice were challenged with 200 LD₅₀ of NIBRG-14 virus and monitored for survival (C).

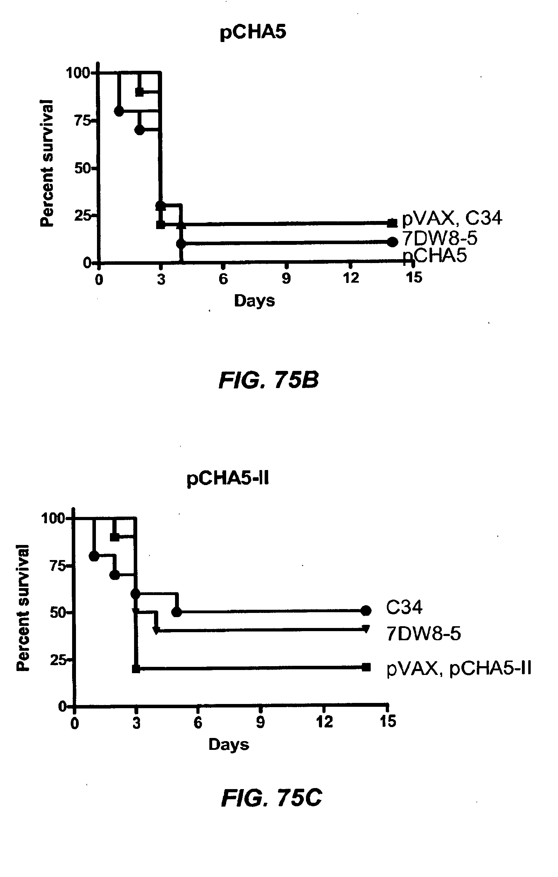

Figures 75(A-C) show comparison of the adjuvant effects of glycolipids for pCHA5 and pCHA5-II vaccine. Two weeks after booster injection for vaccination with pCHA5 or pCHA5-II (30 µg) +/- glycolipids (2 µg) through IM/EP route at week 0 and three, sera were collected and tested anti-HA antibody titers by ELISA (A), and, mice which were vaccinated with pCHA5 (B) and pCHA5-II (C) were challenged with E319 virus and survivals were monitored.

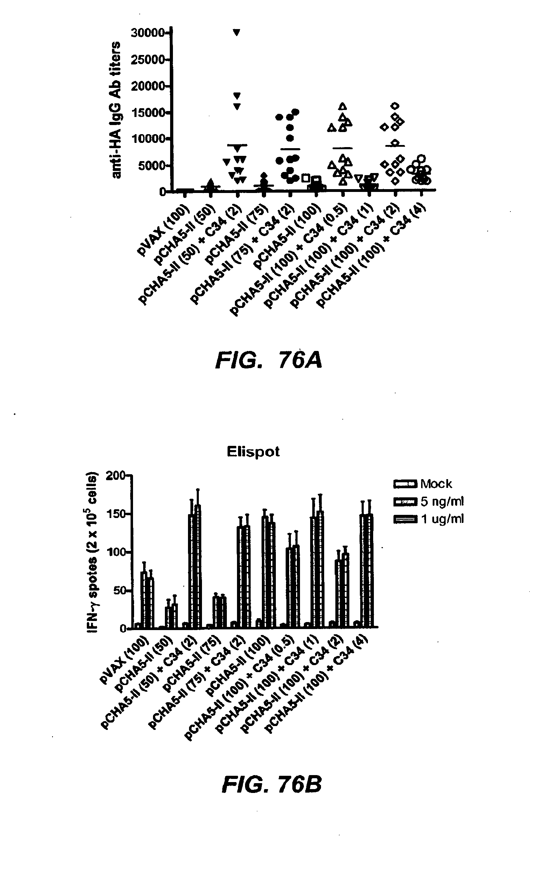

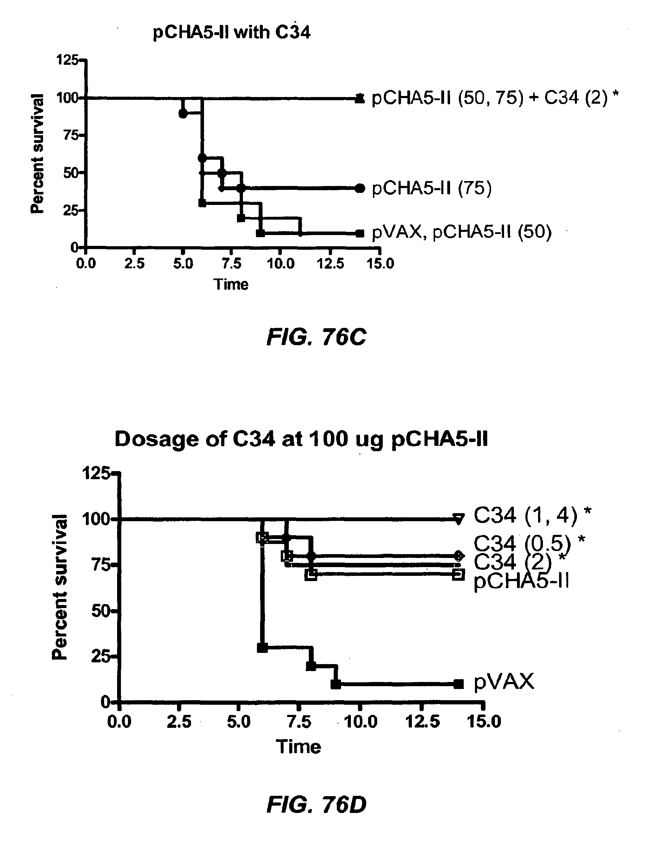

Figures 76(A-D) show dose effects of pCHA5-II and C34 on HA-specific antibody production and immune protection in mice. Three weeks after vaccination with single dose of pCHA5-II (100,75,50 µg) with or without C34 (0.5, 1,2,4 µg) as adjuvant through IM route, sera were collected and HA-specific antibody titers were assayed by ELISA (A). At the same time, mice were sacrificed and splenocytes were harvested for ELISPOT assay (B). Fig. 76C shows survivals of mice treated with different doses of pCHA5-II and 2 µg C34 adjuvant. Fig. 76D shows survivals of mice treated with 100 µg pCHA5-II and C34 at various dosages.

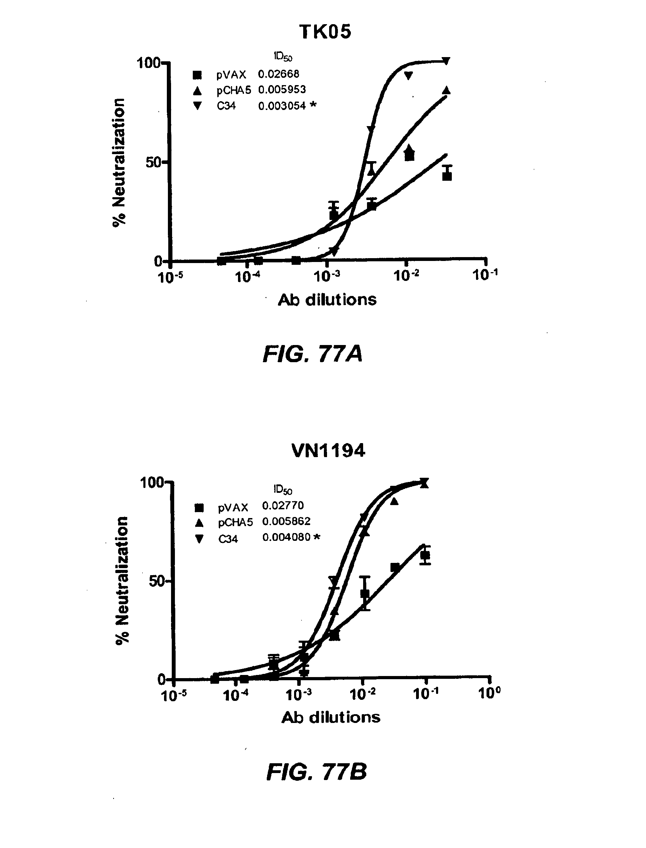

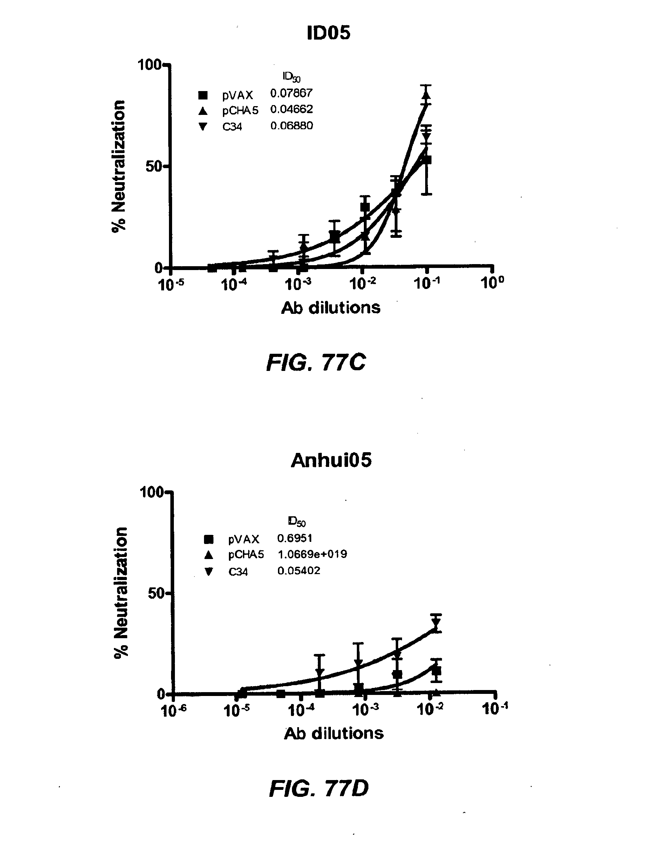

Figures 77(A-D) show adjuvant effect of C34 on neutralization antibody production against various HA-pseudotyped viruses. Nonlinear regression analysis for serum dilutions after IM/EP vaccination with 0.2 µg of pCHA5 dissolved in PBS containing 2 µg of C34 on week 0 and 3, giving 50% of HA-pseudotyped virus neutralization (ID₅₀) for TK (A), VN1194 (B), ID05 (C), and Anhui05 (D) are shown. Value of p was the comparison of fits. *p< 0.05 and presented statistical significant when compare C34-adjuvanted group with pCHA5 only group.

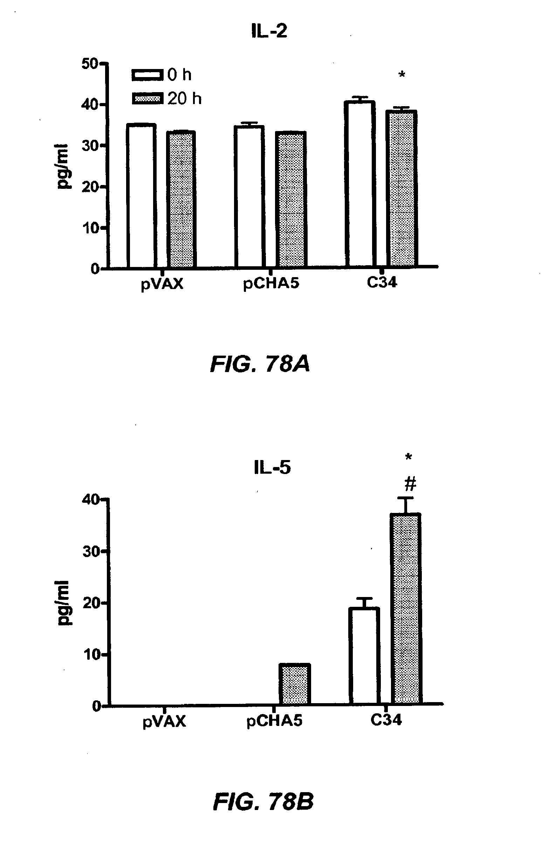

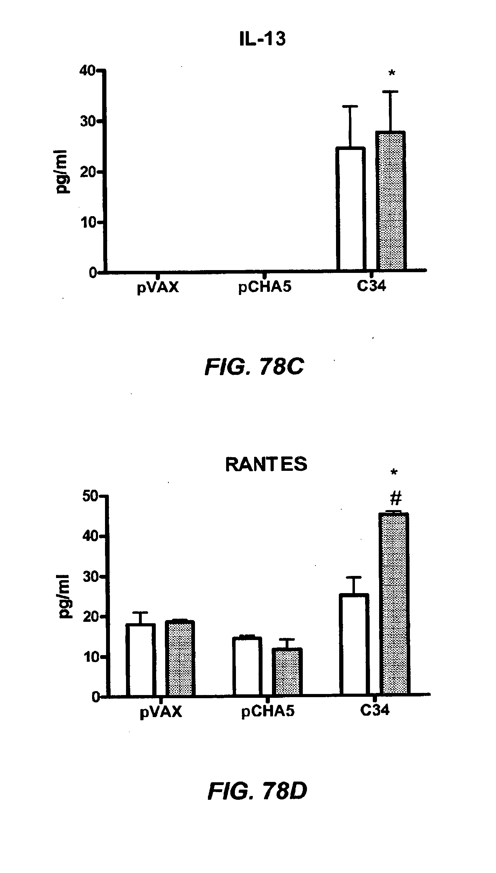

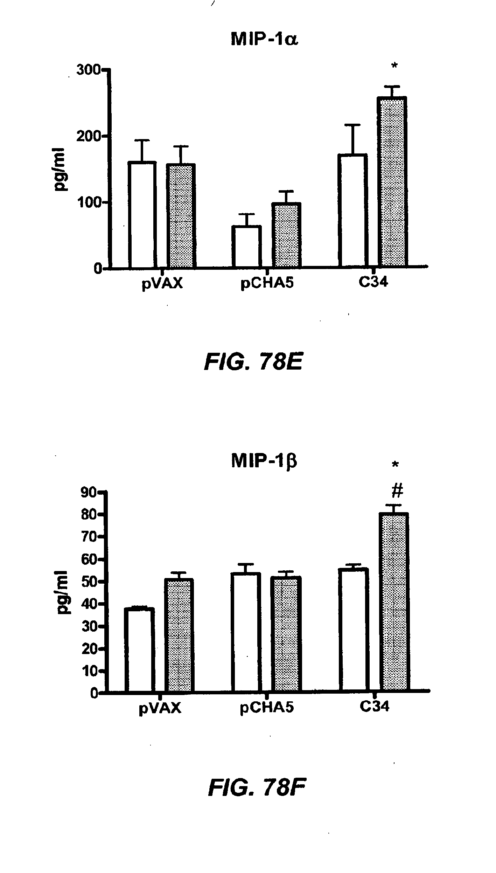

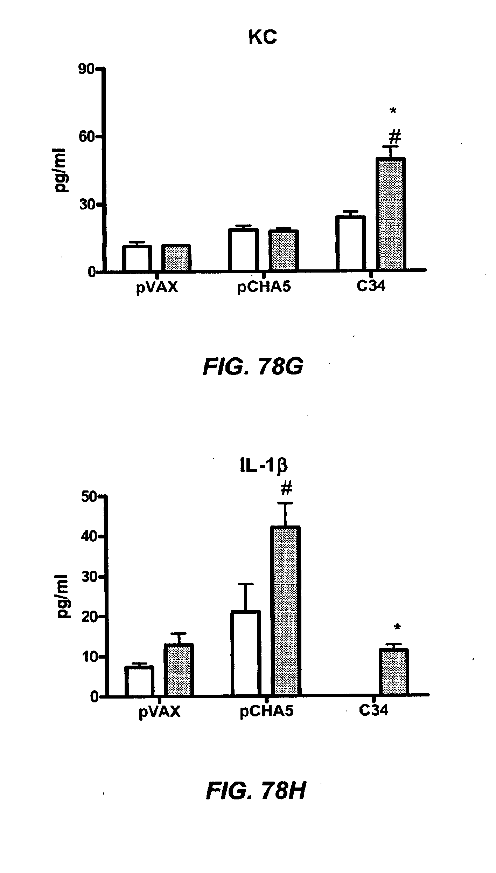

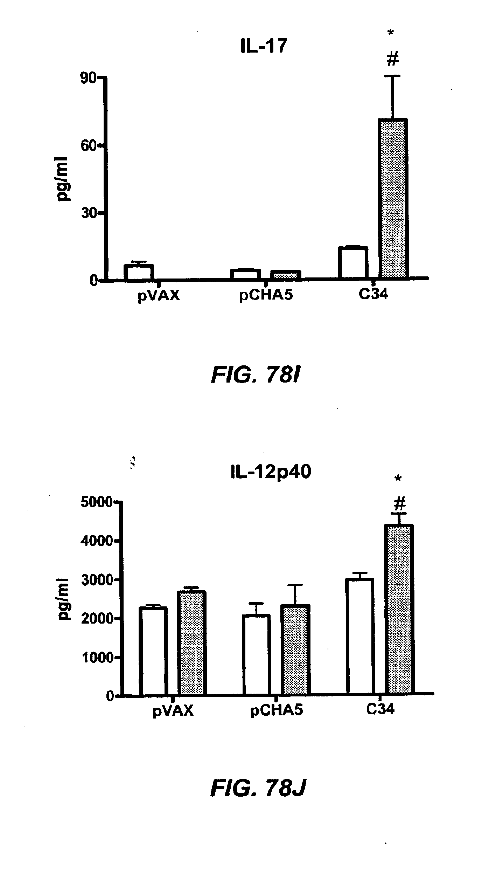

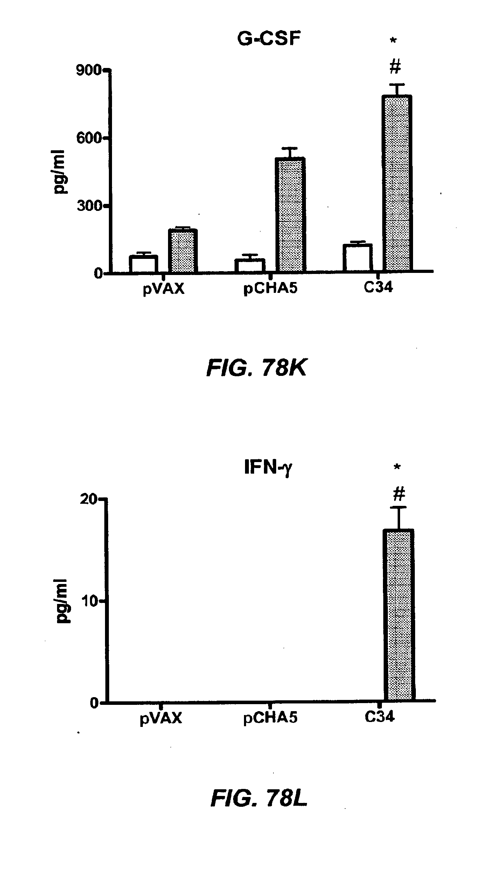

Figures 78(A-L) show serum cytokine expression profiles in mice receiving pCHA5 with or without C34 as adjuvant. Serum concentrations after vaccination with 0.2 µg pCHA5 with or without C34 through IM/EP route at week 0 and 3 are shown with respect to IL-2 (A), IL-5 (B), IL-13 (C), RANTES (D), MIP-1α (E), MIP-1β (F), KC (G), IL-1β (H), IL-17 (I), IL-12p40 (J), G-CSF (K), and IFN-γ (L).

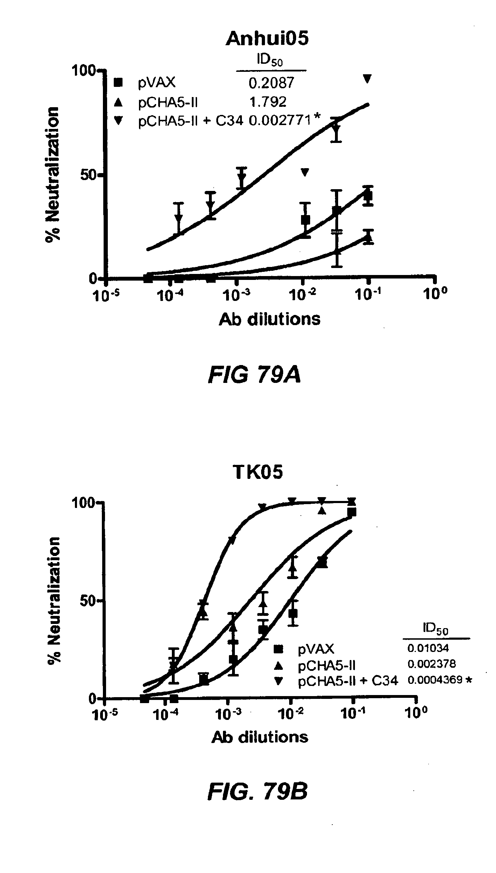

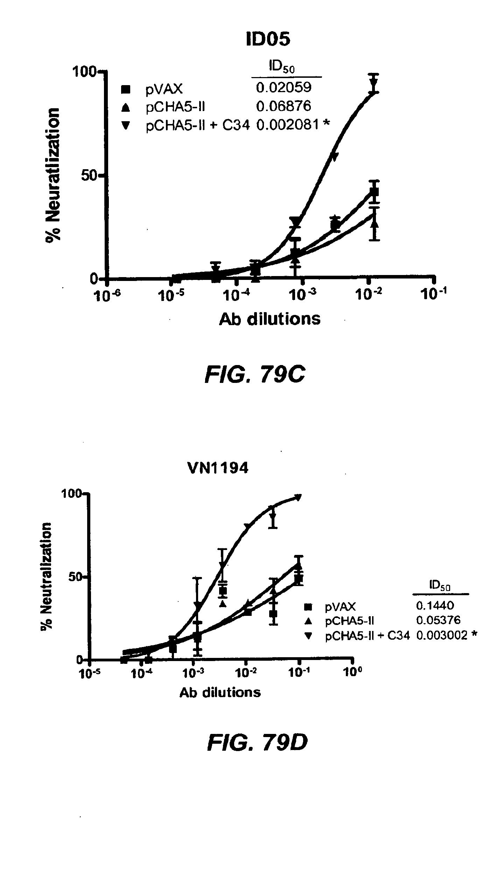

Figures 79(A-D) show neutralization abilities of antisera were induction by C34 through single dose intramuscular injection. Results of HA-pseudotyped virus neutralization assay performed three weeks after vaccination with 50 µg of pCHA5-II dissolved in PBS containing 2 µg of C34 are shown with respect to Anhi05 (A), TK05 (B), ID05 (C), and VN1194 (D). Value of p was the comparison of fits. * p < 0.05 and presented statistical significant when compare C34-adjuvanted group with pCHA5-II only group.

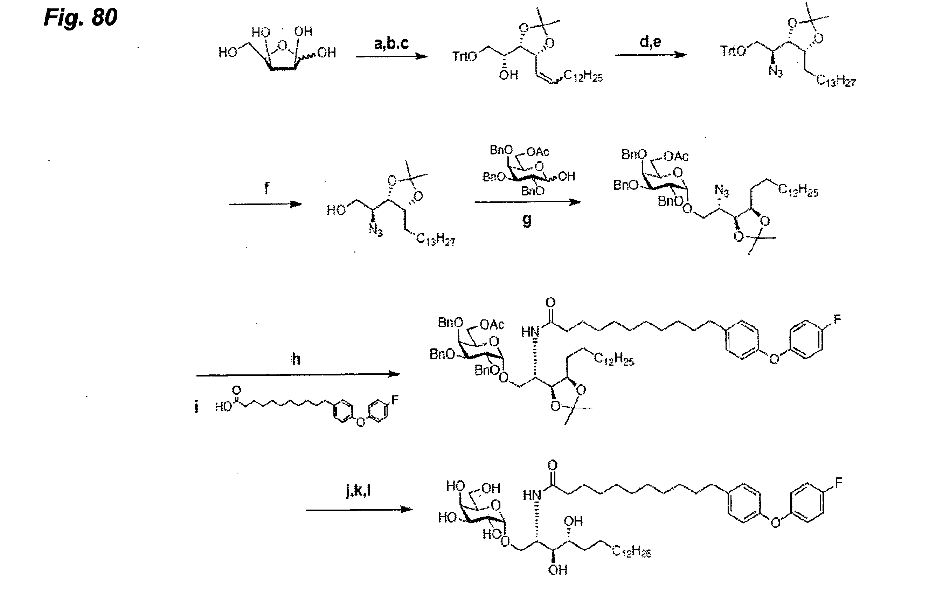

Figure 80 shows a synthetic scheme for C34 α-GalCer analog of the present disclosure. Reagents and conditions shown are as follows: (a) 2-Methoxypropene, CSA, DMF, 83%; (b) Ph₃CCl, Pyridine. 77%; (c) C₁₃H₂₇*PPh₃⁻Br, LHMDS, THF: 75%; (d) H₂, Pd(OH)₂, EA, 90% (e) Tf₂O. 2,6-Lutidine, TAlGA, CH₂Cl₂; (f) TFA/TFAA, CH₂Cl₂, two steps 63%; (g) Tf₂O, Me₂S, 2-Cl-Pyridine, MS4A, CH₂Cl₂, 60%; (h) PPh₃ Pyridine/H₂O; (i) EDC, HBTU, TEA, CH₂Cl₂, two steps 88%; 0) NaOMe, MeOH/ CH₂Cl₂; (k) AcOH(aq.); (I) H₂, Pd(OH)2, MeOH/CHCl₃, three steps 40%.

### DETAILED DESCRIPTION OF THE DISCLOSURE

All scientific terms are to be given their ordinary meanings as understood by those of skill in the art, unless an alternate meaning is set forth below. In case of conflict, the definitions set forth in this specification shall control.

As used herein, the term "lipid" refers to any fat-soluble (lipophilic) molecule that participates in cell signaling pathways.

As used herein, the term "glycolipid" refers to a carbohydrate-attached lipid that serves as a marker for cellular recognition.

As used herein, the term "aipha-galactosyl ceramide" and "α-GalCer" refers to a glycolipid that stimulates natural killer T cells to produce both T helper (T_{H})1 and T_{H}2 cytokines.

As used herein, the term "glycan" refers to a polysaccharide, or oligosaccharide. Glycan is also used herein to refer to the carbohydrate portion of a glycoconjugate, such as a glycoprotein, glycolipid, glycopeptide, glycoproteome, peptidoglycan, lipopolysaccharide or a proteoglycan. Glycans usually consist solely of O-glycosidic linkages between monosaccharides. For example, cellulose is a glycan (or more specifically a glucan) composed of beta-1,4-linked D-glucose, and chitin is a glycan composed of beta-1,4-linked N-acetyl-D-glucosamine. Glycans can be homo or heteropolymers of monosaccharide residues, and can be linear or branched. Glycans can be found attached to proteins as in glycoproteins and proteoglycans. They are generally found on the exterior surface of cells. O- and N-linked glycans are very common in eukaryotes but may also be found, although less commonly, in prokaryotes. N-Linked glycans are found attached to the R-group nitrogen (N) of asparagine in the sequon. The sequon is a Asn-X-Ser or Asn-X-Thr sequence, where X is any amino acid except proline

As used herein, the term "glycoprotein" refers to a protein covalently modified with glycan(s). There are four types of glycoproteins: 1) N-linked glycoproteins, 2) O-linked glycoproteins (mucins), 3) glucosaminoglycans (GAGs, which are also called proteoglycans), 4) GPI-anchored. Most glycoproteins have structural micro-heterogeneity (multiple different glycan structures attached within the same glycosylation site), and structural macro-heterogeneity (multiple sites and types of glycan attachment).

As used herein, the term "analog" refers to a compound, e.g., a drug, whose structure is related to that of another compound but whose chemical and biological properties may be quite different.

As used herein, the term "antigen" is defined as any substance capable of eliciting an immune response.

As used herein, the term "pathogen" is a biological agent that causes disease or illness to ifs host. The body contains many natural defenses against some of the common pathogens (such as *Pneumocysti*s*)* in the form of the human immune system.

As used herein, the term "immunogen" refers to an antigen or a substance capable of inducing production of an antigen, such as a DNA vaccine.

As used herein, the term "immunogenicity" refers to the ability of an immunogen, antigen, or vaccine to stimulate an immune response.

As used herein, the term "immunotherapy" refers to an array of treatment strategies based upon the concept of modulating the immune system to achieve a prophylactic and/or therapeutic goal.

As used herein, the term "CD1d" refers to a member of the CD1 (cluster of differentiation 1) family of glycoproteins expressed on the surface of various human antigen-presenting cells. CD1d presented lipid antigens activate natural killer T cells. CD1d has a deep antigen-binding groove into which glycolipid antigens bind. CD1d molecules expressed on dendritic cells can bind and present glycolipids.

As used herein, the term "adaptive immune system" refers to highly specialized, systemic cells and processes that eliminate pathogenic challenges. The cells of the adaptive immune system are a type of leukocyte, called a lymphocyte. B cells and T cells are the major types of lymphocytes.

As used herein, the term "T cells" and "Ts" refer to a group of white blood cells known as lymphocytes, that play a central role in cell-mediated immunity. T cells can be distinguished from other lymphocyte types, such as B cells and NKs by the presence of a special receptor on their cell surface called the T cell receptor (TCR). Several different subsets of T cells have been described, each with a distinct function. Helper T (T_{H}) Cells are the "middlemen" of the adaptive immune system. Once activated, they divide rapidly and secrete small proteins called cytokines that regulate or "help" the immune response. Depending on the cytokine signals received, these cells differentiate into T_{H}1, T_{H}2, T_{H}17, or one of other subsets, which secrete different cytokines.

As used herein, the term "antigen-presenting cell" (APC) refers to a cell that displays foreign antigen complexed with major histocompatibility complex (MHC) on its surface. T-cells may recognize this complex using their TCR. APCs fall into two categories: professional or non-professional. Dendritic cells (DCs) fall under the professional category and are capable of presenting antigen to T cells, in the context of CD1. As described herein, the DCs may be of any of several DC subsets, which differentiate from, in one implementation, lymphoid or, in another implementation, myeloid bone marrow progenitors.

As used herein, the term "naïve cell" refers to an undifferentiated immune system cell, for example a CD4 T-cell, that has not yet specialized to recognize a specific pathogen.

As used herein, the term "natural killer cells" and "NKs" refers to a class of lymphoid cells which are activated by interferons to contribute to innate host defense against viruses and other intracellular pathogens.

As used herein, the term "natural killer T cells" (NKTs) refers to a subset of T cells that share characteristics / receptors with both conventional Ts and NKs. Many of these cells recognize the non-polymorphic CD1d molecule, an antigen-presenting molecule that binds self-and foreign lipids and glycolipids. The TCR of the NKTs are able to recognize glycolipid antigens presented (chaperoned) by a CD1d molecule. A major response of NKTs is rapid secretion of cytokines, including IL-4, IFN-γ and IL-10 after stimulation and thus influence diverse immune responses and pathogenic processes. The NKTs may be a homogenous population or a heterogeneous population. In one exemplary implementation, the population may be "non-invariant NKTs", which may comprise human and mouse bone marrow and human liver T cell populations that are, for example, CD1d-reactive noninvariant T cells which express diverse TCRs, and which can also produce a large amount of IL-4 and IFN-γ. The best known subset of CD1d -dependent NKTs expresses an invariant TCR-alpha (TCR-α) chain. These are referred to as type I or invariant NKTs (iNKTs). These cells are conserved between humans (Vat24i NKTs) and mice (Vα14i NKTs) and are implicated in many immunological processes.

As used herein, the term "cytokine" refers to any of numerous small, secreted proteins that regulate the intensity and duration of the immune response by affecting immune cells differentiation process usually involving changes in gene expression by which a precursor cell becomes a distinct specialized cell type. Cytokines have been variously named as lymphokines, interleukins, and chemokines, based on their presumed function, cell of secretion, or target of action. For example, some common interieukins include, but are not limited to, IL-12, IL-18, IL-2, IFN-γ, TNF, IL-4, IL-10, IL-13, IL-21 and TGF-β.

As used herein, the term "chemokine" refers to any of various small chemotactic cytokines released at the site of infection that provide a means for mobilization and activation of lymphocytes. Chemokines attract leukocytes to infection sites. Chemokines have conserved cysteine residues that allow them to be assigned to four groups. The groups, with representative chemokines, are C-C chemokines (RANTES, MCP-1, MIP-1α, and MIP-1β), C-X-C chemokines (IL-8), C chemokines (Lymphotactin), and CXXXC chemokines (Fractalkine).

As used herein, the term "T_{H}2-type response" refers to a pattern of cytokine expression such that certain types of cytokines, interferons, chemokines are produced. Typical T_{H}2 cytokines include, but are not limited to, IL-4, IL-5, IL-6 and IL-10.

As used herein, the term "T_{H}1-type response" refers to a pattern of cytokine expression such that certain types of cytokines, interferons, chemokines are produced. Typical T_{H}1 cytokines include, but are not limited to, IL-2, IFN-γ, GM-CSF and TNF-β.

As used herein, the term "T_{H}1 biased" refers to am immunogenic response in which production of T_{H}1 cytokines and/or chemokines is increased to a greater extent than production of T_{H}2 cytokines and/or chemokines.

As used herein, the term "epitope" is defined as the parts of an antigen molecule which contact the antigen binding site of an antibody or a T cell receptor.

As used herein, the term "vaccine" refers to a preparation that contains an antigen, consisting of whole disease-causing organisms (killed or weakened) or components of such organisms, such as proteins, peptides, or polysaccharides, that is used to confer immunity against the disease that the organisms cause. Vaccine preparations can be natural, synthetic or derived by recombinant DNA technology.

As used herein, the term "antimicrobial" refers to a substance that kills or inhibits the growth of microbes such as bacteria, fungi, or viruses.

As used herein, the term "toxoid" refers to a bacterial toxin whose toxicity has been weakened or suppressed either by chemical (formalin) or heat treatment, while other properties, typically immunogenicity, are maintained. Toxoids are used in vaccines as they induce an immune response to the original toxin or increase the response to another antigen. For example, the tetanus toxoid is derived from the tetanospasmin produced by *Clostridium tetani* and causing tetanus. The tetanus toxoid is used by many plasma centers in the United States for the development of plasma rich vaccines.

As used herein, the term "DNA vaccine" refers to a DNA construct that is introduced into cells and subsequently translated into specific antigenic proteins.

As used herein, the term "plasmid" refers to an extrachromosomal circular DNA capable of replicating, which may be used as a cloning vector.

As used herein, the term "microorganism" and "microbe" refers to an organism that is microscopic (too small to be seen by the naked human eye). Microorganisms are incredibly diverse and include, but are not limited to, bacteria and fungi.

As used herein, the term "immunologic adjuvant" refers to a substance used in conjunction with an immunogen which enhances or modifies the immune response to the immunogen. In an exemplary implementation, the α-GalCer analogs of the present disclosure are used as immunologic adjuvants to modify or augment the effects of a vaccine by stimulating the immune system of a patient who is administered the vaccine to respond to the vaccine more vigorously.

As used herein, the term "alum adjuvant" refers to an aluminum salt with immune adjuvant activity. This agent adsorbs and precipitates protein antigens in solution; the resulting precipitate improves vaccine immunogenicity by facilitating the slow release of antigen from the vaccine depot formed at the site of inoculation.

As used herein, the term "anti-tumor immunotherapy active agent" refers to an α-GalCer analog of the present disclosure that inhibits, reduces and/or eliminates tumors.

As used herein, the term "granulocyte-macrophage colony-stimulating factor" (GM-CSF) refers to a cytokine which serves as a colony-stimulating factor that stimulates production of white blood cells, particularly granulocytes (neutrophils, basophils, and eosinophils), macrophages, and cells in the bone marrow that are precursors of platelets.

As used herein, the term "antigen specific" refers to a property of a cell population such that supply of a particular antigen, or a fragment of the antigen, results in specific cell proliferation.

As used herein, the term "Flow cytometry" or "FACS" means a technique for examining the physical and chemical properties of particles or cells suspended in a stream of fluid, through optical and electronic detection devices.

As used herein α-GalCer analogs or synthetic α-GalCer analogs, unless otherwise noted, refer to structure-based synthetic glycolipid analogs based on alpha-galactosyl ceramide.

Amino acid residues in peptides shall hereinafter be abbreviated as follows: Phenylalanine is Phe or F; Leucine is Leu or L; Isoleucine is IIe or I; Methionine is Met or M; Valine is Val or V; Serine is Ser or S; Proline is Pro or P; Threonine is Thr or T; Alanine is Ala or A; Tyrosine is Tyr or Y; Histidine is His or H; Giutamine is Gin or Q; Asparagine is Asn or N; Lysine is Lys or K; Aspartic Acid is Asp or D; Glutamic Acid is Glu or E; Cysteine is Cys or C; Tryptophan is Trp or W; Arginine is Arg or R; and Glycine is Gly or G. For further description of amino acids, please refer to Proteins: Structure and Molecular Properties by Creighton, T. E., W. H. Freeman & Co., New York 1983.

Mammalian and mycobacterial lipids are known to be presented by human CD1a, CD1b, CD1c, and CD1d. α-Galactosyl ceramide, a lipid found in the marine sponge Age/as mauritianus, has been the most extensively studied ligand for CD1d. It has been shown that *in vitro* stimulation of mouse spleen cells by α-GalCer led to the proliferation of NKTs and production of both IFN-γ and IL-4, a T_{H}1-type and T_{H}2-type response, respectively. Murine studies have shown that cells can be rapidly activated by immature dendritic cells (iDCs) bearing α-GalCer and that the activated iNKTs can in turn induce full maturation of DCs.

In one aspect, the present disclosure provides a series of novel lipid portions of the α-GalCer analogs are capable of binding with a binding-groove on a CD1 molecule to form CD1-analog complexes. The compound of the invention may be for use in a method in which these CD1-analog complexes are presented to CD1-restricted T cells (NKTs) by means of T cell receptor recognition, and are capable of TCR activation, T_{H}1 and T_{H}2 cytokine release, and NKT expansion. In an exemplary implementation, an α-GalCer analog of the present disclosure is designed such that it has a strong binding affinity with the bindinggroove on the CD1 molecule, correlating with a T_{H}1-biased immunogenic response. In another exemplary implementation, an α-GalCer analog of the present disclosure is designed such that it has a strong binding affinity with the binding-groove on the CD1 molecule, correlating with a T_{H}2-biased immunogenic response.

In another aspect of the present disclosure, the compound of the invention is for use in a method in which the α-GalCer analogs may be used as immunotherapies. In an exemplary implementation, the compound of the invention is for use in a method in which the α-GalCer analogs may be used for cancer immunotherapy. In an exemplary implementation, the compound of the invention is for use in a method in which the α-GalCer analogs may be used for adjuvant immunotherapy. In another exemplary implementation, the compound of the invention is for use In a method in which the α-GalCer analogs may be used for antimicrobial immunotherapy, which includes vaccination. In still another exemplary implementation, the compound of the invention is for use in a method in which the α-GalCer analogs may be used for immunosuppression for the treatment of autoimmune diseases.

According to another aspect, the compositions disclosed herein can be included in a pharmaceutical or nutraceutical composition together with additional active agents, carriers, vehicles, excipients, or auxiliary agents identifiable by a person skilled in the art upon reading of the present disclosure.

The pharmaceutical or nutraceutical compositions preferably comprise at least one pharmaceutically acceptable carrier. In such pharmaceutical compositions, the compositions disclosed herein form the "active compound," also referred to as the "active agent." As used herein the language "pharmaceutically acceptable carrier" includes solvents, dispersion media, coatings, antibacterial and antifungal agents, isotonic and absorption delaying agents, and the like, compatible with pharmaceutical administration. Supplementary active compounds can also be incorporated into the compositions. A pharmaceutical composition is formulated to be compatible with its intended route of administration. Examples of routes of administration include parenteral, e.g., intravenous, intradermal, subcutaneous, oral (e.g., inhalation), transdermal (topical), transmucosal, and rectal administration. Solutions or suspensions used for parenteral, intradermal, or subcutaneous application can include the following components: a sterile diluent such as water for injection, saline solution, fixed oils, polyethylene glycols, glycerine, propylene glycol, or other synthetic solvents; antibacterial agents such as benzyl alcohol or methyl parabens; antioxidants such as ascorbic acid or sodium bisulfite; chelating agents such as ethylenediaminetetraacetic acid; buffers such as acetates, citrates, or phosphates and agents for the adjustment of tonicity such as sodium chloride or dextrose. pH can be adjusted with acids or bases, such as hydrochloric acid or sodium hydroxide. The parenteral preparation can be enclosed in ampoules, disposable syringes, or multiple dose vials made of glass or plastic.

Subject as used herein refers to humans and non-human primates (e.g., guerilla, macaque, marmoset), livestock animals (e.g., sheep, cow, horse, donkey, and pig), companion animals (e.g., dog, cat), laboratory test animals (e.g., mouse, rabbit, rat, guinea pig, hamster), captive wild animals (e.g., fox, deer), and any other organisms who can benefit from the agents of the present disclosure. There is no limitation on the type of animal that could benefit from the presently described agents. A subject regardless of whether it is a human or non-human organism may be referred to as a patient, individual, animal, host, or recipient.

Pharmaceutical compositions suitable for an injectable use include sterile aqueous solutions (where water soluble) or dispersions and sterile powders for the extemporaneous preparation of sterile injectable solutions or dispersion. For intravenous administration, suitable carriers include physiological saline, bacteriostatic water, Cremophor EL.TM. (BASF, Parsippany, N.J.), or phosphate buffered saline (PBS). In all cases, the composition should be sterile and should be fluid to the extent that easy syringability exists. It should be stable under the conditions of manufacture and storage and be preserved against the contaminating action of microorganisms such as bacteria and fungi. The carrier can be a solvent or dispersion medium containing, for example, water, ethanol, polyol (for example, glycerol, propylene glycol, and liquid polyetheylene glycol, and the like), and suitable mixtures thereof. The proper fluidity can be maintained, for example, by the use of a coating such as lecithin, by the maintenance of the required particle size in the case of dispersion and by the use of surfactants. Prevention of the action of microorganisms can be achieved by various antibacterial and antifungal agents, for example, parabens, chlorobutanol, phenol, ascorbic acid, thimerosal, and the like. In many cases, it will be preferable to include isotonic agents, for example, sugars, polyalcohols such as manitol, sorbitol, or sodium chloride in the composition. Prolonged absorption of the injectable compositions can be brought about by including in the composition an agent which delays absorption, for example, aluminum monostearate and gelatin.

Sterile injectable solutions can be prepared by incorporating the active compound in the required amount in an appropriate solvent with one or a combination of ingredients enumerated above, as required, followed by filtered sterilization. Generally, dispersions are prepared by incorporating the active compound into a sterile vehicle which contains a basic dispersion medium and the required other ingredients from those enumerated above. In the case of sterile powders for the preparation of sterile injectable solutions, methods of preparation include vacuum drying and freeze-drying, which yields a powder of the active ingredient plus any additional desired ingredient from a previously sterile-filtered solution thereof.

Oral compositions generally include an inert diluent or an edible carrier. For the purpose of oral therapeutic administration, the active compound can be incorporated with excipients and used in the form of tablets, troches, or capsules, e.g., gelatin capsules. Oral compositions can also be prepared using a fluid carrier for use as a mouthwash. Pharmaceutically compatible binding agents, or adjuvant materials can be included as part of the composition. The tablets, pills, capsules, troches and the like can contain any of the following ingredients, or compounds of a similar nature: a binder such as microcrystalline cellulose, gum tragacanth or gelatin; an excipient such as starch or lactose, a disintegrating agent such as alginic acid, Primogel, or corn starch; a lubricant such as magnesium stearate or Sterotes; a glidant such as colloidal silicon dioxide; a sweetening agent such as sucrose or saccharin; or a flavoring agent such as peppermint, methyl salicylate, or orange flavoring.

For administration by inhalation, the compounds are delivered in the form of an aerosol spray from pressured container or dispenser which contains a suitable propellant, e.g., a gas such as carbon dioxide, or a nebulizer.

Systemic administration can also be transmucosal or transdermal. For transmucosal or transdermal administration, penetrants appropriate to the barrier to be permeated are used in the formulation. Such penetrants are generally known in the art, and include, for example, for transmucosal administration, detergents, bile salts, and fusidic acid derivatives. Transmucosal administration may be accomplished through the use of nasal sprays or suppositories. For transdermal administration, the active compounds are formulated into ointments, salves, gels, or creams as generally known in the art. The compounds can also be prepared in the form of suppositories (e.g., with conventional suppository bases such as cocoa butter and other glycerides) or retention enemas for rectal delivery.

According to implementations, the active compounds are prepared with carriers that will protect the compound against rapid elimination from the body, such as a controlled release formulation, including implants and microencapsulated delivery systems. Biodegradable, biocompatible polymers can be used, such as ethylene vinyl acetate, polyanhydrides, polyglycolic acid, collagen, polyorthoesters, and polylactic acid. Methods for preparation of such formulations will be apparent to those skilled in the art. The materials can also be obtained commercially from Aiza Corporation and Nova Pharmaceuticals, Inc. Liposomal suspensions (including liposomes targeted to infected cells with monoclonal antibodies to cell-specific antigens) can also be used as pharmaceutically acceptable carriers. These can be prepared according to methods known to those skilled in the art, for example, as described in U.S. Pat. No. 4,522,811, which is incorporated by reference herein.

It is advantageous to formulate oral or parenteral compositions In dosage unit form for ease of administration and uniformity of dosage. Dosage unit form as used herein refers to physically discrete units suited as unitary dosages for the subject to be treated; each unit containing a predetermined quantity of active compound calculated to produce the desired therapeutic effect in association with the required pharmaceutical carrier.

Toxicity and therapeutic efficacy of such compounds may be determined by standard pharmaceutical procedures in cell cultures or experimental animals, e.g., for determining the LD50 (the dose lethal to 50% of the population) and the ED50 (the dose therapeutically effective in 50% of the population). The dose ratio between toxic and therapeutic effects is the therapeutic index and it can be expressed as the ratio LD50/ED50. Compounds which exhibit high therapeutic indices are preferred. While compounds that exhibit toxic side effects can be used, care should be taken to design a delivery system that targets such compounds to the site of affected location to minimize potential damage to uninfected cells and, thereby, reduce side effects.

The data obtained from the cell culture assays and animal studies can be used in formulating a range of dosage for use in humans. The dosage of such compounds lies preferably within a range of circulating concentrations that include the ED50 with little or no toxicity. The dosage can vary within this range depending upon the dosage form employed and the route of administration utilized. For any compound used in the method described herein, the therapeutically effective dose can be estimated initially from cell culture assays. A dose can be formulated in animal models to achieve a circulating plasma concentration range that includes the IC50 (i.e., the concentration of the test compound which achieves a half-maximal inhibition of symptoms) as determined in cell culture. Such information can be used to more accurately determine useful doses in humans. Levels in plasma may be measured, for example, by high performance liquid chromatography.

As defined herein, a therapeutically effective amount of the active compound (i.e., an effective dosage) may range from about 0.001 to 100 g/kg body weight, or other ranges that would be apparent and understood by artisans without undue experimentation. The skilled artisan will appreciate that certain factors can influence the dosage and timing required to effectively treat a subject, including but not limited to the severity of the disease or disorder, previous treatments, the general health or age of the subject, and other diseases present.

As described herein, one or more kits of parts can be envisioned by the person skilled in the art, the kits of parts to perform at least one of the methods described herein, the kit of parts comprising two or more compositions, the compositions comprising alone or in combination an effective amount of the compositions disclosed herein according to the at least one of the above mentioned methods.

The kits possibly include also compositions comprising active agents, identifiers of a biological event, or other compounds identifiable by a person skilled upon reading of the present disclosure. The term "identifier" refers to a molecule, metabolite or other compound, such as antibodies, DNA or RNA oligonucleotides, able to discover or determine the existence, presence, or fact of or otherwise detect a biological event under procedures identifiable by a person skilled in the art; exemplary identifiers are antibodies, exemplary procedures are western blot, nitrite assay and RT-PCR, or other procedures as described in the Examples.

The kit can also comprise at least one composition comprising an effective amount of the compositions disclosed herein or a cell line. The compositions and the cell line of the kits of parts to be used to perform the at least one method described herein according to procedure identifiable by a person skilled in the art.

### T CELL RECEPTOR PRECOGNITION AND ACTIVATION VIA THE α-GalCer ANALOGS AND THE RESULTANT IMMUNE RESPONSE

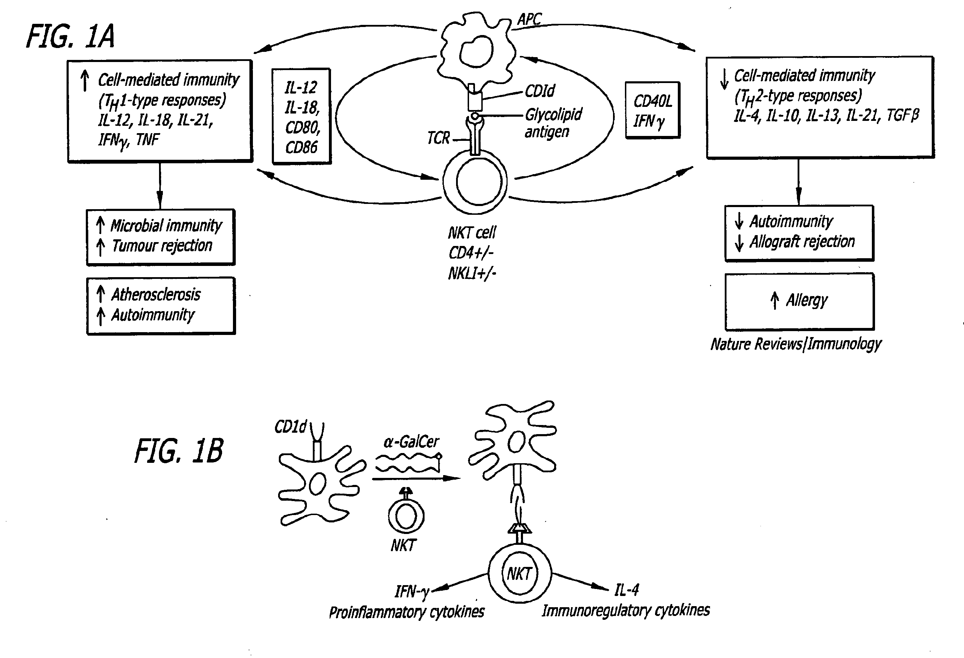

Figure 1A is a schematic illustration showing how invariant NKT cell recognition of glycolipid antigens presented by CD1d leads to a cascade of events. The lipid portions of the glycolipid antigens become inserted into a hydrophobic binding groove of the CD1 molecule to form CD1-antigen complexes, which are able to contact T-cell receptors (TCRs) on the NKTs, which leads to the cascade of events involving cytokines, chemokines and co-stimulatory molecules. The diversity and extent of cytokine production can have a broad range of effects, ranging from enhanced cell-mediated immunity (T_{H}1-type responses) to suppressed cell-mediated immunity (T_{H}2-type responses). Figure 1B is a schematic illustration showing how NKT cell recognition of α-GalCer or an α-GalCer analog of the present disclosure presented by CD1d stimulates a rapid T_{H}1 and T_{H}2 cytokine response. In an exemplary implementation, a T_{H}1 cytokine response is initiated. In another exemplary implementation, a T_{H}2 cytokine response is initiated. In yet another exemplary implementation, both a T_{H}1 and T_{H}2 cytokine response is initiated.

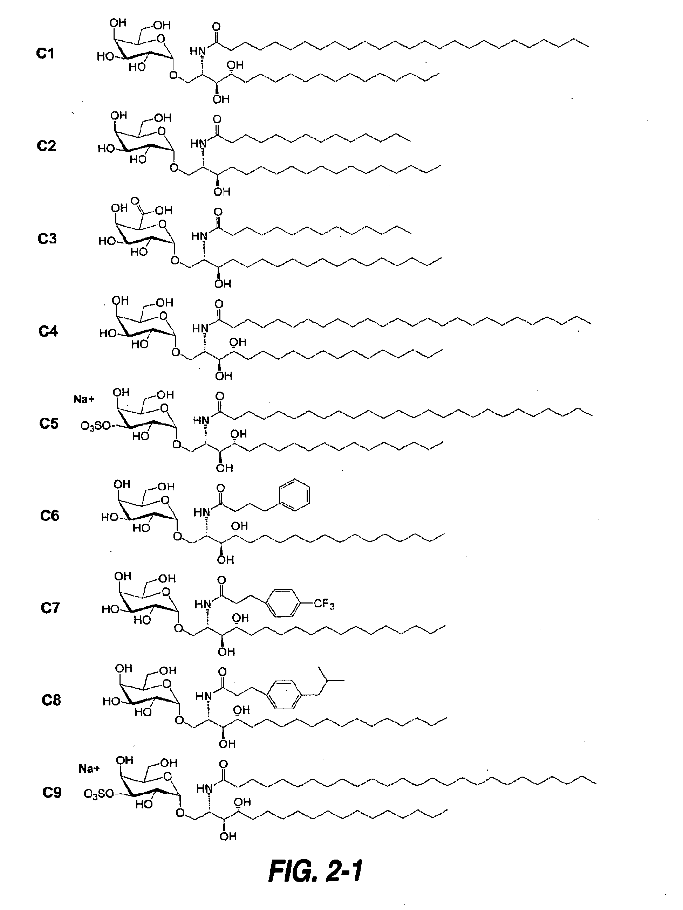

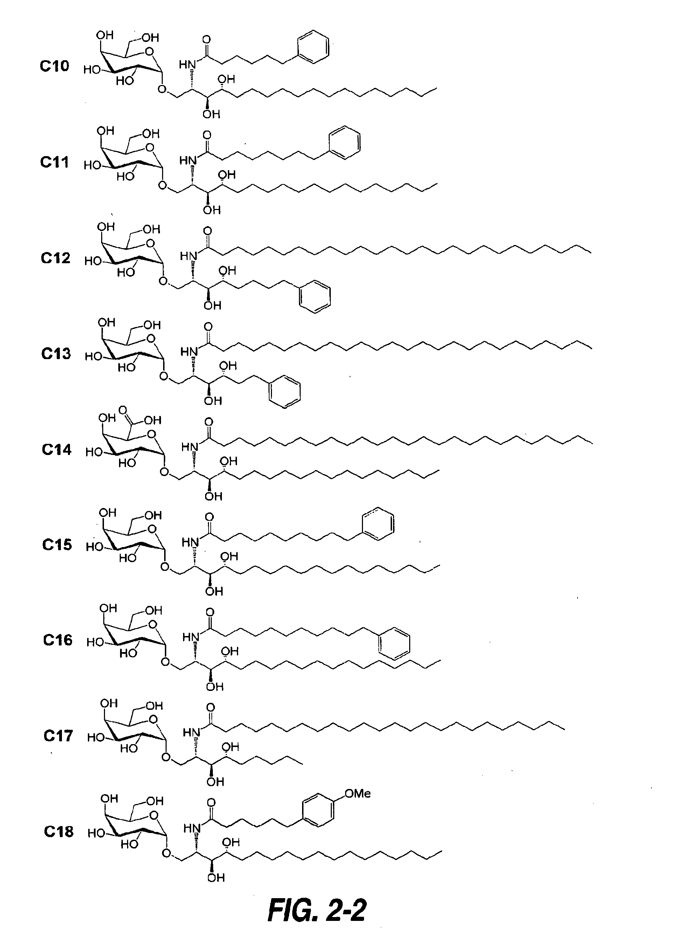

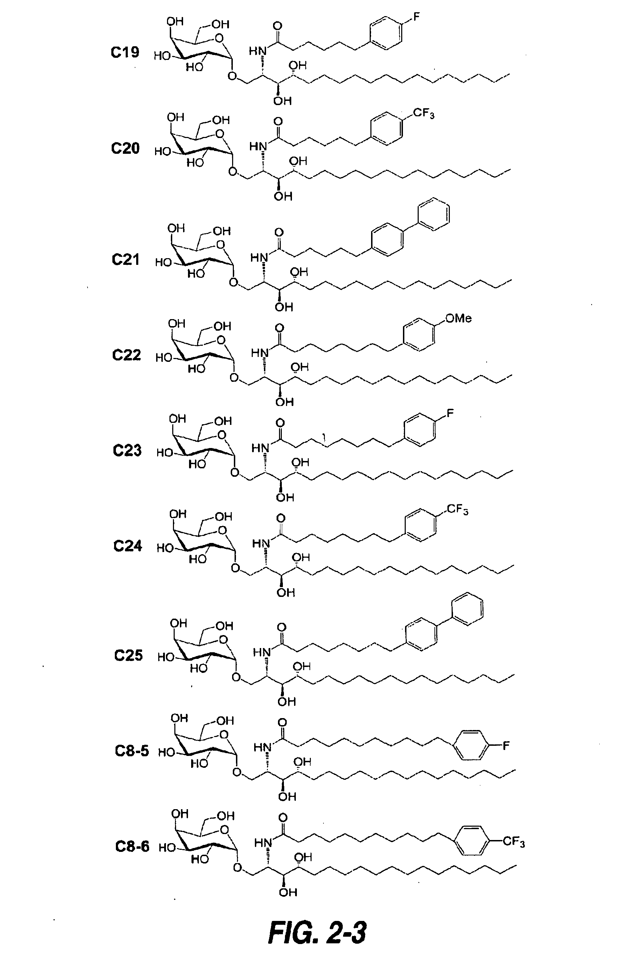

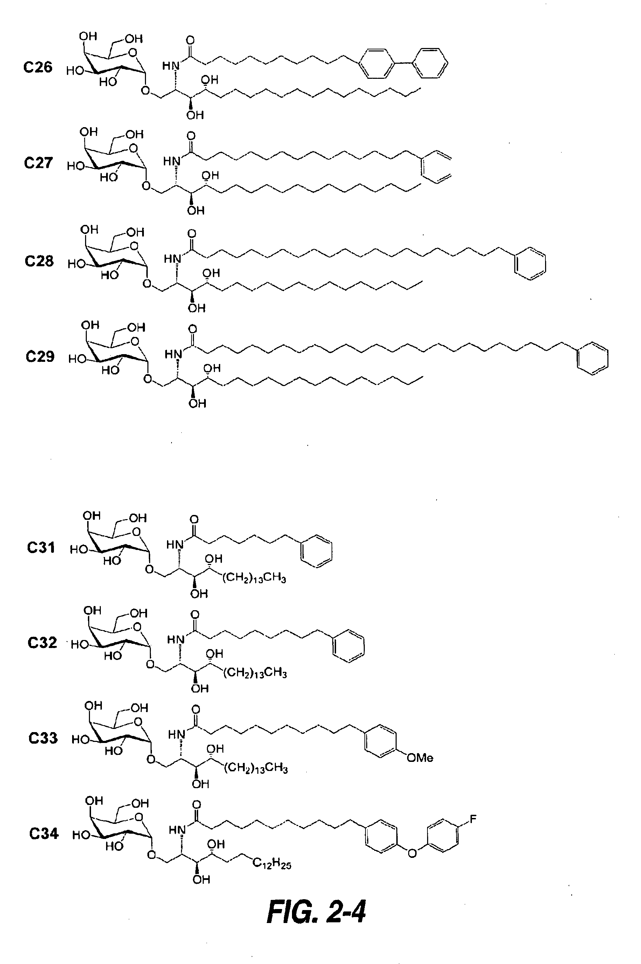

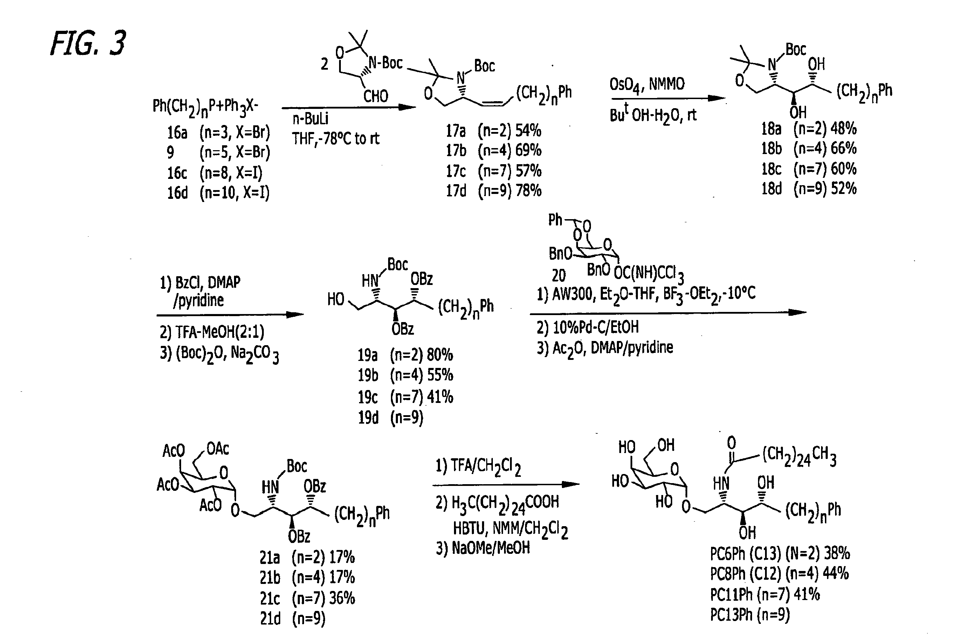

The chemical structures of α-GalCer, as well as the synthetic α-GalCer analogs are shown in Figure 2. The α-GalCer analogs include α-GalCer analogs of bacterial origin (Group I: C2, C3 and C14), α-GalCer analogs modified with sulfonation (Group II: C4, C5 and C9), phenyl-alkyl chain α-GalCer analogs (Group III: C6-C8, C10-11, C15-C16, C18-C34 (C34 being of the present invention), C8-5 and C8-6) and phytosphingosine truncated α-GalCer analogs (Group IV: C12, C13 and C17). Figure 3 shows an example of the synthesis of glycosphingolipid α-GalCer analogs C12 and C13.

In one aspect, the synthetic α-GalCer analogs are capable of forming complexes with a CD1d molecule. In another aspect, the synthetic α-GalCer analogs are capable of being recognized by NKTs T-cell receptors. In yet another aspect, the synthetic α-GalCer analogs are capable of eliciting a T_{H}1-type, a T_{H}2-type or a T_{H}1-type and a T_{H}2-type response. In an exemplary implementation, the α-GalCer analogs are capable of activating NKTs in vitro. In another exemplary implementation, the α-GalCer analogs are capable of activating NKTs *in vivo.*

Described herein is a method for stimulating or enhancing cytokine production in tissue, cells and/or in a subject, the method including: administering to the subject any one of the synthetic α-GalCer analogs, wherein a NKT in the subject is activated following contact with the α-GalCer analog and a cytokine response is initiated. The cytokine may be, for example, interferon-γ (IFN-g) or interleukin-4 (IL-4).

Also described herein is a method of activating a cytokine response in tissue, cells and/or a subject whereby an effective amount of a compound or a salt or a mixture is administered, the compound is selected from the group consisting of C2-C8, C8-5, C8-6 and C9-C34 (C34 being of the present invention), and wherein the subject has an adaptive immune system that includes a population of cells, the population including at least one lymphocyte and at least one antigen-presenting cell; forming a complex between the compound and the antigen-presenting cell, wherein the formation of the complex results in the activation of a receptor on the lymphocyte; and activating the lymphocyte to produce the cytokine response.

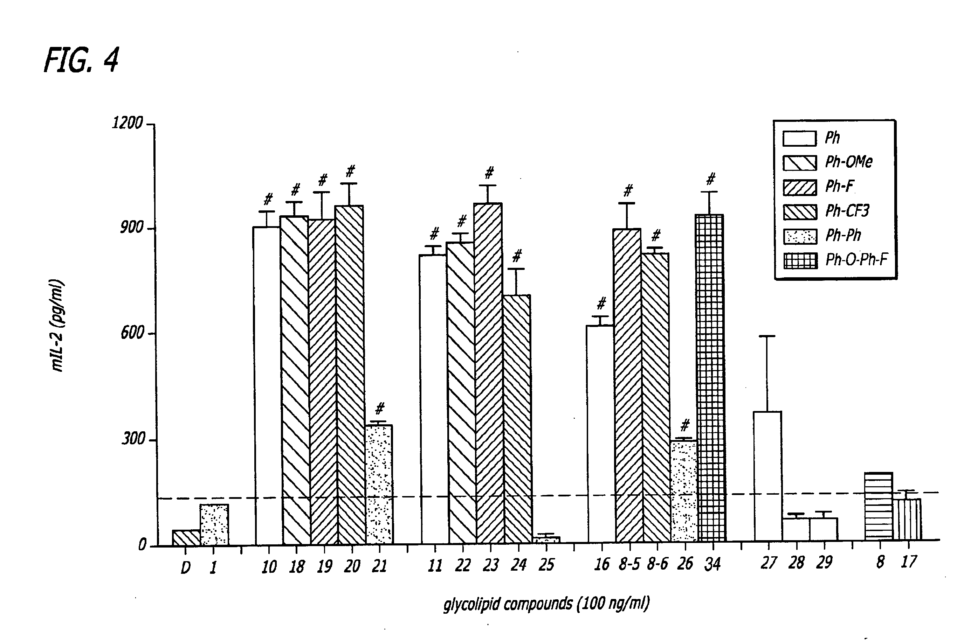

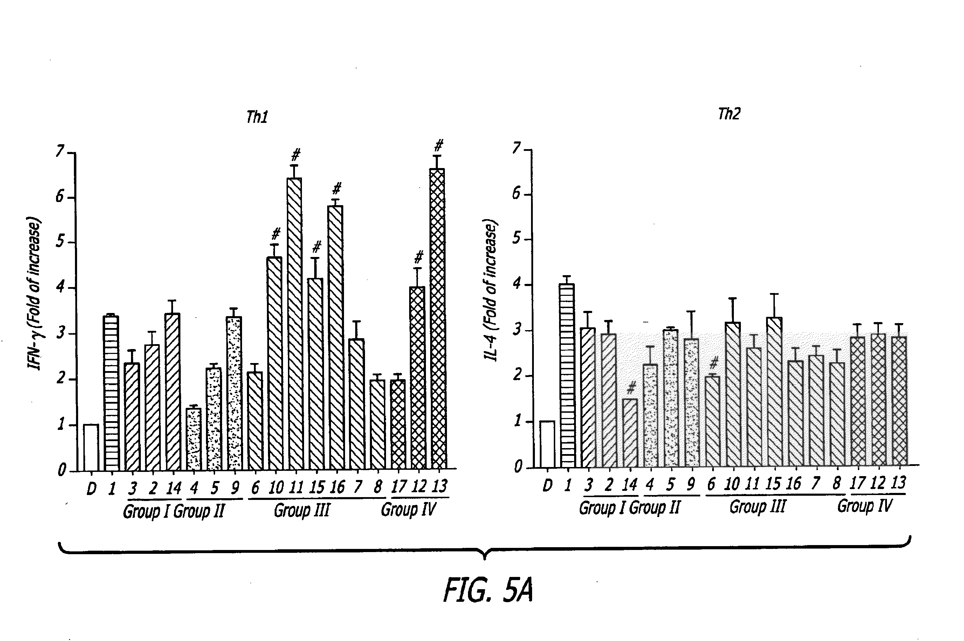

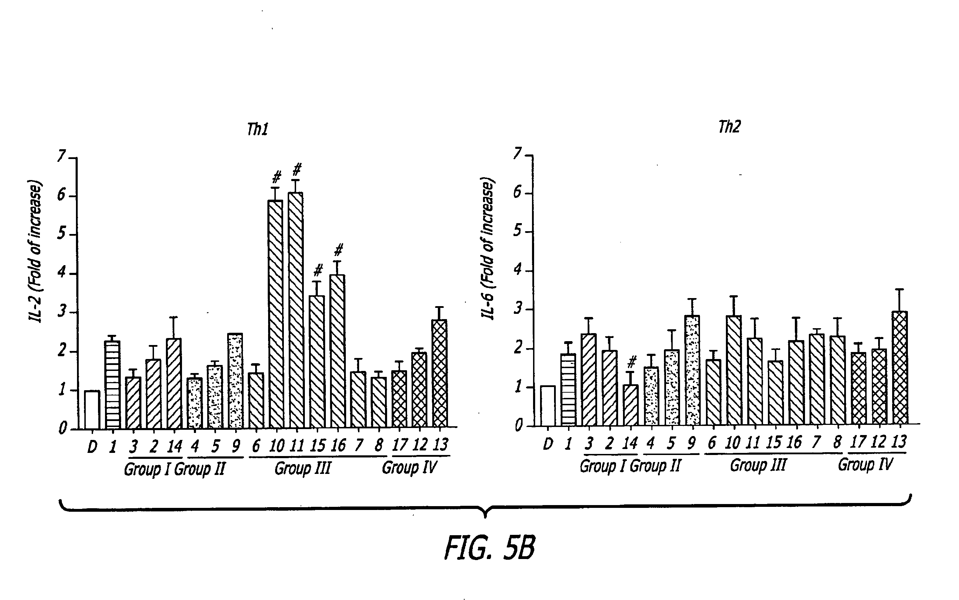

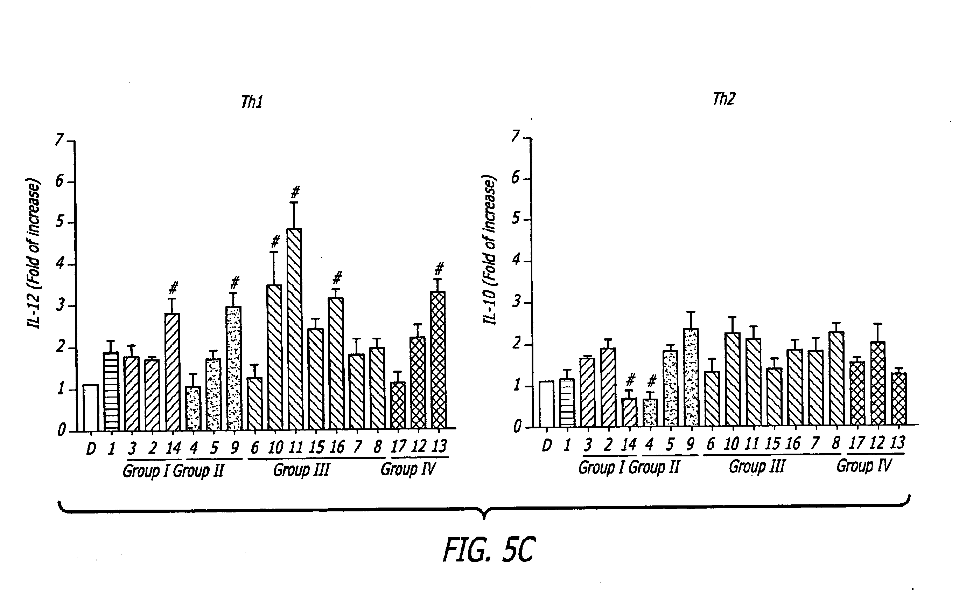

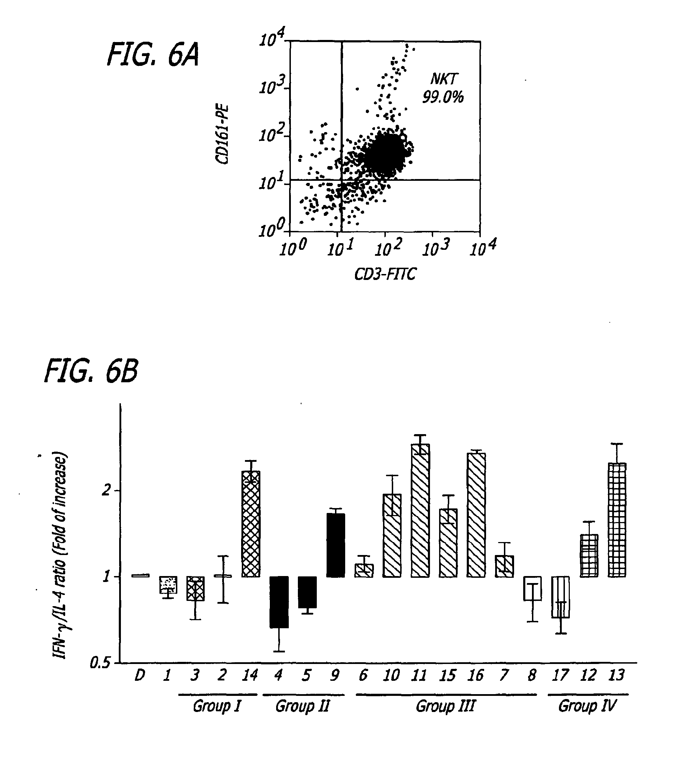

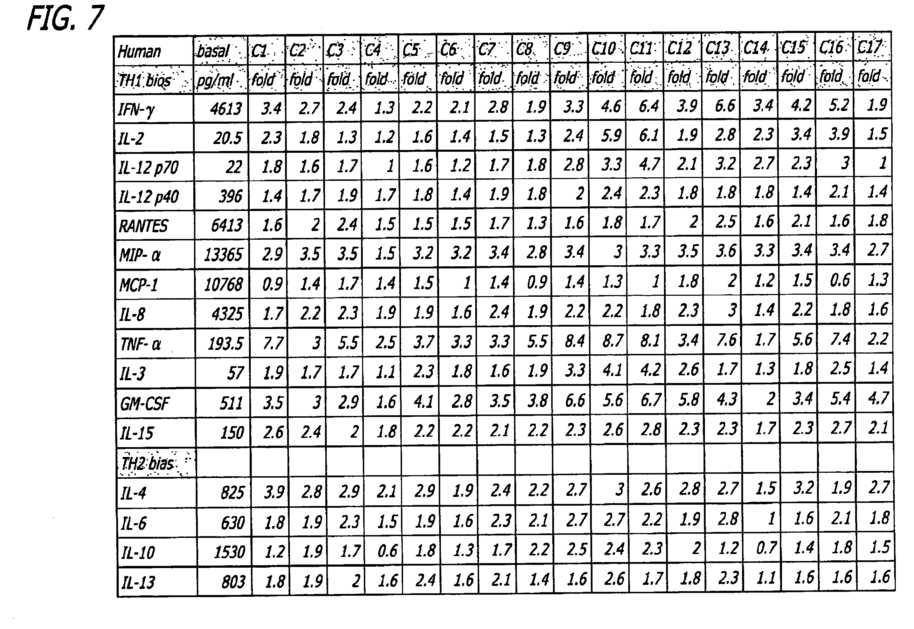

As described herein, murine 1.2 hybridomas (CD1d-reactive Vα14/T cell hybridomas) were cultured in mCD1d-coated 96 well plate and pulsed with control DMSO, α-GalCer (C1) or the indicated α-GalCer analogs at 100 ng/ml. IL-2 release into the tissue culture medium was measured after an 18 hour culture, as seen in Figure 4. Most of the α-GalCer analogs induced greater IL-2 production than α-GaiCer. When the α-GalCer analogs were examined for their capacity to elicit cytokine/chemokine production in human naïve NKTs (CD161⁺CD3⁺) *in vitro,* similar results were found. Human naïve CD161⁺CD3⁺ NKTs were cultured with autologous immature dendritic cells (CD14⁺ DCs) and pulsed with control DMSO, α-GalCer or the indicated α-GalCer analogs at 10 µg/ml. Cytokines released into the tissue culture medium was measured after an 18 hour culture, as seen in Figure 5. The α-GaiCer analogs were potent inducers of T_{H}1 and T_{H}2 cytokine secretion. Figure 5A shows induction of IFN-γ and IL-4, Figure 5B shows induction of IL-2 and IL-6 and Figure 50 shows induction of IL-12 and IL-10. Aromatic compounds from Group III and IV, especially C11, C16 and C13, induced a significantly greater secretion of IFN-γ than α-GalCer, whereas, all α-GalCer analogs elicited slightly less IL-4 than α-GalCer. Figure 6 shows the purity of human CD161⁺CD3⁺ NKTs (top) and the ratio of IFN-γ/IL-4, normalized to DMSO control (bottom). When expressed as IFN/IL-4 ratio, C9, C12, C13, C14 and all Group III compounds were more T_{H}1-biased; whereas C1, C3, C4, C5, C8 and C17 were more T_{H}2-biased. The induction of the cytokines and chemokines from the human CD161⁺CD3⁺ NKTs are listed in Figure 7. The top five values for each cytokine are marked in bold. Some of the α-GalCer analogs tested showed a greater induction In chemokines than did α-GalCer, for example, C13 elicited a striking increase in chemokines such as MIP-1a, MCP-1, and IL-8. Aromatic compounds C10, C11, and C16 displayed a greater induction of IL-3, granulocyte/macrophage colony-stimulating factor (GM-CSF), and IL-15.

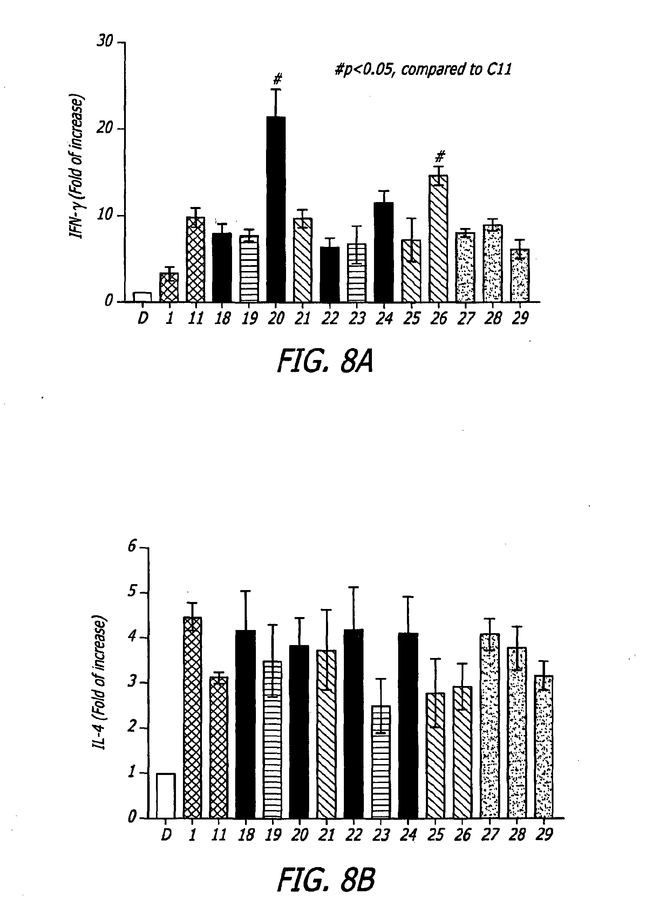

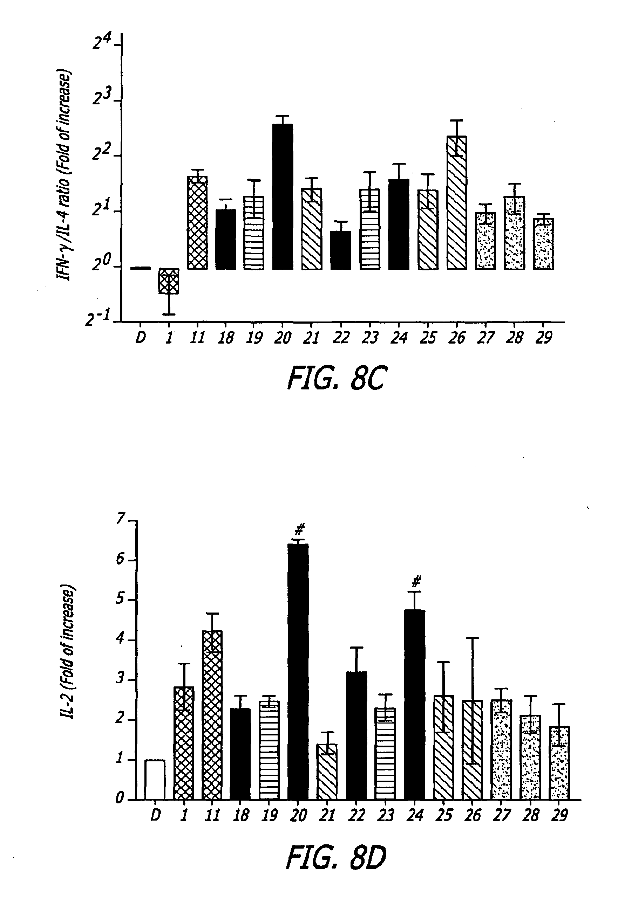

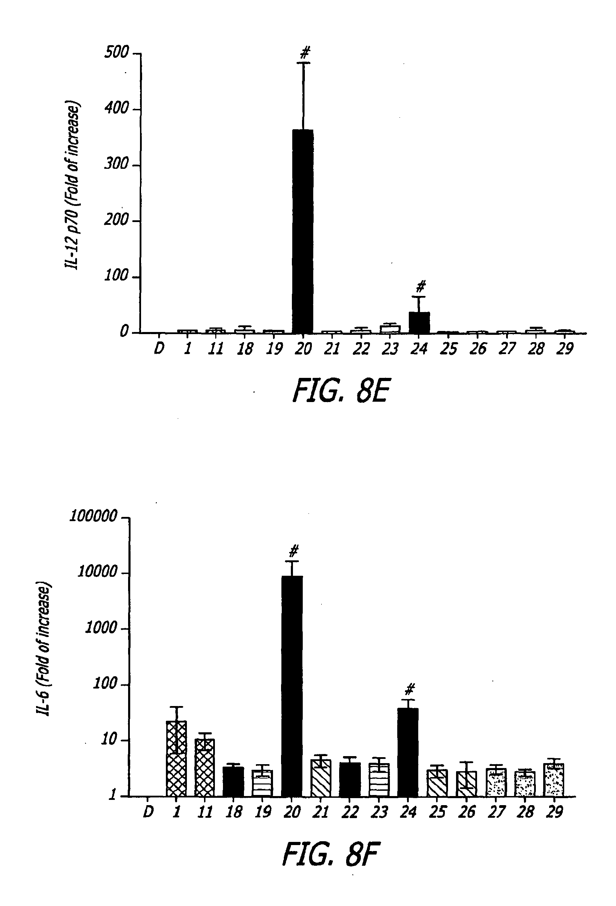

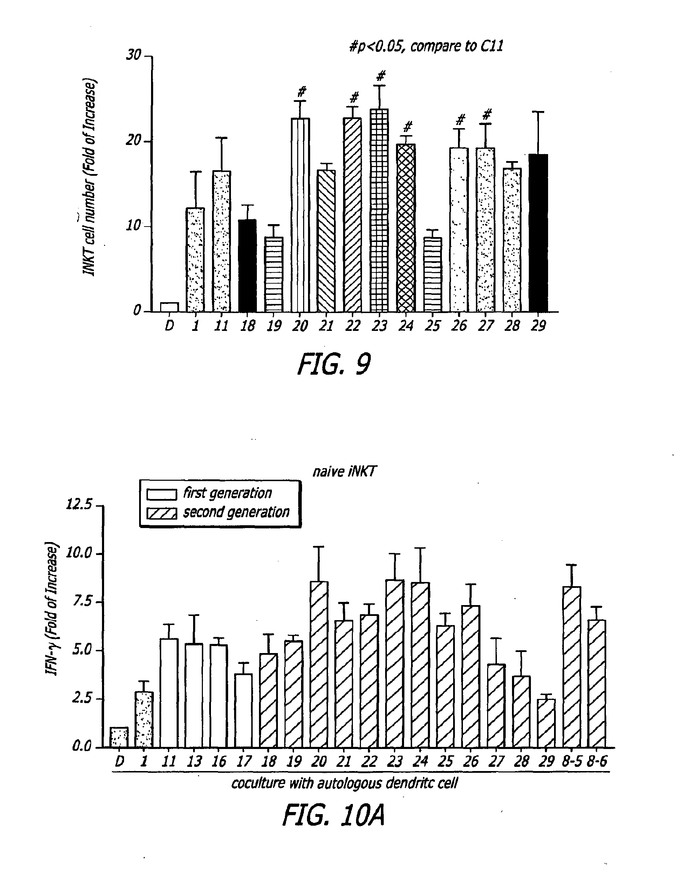

Figure 8 shows more in vitro results for the capacity of the α-GalCer analogs to elicit cytokine/chemokine production in primary naïve human *i*NKTs. Primary naïve human *i*NKTs were cultured with autologous immature DCs and pulsed with control DMSO, α-GalCer or the indicated α-GalCer analogs (C11 and C18-C29). As shown in Figure 8A, all of the tested α-GalCer analogs induced higher levels of INF-γ secretion than C1. α-GalCer analogs induced comparable levels of IL-4 (see Figure 8B). α-GalCer analogs induced higher IFN-γ/L4 ratios, i.e., the T_{H}1/T_{H}2 bias than C1 (See Figure 80). α-GalCer analogs C20, C24 and C26 were significantly more potent in eliciting IFN-γ production, higher IFN-γ /IL4 ratio, and higher levels of IL-2 (See Figure 8D) than α-GalCer analog C11. α-GalCer analogs C20 and C24 induced IL-12 production and also elicited more IL-6 release than the other α-GalCer analogs tested (see Figures 8E and 8F). Figure 9 shows the expansion of human *i*NKTs by α-GalCer analogs C11 and C18-C29. α-GalCer analogs C20, C22-C24 and C26-C27 induced significant greater expansion of CD1d-restricted human *i*NKT cells than C1 and C11.

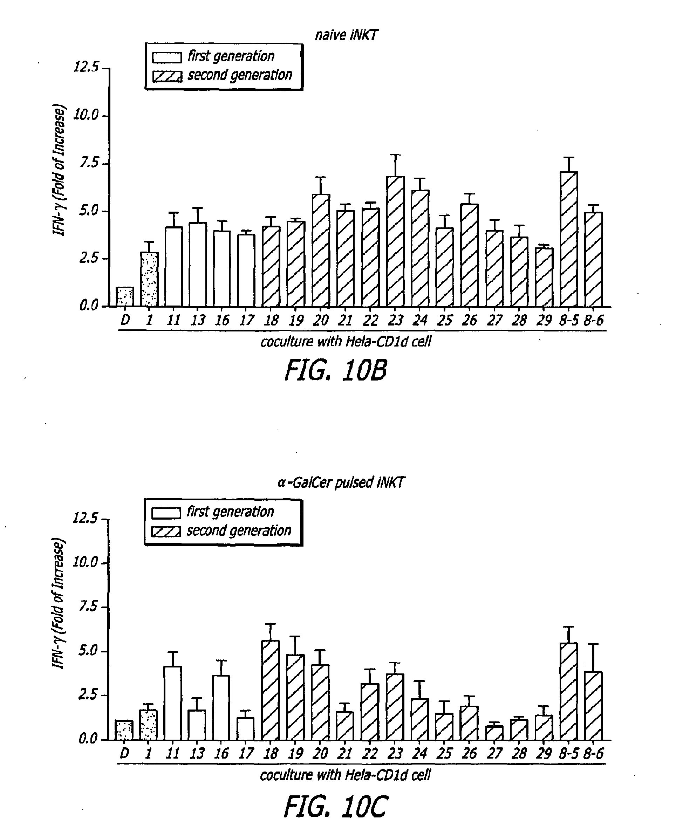

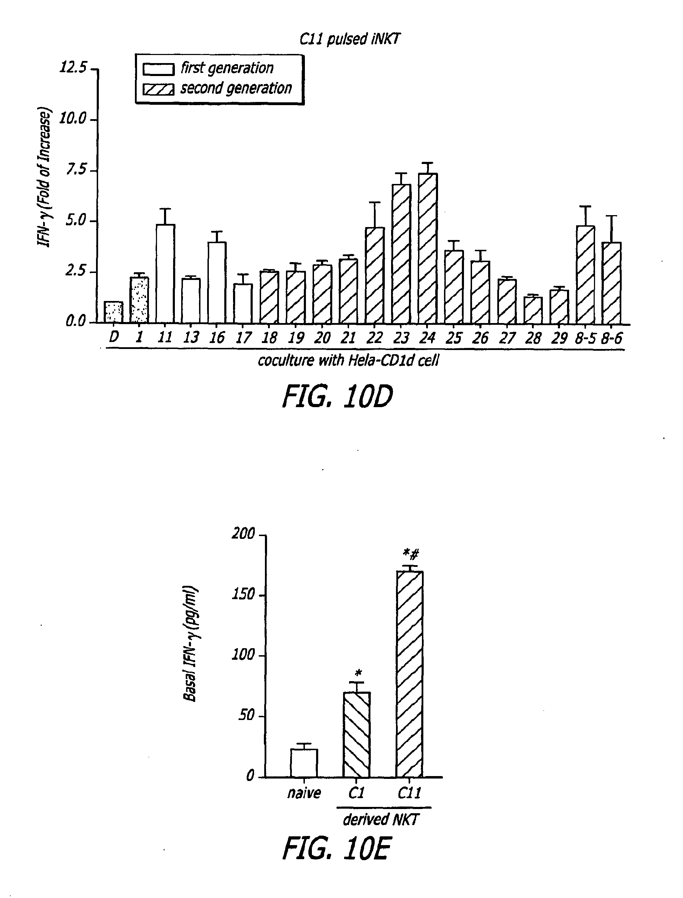

Figure 10 shows different IFN-γ secretion levels between naïve and various α-GalCer analog-pulsed human NKTs. Figure 10A shows the IFN-γ secretion from human naïve *i*NKTs (Vα24⁺) cultured with immature CD14⁺ DCs, and pulsed with control DMSO, α-GalCer or the indicated α-GalCer analogs. Figure 10B-D show IFN-γ secretion in response to the α-GalCer analogs in three different sources of *i*NKTs: (B) Human naïve *i*NKTs, (C) α-GalCer pulsed *i*NKTs and (D) C11 pulsed *i*NKTs. The *i*NKTs were cultured with HeLa-CD1d cells, and pulsed with control DMSO, α-GalCer or the indicated α-GalCer analogs for 18 hours. Figure 10E shows different basal levels of IFN-γ in human naïve iNKTs, α-GalCer pulsed iNKTs and C11 pulsed iNKTs.

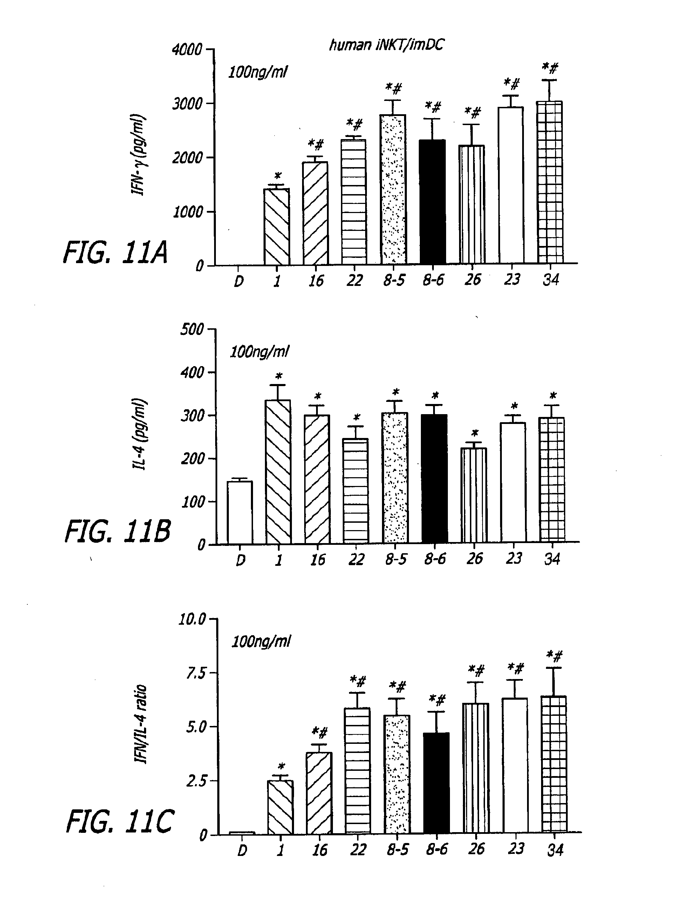

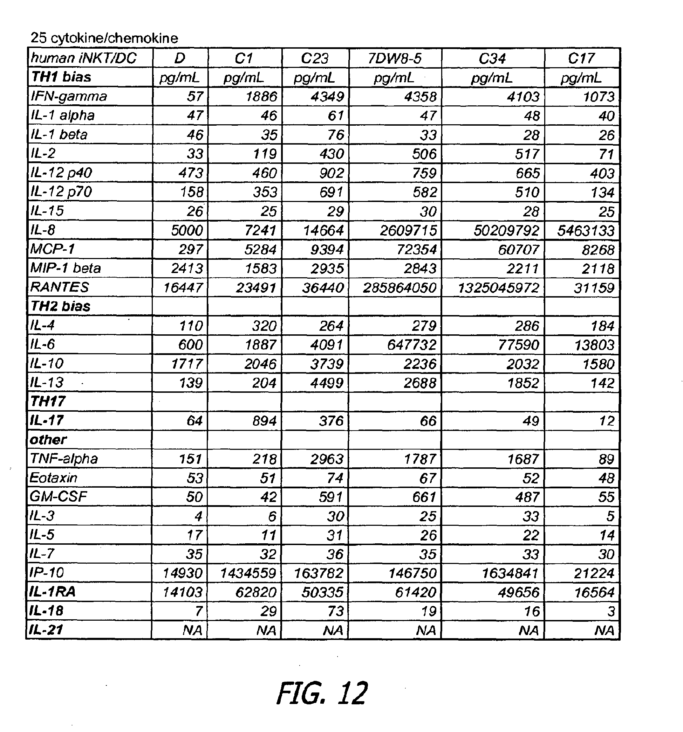

Figure 11 shows T_{H}1/T_{H}2 cytokine production by invariant human naive NKTs in response to the α-GalCer analogs. Human Vα24+ *i*NKTs were cultured with autologous immature CD14+ DCs pulsed with control DMSO, α-GalCer or the indicated α-GalCer analogs for 18 hours. Figure 11 (A) shows the induction of IFN-γ, (B) shows the induction of IL-4 and (C) shows the ratio of IFN-γ over IL-4, normalized to DMSO control. The induction of cytokines and chemokines from the naive human Vα24+*i*NKTs are listed in Figure 12.

### EXPANSION AND ACTIVATION OF NKTs USING α-GalCer ANALOGS

In one aspect, the compound of the invention is for use in a method in which the synthetic α-GalCer analogs are capable of expanding and activating NKs and *i*NKTs. Because decreased numbers of *i*NKTs in human peripheral blood mononuclear cells has been documented in patients with malignancies, expansion and activation of such patients' *i*NKTs with the α-GalCer analogs may be therapeutically beneficial. In an exemplary implementation, the α- GalCer analogs of are capable of expanding human iNKTs *in vitro.*

A method is described for producing an isolated, culture-expanded NKT population, comprising contacting Vα14*i* or Vα24*i*T cells with dendritic cells and an α-GalCer analog, for a period of time resulting In analog-specific T cell expansion and isolating the expanded T cells thus obtained, thereby producing an isolated, culture-expanded NKT population. The method for producing an isolated culture-expanded NKT population may further comprise the step of adding a cytokine or growth factor to the dendritic cell, NKT cell culture.

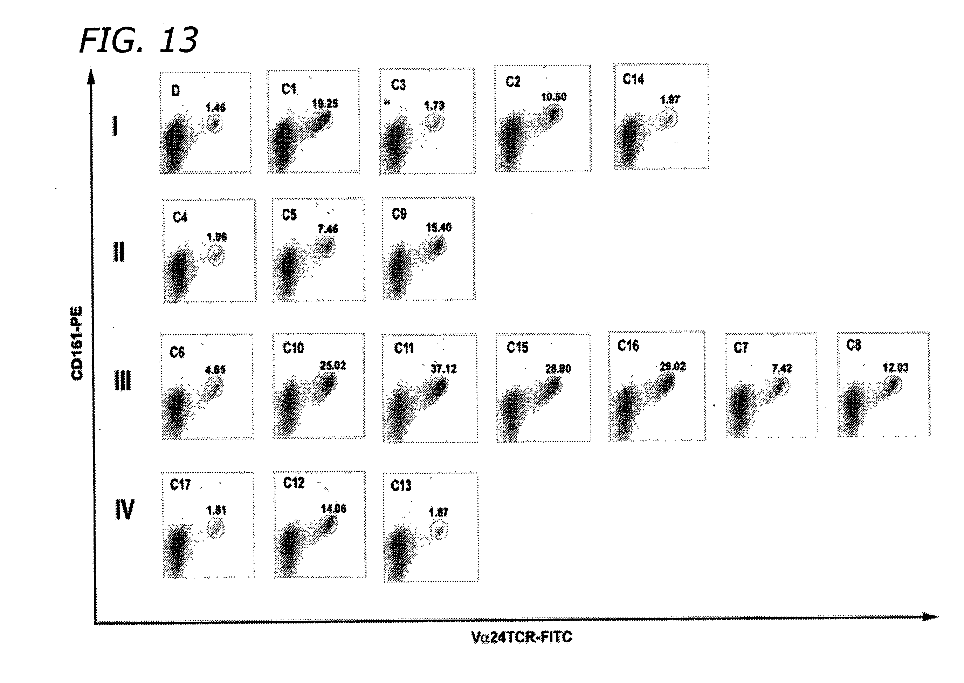

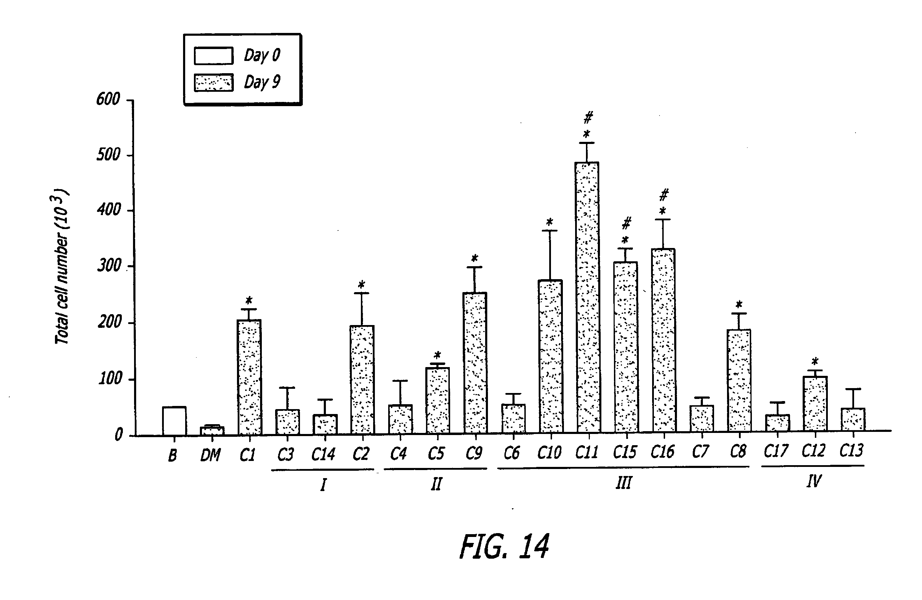

Human CD56+cells (NK/NKT cell mixtures) were cultured with autologous immature CD14+ DCs and pulsed with DMSO, α-GalCer or various α-GalCer analogs. On day 9 after exposure, the expansion/survival of NKs and NKTs and of a subpopulation of NKTs, *i*NKTs (CD161+*i*Vα24+/ CD56+/ CD3+), was determined by flow cytometry. As shown in Figures 13 and 14, a significant increase in *i*NKTs over control was noted upon stimulation with C2, C8-C12 and C15-C16. Among the α-GalCer analogs tested, several of the aromatic compounds from Group III, especially C11, C15 and C16, were more effective than C1.

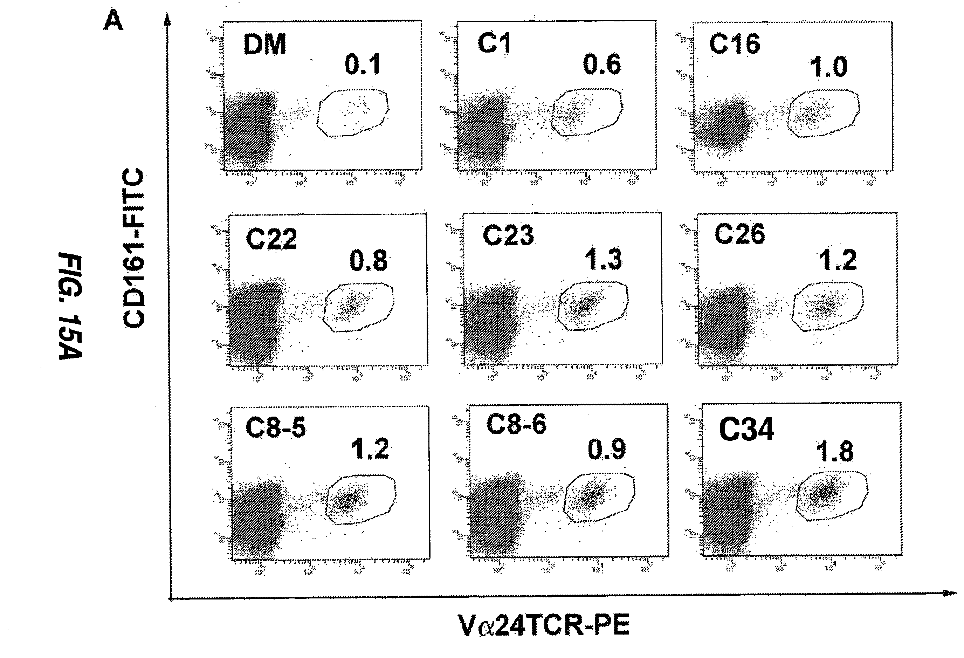

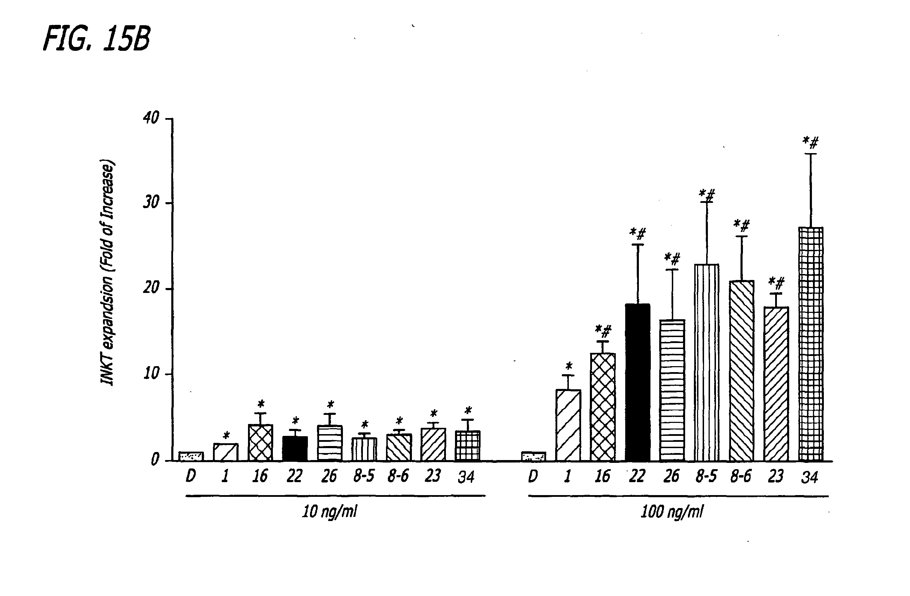

As shown in Figure 15, human CD56+ cells (NK/NKT mixtures) were cultured with autologous immature CD14+ DCs and pursed with DMSO, α-GalCer or various α-GalCer analogs at 10 or 100 ng/ml on day 2 for 18 hours. The percentage of CD161+ / Vα24 TCR +cells in the NK/NKT mixtures were gated by flow cytometry on day 9. Figure 15A shows the percentage of Vα24*i* NKTs in response to 100 ng/ml. Figure 15B shows the fold changes in total number of Vα24*i* NKTs in response to different doses.*, p <0.05, compared with DMSO; #, p < 0.05, compared with C1.

### MATURATION AND ELONGATION OF DENDRITIC CELLS USING a GalCer ANALOGS

The most efficient antigen-presenting cells (APCs) are mature, immunologically competent dendritic cells (DCs). DCs are capable of evolving from immature, antigen-capturing cells to mature, antigen-presenting, T cell-priming cells; converting antigens into immunogens and expressing molecules such as cytokines, chemokines, costimulatory molecules and proteases to initiate an immune response. The types of T cell-mediated immune responses (tolerance vs. immunity, T_{H}1 vs. T_{H}2) induced can vary, however, depending on the specific DC lineage and maturation stage in addition to the activation signals received from the surrounding microenvironment.

The ability of DCs to regulate immunity is dependent on DC maturation. Consequently, maturation of DCs is critical to the initiation of the immune response. A variety of factors can induce maturation following antigen uptake and processing within DCs. During their conversion from immature to mature cells, DCs undergo a number of phenotypical and functional changes. The process of DC maturation, in general, involves a redistribution of major histocompatibility complex (MHC) molecules from intracellular endocytic compartments to the DC surface, downregulation of antigen internalization, an increase in the surface expression of costimulatory molecules, morphological changes (e.g. formation of dendrites), cytoskeleton re-organization, secretion of chemokines, cytokines and proteases, and surface expression of adhesion molecules and chemokine receptors.

In one aspect, the compound of the invention is for use in a method In which the synthetic α-GalCer analogs are capable of promoting the maturation of human DCs. Dendritic cell maturation may lead to enhanced adaptive immune responses. A method is disclosed for the maturation of dendritic cells that includes: providing immature dendritic cells; and incubating the immature dendritic cells with a concentration of α-GalCer analogs for a period of time such that the immature dendritic cells become mature. In an exemplary implementation, the compound of the invention is for use in a method in which these mature denritic cells may then be used as immunotherapies, such as for example, cancer immunotherapies and adjuvant immunotherapies. In another exemplary implementation, the compound of the invention is for use in a method in which the α-GalCer analogs may be combined with immature denritic cells or mature denritic cells and then used as immunotherapies, such as for example, cancer immunotherapies and adjuvant immunotherapies.

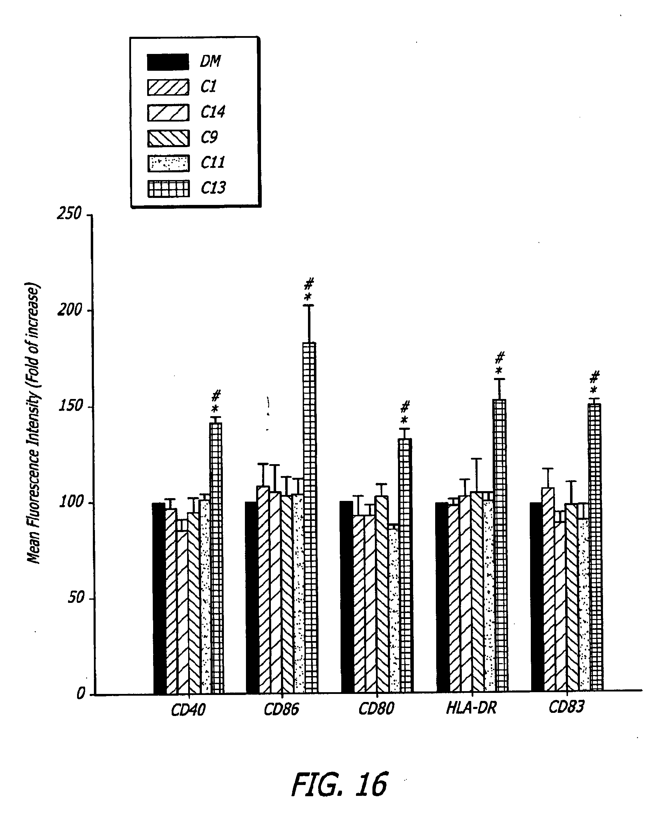

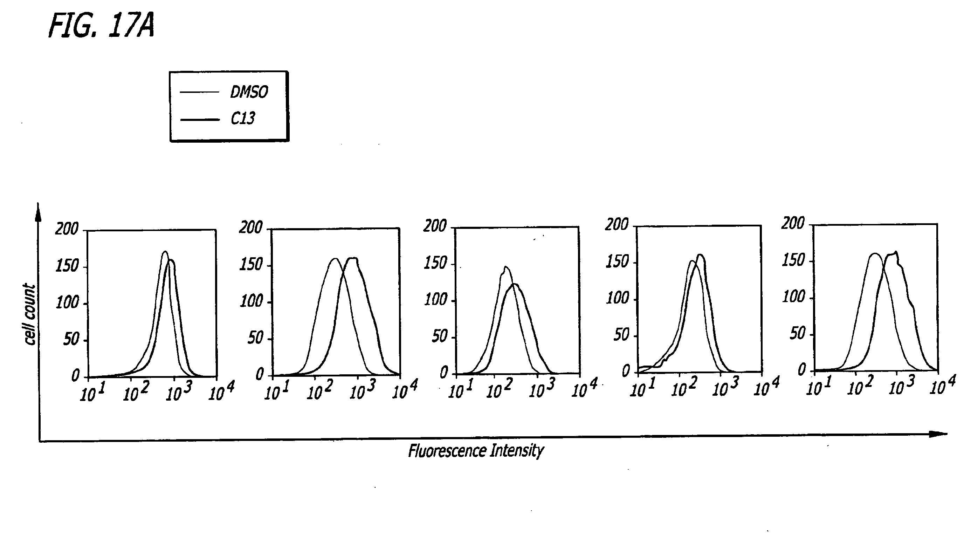

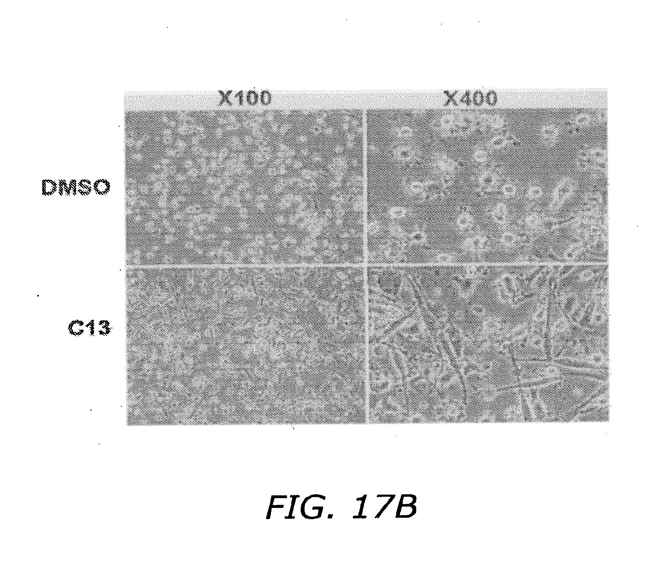

The α-GalCer analogs are capable of inducing mouse splenic DC maturation. In vitro, the α-GalCer analogs were able to directly augment the expression levels of various surface maturation markers, including CD40, CD54, CD80, CD83, CD86, CD209, and HLA-DR (MHC II molecule) on human DCs, along with dendritic elongation. As shown in Figure 16, C13 (not of the present invention) showed a significant increase in the expression levels of CD40, CD80, CD83, CD86 and HLA-DR and promotes maturation of human monocyte-derived DCs. Figure 17A shows histograms for CD40, CD80, CD83, CD86 and HLA-DR expression in DCs in response to C13. Figure 17B shows the morphology of DCs incubated with C13 for 48 hours.

### CD1d-DEPENDENT TCR ACTIVATION OF NKTs USING α-GalCer ANALOGS

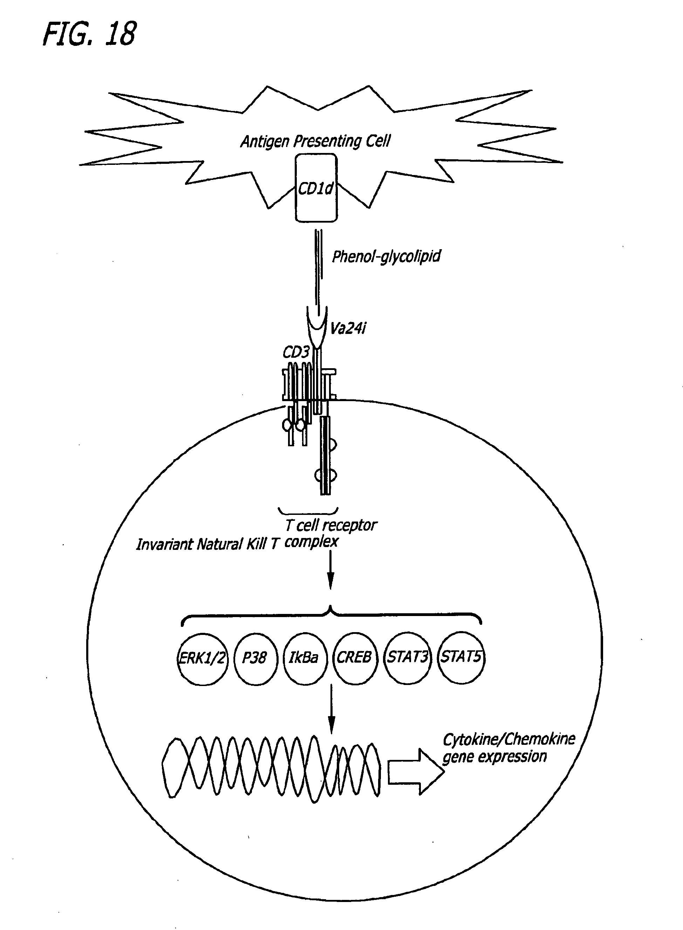

As described herein, the synthetic α-GalCer analogs are capable of inducing CD1d dependent TCR activation. Figure 18 shows a schematic illustration summarizing TCR signaling pathways in NKTs. *i*NKTs recognize glycolipid antigens presented in the context of CD1d on the surface of antigen presenting cells (APCs) via T cell receptor complexes. The binding of glycolipid antigens activates cytosolic kinases in *i*NKTs, including phosphorylation of ERK1/2, p38, IKBα, CREB, STAT3 and STAT5. These signaling cascades lead to i*N*KT proliferation and cytokine/chemokine production.

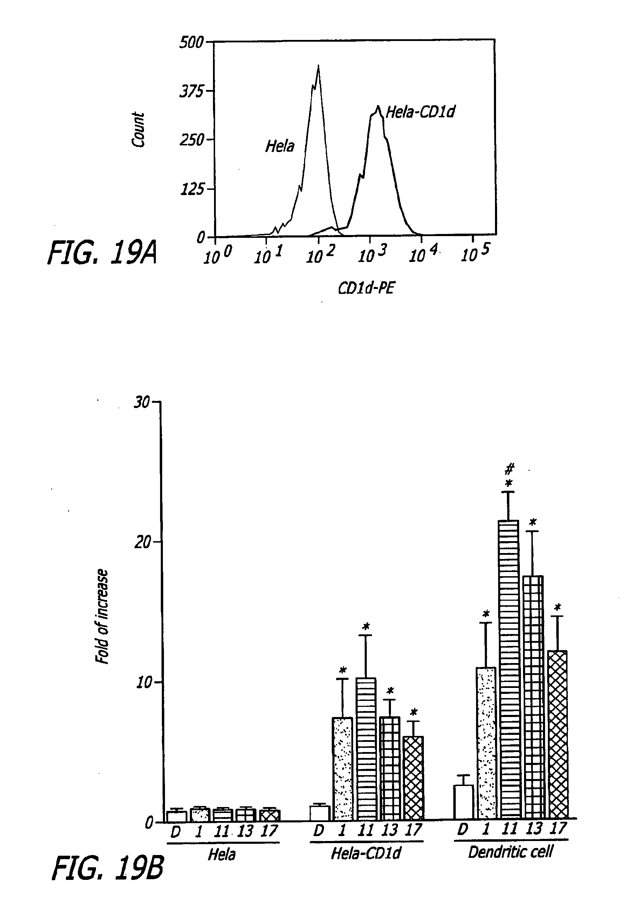

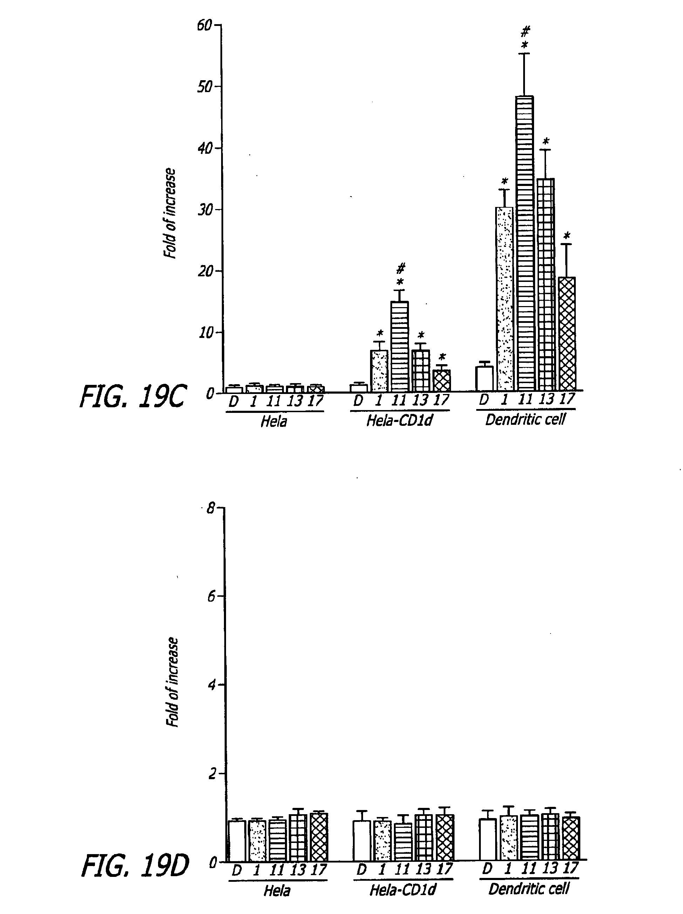

As described herein, the α-GalCer analogs are capable of inducing CD1ddependent TCR activation of naive human NKTs. To discern whether TCR activation is CD1d-dependent, the effects of various α-GalCer analogs presented by HeLa-CD1d, overexpressing human CD1d, and control HeLa cells was determined. Also, the capacity of HeLa-CD1d (nonprofessional ARCs) were compared with immature DCs (professional APCs) in presenting the various α-GalCer analogs to NKTs. As shown in Figure 19, C1 and the α-GalCer analogs C11, C13 and C17 increased intracellular values of phospho-CD3ε by 7.3, 10, 7.3 and 5.9 folds of control, respectively, when presented by HeLa-CD1d cells and 10.8, 21.3, 17.3 and 12 folds respectively, when presented by DCs. For phospho- ERK1/2, C1 and the α-GalCer analogs C11, C13 and C17 induced 6.6, 14.6, 6.6 and 3.3 folds increase respectively, with HeLa-CD1d cells and 30, 48.3, 35 and 18.6 folds respectively, with DCs. The induction of phospho-CREB is even more surprising; C1 and the α-GalCer analogs C11, C13 and C17 induced 2, 117, 41 and 20 folds expression respectively, when presented by HeLa-CD1d cells and 68, 204, 158 and 49 folds increase respectively, when presented by DCs. None of the α-GalCer analogs tested had any effect on the phosphorylation of Syk, a protein kinase, known to play a role in B cell receptor signaling but not in the TCR pathway. These findings suggest that aromatic α-GalCer analogs induced a strong TCR activation in a CD1d-dependent manner, and the extent of activation is greatly enhanced when presented by professional APCs as compared to non-professional APCs. None of the α-GalCer analogs showed any effect on phosphorylation of CD3ε, ERK1/2 or CREB in NKT cells when co-cultured with control HeLa cells. Overall, compounds C11 and C13 appeared to be stronger in TCR activation than compounds C1 and C17, which were consistent with their greater induction of T_{H}1-biased cytokine profile triggered by C11 as compared with C1, because ERK1/2 and CREB activations have been reported to play a role in the induction of many T_{H}1 cytokines, such as IL-12 and IFN-γ. C13 also triggered significant activation of TCR, presumably as a consequence of the unique ability of C13 to enhance expression of co-stimulatory molecules on DCs. For the four α-GalCer analogs examined, the TCR was activated more potently when presented by DCs than by HeLa-CD1d cells, especially with C13. Higher levels of phosphorylated CD3ε, ERK1/2 and CREB induced by the α-GalCer analog C11 than by C1 is consistent with the notion that stronger binding of glycolipid to CD1d induces a greater stimulation of TCR on NKTs.

Figure 20 shows how α-GalCer analogs are capable of inducing CD1d-dependent TCR activation. Various α-GalCer analogs (specifically C16, C23, C26, C8-5 and C8-6) are capable of activating TCR signaling pathways in human *i*NKTs (Vα 24⁺ T cells) with phosphorylation of ERK1/2, p38, IKBα, CREB, STAT3 and STAT5. To discern whether TCR activation is CD1d-dependent, the effects of various α-GalCer analogs presented by HeLa-CD1d, overexpressing human CD1d, and control HeLa cells was determined. Figure 20A shows the determination of isolated Vα24⁺ T cells by flow cytometry which contained 92% naïve Vα24⁺ /CD3⁺ T cells. C1 and the α-GalCer analogs, specifically C16, C23, C26, C8-5 and C8-6, increased intracellular values of (B) phospho-CD3ε (Phospho-tyrosine), (C) phospho-CREB (Ser133), (D) phospho-ERK1/2 (Thr185/Tyr187), (E) phospho-p38 (Thr180/Tyr182), (F) phospho- IκBα (Ser32), (G) phospho-Lck, (H) phospho-Lat, (I) phospho-STAT3 (Ser727), (J) phospho- STAT5 A/B (Tyr 694/699), (K) phospho-Syk (Phospho-tyrosine) and (L) phospho-Zap-70 (Phospho-tyrosine). *, p < 0.05, compared with DMSO; #, p < 0.05, compared with C1.

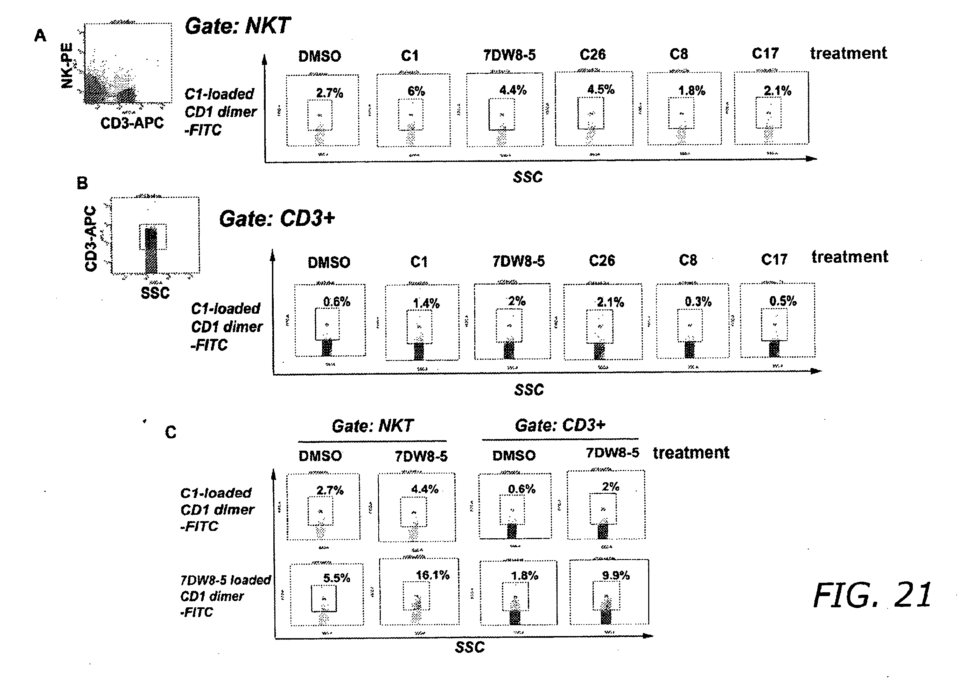

The α-GalCer analogs also exhibit higher binding affinity to CD1 d-restricted mouse NKT/Ts in vitro (Figure 21) and CD1d-dependent activation of two subset NKTs and NKs *in vivo* (Figure 22). As shown in Figure 21, spleens from BALB/c mice were harvested 72 hours after intravenous (IV) injection of 0.1 µg/mouse of the indicated α-GalCer analogs (C1, 7DW8-5, C26, C8, C17) or vehicle. Percentage of mouse NKTs cells (Figure 21 A) or T cells (Figure 21 B) were stained with mCD1d tetramer loaded with α-GalCer (10 mole per µg). Figure 21C shows different binding affinity of α-GalCer and phenol α-GalCer analog 7DW8-5 to CD1d-restricted NKTs and T cells. Figure 22 shows CD1-dependent expansion of two NKTs subsets. Spleens from BALB/c wild type (WT) or CD1 Knock out (KO) mice were harvested 72 h post-injection of DMSO control, α-GalCer or the indicated α-GalCer analogs C8, C16, C22, C23, C26, 7DW8-5 and 7DW8-6 IV. Total numbers of NKTs, and its two subtypes, designated as Type II NKT (CD3⁺/NK⁺/CD49⁺ /CD69.) and Type I NKT (CD3⁺/NK⁺/CD49/CD69⁺) in (B) wild type or (C) CD1 knockout mice in response to the indicated α-GalCer analogs were assessed by FACS. (D) shows CD1d-dependent activation of NKs. The expansion of total number of active NKs (CD3⁻/NK⁺/CD69⁺) in WT or CD1KO mice in response to the indicated α-GalCer analogs was assessed by FACS. *, p< 0.05, compared with DMSO; #, p < 0.05, compared with C1.

### IN VIVO T_{H} CELL ACTIVATION, EXPANSION/ACTIVATION OF SPLENOCYTES AND CD1d-DEPENDENT TCR ACTIVATION OF NKTs USING α GalCer ANALOGS

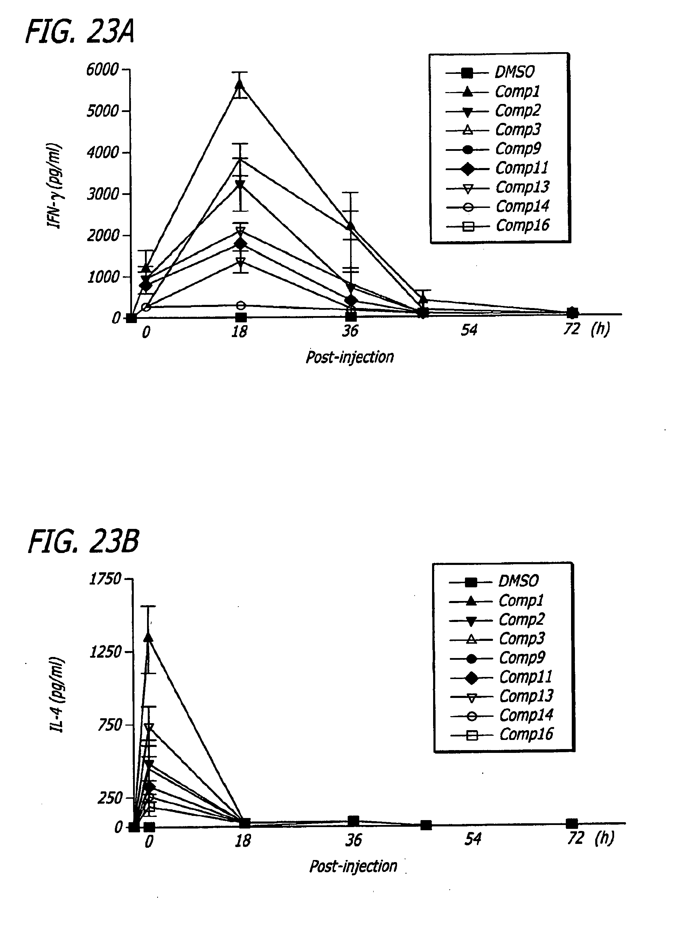

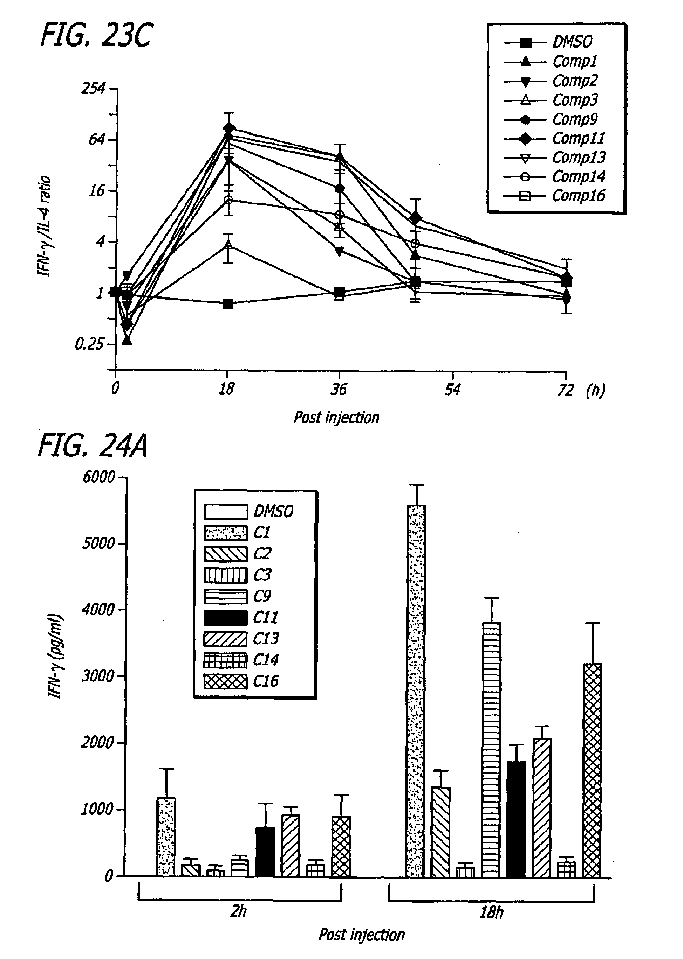

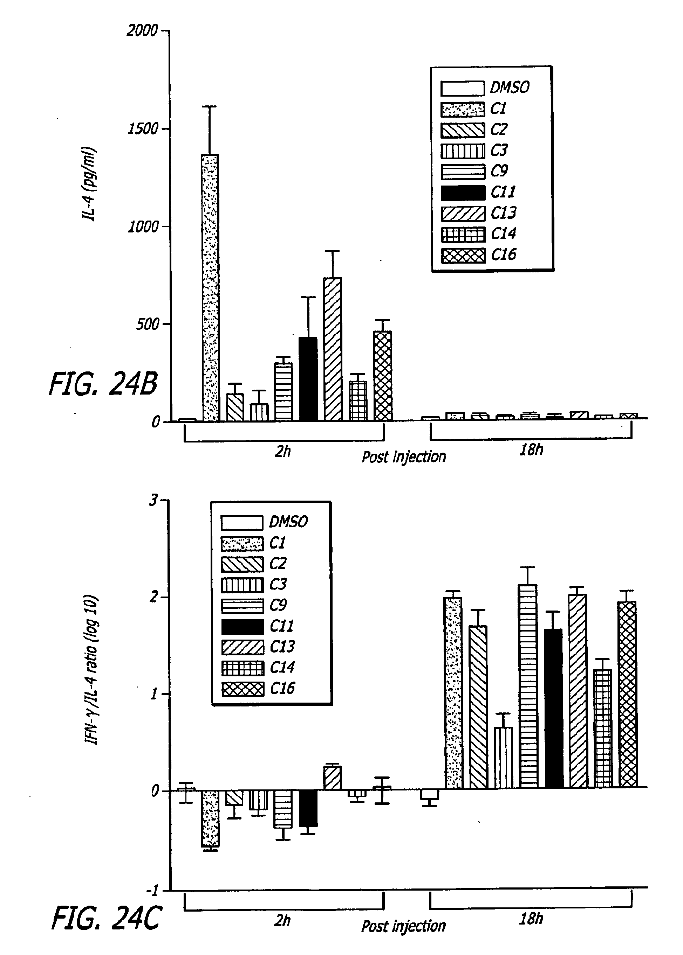

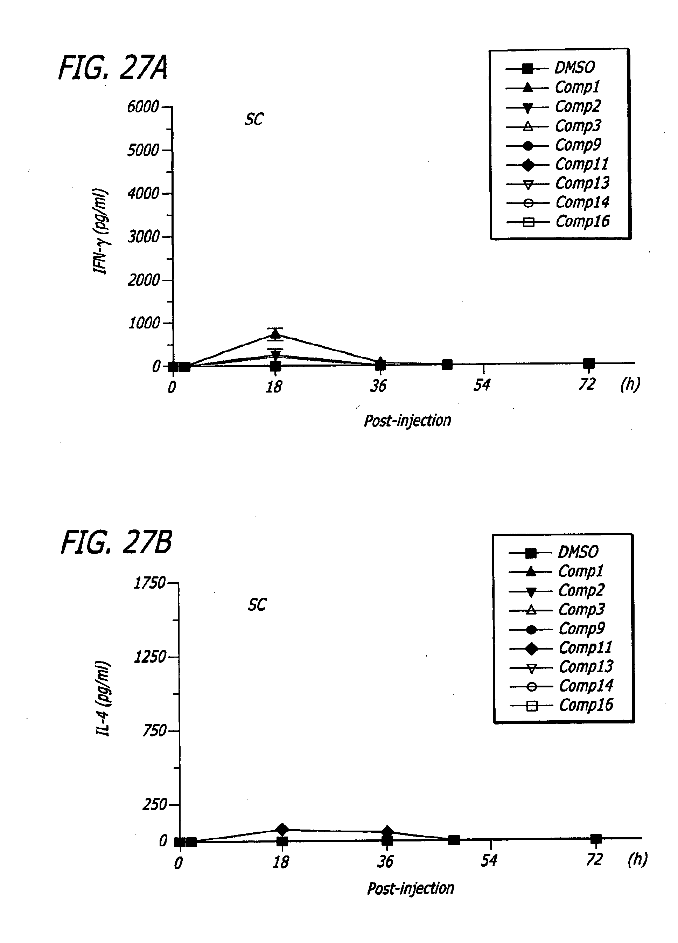

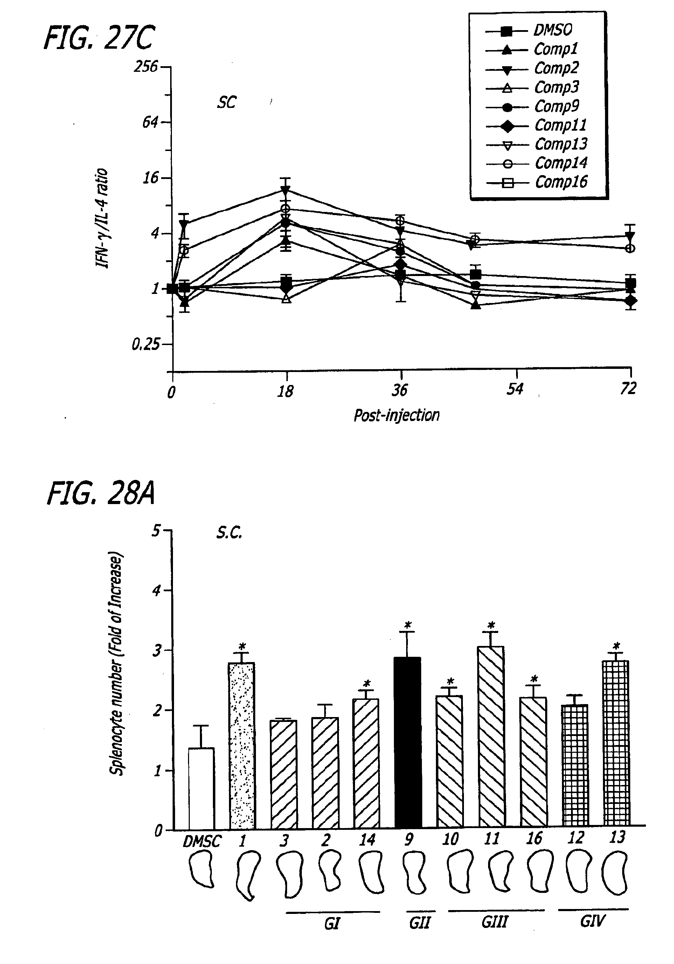

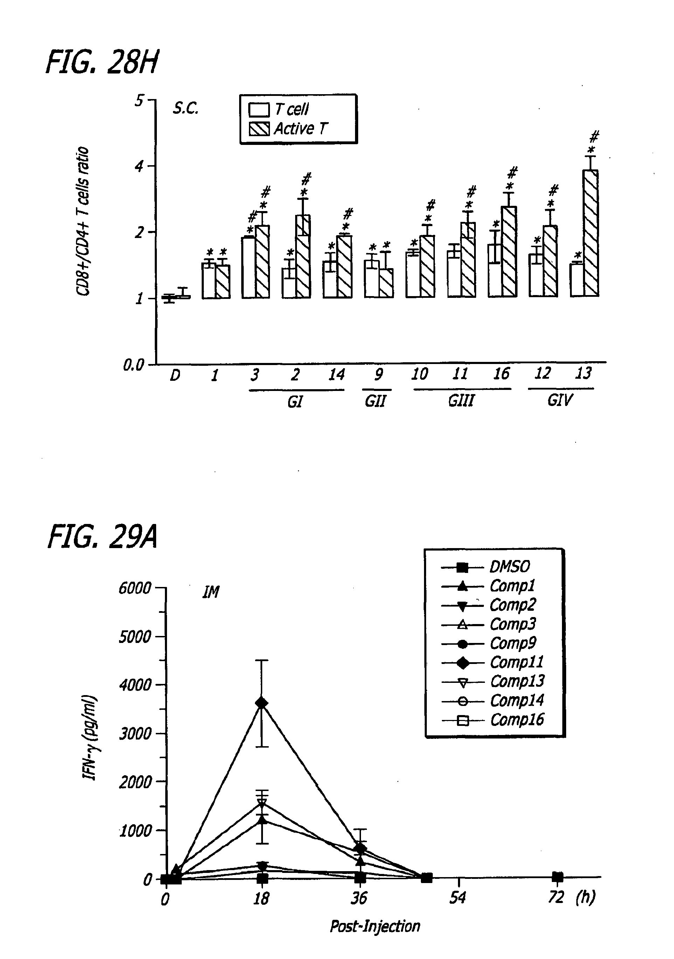

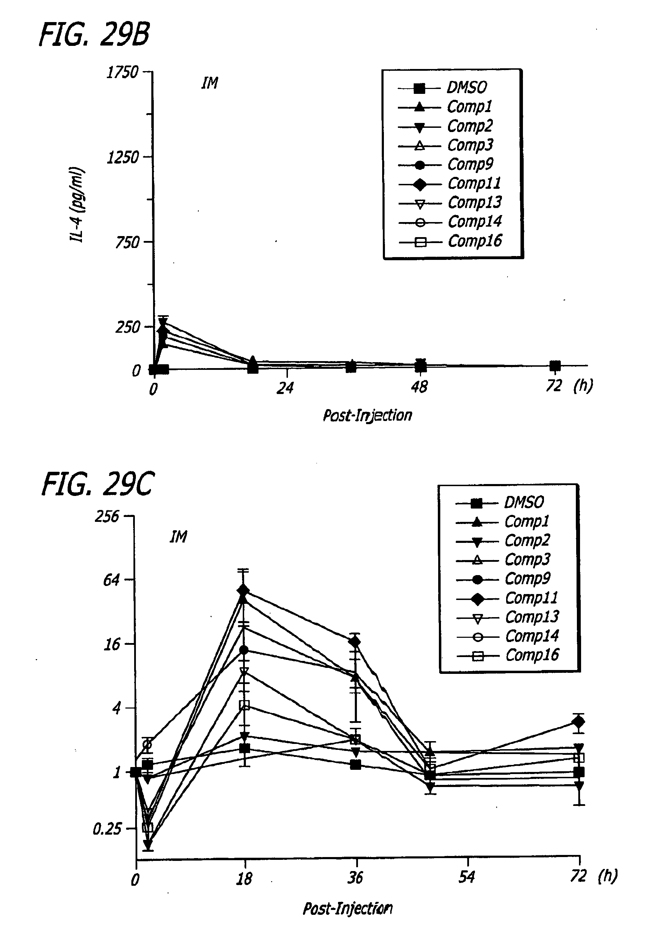

As described herein, the α-GalCer analogs are capable of activating T_{H} cells *in vivo.* To evaluate the impact of administration route on cytokine secretion, α-GalCer and seven α-GalCer analogs were injected into BALB/c mice by either intravenous (IV), subcutaneous (SubQ) or intramuscular (IM) routes and the impact on cytokine production was determined. Figures 23A, 27A and 29A show the serum level of IFN-γ over a period of 72 hours after injection of various α-GalCer analogs through different routes. In general, an increase in cytokine production was detectable as early as 2 hours, peaked at 18 hours and gradually dropped down to the baseline level by 48 hours. When introduced through the IV route (Figure 23A), the α-GalCer analog C9 and the α-GalCer analog C16 showed a level of activity close to that of C, followed by the α-GalCer analogs C13, C11, C2, C14 and C3 (none of which are of the present invention). Notably, the level of IFN-γ induced by SubQ administration (Figure 27A) of the same α-GalCer analogs was much lower than that of the IV route, whereas the level of IM route (Figure 29A) was intermediate. Although C1 induced the highest level of IFN-γ when given IV, the α-GalCer analog C9 surpassed C1 when given by SubQ and IM routes. Figures 23B, 27B, and 29B, show the levels of IL-4 after injections of the α-GalCer analogs through the different routes. All the α-GalCer analogs tested, as well as α-GalCer, showed little induction of IL-4 when introduced through the SubQ route, whereas intermediate levels of IL-4 were induced by all α-GalCer analogs when given by IM administration. When the data are expressed as IFN-γ/IL-4 ratio (Figure 23C, 27C and 29C) to reflect the T_{H}1/T_{H}2 bias, the aromatic α-GalCer analogs C11, C13, C16 and C14 of bacterial origin elicited less T_{H}2 responses than C1 at 2 hours via the IV route, and all α-GalCer analogs induced T_{H}1 bias responses during the period of 18- 72 hours, as shown in Figures 23C, 27C and 29C. Furthermore, when administered by the SubQ route, all the tested α-GalCer analogs showed a higher T_{H}1/T_{H}2 ratio than C1 during the entire period of 2-72 hours except α-GalCer analogs C2 and C3. On the other hand, when given by IM injection, all the α-GalCer analogs showed a T_{H}2 biased response at 2 hours and again shifted to a more T_{H}1 biased response during the period of 18-72 hours except for C14. The latter showed a more T_{H}1 biased response at 2 hours and remaining T_{H}1 bias during the entire period of 2-72 hours. In another view, Figure 24 shows mouse serum levels of secreted (A) IFN-γ, (B) IL-4 and (C) ratio of IFN-γ/IL-4 at 2 and 18 h following IV administration of indicated α-GalCer analogs.

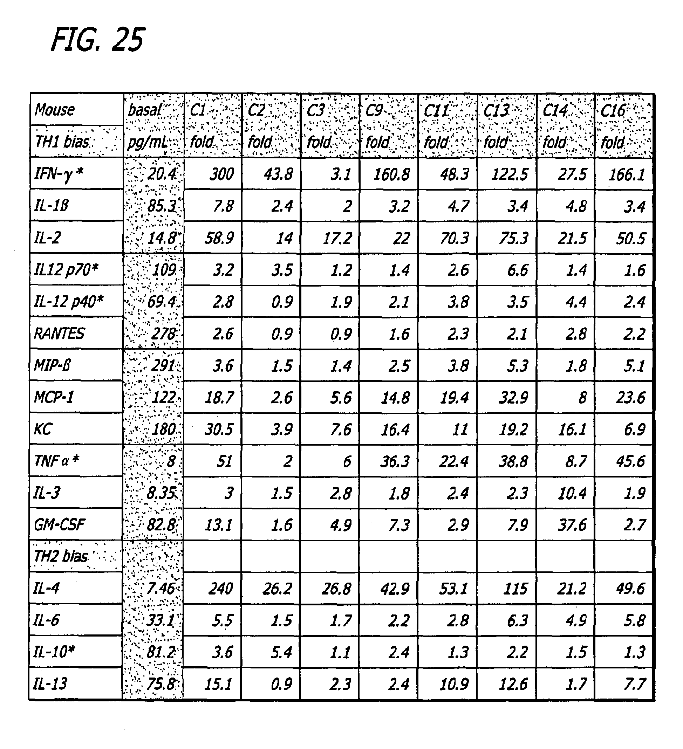

Along with IFN-γ and IL-4, other cytokines and chemokines also increased significantly in sera in response to these α-GalCer analogs. These included IL-2, IL-6, KC, IL-10, IL-12, IL-13, GM-CSF, TNFα, RANTES, MCP-1. and MIP-1, which are listed in the Table in Figure 25. In IV administration, these α-GalCer analogs elicit a greater T_{H}1 biased cytokine and chemokine response than C1. For example, aromatic α-GalCer analogs C11, C13 and C16 induce striking rises in IL-2, IL-12, MIP-1β and MCP-1, and C14 showed greater inductions of IL-3, GM-CSF and IL-12.

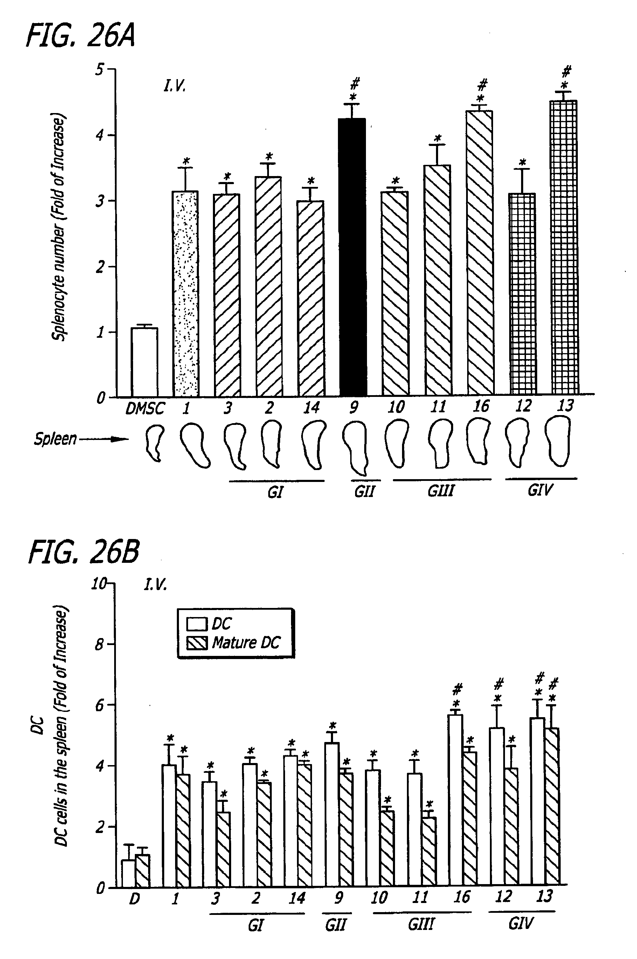

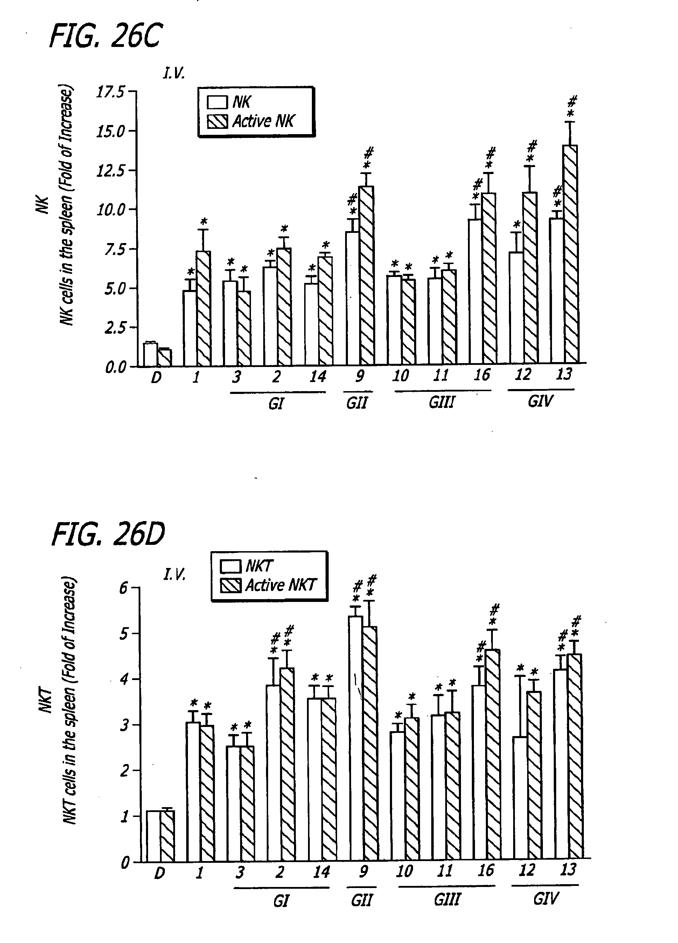

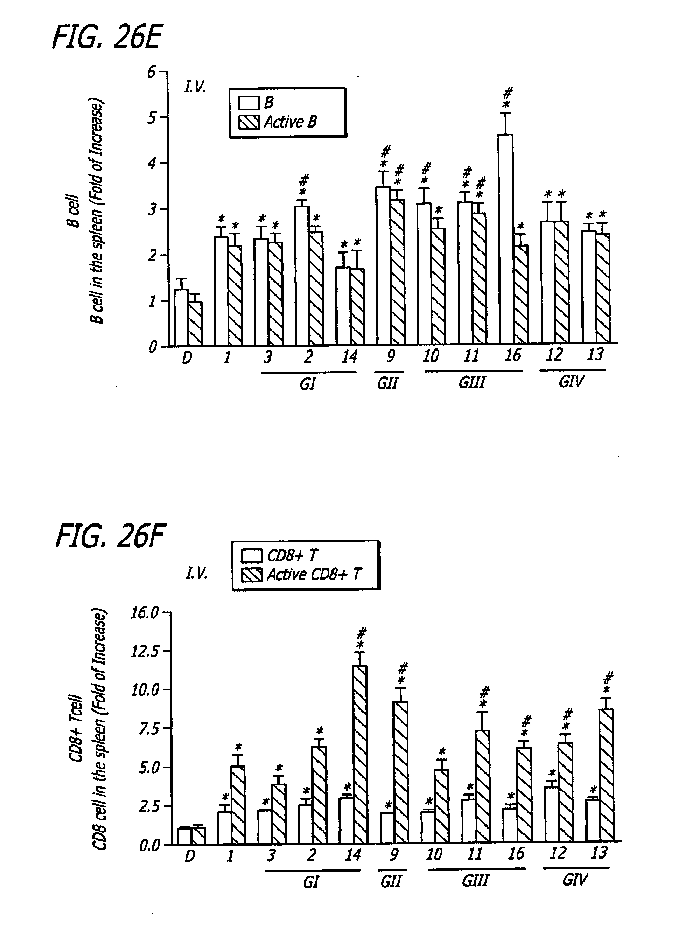

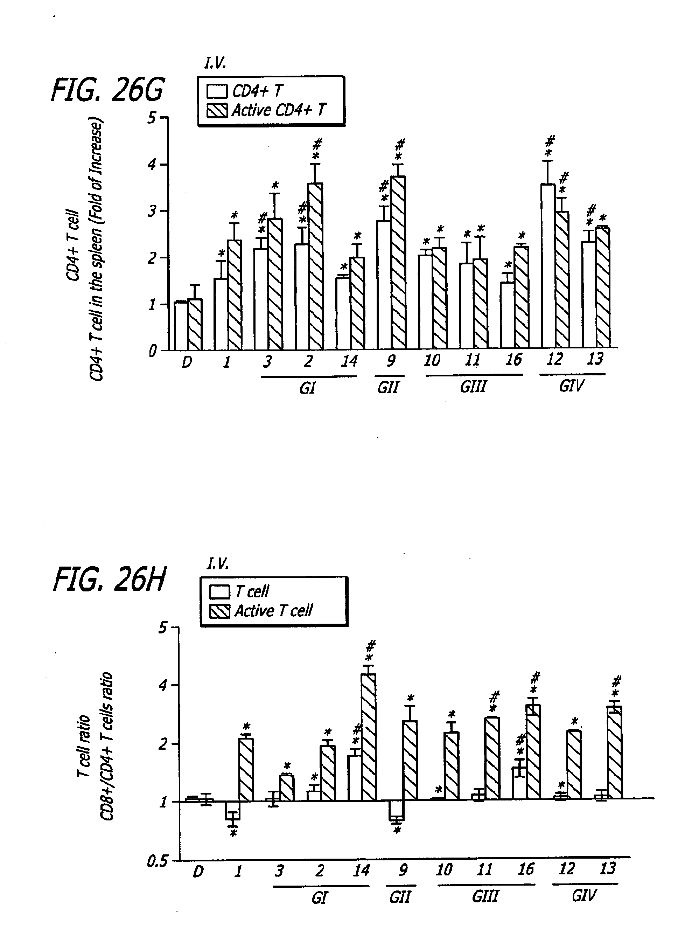

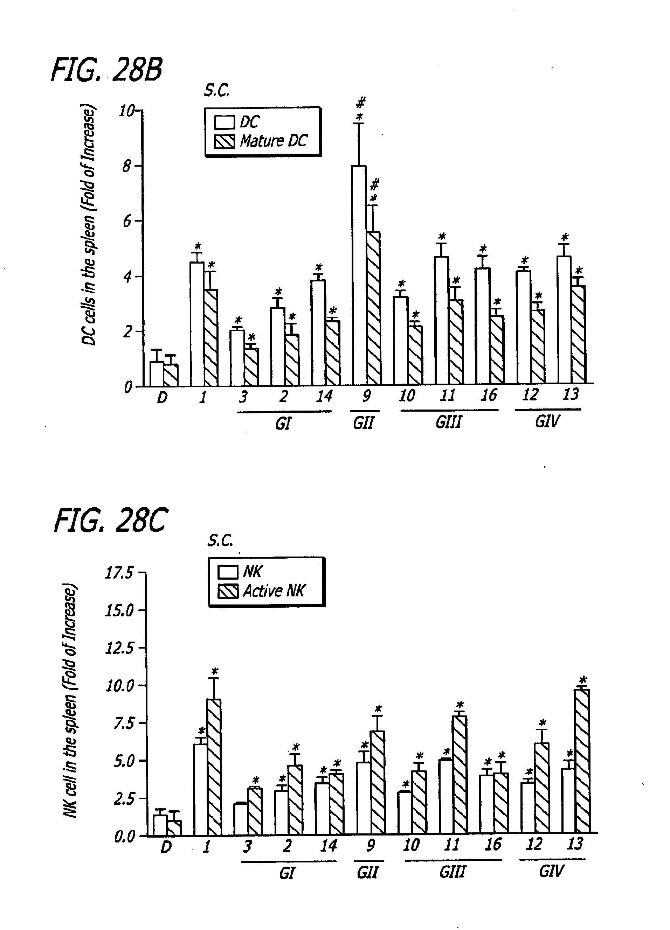

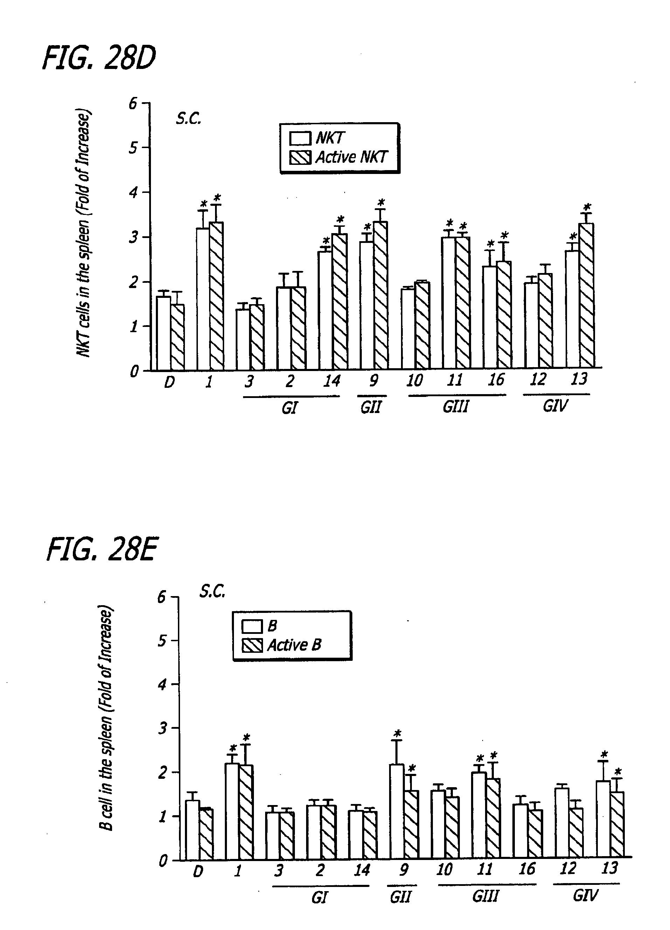

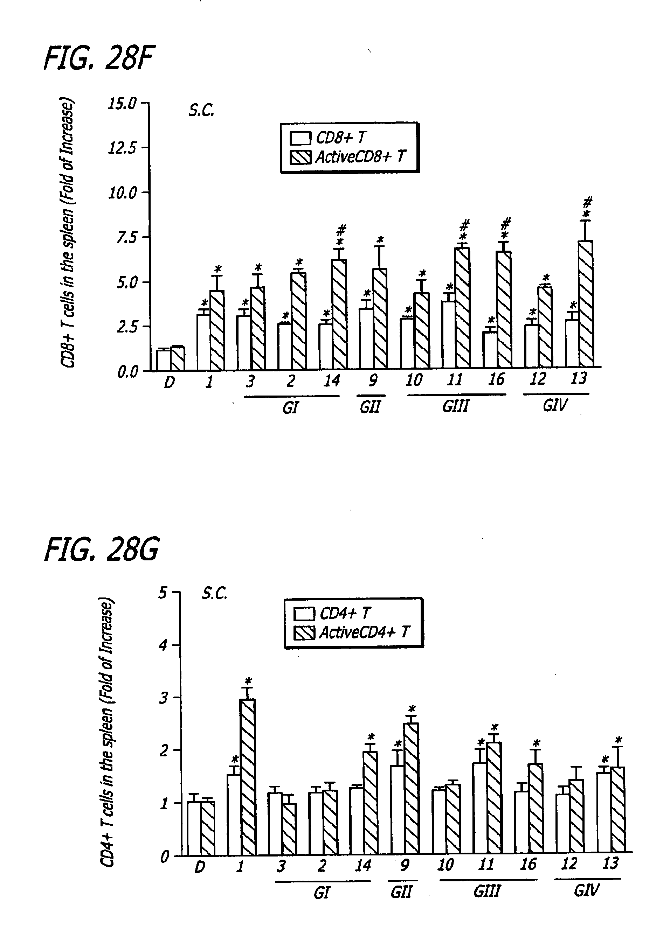

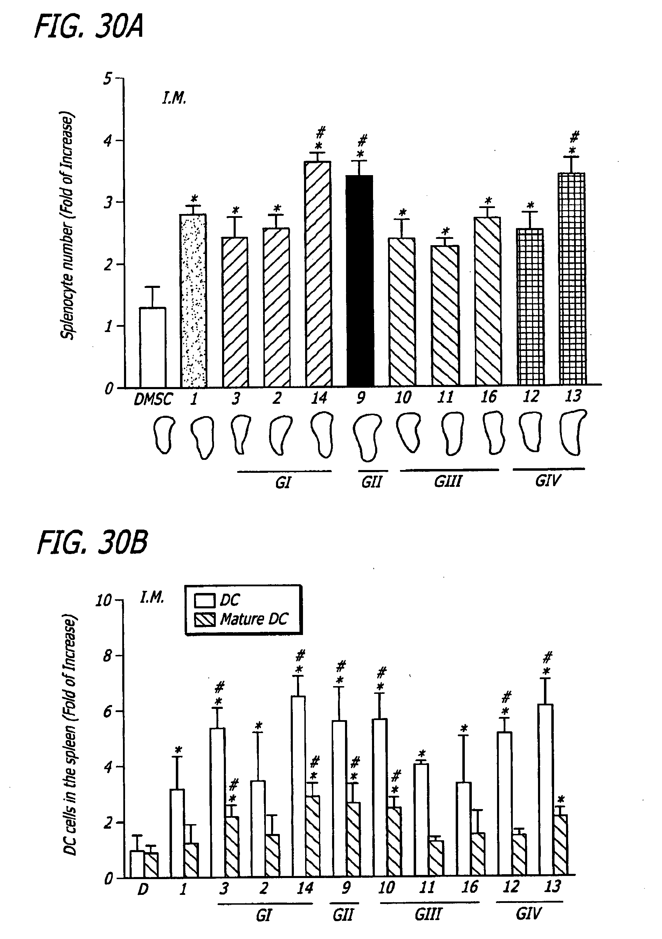

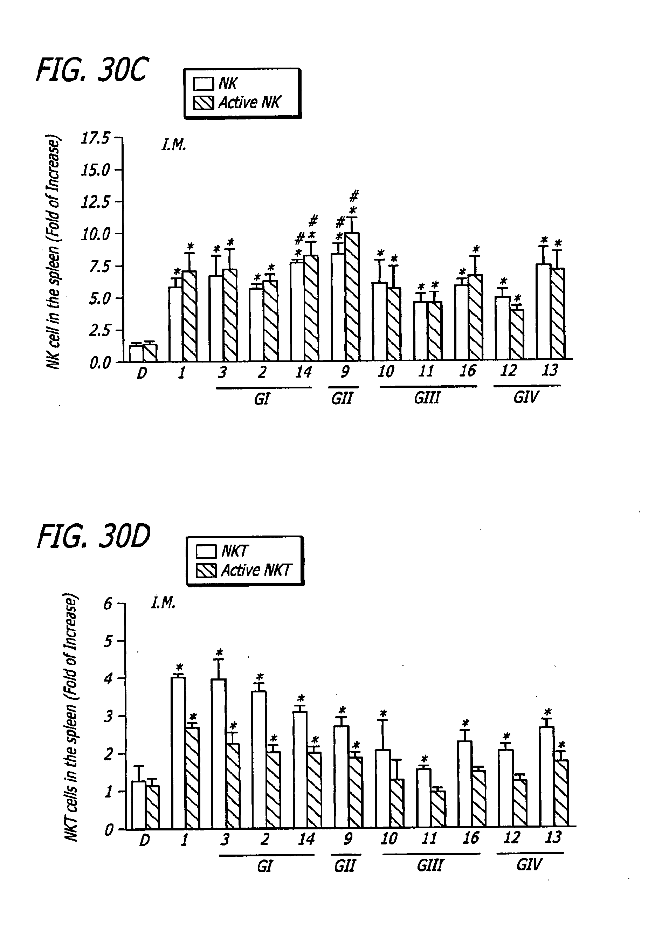

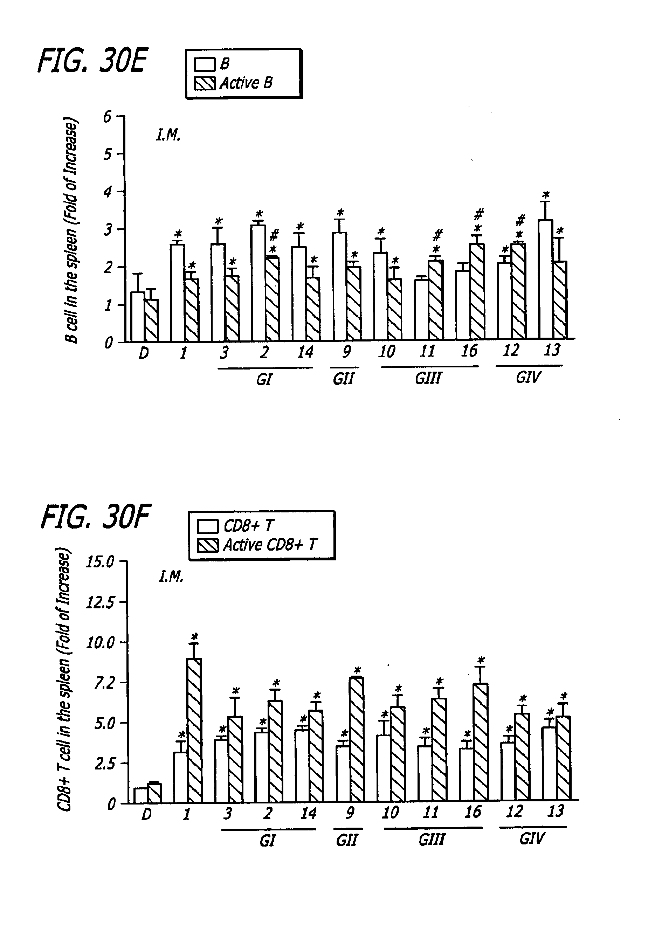

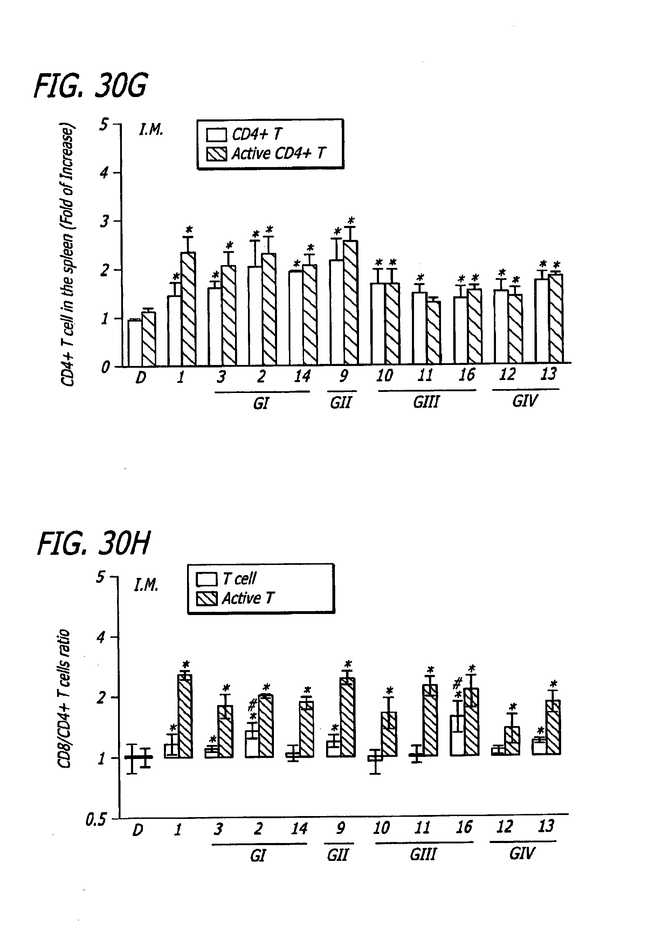

To determine the populations of immune cells in the spleens of BALB/c mice injected with α-GalCer or the indicated α-GalCer analogs, BALB/c mice were injected and then examined 72 hours after injection. As shown in Figure 26, after IV administration all of the α-GalCer analogs tested induced significant expansion in (A) splenocytes, with C9, C13 and C16 showing greater potency than C1., (B) DCs, (C) NKs, (D) NKTs, (E) B cells, (F) CD8⁺ T cells, (G) CD4⁺ T cells and (H) activated CD8⁺/CD4⁺ ratios. As shown in Figures 28 after SubQ administration, none of the α-GalCer analogs tested showed a significant effect on the expansion of (A) splenocytes, as compared with that of C1. As shown in Figures 30, after IM administration all of the α-GalCer analogs tested induced (A) splenocyte expansion, with C9, C13 and C14 having greater effects than C1. Aromatic α-GalCer analogs C12, C13 and C16 induced significantly greater rises in total and mature DCs than C1 (Figures 26B, 28B and 30B). α-GalCer analogs C9, C12, C13 and C16 displayed the best capacity for expansion/activation of NKs and NKTs (Figures26C-D, 28C-D and 30C-D). α-GalCer analog C16 was most effective in B cell expansion, and α-GalCer analogs C2, C9, C10, and C11 were also more active than C1 (Figures 26E, 28E and 30E). For CD8⁺ T cells, α-GalCer analog C14 was most effective in cell expansion/activation, although α-GalCer analogs C9, C11, C16, C12 and C13 were also more active than C1 (Figures 26F, 28F and 30F). α-GalCer analog C9 was most effective in CD4⁺ T cell expansion/activation than C1 (Figures 26G, 28G and 30G). Among the T cell subpopulations, all of the α-GalCer analogs tested induced a rise in CD8⁺/CD4⁺ ratio, with α-GalCer analogs C11, C13, C14 and C16 being more potent than C1 (Figures 26H, 28H and 30H). In mice treated with the α-GalCer analogs by the SubQ route, α-GalCer analog C9 induced significantly greater expansion of total and mature DCs than C1, while the remaining α-GalCer analogs were comparable to C1 (Figure 28B). For NK and NKT expansion/activation, α-GalCer analogs C9, C11, C13, C14 and C16 showed comparable activities as C1, and the remaining α-GalCer analogs seemed less active (Figure 28C-D). For B cell expansion/activation, α-GalCer analogs C1, C9, C11 and C13 showed significant activities (Figure 28E). For CD8⁺ T cells, α-GalCer analogs C9, C11, C13, C14 and C16 showed more activity than C1, and the remaining α-GalCer analogs appeared to be comparable activities as C1 (Figure 28F). For CD4⁺ T cells, C1 was most effective, although α-GalCer analogs C9, C11, C13, C14 and C16 were also more active over control (Figure 28G). For T cells, most α-GalCer analogs tested elicited a greater increase in CD8⁺/CD4⁺ ratio than C1 (Figure 28H). When the α-GalCer analogs were introduced through the IM route, all induced significant increases in DCs, NK, NKT, B cells and CD8⁺/CD4⁺ ratio. The majority of novel α-GalCer analogs elicited greater expansion of DCs than C1 (Figure 30B). α-GalCer analogs C9 and C4 displayed stronger induction of NK cells (Figure 30C) than C1, but comparable or less effects on NKT cells (Figure 30D). α-GalCer analogs C2, C11, C12 and C16 showed stronger activations of B cells than C1 (Figure 30E). For CD8⁺ T cells, α-GalCer analogs C9 and C16 showed comparable activities as C1 in cell expansion/activation, and the remaining α-GalCer analogs induced significant increases over the control (Figure 30F). For CD4⁺ T cells, α-GalCer analogs C2 and C9 showed comparable activities as C1 in cell expansion/activation, and the remaining α-GalCer analogs induced significant increases over the control (Figure 30G). α-GalCer analogs C9, C11 and C16 showed similar activities as C1 in raising CD8⁺/CD4⁺ ratio (Figure 30H).

Figure 31 shows the effects of route of administration of α-GalCer analogs on cytokine kinetics and splenocytes expansion/activation. Figure 31(A-C) shows the kinetics of cytokines in response to DMSO vehicle, α-GalCer or α-GalCer analog C16 given by different routes. BALB/c mice were injected with vehicle, C1 or C16 (2 µg per mouse) IV, SubQ or IM. Serum samples collected at 0, 2, 18, 36, 48, 72 h were analyzed for cytokines: (A) IFN-γ, (B) IL-4 and (C) the ratio of IFN-γ over IL-4, normalized to DMSO vehicle. Figure 31 (D-K) shows the expansion/activation of splenocytes in response to vehicle, C1 and C16 given by different routes. Spleens from BALB/c mice were harvested 72 h after injection of C1, C16 (2 µg per mouse) or vehicle IV, SubQ or IM. (D) shows the total number of nucleated cells, (E-G) shows the population of innate immune cells including mature dendritic cells (CD11C⁺/CD80⁺/CD86⁺), activated NKs (U5A2-13Ag⁺/CD3-/CD69⁺), activated NKTs (U5A2⁻13Ag/CD3⁺/CD69⁺), (H-J) shows adaptive immune cells including activated B cells (CD45R⁺/CD23⁺/CD69⁺), activated CD8 T cells (CD3⁺/CD4⁻/CD8⁺/CD69), and activated CD4 T cells (CD3⁺/CD4⁺/CD8⁻/CD69⁺). (K) shows the ratio of CD8/CD4, normalized to DMSO. ** p < 0.05, compared with C1.

As described herein, the α-GalCer analogs were administered to mice at various doses to determine whether a dose-response is noticeable for the expansion/activation of splenocytes. As shown in Figure 32A-H, spleens from BALB/c mice were harvested 72 h after IV injection of vehicle or α-GalCer analog C11 (2 or 0.1 µg per mouse). (A) shows the total number of nucleated cells, (B-H) shows the population of innate immune cells including mature DCS (CD11C+/CD80⁺/CD86⁺), activated NKs (U5A2⁻13Ag⁺/CD3⁻/CD69⁺), activated NKTs (U5A2⁻13Ag⁺/CD3⁺/CD69⁺), monocyte (CD11b⁺Gr1⁻), granulocyte (CD11b⁻ Gr1⁺); (F-H) shows adaptive immune cells including activated CD4 T cells (CD3⁺/CD4⁺/CD8⁻/CD69⁺), activated CD8 T cells (CD3⁺/CD4-/CD8⁺/CD69⁺), and activated B cells (CD45R⁺/CD23⁺/CD69⁺). *, p < 0.05, compared with DMSO, #, p < 0.05, compared with C11 (2 µg per mouse).

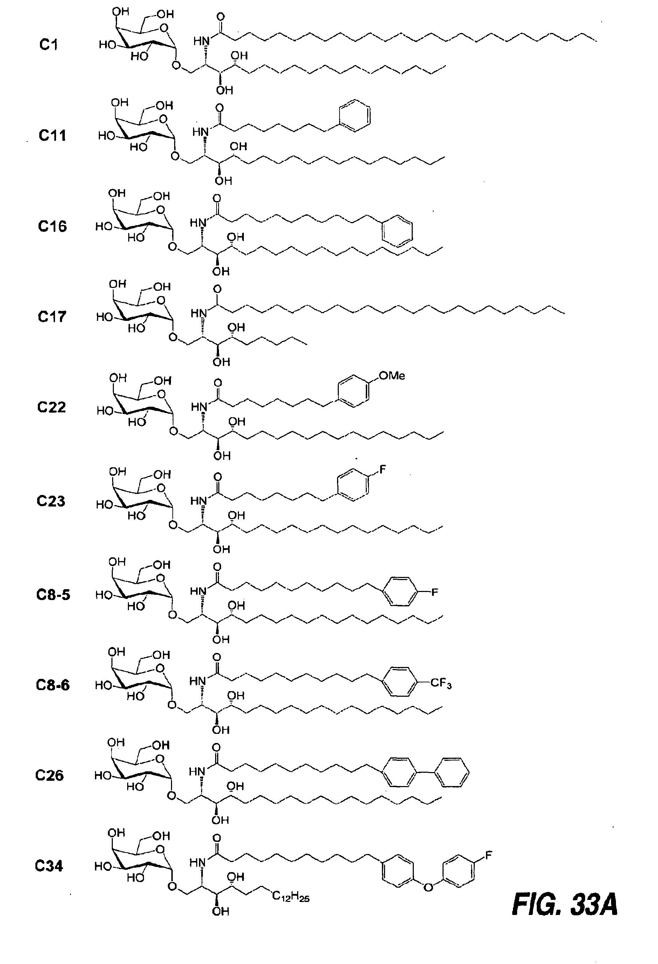

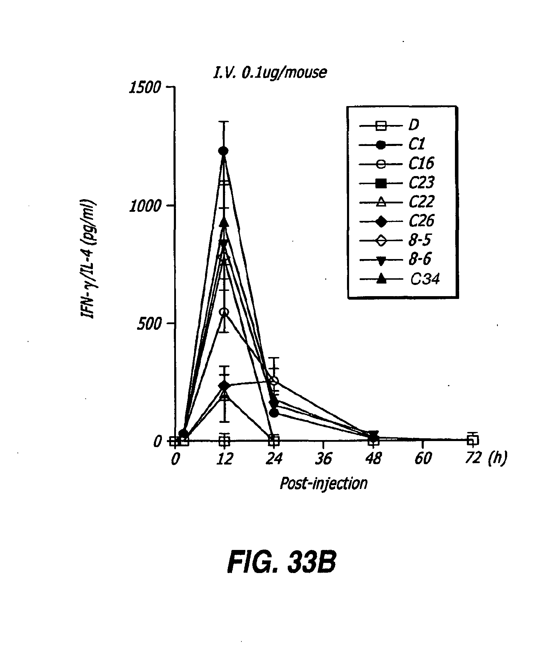

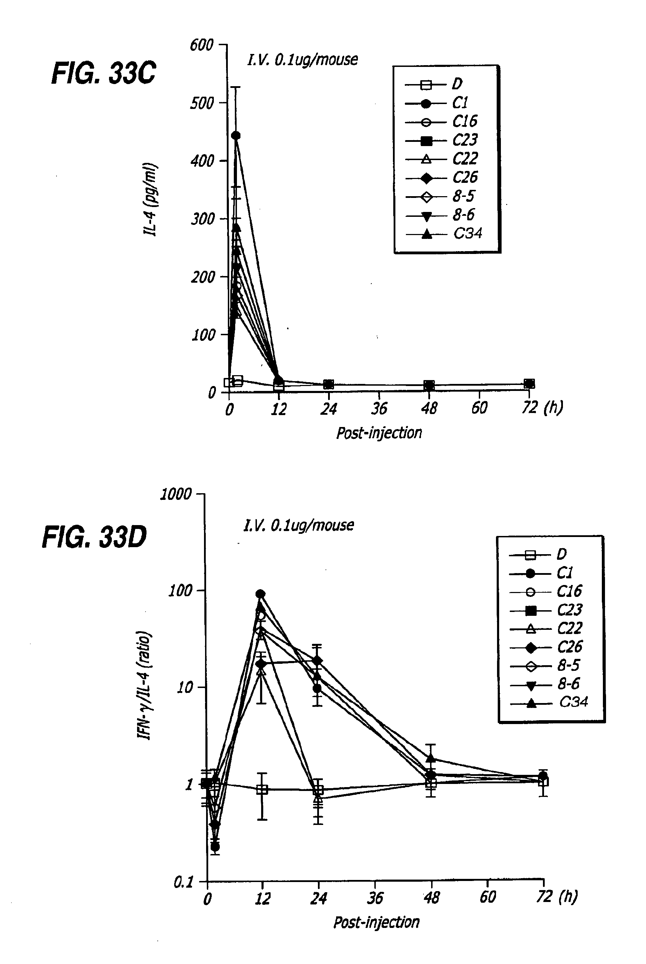

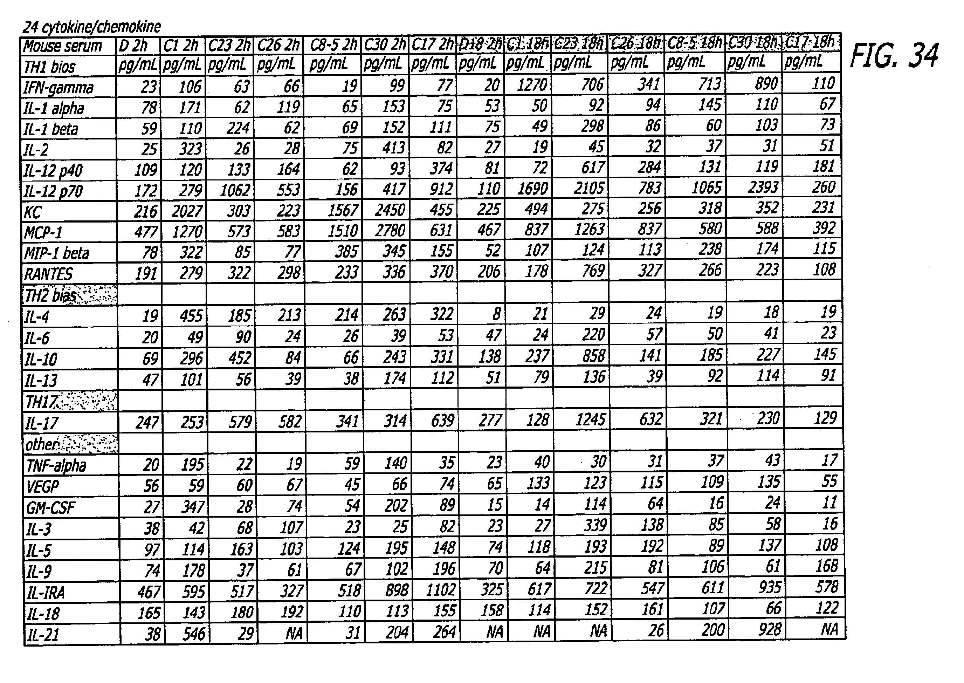

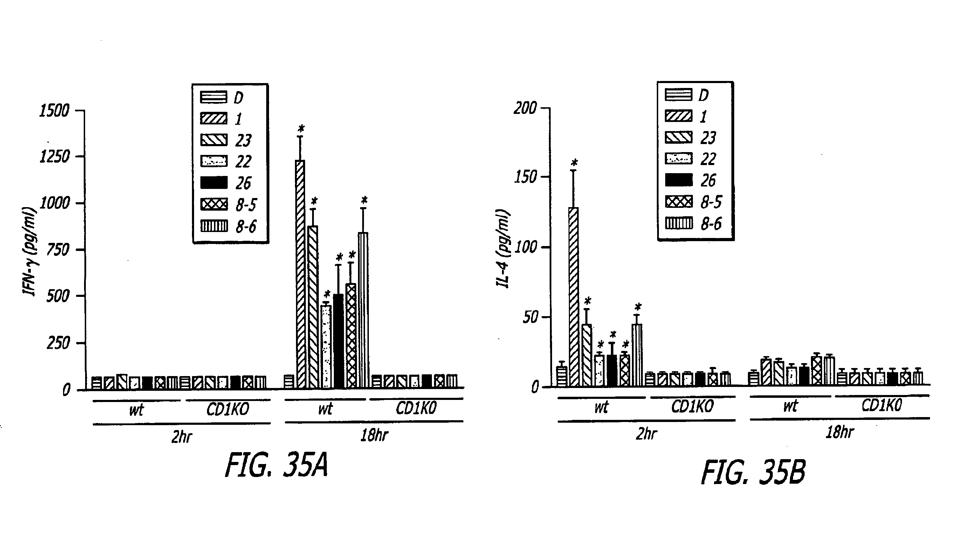

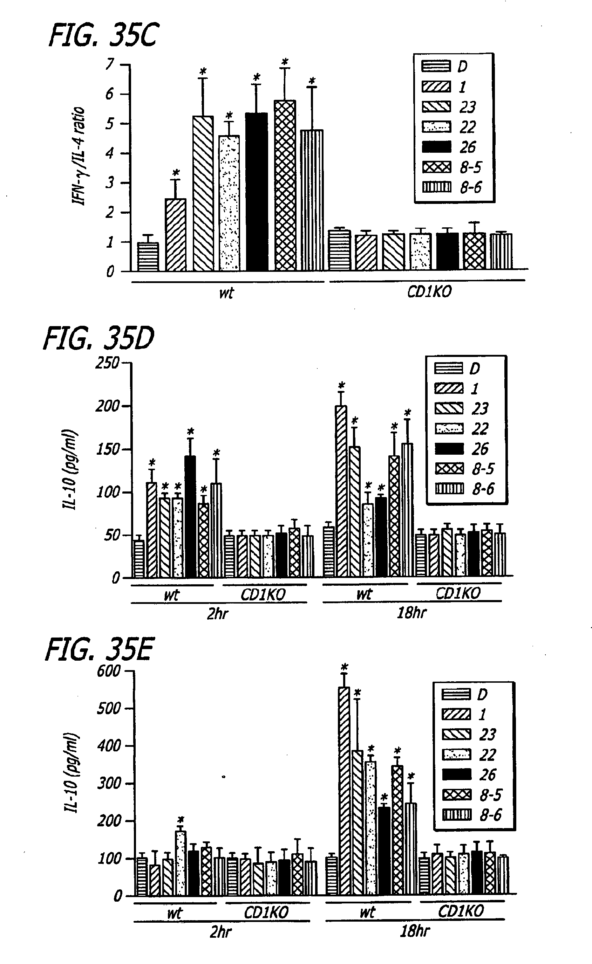

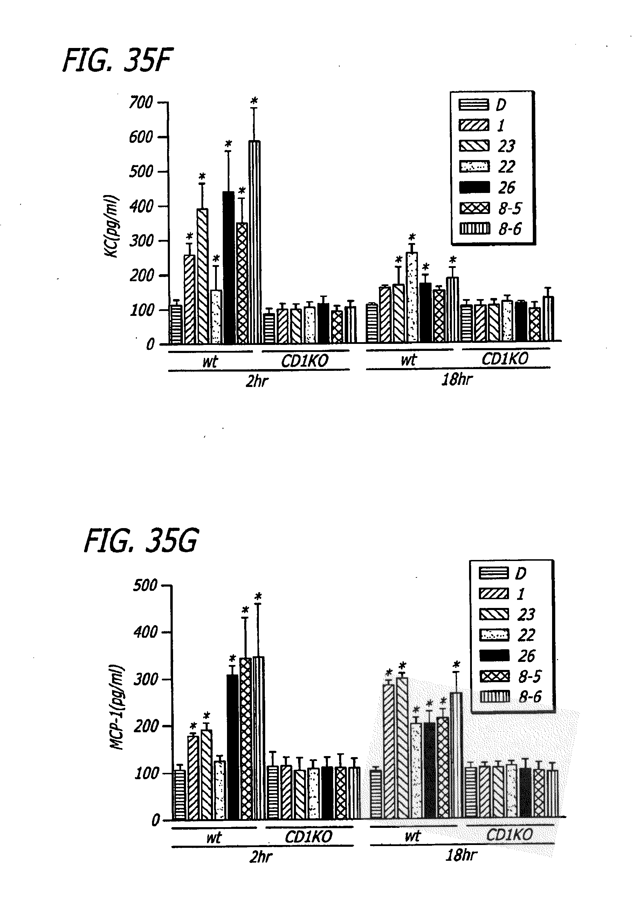

As described herein, the kinetics of T_{H}1/T_{H}2 cytokines induced by various α-GalCer analogs was assessed (Figure 33). BALB/c mice were injected IV with vehicle, C1 or the indicated α-GalCer analogs (see A, 0.1 µg per mouse). Serum samples were collected at 0, 2, 12, 24, 48, and 72 h, and then assessed for the secretions of (B) IFN-γ, (C) IL-4 and (D) the ratio of IFN-γ over IL-4, normalized to DMSO control. These potent α-GalCer analogs elicited cytokines/chemokines as can be seen from the Table in Figure 34 which shows serum samples collected at 2 and 18 h. α-GalGer analogs of the present disclosure were administered IV to wild type (WT) and CD1d knockout (CD1KO) BALB/c mice (at 0.1 µg per mouse), see Figure 35. Serum samples were collected at 2 and 18 hour, and then analyzed for cytokines/chemokines, including (A) IFN-γ, (B) IL-4, (C) IFN-γ/IL-4 ratio, (D) IL-10, (E) IL-12p70, (F) KC) and (G) MCP-1. *, p <0.05, compared with DMSO. The results indicate that the α-GalCer analogs elicit CD1-dependent cytokines/chemokines secretion in mice.

Figure 36 shows the expansion/activation of splenocytes and CD1d-dependent activation of two NKT subsets after injection with various α-GalCer analogs. (A-F) shows the expansion/activation of splenocytes in response to the α-GalCer analogs tested. Spleens from C57BL/6 mice were harvested 72 h after IV injection of vehicle, α-GalCer or the indicated α-GalCer analogs (0.1 µg per mouse). (A) shows the total number of nucleated cells, (B-F) show the population of mature dendritic cells (CD11C⁺/CD80⁺/CD86⁺). activated NKs (NK1.1⁺/CD3⁻/CD69⁺), activated CD4 T cells (CD3⁺/CD4⁺/CD8⁻/CD69⁺), activated CD8 T cells (CD3⁺/CD4⁻/CD8⁺/CD69⁺), and CD8/CD4 ratio, normalized to DMSO. *, p < 0.05, compared with DMSO. (G-H) shows the CD1-dependent expansion of two NKT subsets. Spleens from C57BL/6 wild type (Wt) or CD1 knockout (CD1K0) mice were harvested 72 h post IV injection of vehicle, C1, 7DW8-5, C22, C23, C26, C34 (C34 being of the present invention) and C17, 0.1 µg per mouse. (G) shows the determination of mouse NKTs by flow cytometry (*lower*-*left panel*). An increase of total number of NKTs (*upper-left panel*) and its two subtypes including Type II NKT (CD3⁺/NK1.1⁺/CD49⁺/CD69⁻) (*upper-right panel*) and Type I NKT (CD3⁺ /NK1.1⁺/CD49⁻/CD69⁺) (*lower-right panel)* in Wt was noted by FACS. (H) shows the total number of NKTs in CD1 KO mice and (I) shows the total number of Treg cells (CD4⁺/CD254⁺/FoxP3⁺) in Wt C57BL/6 mice in response to the α-GalCer analogs. *, p < 0.05, compared with DMSO; #, p < 0.05, compared with C1.

### IMMUNOTHERAPY

The immune system effectively prevents our body's from being overtaken by scavenging germs. Without an effective immune system, people are subject to developing all sorts of infections from bacteria, viruses, protozoa, parasites and fungi. They are also more likely to develop cancer. Because NKTs play a regulatory role in the immune system, they are attractive targets for immunotherapy. The activation of NKTs paradoxically can lead either to suppression or stimulation of immune responses. For example, the production of T_{H}1 cytokines are thought to correlate with antitumor, antiviral/antibacterial, and adjuvant activities, whereas T_{H}2 cytokine production is thought to subdue autoimmune diseases.

### ANTI-TUMOR IMMUNOTHERAPY

It is now understood that there is a firm link between the immune system and cancer, and that by properly stimulating the immune system, there is the possibility of impacting many cancers. Treatment of mice with α-GalCer has been shown to suppress tumor metastasis to liver, lung and lymph nodes. In two phase I clinical trials in patients with advanced cancers who were injected with α-GalCer or α-GalCer-loaded iDCs, a distinct activation of the immune.system was observed in those patients who had a detectable Vα24⁺Vβ11 NKT number prior to treatment. Although there was no durable tumor regression, stable disease was noted in several patients, without any toxicity, and some patients even showed a transient reduction of serum tumor markers or tumor size. The lack of significant anti-cancer activity of α-GalCer in several clinical trials may be due to the effect of IFN-γ (a T_{H}1 cytokine) counteracted by IL-4 (a T_{H}2 cytokine), resulting in no net benefit.

As described herein, the synthetic α-GalCer analogs have use as anti-tumor immunotherapy active agents. The α-GalCer analogs may be designed such that they are T_{H}1-biased. These T_{H}1-biased α-GalCer analogs are capable of eliciting a T_{H}1 cytokine response, increasing survival time of animals afflicted with cancer, slowing down tumor growth in animals afflicted with cancer and increasing the tumor-infiltrating lymphocytes, including T, CD8T, NK and NKT cells.

As described herein, the α-GalCer analogs act as therapeutic drugs in anti-tumor immunotherapy. The α-GalCer analogs may be administered as cancer vaccines. As described herein, the α-GalCer analogs may be used in combined immunotherapy, where the α-GalCer analogs are combined with an already existing cancer vaccine. A subject treated with any of the α-GalCer analogs may be afflicted with cancer, may be at an elevated risk for cancer or may have precancerous precursors.

Also described herein is an anti-tumor immunotherapy comprising administering an effective amount of a compound or a salt or a mixture thereof to a subject, the compound selected from the group consisting of C3, C10-C17, C19-C28, C34 (C34 being of the present invention) and C8-5.

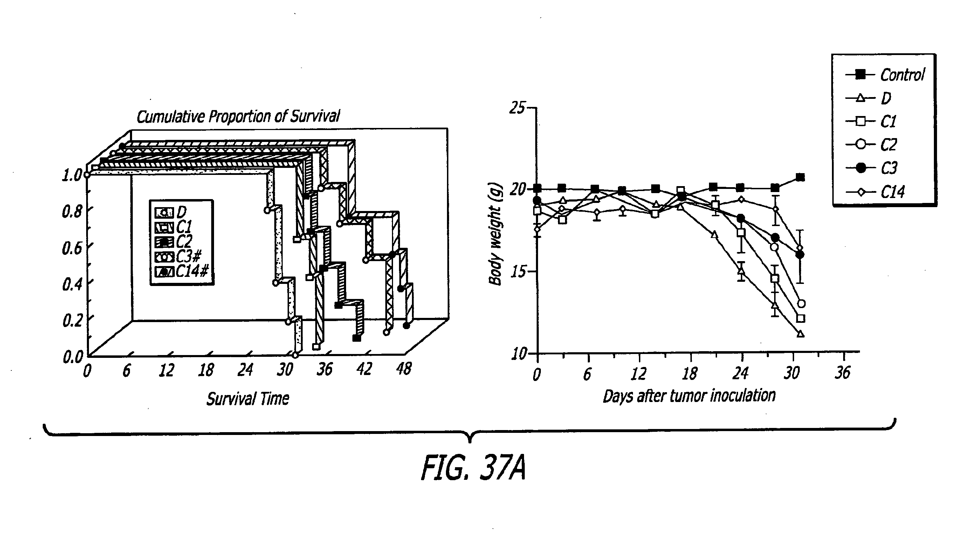

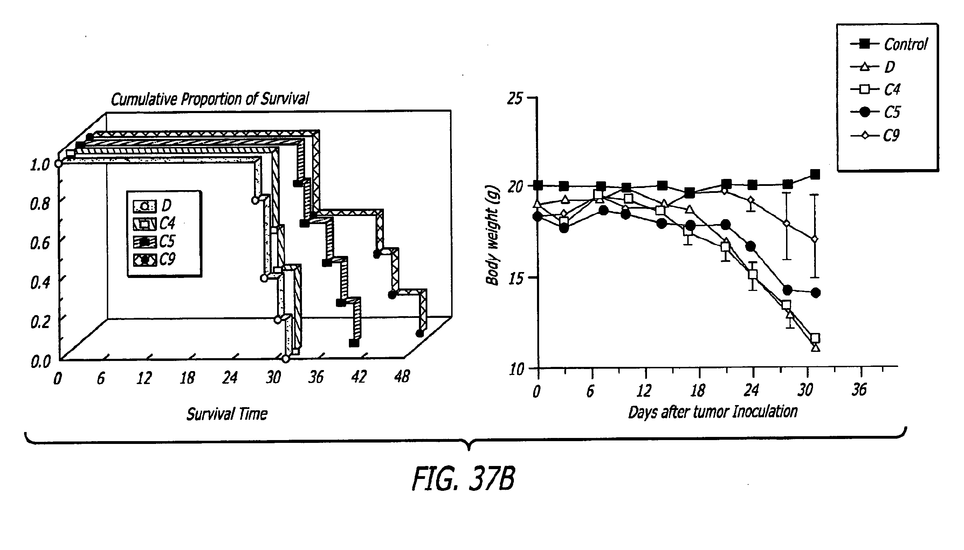

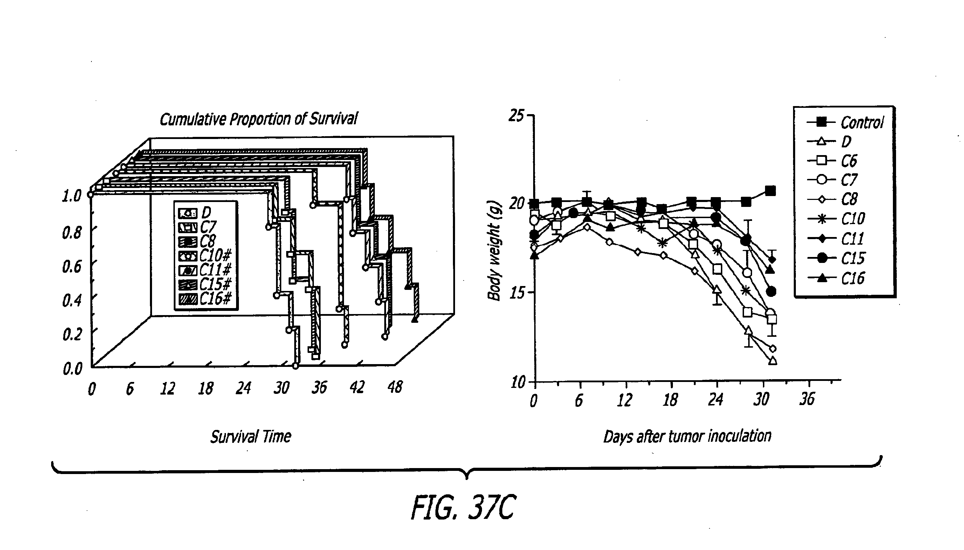

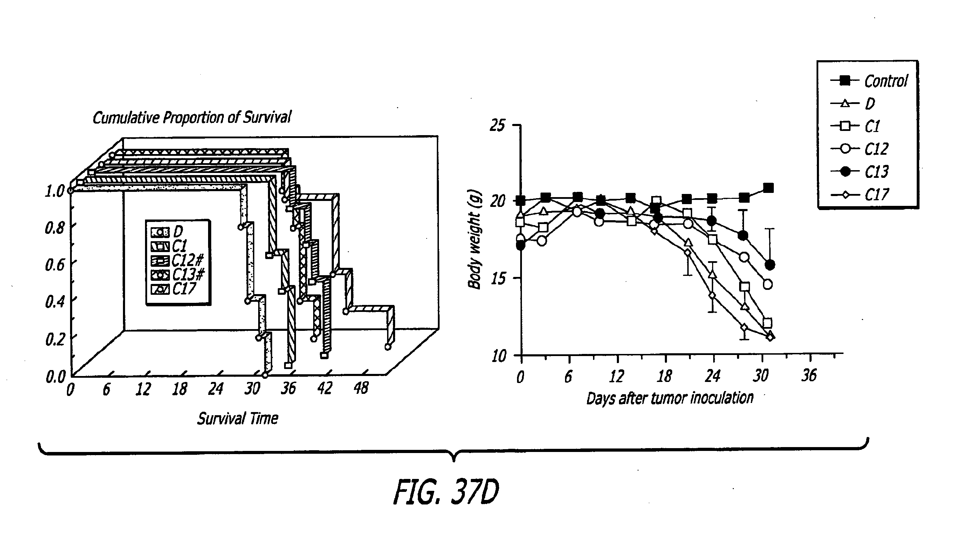

In order to determine the anticancer efficacy of the α-GalCer analogs, mouse models of metastatic lung cancer with TC1 cell line, and SubQ tumor model of breast cancer with 4T1 cell line in syngeneic immunocompetent mice (C57BL/6 and BALB/c, respectively) were studied. Figure 38A shows the result of a representative experiment with reduced number of tumor nodules on the lung surface of mice treated with α-GalCer analog C11. The effects of IV administration of various α-GalCer analogs from groups I-IV and C1 on the survival of TC1 tumor-bearing mice are shown in Figure 37. Significant prolongation of survival and reduced weight loss were observed with many of the α-GalCer analogs tested, except for C4, C6, C7, C8 and C17. Moreover, eight of the α-GalCer analogs tested, C3, C10, C11, C12, C13, C14, C15 and C16, have significantly greater anticancer efficacy than C1. Next, the anti-tumor efficacy of eight α-GalCer analogs and C1 administered IV on mice bearing 4T1 breast cancer was assessed. The reduced tumor size of mice 16 days after treatment with α-GalCer analog C11 is shown in Figure 38B as an example. All of the α-GalCer analogs tested were able to suppress tumor growth and prolong survival as compared to the control, and all were more effective than C1, Figure 39A. Based on these findings, the effect of the SubQ delivery of some of the most active α-GalCer analogs (C9, C11, C13, C14, C16) and C1 were tested. SubQ delivery of the α-GalCer analogs tested were able to suppress tumor growth and prolong survival as compared to control. α-GalCer analogs C13, C14 and C16 achieved significantly greater suppression of tumor size than C1, although their effects on survival did not differ significantly from that of C1 (Figure 39B). C1 showed a statistically better efficacy with SubQ delivery over IV route (Figure 40), whereas the route of administration did not significantly affect the anti-tumor effects of the remaining α-GalCer analogs tested (Figure 39A-B). Mice receiving a SubQ injection of α-GalCer analogs appeared to be less morbid than those treated IV, which is consistent with lower serum levels of cytokines/chemokines following SubQ administration.

In order to optimize the therapeutic protocol of these α-GalCer analogs, we assessed the anticancer efficacy in tumor-bearing mice, with special focus on the routes, frequency, and dosage of administration (see Figure 41-44). The results showed optimal dose schedule to be IV adminstration of 0.1 µg α-GalCer per mice, once per week., This is applicable to the treatment of mice bearing breast and lung cancer, as well as melanoma (see, Figures 43 and 44). Treatment with α-GalCer analogs led to an increase in the tumor-infiltrating lymphocytes, including T, CD8T, NK, and NKT (see, Figure 45). Figure 41A-B, show the impacts of different routes of administration. (A) BALB/c mice were SubQ inoculated with mouse breast cancer cells, 4T-1. Three days after tumor inoculation, the mice were treated (IV or SubQ) with vehicle, α-GalCer or the indicated α-GalCer analogs (2 µg per mouse) twice per week for four weeks. The tumor volume was recorded every 3 days for 33 days and survival was monitored for up to 70 days. *Left panel,* Kaplan Meier survival curve of mice bearing breast cancer; *right panel,* tumor growth curve. (B) C57BL/6 mice were IV inoculated with mouse lung cancer cells, TC-1, and then treated (IV or SubQ) with vehicle, α-GalCer or the indicated α-GalCer analogs (2 µg per mouse) twice per week for four weeks. *Left panel,* Kaplan Meier survival curve of mice bearing lung cancer; right panel, changes of body weight.

(C) shows the impacts of frequency of administration. C57BL/6 mice were IV inoculated with mouse lung cancer cells, TC-1, and then treated (IV or SubQ) with vehicle, α-GalCer or the indicated α-GalCer analogs (2 µg per mouse) twice per week or once per week for four weeks. *Left panel,* Kaplan Meier survival curve of mice bearing lung cancer, *right panel,* changes of body weight

Figures 43 and 44 show the evaluation of the anticancer efficacy of a-GalCer analogs with the optimized protocol. Figure 43 shows C57BL/6 mice were inoculated with lung cancer (TC1) IV or with melanoma (B16) cells SubQ, and then treated IV (0.1 µg per mouse) with vehicle, α-GalCer or the indicated α-GalCer analogs (C23, C26, C34 (C34 being of the present invention), 7DW8-5) once per week for four weeks. (A) shows the Kaplan Meier survival curve of mice bearing TC1, (B) shows growth curves of B16 tumor. All of the α-GalCer analogs tested showed a significant increase in the survival time of mice bearing TC1. Also, when mice bearing B16 were treated with the α-GalCer analogs, there was a significant decrease in the size of the tumors. Figure 44(A-B) show the real time assessment of tumor growth in mice. C57BL16 mice were SubQ inoculated with (A) lung cancer (TC1-GFP-Luciferase) or (B) breast cancer (4T1-GFP-Luciferase) cells, and then treated IV (0.1 µg per mouse) with vehicle, α-GalCer or the indicated α-GalCer analogs (C23, C34 (C34 being of the present invention), 7DW8-5 and C17) once per week for four weeks. The pixel of the bioluminescence of the tumor *in vivo* was assessed and calculated by IVIS system. *Left panel,* the quantitative data of bioluminescence; *Right panel,* the representative images of mice bearing tumor. *, p < 0.05, compared with DMSO; #, p < 0.05, compared with C1. In mice inoculated with lung cancer, the α-GalCer analogs C34 (C34 being of the present invention), C23 and C8-5 showed a significant decrease in tumor growth compared with both control and α-GalCer. Interestingly, these α-GalCer analogs, C34 (C34 being of the present invention), C23 and C8-5, all have been shown to produce a T_{H}1-biased response, as shown in the results above. In mice inoculated with breast cancer, the α-GalCer analog C8-5 showed a significant decrease in tumor growth compared with both control and α-GalCer. The α-GalCer analog C17 showed a significant decrease in tumor growth compared with control, but had a similar result to α-GalCer. Interestingly, the α-GalCer analog C17, has been shown to produce a T_{H}2-biased response, as shown in the results above. These results confirm the idea that the production of T_{H}1 cytokines are thought to correlate with antitumor activities.

Figure 45 shows in an exemplary implementation, how the α-GalCer analogs elicit T_{H}1-biased tumor infiltrating lymphocytes in lung and melanoma tumors. (A-D) show tumor infiltrating lymphocytes in lung cancer. Single cell suspensions of tumors removed on day 21 from the C57BL/6 mice bearing TC1 tumor treated with vehicle, α-GalCer or the indicated α-GalCer analogs (C23, C34 (C34 being of the present invention), C8-5; 0.1 µg /mouse, once/week) were stained for (A) CD3⁺ T cell, (B) CD8 T cells (CD3⁺/CD4/CD8⁺), (C) NKs (NK1.1⁺/CD3) and (D) NKTs (NK1.1⁺/CD3⁺), normalized to DMSO. The α-GalCer analog C34 (of the present invention), showed a significantly significant increase in the number of T_{H}1-biased tumor infiltrating lymphocytes in lung cancer, as compared with both control and α-GalCer. The α-GalCer analogs C23 and C8-5 also showed a significantly significant increase in the number of tumor infiltrating lymphocytes in lung cancer, as compared with control (for CD3⁺ T cells) and as compared with both control and α-GalCer (for CD8 T cells, NKs and NKTs). (E-H) show tumor infiltrating lymphocytes in melanoma. Single cell suspensions of tumors removed on day 21 from C57BL/6 mice bearing B16 melanoma treated with the vehicle, α-GalCer or the indicated α-GalCer analogs (C23, C34, C8-5; 0.1 µg/mouse, once/week), were stained for (E) CD3⁺ T cell, (F) CD8 T cells (CD3⁺/CD4⁻/CD8⁺), (G) NKs (NK1.1⁺/CD3⁻) and (H) NKTs (NK1.1⁺/CD3⁺) and normalized to DMSO. The α-GalCer analogs C23, C8-5 and C34 (C34 being of the present invention), all showed a significantly significant increase in the number of T_{H}1-biased tumor infiltrating lymphocytes in melanoma, as compared with both control and α-GalCer. *, p < 0.05, compared with DMSO; #, p < 0.05, compared with C1.

### ADJUVANT IMMUNOTHERAPY

### Adjuvant Effects on Peptide, Protein, Polysaccharide arid DNA Immunogens

Adjuvants are compounds that, when combined with an antigen, potentiate an immune response in an immunized species. For over eighty years, adjuvants have been used to boost the effectiveness of vaccines. Live vaccines, containing weakened forms of an infectious organism, generally work fine by themselves. But vaccines containing dead organisms (inactivated vaccines) or pieces of the infectious organisms or their toxins (acellular or recombinant vaccines) generally need adjuvants to boost their effectiveness. In most situations, the type of response induced (type 1 or type 2) has a significant impact on the protective efficacy of the vaccine. Alternative adjuvants tend to favor specific types of responses. However, adjuvant selection is complicated by functional unpredictabilities and also by commercial constraints and availability.

Aluminum salts, known as alum, are the only adjuvant approved for use in the United States for routine preventive vaccines. However, aluminum salts have been shown to increase in humans, as well as in animals, exclusively a shift to T_{H}2-type responses (e.g., IL-4 production). The inability of aluminum salts to elicit a T_{H}1 cell-mediated immune responses (e.g., IFN-γ production) is a major limitation of its use as adjuvant. Particularly for vaccines against intracellular viral and bacterial infections, the lack of cytotoxic T cell responses is fatal.

The α-GalCer analogs may be synthesized such that a T_{H}1 biased immunogenic response is initiated. Therefore, improved vaccines which show a T_{H}1-type directed immune response or vaccines which allow-in addition to a T_{H}2-type response-also a T_{H}1-type shift of the immune reaction may be achieved using the α-GalCer analogs as adjuvants. As such, one or more α-GalCer analogs are administered as an adjuvant in conjunction with administration of a vaccine. Moreover, vaccines already available can be provided in an improved form, when the α-GalCer analogs are added to them, which allows the induction of a T_{H}1-type response.

Also described herein is a vaccine comprising an effective amount of a compound or a salt or a mixture thereof selected from the group consisting of C3, C11, C13-C14, C16-C18, C20, C22-C24, C26, C8-5 and C8-6; and a vaccine agent. In some instances the vaccine agent is selected from the group consisting of a killed microorganism, a live attenuated virus microorganism, a toxoid and a fragment of an inactivated or attenuated microorganism. In some instances the microorganism is a bacteria or a fungi. In some instances the toxoid is a tetanus or a diphtheria. In some instances the vaccine agent is capable of eliciting an immune response in a subject that is administered the vaccine. In some instances the compound acts as an immunologic adjuvant and is capable of modifying or augmenting the immune response elicited by the vaccine agent by stimulating the immune system which results in the subject responding to the vaccine more vigorously than without the compound.

In one aspect, appropriate vaccines may comprise peptide, protein, polysaccharide or DNA immunogens. In another aspect, the vaccine may be selected from one or more commercially available vaccines, such as, but not limited to, vaccines for Hepatitis A, Hepatitis B, Rotavirus, Diptheria, Tetanus, Pertussis, Haemophilus influenza type b, Pneumococcal, Poliovirus, Influenza, Measles, Mumps, Rubella, Varicella, Meningiococcal, Human Papillomavirus, Herpes Zoster, Borrelia burgdorferi, Typhoid, Japanese encephalitis, Rabies, Tick Borne encephalitis, Cholera, Yellow Fever, H5N1, West Nile, Parvovirus, Feline Rhinotracheitis, Calicivirus, Panleukopenia virus, Chiamydia psittaci, Feline leukemia, Canine Distemper, Canine Adenovirus, Canine Parainfluenza, Bordetella Bronchiseptica, Canine Coronavirus, Giardia lamblia, Leptospira bacterin, Infectious Bovine Rhinotracheitis virus, Parainfluenza 3 virus, Bovine Repiratory Syncytial virus, Bovine Viral Diarrhea virus, Clostridium Chauvoei, Septicum Haemolyticum, Septicum Novyi, Tetani, Sordellii Perfringens, Moraxella bovis, Mannheimia haemolytica, Pateurella multocida, Leptospira pomona, Leptospira hardjo, Leptospira grippotyphosa, Leptospira canicola, and Leptospira icterohaemorrhagiae.

Also described is a method for enhancing immunogenicity of a compound, composition, or vaccine in a subject, the method including: administering to the subject a compound, composition or vaccine further comprising an adjuvant according to the present disclosure, wherein the adjuvant enhances the immunogenicity of the compound, composition or vaccine.

### Adjuvant Effect on Protein Vaccines

α-GalCer and the α-GalCer analogs were tested for the ability to enhance immune responses to existing protein based vaccine such as tetanus toxoid (TT) inactivated toxin. Mice were vaccinated TT without or with α-GalCer analogs on day 0 and day 28. Serum was harvested weekly for determination of anti-TT-specific antibodies. Figure 46A shows adjuvant effects of α-GalCer analogs on antibody response to TT. As shown in Figure 46A, production of anti-TT-specific IgG antibody was enhanced by α-GalCer (C1) and the α-GalCer analog C11. Although the kinetics of anti-TT production was similar to that induced by conventional adjuvant alum ("Alum"), C1 elicited significantly greater antibody production than Alum. When the conventional TT + Alum was combined with C1 or C11, the antibody response was further augmented to ∼2 fold of conventional vaccine. These findings indicate that C1 and C11 had adjuvant effects which are synergistic with Alum to further augment immune responses. The adjuvant effects of the α-GalCer analog C11 were remarkably durable. Twenty weeks after the second Immunization, a booster dose of TT alone (without Alum or α-GalCer analog C11) in mice led to a rapid rise of anti-TT antibody 1 week later. Figure 46B shows the effects of α-GalCer analog C11 on delayed antigen boost twenty weeks after the second vaccination. The level of antibody in mice treated with C1 or C11 was twice as high as those given TT + Alum, and more than 25 fold higher than those injected with TT only as shown in Figure 46B. These findings suggested that C1 or the α-GalCer analog C11 have effects on the memory T and B cells leading to an augmented booster immune response.

### Adjuvant Effect on Peptide Vaccines

The adjuvant effects were evaluated with peptide vaccine containing the extracellular domain of the M2 protein of the H1N1 subtype of the Influenza A virus. The amino acid sequence of the peptide vaccine was MSLLTEVETPIRNEWGCRCN. Female BALB/c mice were vaccinated with 5 or 45 µg of M2e peptide without or with various α-GalCer analogs (C9, C11, C14, C17) on week 0, 3, and 6. Figure 47 shows adjuvant effects of various α-GalCer analogs on M2e peptide vaccine. As shown in Figure 47, two weeks after the third immunization, the M2e peptide alone induced anti-M2e-specific IgG titer of 1.8 x 10⁵ and 5.4 x 10⁵ for 5 and 45 µg antigen dosage, respectively. When combined with α-GalCer analogs, 10-30 fold higher anti-M2 antibody titers were obtained. Among the α-GalCer analogs tested, C11 had the best adjuvant effect which was equivalent to complete Freund's adjuvant (CFA) but 3 fold higher titer than C1. The remaining α-GalCer analogs (09, C14 and 017) were equivalent to C1. These findings suggest that α-GalCer and its analogs have strong adjuvant activities for peptide antigens with those containing aromatic ring in the acyl tail such as C11 being most potent.

### Adjuvant Effect on DNA Vaccines

An H5 DNA construct (pHA) was prepared as a plasmid containing full length H5 consensus sequence of avian influenza viruses. Briefly, in order to cover the genetic variability and thus induce cross-protection across different H5N1 strains, a consensus HA sequence was deduced from HA gene of 500 H5N1 virus strains and used for a vaccine development effort. The consensus sequences of HA were constructed into a pVAX vector as DNA vaccine candidates, based on a similar strategy for ADVAX, a DNA vaccine for HIV, developed by Ho et al. (Jin et al., (2002) J. Virol. 76 (5):2306-2216). The effects of H5 DNA vaccine (pHA) dosage without and with α-GalCer (C1) on anti-H5 titers in mice at three weeks after first immunization are shown in Figure 48A. Immunization of mice with 5-45 µg H5 DNA vaccine without or with α-GalCer showed that the anti-H5 responses were enhanced by α-GalCer at 5-30 µg H5 DNA, but reached a plateau at 45 µg. Figure 48B shows the effects of low dose H5 DNA vaccine and α-GalCer (C1) on anti-H5 titers two weeks after second immunization. When H5 DNA dose was reduced to 0.2-5 µg, the adjuvant effect of α-GalCer was evident for all low dosages tested. Figure 480 shows protection against viral challenge by Vietnam reassortant influenza strain NIBRG-14 two weeks after low dose H5 DNA vaccine without or with C1. None of the animals treated with <2 µg survived viral challenges with 20 LD₅₀ of NIBRG-14 without α-GalCer, while 80% protection was noted among those treated with 0.2 to 1 µg pHA with α-GalCer (Figure 48C). These findings confirm the adjuvant effects of α-GalCer when used with low dose pHA vaccine on induction of protective immunity against NIBRG-14.

Other α-GalCer analogs were also tested as adjuvants with the pHA vaccine in mice with a similar protocol and schedule as used above, differences are noted. 6-7 week old female BALB/C mice were vaccinated by electrotransfer in muscle with α-GalCer or the indicated α-GalCer analogs with pHAc and boosted once with the same formulation four weeks later. Blood samples were collected at 2 weeks after the second vaccination and tested for anti-HAc-speafic IgG antibody titers by ELISA. Figure 49A shows titers of anti-HA specific IgG antibody (AY3) in mice following immunization with 0.2 µg pHA without or with α-GalCer or α-GalCer analog C3, C11, C13, C14 and C16. Figure 49B shows titers of anti-HA specific IgG antibody (AY4) in mice following immunization with 0.2 µg pHA without or with α-GalCer or α-GalCer analog C10, C13, C18, C19 and C20. Figure 49C shows percent mouse survival following viral challenge as above for some of the α-GalCer analogs tested. Figure 50A shows anti-HA specific IgG antibody (AY4) following immunization with 0.5 µg pHA and indicated α-GalCer analogs. Figure 50B shows percent survival following viral challenge as described above. Figure 51 shows mouse titer of anti-HA specific IgG antibody (AY5) following immunization with either (A) 0.1 µg pHA (pHA_{0.1} vs pHA_{0.1} + C26: p < 0.01 in one-way ANOVA Kruskal-Walls test) or (B) 0.2 µg pHA (pHA_{0.2} vs pHA_{0.2} + C17: p <0.01, pHA_{0.2} vs pHA_{0.2} + C26: p < 0.05 in one-way ANOVA Kruskal-Walis test) and the indicated α-GalCer analog. Figure 52 shows mouse titers of anti-HA specific IgG antibody (AY6) following immunization with either (A) 0.1 µg pHA or (B) 0.2 µg pHA and the indicated α-GalCer analog at 0.1 µg or 1 µg. α-GalCer analog particularly effective as adjuvants at 0.2 µg pHA dose were C13, C17, C20 and C26.

Figure 53 shows mouse titers of anti-HAc specific IgG antibody (A) AY3. (B) AY4, (C) AY5 and (D) AY15 following immunization with 0.2 µg pHAc and α-GalCer or the indicated α-GalCer analog C3, C10, C11, C13, C14, C16, C17, C18, C19, C20, C23, C24, C26, 7DW8-5, and alum. The results indicate that C1, C13, C14, C17, C26 and 7DW8-5 had the better adjuvant activities than the others in enhancing the antibody titer. To investigate whether the HA specific CD8 T cell response would be enhanced by the use of an α-GalCer analog as an adjuvant, C1, C26 and 7DW8-5 were assessed further. As shown in Figure 54, the IFN-γ secreting cells increased in α-GalCer analog -adjuvanted groups. Furthermore, after NIBRG-14 virus challenge, the survival percentage of C1, C26 and 7DW8-5 adjuvanted groups were higher than alum-adjuvanted or pHA only groups (Figure 55).

The adjuvant effects of α-GalCer analogs was also evident after single dose of pHA vaccination. At three weeks after one dose immunization, anti-HA-specific IgG antibody was enhanced in mice treated with C26 and C1 as adjuvant (Figure 56). Mice treated with C1, C26 or 7DW8-5 were protected effectively from lethal challenge by NIBRG-14 virus challenge, with the survival rates ranged from 87.5% to 100% These findings indicate that C1, C26 and 7DW8-5 have good adjuvant activities in the setting of single vaccination procedure.

### Adjuvant Effect on Polysaccharide Immunogens

Globo H, a hexasaccharide (Fucα1 → 2Galβ1- 3GalNAcβ1 → 3Galα1 → 4Galβ1 → 4Glcβ1) had been shown to be overexpressed on a variety of epithelial cell tumors such as colon, ovarian, gastric, pancreatic, endometrial, lung, prostate and breast cancers, with the use of monoclonal antibodies MBr1 (IgM) and VK-9 (IgG3). In normal tissues, globo H is limited to the apical surface of epithelial cells at the lumen border, a site that appears not to be accessible to the immune system. Therefore, globo H is an ideal target antigen for immunotherapy of breast cancer and other epithelial cancers.

The adjuvant effects of α-GalCer and the α-GalCer analogs C23 and 7DW8-5, were evaluated for globo H conjugated to diphtheria toxoid (GH-DT) vaccine. BALB/c mice were injected IM with globo H-DT/α-GalCer or globo H-DT/α-GalCer analogs three times at two weeks interval. Sera was collected two weeks after the third vaccination and tested for IgG and IgM anti-globo H-specific antibody at 1:480 and 1:240 dilution, respectively, using a glycan microarray. As shown in Figure 57A, GH-DT alone did not induce any anti-globo H antibody, but the addition of C1 or 7DW8-5 elicited significant IgG antibody production. On the other hand, the production of 1gM was observed only in 7DW8-5-adjuvanted groups but not in C1 treated group (Figure 57B). In conclusion, adding C1 or 7DW8-5 into GH-DT vaccine could enhance specific antibody production against carbohydrate antigen.

### ANTIMICROBIAL IMMUNOTHERAPY

As described herein, an α-GalCer analog has use, for example, in treatment methods for infectious diseases resulting, for example, from the presence of pathogenic microbial agents, including viruses, bacteria, fungi, protozoa, multicellular parasites, and aberrant proteins (prions).

Described herein is an anti-microbial immunotherapy for a subject comprising: administering an effective amount of a compound or a salt or a mixture thereof to a subject, the compound selected from the group consisting of C9, C11, C13-C16, C23 and C34 (C34 being of the present invention).

### Antiviral Effects:

Antiviral drugs are a class of medication used specifically for treating viral infections. Like antibiotics, specific antivirals are used for specific viruses. They are relatively harmless to the host, and therefore can be used to treat infections. Antiviral drugs are available to treat only a few viral diseases. Two useful antivirals are: the nucleoside analogues and the interferons. There are three classes of interferons: *alpha-beta-*and *gamma-interferons.* The alpha and beta interferons are cytokines which are secreted by virus infected cells. They bind to specific receptors on adjacent cells and protect them from infection by viruses. They form part of the immediate protective host response to invasion by viruses. In addition to these direct antiviral effects, alpha and beta interferon also enhance the expression of class I and class II MHC molecules on the surface of infected cells, in this way, enhancing the presentation of viral antigens to specific immune cells. Their presence can be demonstrated in body fluids during the acute phase of virus infection. Recombinant alpha and beta interferons are now available and have been used for the treatment of Chronic hepatitis B and C virus infections. However, side effects such as fever, malaise and weight loss have limited the use. Gamma Interferon (immune interferon) is a cytokine secreted by TH1 CD4 cells. Its function is to enhance specific T cell mediated immune responses.

The mechanism of action of the interferons include: 1) enhancement of the specific immune response. By increasing the expression of MHC class I molecules on the surface of infected cells, the interferons increase the opportunity for specific cytotoxic T cells to recognise and kill infected cells; and 2) Direct antiviral effect: a) degradation of viral mRNA and b) inhibition of protein synthesis, which prevents the infection of new cells.

In one aspect, the synthetic α-GalCer analogs have use for antiviral treatment of and prophylaxis for various infectious viruses. Examples of infectious virus to which stimulation of a protective immune response is desirable, which may be accomplished utilizing the NKTs, vaccines or compositions of the present disclosure include, but are not limited to, Retroviridae (e.g., human immunodeficiency viruses, such as HIV-1 (also referred to as HTLV-III, LAV or HTLV-III/LAV, or HIV-III; and other isolates, such as HIV-LP; Picomaviridae (e.g., polio viruses, hepatitis A virus; enteroviruses, human coxsackie viruses, rhinoviruses, echoviruses); Calciviridae (e.g., strains that cause gastroenteritis); Togaviridae (e.g., equine encephalitis viruses, rubella viruses); Flaviridae (e.g., dengue viruses, encephalitis viruses, yellow fever viruses); Coronaviridae (e.g., coronaviruses); Rhabdoviridae (e.g., vesicular stomatitis viruses, rabies viruses); Filoviridae (e.g., ebola viruses); Paramyxoviridae (e.g., parainfluenza viruses, mumps virus, measles virus, respiratory syncytial virus); Orthomyxoviridae (e.g. influenza viruses); B ungaviridae (e.g., Hantaan viruses, bunga viruses, phleboviruses and Nairo viruses); Arena viridae (hemorrhagic fever viruses); Reoviridae (erg., reoviruses, orbiviurses and rotaviruses); Bimaviridae; Hepadnaviridae (Hepatitis B virus); Parvoviridae (parvoviruses); Papovaviridae (papilloma viruses, polyoma viruses); Adenoviridae (most adenoviruses); Herpesviridae (herpes simplex virus (HSV) I and 2, varicella zoster virus, cytomegalovirus (CMV), herpes viruses); Poxviridae (variola viruses, vaccinia viruses, pox viruses); and Iridoviridae (e.g. African swine fever virus); and unclassified viruses (e.g., the etiological agents of Spongiform encephalopathies, the agent of delta hepatities (thought to be a defective satellite of hepatitis B virus), the agents of non-A, non-B hepatitis (class 1= internally transmitted; class 2=parenterally transmitted (i.e., Hepatitis C); Norwalk and related viruses, and astroviruses).

### Viral Challenge - Influenza Virus H1N1 Infection

### Treatment via IP Injection of ct-GalCer Analogs

Figure 58 shows mouse survival at 0 to 12 days post influenza virus H1N1 infection. Mice were treated (IP injection) with 2 µg of α-GalCer (C1) or the α-GalCer analogs C2, C3, C9, C11, C13, C14 and C16, and compared to control DMSO. Three different treatment schedules were tested. Figure 58A shows survival rate when BALB/c mice were treated starting at 30 minutes post- H1 N1 virus challenge. P values compared to control were C1: 0.4554, C2: 0.5149, C3: 0.5764, C9: 0.5466, C11 0.2031, C16: 0.0359. Figure 58B shows survival rate when BALB/c mice were treated starting at two weeks prior to virus challenge with H1N1 (WSN). Mice were treated at -14 days, -10 days, -3 days, 0.5 hour, 2 days, 4 days, 6 days 8 days 10 days and 12 days with 2 µg (IP injection) of control, α-GalCer (C1) or the α-GalCer analogs. When treatment started two weeks before virus challenge and was given two times per week, mice exhibited significantly enhanced survival with α-GalCer analog treatment with all analogs tested (C9, C11, C13 and C14). P values compared to control were C1: 0.000116, C9: 0.000126, C11: 0.02627, C13: 0.000027, and C14: 0.000147. Figure 59 shows cumulative proportion of survival with mice that were infected with a higher dose of influenza virus H1N1. In Figure 59A, BALB/c mice were treated starting at two weeks prior to virus challenge with H1N1 (WSN). Mice were treated at -14 days, -10 days, -3 days, 0.5 h, 2 days, 4 days, and 6 days with 2 µg (IP injection) of control, α-GalCer (C1) or the α-GalCer analogs. Group 1 is the control group. Group 6 were treated with a-GalCer (C1). Group 7 were treated with α-GalCer analog C13. Group 8 were treated with α-GalCer analog C14. Group 9 were treated with α-GalCer analog C16. α-GalCer analog C16 showed prolonged survival, indicative of C16 having a direct anti-viral effect.

### Treatment via Intranasal Administration of α-GalCer Analogs

Figure 59B shows cumulative proportion of survival with mice infected with H1N1. BALB/c mice were treated via intranasal route with control, α-GalCer (C1) or the α-GalCer analogs C13, C14 or C16 at one hour prior to virus challenge with H1N1 (WSN). C13 showed prolonged survival, suggestive of direct anti-viral effects. In general, certain α-GalCer analogs may exert direct anti-viral effects, or act indirectly via immune stimulation. Figure 60 shows the cytopathetic effect (CPE) of Madin-Darby canine kidney (MDCK) cells *in vitro.* MDCK cells were pretreated with vehicle, α-GalCer or one of the α-GalCer analogs C13, C14 or C16 at 10 µg /ml for four hours, followed by infection with FLU-A virus serotype H1 N1 (WSN) at 10TCID50. The virus titer in MDCK cells was determined at 48 hours post-infection *(right panel).* α-GalCer, as well as the three α-GalCer analogs tested showed slight inhibition of the entry/replication of H1 N1 virus *in vitro.*

### Antibacterial Effects:

Since the introduction of penicillin into clinical use in the 1940s, antibacterials have saved millions of lives. However, the lengthening shadow of antimicrobial resistance threatens a return to the pre-antibiotic era. Synthetic glycolipids such as α-GalCer and natural bacterial glycolipids were demonstrated as CD1-d ligands that activated NKT cells and contributed the antibacterial functions of the hosts. The antilbacterial activities of α-GalCer were documented in the amelioration of mycobacterium tuberculosis infections, clearance of lung infection by Pseudomonas aeruginosa. Infections by Sphingomonas capsulate and Ehrlichia muris were also attenuated in mice by the activation of NKT cells via glycolipids.

Examples of infectious bacteria to which stimulation of a protective immune response is desirable, which may be accomplished utilizing the NKTs, vaccines or compositions of the present disclosure include, but are not limited to, Helicobacter pylon, Borellia burgdorferi, Legionella pneumophilia, Klebsiella Pneumoniae, Mycobacteria sps (e.g. M. tuberculosis, M. avium, M. intracellulare, M. kansaii, M. gordonae), Staphylococcus aureus, Neissenia gonorrhoeae, Neisseria meningitidis, Listeria monocytogenes, Streptococcus pyogenes (Group A Streptococcus), Streptococcus agalactiae (Group B Streptococcus), Streptococcus (viridans group), Streptococcus faecalis, Streptococcus bovis, Streptococcus (anaerobic sps.), Streptococcus pneumoniae, pathogenic Campylobactersp., Enterococcus sp., Chiamidia sp., Haemophilus influenzae, Bacillus antracis, corynebacterium diphtheriae, corynebacteriu m sp., Erysipelothrix rhusiopathiae, Clostridium perfringers, Clostridium tetani, Enterobacter aerogenes, Klebsiella pneumoniae, Pasturelia multocida, Bacteroides sp., Fusobacterium nucleatum, Streptobacillus moniliformis, Treponema pallidium, Treponema pertenue, Leptospira, Actinomyces israelli, Sphingomonas capsulata and Francisella tularensis.

### Enhanced Bacterial Clearance -Sphingomonas Capsulate Infected Mice

Sphingomonas capsulata is a common environmental bacterial strain that is found in many places such as the air and water. It can be easily identified on nutrient agar plates because of its yellow colony color. Unlike most Gram negative bacteria, Sphingomonas capsulata does not contain lipopolysaccharide (LPS) that is used by animals for the activation of the host antibacterial activities. Since the antibacterial activities of glycolipid antigens are mediated through the activation of NKT cells by glycolipid bourid-CD1-d molecules, evaluation of the antibacterial efficacies using the disease model of Sphingomonas capsulata infection will focus on the impact of the NKT mediated pathway that is activated by glycolipid bindings. Six to eight week old female C57BL/6 mice were injected IP with Sphingomonas capsulate cells. Four hours after the infection, mice were injected IP with control, α-GalCer (C1) or the α-GalCer analogs (C3, C9, C11, C14, C16 or C17) at 50 or 100 µg /kg. Twenty-four hours after bacterial infection, livers were removed from mice and homogenized. Colony formation units (CFU) of Sphingomonas capsulate in liver homogenates were determined by plating diluted samples on nutrient plates. Colonies were counted after incubation for 48 hours at 37°C. Figure 61A shows that the CFU numbers of the groups treated with α-GalCer and C11, C14, and C16 at 100 µg /kg, 24 hour after bacterial infections, are significantly lower than the control group. To confirm the antibacterial efficacies of these α-GalCer analogs, another study was conducted to repeat the study by treating infected mice with 50 µg /kg in the same disease model. Figure 61B shows that the antibacterial efficacies of mice treated with C11, C14, C16, and also C15 are significant in comparison to the untreated group. Among the three efficacious groups, C1, C11, and C15, the difference in the values of the CFU per gram liver is not statistically significant. Figure 63 shows that the CFU numbers (in lungs) of the groups treated with C23 and C34 (C34 being of the present invention) at 50 µg/kg, are significant in comparison to the untreated group. Similar results were found in the CFU numbers in livers after mice were treated with C23 and C34.

### Enhanced Bacterial Clearance -Klebsiella Pneumoniae Infected Mice

K. pneumoniae is a Gram negative bacterium that causes liver abscess and is becoming a serious disease in Taiwan among diabetic patients. Figure 62 shows that both C1 and C14 can significantly reduce the bacterial loads in mouse lung and liver after injection. BALB/cByl female mice were administered a single dose of live K. pneumoniae by oral gavage. Mice were injected with control, α-GalCer or the α-GalCer analog C14 at 100 µg /kg twice at 4-hour and 8-hour after bacterial infection. Twenty four hours after infection, both the liver and lungs were collected from each mouse, and homogenized. Bacterial counts were determined similarly as described above.

The extent of bacterial clearance by C14 is found to be greater than the clearance by C1 as shown in Figure 62.

### Antifungal Effects:

T helper cell type 1 (T_{H}1) cell-mediated immunity plays a critical role in protection against various infectious fungi. In still another aspect, the α-GalCer analogs may be used in antifungal therapies. Antifungal drugs are used to treat infections caused by fungus and to prevent the development of fungal infections in patients with weakened immune systems. Fungal infections have become one of the leading factors contributing to morbidity and mortality in immunosuppressed patients.

The innate host defense against fungal diseases is based on the action of phagocytic cells (PMNLs and macrophages); both the number and the function of these cells can be regulated by the colony-stimulating factors (CSFs). On the other hand, acquired defense involves cellular and humoral immunity that requires interactions between antigen-presenting cells, T lymphocytes, B lymphocytes, and NKs that are driven and regulated by cytokines such as IL-2 and IFN-γ. The potential importance of immune activation via cytokines in the host defense against opportunistic fungi has been the subject of several studies and has raised some intriguing questions about novel antifungal strategies for candida and aspergillus infections. Different potential roles for cytokines have been described. First, exposure to fungi and their antigens may induce release of IL-2, IFN-γ, tumor necrosis factor-α (TNF-α), granulocyte colony-stimulating factor (G-CSF), and granulocyte macrophage colony-stimulating factor (GM-CSF). These cytokines may in turn activate or enhance the antifungal function of phagocytes against Candida and *Aspergilus* species.

Examples of infectious fungi to which stimulation of a protective immune response is desirable, which may be accomplished by administering an α-GalCer analog alone or in combination with an antifungal drug include, but are not limited to, Cryptococcus neoformans, Histoplasma capsulatum, Coccidioides immitis, Blastomyces dermatitidis,Chiamydia trachomatis, Candida albicans. Other infectious organisms (i.e., protists) include: Plasmodium sp., Leishmania sp., Schistosoma sp. and Toxoplasma sp.

### SYNTHETIC α-GALACTOSYL CERAMIDE ANALOGS SUPPRESSING BACTERIAL AND VIRAL INFECTIONS IN MURINE DISEASE MODELS

Natural killer T cells contribute to a variety of immunological process through the recognition of lipid and glycolipid antigens presented to CD1-d molecules. CD1-d are major histocompatibility complex class I like proteins and are expressed in most monocytes, macrophages, dendritic cells, B cells, as well as nonlymphoid cells. CD1-d presents glycolipids antigens to CD1d-restricted T cells (or NKT cells) that are implicated in the host innate defense system through the production of Th1 and Th2 types of cytokines, such as IFN-γ and IL-4. Among the variety of ligand that binds CD1-d, the most well-studied ligand is alpha-Galactosyl Ceramide (α-GalCer) that is the synthetic, structurally optimized α-linked glycosphingolipid from the marine sponge, *Agelas mauritianus.* Synthetic glycolipids such as alpha-galactosyl ceramide (α-GalCer) and natural bacterial glycolipids were reported as CD1-d ligands that activated NKT cells. Mice treated with α-GalCer launched the protection of a variety of infections. Many α-GalCer analogs were synthesized. Their immune modulating activities were shown to be related to the binding to CD1-d. Recent studies using CD1-d array binding established that the secretions of IFN-γ and IL-4 by NKT cells are determined by the binding constants of the α-GalCer analogs to the CD1-d binding pocket, and the ratios of the Th1 and Th2 cytokines secreted are one of the dominate factors for the immune modulating properties of these molecules.

The efficacies of some of the synthetic glycolipids for the protection of both bacterial and viral infections were determined using several murine infectious disease models. Among all of the tested synthetic α-GalCer analogs, the 4-(4 fluorophenoxy)phenyl octanoyl modified α-GalCer **(C34 -** being of the present invention) is most active in the protection against the bacterial and viral infections in murine models. The protective effects of C34 are most effective when given in a prophylactic manner or shortly after infections.

### Antibacterial efficacy of α-GalCer analogs using Sphingomonas capsulata infected mice

The *Sphingomonas capsulata* infected mice were used as the infection model to evaluate the antibacterial efficacy of some of the synthetic glycolipids. S. *capsulata* is a common environmental bacterial strain that is found in many places such as air arid water. It can be easily identified on nutrient agar plates because of its yellow colony color. Unlike most Gram negative bacteria, *Sphingomonas capsulata* does not contain lipopolysaccharide (LPS) that is used by animals for the activation of the host antibacterial activities. The antibacterial activities of glycolipids are mediated through the activation of NKT cells by glycolipid bound-CD1-d molecules, evaluation of the antibacterial efficacies using the disease model of *Sphingomonas capsulata* infection will focus on the impact of the NKT mediated pathway activated by glycolipid bindings. Figure 65 shows that the CFU numbers of the groups treated with 100 µg/kg C1, C11, C14, or C16 at 24 hour after bacterial infections are significantly lower than the control group. In contrast, the CFU differences of the groups treated with C3, C9 and C17 are not significant in comparison to the CFU found among mice in the control group. Additional antibacterial efficacy studies of these glycolipids were conducted to treat infected mice with 50 µg /kg glycolipids in the same disease model. Significantly reduced CFU values were also observed in the livers of mice treated with C1, C11, C14, and C16 in comparison to the untreated group (data not shown). α-GalCer appeared to be more potent in suppressing S. *capsulate* in vivo than C11, C14 and C16; even though the differences are not significant.

The production of Th1 cytokines is thought to correlate with the anti-tumor, anti-bacterial and anti-viral effects of glycolipids while the ability to induce the Th2 cytokines is thought to correlate with the amelioration of certain autoimmune diseases, such as type 1 diabetes. Many α-GalCer analogs were synthesized recently, and their cytokine induction profiles were characterized in vitro or in vivo. The anti-bacterial and anti-viral properties of α-GalCer and three analogs, 7DW8-5, C23, and C34 (C34 being of the present invention) that are known to induce higher levels of IFN-γ secretions in NKT cells were studied. These four glycolipids were first evaluated using the S. *capsulata* infection model in C57/b mice. The table of Figure 72 shows that all four glycolipids at 50 µg /kg are able to suppress S. *capsulata* infection to greater extents than vehicle treated mice with p values small than 0.001. However, among the four glycolipids, their efficacies were not significantly different in the *S*. *capsulate* infection model.

### Antibacterial activities of α-GalCer analogs in the thigh-wound mouse model using luminescence Staphylococcus aureus (Xen29)

Intraperitoneal injection of C57/B mice by *S*. *capsulata* often results in transient infections, and treatments with glycolipids hasten the clearance in 24 hour post infection. However, in the absence of any treatments, the infections often are cleared in 2-3 days. To evaluate the antibacterial efficacy of the α-GalCer analogs, a more relevant disease model of mouse deep thigh wounds by injection at mouse hind thigh muscle with luminescence S. *aureus* Xen29 was used. The S. *aureus* Xen29 infection can be monitored by image analyses of live mice to reduce the numbers of experimental mice and to allow repeated examination of the extent of infection throughout the antibacterial studies. Figure 66 (A-B) shows that treatment of the thigh-muscle infected mice with α-GalCer and its analogs resulted in profound clearance of most treated mice; however, statistically significance difference (p< 0.01) in comparison to the vehicle treated group is noted only in the group receiving C34 (C34 being of the present invention). This result thus suggests that C34 treatment may have high probability to be beneficial to bacterial clearance in this disease model.

### Antiviral evaluation of α-GalCer analogs using the Japanese Encephalitis Virus (JEV) infection animal model

To determine whether glycolipids elicit protective immunity against Japanese encephalitis virus (JEV) infection, a two-dose protocol was used, in which that glycolipid was given one day before and one day after viral challenge in mice. The prototype glycolipid, αGalCer (C1), and a glycolipid derivative C23 increased the mice survival from 21% to 57% as compared to solvent control (Figure 67). Furthermore, the protective effects were even more profound in mice receiving the glycolipids such as C34 (C34 being of the present invention) and 7DW8-5 with a survival rate of 64% and 71%, respectively (Fig. 67). These results strongly suggest that glycolipid-stimulated NKT cells likely contribute to host defense against JEV infection. Similarly, those analogs effective in protecting JEV infections were also found to prolong the survival of influenza infected Balb/C mice (data not shown). To further dissect the requirement for the glycolipid-mediated immune protection, tests were conducted to determine whether the beneficial effects of C34 and 7DW8-5 were still noted in certain immune deficient mice. Apparently, both of the innate and adaptive immune components are required for these glycolipids to trigger a protective immunity against JEV, as no beneficial effect was noted in mice lacking Stat-1, immunoglobulin µ-chain, or CDBα-chain (Figure 68 (A-C)).

### The anti-infectious activities of α-GalCer analogs are most effective when administered in a prophylactic manner

Assessments were performed to determine whether these glycolipids could generate protective immunity if they were administered post viral infection. Besides the two-dose protocol described above, several one-dose protocols were also tested. Glycolipids C34 (C34 being of the present invention) and 7DW8-5 given only once at one day before or at the same day of viral infection, elicited a weaker protective effect as compared to the two-dose protocol (Fig. 69 (A-B)). However, if the glycolipids were given one day after viral infection, no protection against JEV challenge could be noted (Fig. 69 (A-B)), suggesting that glycolipid-mediated immune modulation is an infection time-dependent event. The effects of C34 administration times versus viral infection time were also evaluated in influenza infected Balb/C mice. Administration of C34 one day before influenza infection was most beneficial to mouse survival (P value < 0.0001). Treatment with two doses of C34 administered both one day before and one day after influenza infection could also prolong survival significantly (P value = 0.0002) then the vehicle treated group. Similar to JEV infection, a single dose of C34 given one day after influenza infection was not beneficial to survival (Fig 7). The effects of C34 administration times on bacterial clearance in the *S. aureus* thigh infection model were also studied. When mouse infections were examined at 48 hr post infection, bacterial clearance were most profound in the group of mice administered with C34 immediately after the thigh infections were introduced. For the group receiving C34 at 6 hr after thigh infection, improvement in bacterial clearance is noted, but the difference to the vehicle treated group is not significant (Figure 71). Similar bacterial clearance profiles were observed when examined at 72-hour post infection (data not shown).

Both bacterial and viral infection murine models were used to evaluate the infection suppression efficacies of several α-GalCer analogues and found that the 4- (4-fluorophenoxy)phenyl octanoyl modified α-GalCer (C34 - being of the present invention) is most efficacious in our models. Previous studies showed that α-GalCer treatments are complicated with the association of detrimental side effects and the treatment efficacy was influenced by a variety of parameters. Natural killer (NKT) cells respond to infection associated glycolipids stimulations as well as by cytokines produced by dendritic cells activated by microorganisms. Thus, in experimental mice receiving both glycolipids and infection challenges, Complex stimulation to the murine immune system is expected. The cytokine responses were documented in the influenza infected, α-GalCer-treated, or both influenza infected and α-GalCer-treated mice. Greater serum cytokines were found in the many doubly stimulated mice. However, the profiles are complicated and varied at different observation times.

The efficacy of α-GalCer in the protection against lipopolysaccharide-induced shock was found to be critical on the time of administration while the glycolipid needs to be administer before or within 2 h after LPS challenge. Our in vivo studies using bacterial and viral infection models also showed that C34 (C34 being of the present invention) is most effective in suppressing infections when administered before or shortly after infection. Infections result in varied immune stimulations with different kinetics that are intimately related to the disease progression. It is conceivable that for the protection against infections both the doses and administrations times of glycolipids need to be fine tuned to be beneficial.

### IMMUNOTHERAPY FOR AUTOIMMUNE DISEASES

Autoimmunity results from a breakdown in the regulation in the immune system resulting in an inflammatory response directed at self-antigens and tissues. Autoimmune diseases are the third most common category of disease in the United States after cancer and heart disease; they affect approximately 5%-8% of the population or 14-22 million persons. Autoimmune diseases involving the destruction of self-antigen by T lymphocytes includes, but are not limited to, multiple sclerosis, insulin-dependent diabetes mellitus, and rheumatoid arthritis.

According to the current dogma, inflammatory autoimmune diseases such as myocarditis are primarily attributable to T_{H}1 responses, with IFN-γ as the prototypic cytokine; T_{H}2 responses where IL-4 dominates are believed to reduce autoimmunity. Because the α-GalCer analogs described herein can be designed such that a T_{H}2-biased immunogenic response is initiated, these α-GalCer analogs can be used as immunotherapies for autoimmune diseases.

### ADJUVANT ACTIVITIES OF α-GALCER ANALOGS

### Adjuvant activities of α-GalCer analogs for pCHA5 vaccine administered intramuscularly

Previously, it had been shown that immunization of mice with 30 µg pCHA5 by electroporation/intramuscular (EP/IM) route induced high titers of anti-HA antibodies and conferred 100% protection to lethal challenge of NIBRG-14 virus. With such high dose pCHA5 delivered by EP/IM, the addition of glycolipids could not enhance immune responses further. Therefore, the adjuvant effects of glycolipids for pCHA5 vaccine delivered by intramuscular route instead of EP/IM were tested. Mice were injected with 30 µg pCHA5 with/without 2 µg glycolipids (C1, C23, C26, C34, and 7DW8-5) intramuscularly and boosted with pCHA5 alone two weeks later. Sera were collected and mice were challenged with NIBRG-14 virus 2 weeks after the 2nd vaccination. In pCHA5 only mice, significant anti-HA-specific IgG antibodies were noted while none were detected in mice that received pVAX. In comparison to pCHA5 only group (16180 ± 5261), sera from mice showed slightly higher antibody titers in the group received C1 (24250 ± 3206) or C34 (23622 ± 5516) as adjuvant and similar level in C23 (14538 ± 3223) and C26 (16350 ± 2193) group (Figure 73A). Upon virus challenge none of the mice received pVAX vehicle survived, but all mice which received C1, C23, C26, C34 as adjuvant survived. Although the survival of pCHA5 only group (80%) was not as good as glycolipids-adjuvanted groups, the differences in survival were not statistically significant (Figure 73B). Therefore, the adjuvant effects of these glycolipids were not clearly delineated in this regimen. However, the intramuscular injection route had not been evaluated for vaccine delivery.

Next, the adjuvant effects of glycolipids on single dose of pCHA5 delivered by IM injection were evaluated. Mice were vaccinated intramuscularly with one dose of 50 microgram pCHA5 with/without glycolipids or alum. Three weeks later, sera were collected and mice were challenged with NIBRG-14 virus. Induction of HA specific antibody response were observed in pCHA5 (3692 ± 897.5) vaccinated mice and the transditional alum (2192 ± 547.5) adjuvant didn't show a better effect. However, increased titers were noted in the presence of glycolipid adjuvant C34 (7208 ± 1482) treated groups (Figure 74A). Similarly, enhanced cellular immunity as determined by functional IFN-γ ELISPOT analysis was detected in the presence of glycolipid as adjuvant (Figure 74B). The IFN-γ secreting cells in pCHA5 were 29.5 ± 0.5 per 2 x 10⁵ cells and increased significantly (p < 0.05) in glycolipids adjuvanted groups, such as 66.33 ± 5.3 in C1, 176.7 ± 2.3 in C34, 69.17 ± 1.5 in 7DW8-5, and 68.56 ± 8.72 in alum. Upon challenge with lethal dose of NIBRG-14 virus, none of the animals in the control (pVAX) and 20% for pCHA5 only group survived. The survival of mice with adjuvants was 40% for C1 and 7DW8-5 group, 60% survival for C34, and 0% survival of alum. The survival difference between the alum and glycolipid adjuvant groups is statistically significant (P <0.05) (Figure 74C). Overall, glycolipids could enhance HA-specific antibody titers, raise the number of IFN-γ production cells, and improve the survival after lethal challenge of H5N1 virus.

### Adjuvant activities of a -GalCer analogs for pCHA5-II vaccine

Since the pCHA5 DNA consensus sequences were derived from 467 H5N1 virus strains two years ago, it might be out of date. 1192 full length HA sequences were collected and a new consensus HA sequence, termed pCHA5-II, was obtained. There are 5 residues mutated in pCHA5-II when compared to pCHA5. The adjuvant effect of glycolipids in these two consensus HA DNA vaccines were compared. Groups of mice received EP/IM injection of either 30 microgram pCHA5 or pCHA5-II in the presence/absence of glycolipids as adjuvant Mice received two vaccinations at 3 weeks interval and sera were collected two weeks after the last immunization. The anti-HA antibodies titers were tested by ELISA and no significant difference were observed between pCHA5 and pCHA5-II with or without glycolipids as adjuvant (Figure 75A). However, there were significant difference between pCHA5 and pCHA5-II when challenged with highly pathogenic wild type H5N1 influenza virus (E319). No matter which glycolipid was added as adjuvant, the protection conferred by pCHA5 was not remarkable (Figure 75B). No mice survived in pCHA5 only group whereas about 10% and about 20% of mice survived in 7DW8-5 and C34 adjuvanted group, respectively. In pCHA5-II vaccinated groups, the C34 and 7DW8-5 glycolipids could improve the rate of survival from about 20% to about 50% and about 40%, respectively (Figure 75C). In conclusion, although the antibody titers were similar between pCHA5 and pCHA5-II with or without glycolipids, pCHA5-II conferred better protection than pCHA5 and the protection ability of pCHA5II was enhanced by the glycolipid adjuvants.

Furthermore, in some implementations optimal dosages under certain conditions were evaluated and suggested that a dosage of pCHA5-II and C34 delivered by IM as one shot vaccination in some circumstances is optimal. Various dosages of pCHA5-II (50, 75, and 100 µg) and C34 (0.5, 1, 2, and 4 µg) were used in determining formulations for pCHAS-II/C34 vaccine. 3 weeks after vaccination, sera were collected and anti-HA antibodies titers were tested by ELISA. In pCHAS-II treated groups, only weak anti-HA antibodies were detected and no obvious dose effects were observed. The anti-HA responses were enhanced around 8 ∼ 9 folds by adding 2 µg of C34 as adjuvant. At 100 µg of pCHA5-II, 0.5 and 2 µg of C34 elicited higher antibody titers than 1 and 4 µg (Figure 76A) (p < 0.01). Moreover, in functional IFN-γ secreting ELISPOT test, 100 µg of pCHA5-II induced greater numbers of IFN-γ producing cells (136.5 ±11.26) than either 50 (31.33 ± 11.95) or 75 µg (40 ±3.67) of pCHA5-II (p < 0.01). Such cell-mediated immune responses were increased by adding C34 as adjuvant, The IFN-γ secreting cells in 1 µg (151.2 ± 22.58) and 4 µg (146.5 ± 19.41) of C34 adjuvanted groups were better than 0.5 µg (106.7 ± 19.31) and 2 µg (95.56 ± 10.06) of C34 groups but the differences were not statistically significant (Figure 76B). Upon intranasal challenge with lethal dose NIBRG-14. The dose effects of pCHA5-II were shown in protecting mice against viruse challenge. The survivals of mice were increased from 10% (50 µg) to 40% (75 µg) and 70% (100 µg) in pCHA5-II groups. Furthermore, adding 2µg C34 into 50 µg and 75 µg of pCHA5-II groups could increase the survivals of mice from 10% to 100% and 40% to 100%, respectively (Figure 76C). Although the survivals of mice were not as good in C34 at 0.5 µg (80%) and 2 µg (75%), the differences did not appear, based on these test, to be statistically significant (Figure 76D) under the experimental conditions at the time. The addition of 1 µg C34 as adjuvant to 100 µg pCHA5-II increased the survival of mice from 70% to 100%. These data suggested that 50 µg of pCHA5-II with 2 µg of C34 in one shot vaccination schedule might provide good protection against virus challenge. To ensure the effect of protection, about 100 µg of pCHA5-II with about 1-2 µg of C34 in some implementations appears to provide a safe formulation.

To explore the cross-neutralization capability of antisera in mice immunized with pCHA5 with/without C34, the suboptimal dose of pCHA5 was used and the same vaccination schedule was applied. Two weeks after last vaccination, mice serum was taken and assessed by HA-pseudotyped virus neutralization assay. Four H5N1 influenza virus were selected from clade 1 (VN1194), 2.1 (ID05), 2.2 (TK05), and 2.3.4 (Anhui05). The various HA-pseudotyped viruses were incubated with antisera from control, pCHA5 only, and pCHA5 with C34 adjuvanted mice. The neutralizing titers were measured by the luciferase activity and data were reported as serum dilutions giving 50% of HA-pseudotyped virus neutralization (ID₅₀). As shown by the ID₅₀ (Figure 77), the antisera of pCHA5 only group showed greater neutralizing antibodies against VN1194 (ID₅₀ = 0.005862) and TK05 (ID₅₀ = 0.005953) HA-pseudotyped virus than pVAX group (ID₅₀ = 0.0277 and 0.02668, respectively). Moreover, the neutralization efficacy of C34 adjuvanted group against VN1194 (ID₅₀ = 0.00408) and TK (ID₅₀ = 0.003054) could be significantly enhanced. No difference was observed between the pVAX and pCHA5 with/without C34 group on the ID05 and Anhui05 HA-pseudotyped virus neutralization. These results suggest that C34 as an adjuvant in some implementations can act to increase the neutralization capability against various strains of virus at suboptimal pCHA5 vaccine.

The mechanisms responsible for the adjuvant effect of C34 through IM/EP route remain unclear. To explore the mechanisms of the adjuvant effect of C34, sera were collected at 0 hr and 20 hr after boost and cytokines were assayed by luminex. The analysis revealed that (Figure 78) very low to undetectable levels of IL-1α, IL-3, IL-4, IL-6, IL-10, IL-12p70, IL-15, and TNF-α were found in pCHA5 with or without C34 adjuvant groups. Serum levels of IFN-γ, G-CSF, IL-5, IL-17, KC, MIP-1β, and RANTES were significantly increased after boosting in C34 adjuvanted group. Furthermore, IL-2, IL-5, IL-12p40, IL-13, IL-17, IFN-γ, MIP-1α, MIP-1β, KC, RNATES, and G-CSF were found to be substantially higher in C34 adjuvanted group than in pCHA5 only group. In addition, decreased levels of IL-1β were found in pCHA5 containing C34 group compare with pCHA5 only group. The data shows that, in some exemplary implementations, adjuvant effect of C34 correlate with higher production of proinflammatory cytokines, T helper type I and II cytokines and chemokines involved in cell proliferation and chemotaxis.

The cross-neutralization capability of antisera in mice immunized with pCHA5-II with/without C34 vaccine was also tested, where 50 µg of pCHA5 was used and only single vaccination was applied. Three weeks after vaccination, mice serum was taken and assessed by HA-pseudotyped virus neutralization assay. Four H5N1 influenza viruses were used and the antisera from control, pCHA5-II only, and pCHA5- II with C34 adjuvanted mice were determined. The data presented in Figure 79, the antisera of pCHA5-II only group showed greater neutralizing antibodies against TK05 (ID₅₀ = 0.05376) HA-pseudotyped virus than pVAX group (ID₅₀ = 0.144). Unlike the pCHA5-II only group, the neutralization efficacy (ID₅₀) of C34 adjuvanted group against VN1194 (0.003), TK05 (0.004), Anhui05 (0.0027), and ID05 (0.002) were significantly enhanced. Taken together, these results indicate that C34 adjuvantnot only increase the potency of neutralization but also can act to broaden the spectrum against various virus.

### EXAMPLES

### Glycolipid Analogs of α-GalCer, Reagents and Mice

α-GalCer (C1) and synthetic α-GalCer analogs were synthesized and purified by column chromatography by techniques previously described in Fujio etal. (2006) J. Am. Chem. Soc. 128:9022-9023; Xing etal. (2005) Bioorg. Med. Chem. 13:2907-2916; Kinjo et al. (2005) Nature 434:520-525; Wu et al. (2006) Natl. Acad. Sci. U. S. A 103:3972-3977; and Wu et al. (2005) Proc. Natl. Acad. Sci. U. S. A 102:1351-1356; each of which is hereby incorporated herein by reference.

The synthetic α-GalCer analogs, as shown in Figure 2, were separated into four groups based on their chemical structures. Group I: C2, C3 and C14 are of bacterial origin, Group II: C4, C5 and C9 contain sulphur modification of O-linkage to ceramide (C4) or a sulfate group at 3"-OH of the galactose moiety (C5, C9), Group III: C6-C8, C8-5, C8-6, C10-C11, C15-C16 and C18-C34 (C34 being of the present invention) are modified with an aromatic ring in their acyl tail and Group IV: C12, C13 and C17 contain truncated phytosphingosine. Among these new analogs, C10, C11, C16, C27, C28, C29 are modified with a phenyl group in various length of fatty amide chain (Ph); C18, C22 are modified with methoxy group (-OMe) at the phenyl ring; C19, C23, 7DW8-5 are modified with fluoride group (-F) at the phenyl ring; C20, C24, 7DW8-6 are modified with trifluoromethyl group (-CF3) at the phenyl ring; C21, C25, C26 are modified with phenyl group (-Ph) at the phenyl ring; C34 is modified 1'-oxy-4-fluorophenyl (O-Ph-F). 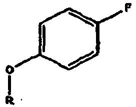 at the phenyl ring. However, substitution of the para-oxy-fluorophenyl (1'-oxy-4'-fluorophenyl) at the phenyl ring with an oxy-fluorophenyl with the F group at a non-para position or one of a difluoro, trifluoro, tetrafluoro and pentafluoro phenyl may also yield useful properties; and C17 contains a truncated phytosphingosine.

Synthesis of glycosphingolipid compounds C12 and C13 are summarized in Scheme 1 (Figure 3). Characterization data for these compounds are described below.

**Compound C13 (lot. MFJ3-017-1):** ¹H NMR (500MHz, CDCl₃-MeOH 4:1) δ: 7.26 (m, 2H), 7.23-7.19 (m, 2H), 7.18-7.14 (m, 1H), 4.90 (d, J = 3.9 Hz, 1H), 4.24-4.19 (m, 1 H), 3.86 (dd, J = 10.8, 5.2 Hz, 1H), 3.82-3.62 (m, 7H), 3.58-3.53 (m, 2H), 2.92-2.84 (m, 1H), 2.67 (ddd, J = 13.7, 9.3, 7.5 Hz, 1H), 2.16 (m, 2H), 2.06-1.98 (m, 1H), 1.74-1.65 (m, 1H), 1.62-1.53 (m, 2H), 1.33-1.19 (m, 44H), 0.88 (t, *J* = 7.0 Hz, 3H). ¹³C NMR (125MHz, CDCl₃-MeOH 4:1.) δ: 174.06, 141.93, 128.25, 128.01, 125.43, 99.48, 74.60, 70.75, 70.44, 69.99, 69.52, 68.66, 67.03, 61.69, 50.15, 50.06, 36.27, 34.13, 31.67, 31.59, 29.43, 29.31, 29.15, 29.09, 25.55, 22.41, 17.60, 13.76. HRMS (ESI-TOF) for C₄₄H₈₀NO₉⁺ [M + H]⁺ calcd 766.5827, found 766.5813.

**Compound C12 (lot. MFJ3-018-1):** ¹H NMR (400MHz, CDCl₃-MeOH 4:1) δ: 7.26 (m, 2H), 7.19-7.13 (m, 3H), 4.91 (d, *J =* 3.8 Hz, 1H), 4.20 (q, *J* = 4.4 Hz, 1H), 3.95-3.85 (m, 2H), 3.83-3.61 (m, 6H), 3.59-3.50 (m, 2H), 2.63 (t, *J* = 7.5 Hz, 2H), 2.20 (t, *J =* 7.5 Hz, 2H), 1.78-1.54 (m, 6H), 1.47-1.17 (m, 46H), 0.89 (t, *J* = 6.9 Hz, 3H). ¹³C NMR (100MHz, CDCl₃-MeOH 4:1) δ: 174.16, 142.27, 127.91, 127.77, 125.14, 99.33, 74.28, 71.38, 70.42, 69.86, 69.33, 68.51, 66.84, 61.40, 50.02, 36.04, 35.52, 31.93, 31.51, 31.21, 29.26, 29.14, 28.99, 28.94, 25.47, 25.08, 22.25, 13.51. HRMS (ESI-TOF) for C₄₆H₈₄NO₉⁺ [M + H]⁺ calcd 794.6140, found 794.6129.

All the synthetic α-GalCer analogs were originally dissolved in 100% DMSO at a concentration of 1-2 mg/ml. For *in vivo* experiments, synthetic α-GalCer analogs were diluted to 20 or I pg/ml in saline just before injection into mice. Pathogen-free BALB/c (wild type or CD1d knockout) and C57BL/6 female mice aged 6-10 weeks were obtained from the National Laboratory Animal Center (Taipei, Taiwan). CD1d-deficient BALB/c and C57BL/6 were obtained from the Jackson laboratory (C.12952-CD1tm1Gru/J, U.S) and provided by Dr. Steve R. Roffler (Academia Sinica, Taiwan), respectively. All the mice were maintained in pathogen free animal facility.

Synthesis of glycosphingolipid compound C34 (C34 being of the present invention) is summarized in Scheme 2 (Figure 80). Phytosphingosine derivative was synthesized from D-lyxose. The 2,3-dihydroxy groups of D-lyxose were selectively protected by 2-methoxypropene in the presence of acid to give actonide intermediate, and then the primary hydroxyl group of this intermediate was subjected to trityl chloride and base condition to give trityl ether. Followed by Wittig olefination, the subsequent intermediate reacted with C₁₃H₂₇⁺PPh3⁻Br in the presence of lithium hexamethyldisilazide (LHMDS) to yield alkene with the E/Z ratio of 2:1 by ¹H NMR spectrometry characterization. After catalytic hydrogenation of unsaturated alkene intermediate to give alkane, the hydroxy group was activated by triflate anhydride and 2,6-lutidine to give a triflate intermediate. The S_{N}2 reaction of triflate intermediate with tetramethylguanidinium azide (TMGA) gave azido compound with inverted configuration. The trityl group of Cl was selectively removed by using triflouroacetic acid (TFA).

Glycosylation of doner-galactose derivative and acceptor phytosphingosine was subjected to a condition using trifluoromethanesulfonic anhydride (Tf₂O) and dimethyl sulfide (Me₂S) as promoters to get the key intermediate. Then, the azido group of this key intermediate was reduced by using Staudinger reaction to give the amine intermediate. The amine was coupled with freshly prepared fatty acid by using EDC and HBTU and then global deprotected to give compound C34.

Characterization data for this compound is described below.

**Compound C34:** ¹H NMR (600MHz, pyridine-d⁵) δ: 8.53 (d, *J =* 8.6 Hz, 1 H), 7.27 (d, *J* = 8.4 Hz, 2H), 7.14 (t, *J* = 8.4 Hz, 2H), 7.05-7.09 (m, 4H), 5.59 (d, *J* = 3.8 Hz, 1H), 5.27 (m, 1H), 4.70-4.65 (m, 2H), 4.55 (d, *J*= 2.6 Hz, 1H), 4.52 (t, *J=* 6.0 Hz, 1 H), 4.45-4.39 (m, 4H), 4.35-4.30 (m, 1H), 2.59 (t, *J =* 7.6 Hz, 2H), 2.45 (t, 2H), 2.30 (m, 1H), 1.91 (m, 2H), 1.81 (m, 2H), 1.68 (m, 2H), 1.59 (m, 2H), 1.47-1.15 (m, 42H), 0.87 (t, *J* = 6.9 Hz, 3H). ¹³C NMR (150MHz, pyridine-d⁵) δ: 173.79, 160.19, 158.60. 156.32, 154.53, 138.89, 130.73, 123.65, 121.06, 121.01, 119.46, 117.25, 117.09, 102.00, 77.17, 73.53, 72.96, 72.09, 71.46, 71.29, 70.79, 69.07, 63.12, 51.95, 37.27, 35.86, 34.82, 32.60, 32.47, 30.84, 30.63, 30.48, 30.41, 30.35, 30.28, 30.25, 30.20, 30.10, 30.03, 26.99, 26.87, 23.42, 14.77. HRMS (ESI-TOF) for C₄₇H₇₆FNO₁₀Na⁺ [M + Na]⁺ calcd 856.5345, found 856.5362.

### Isolation and Generation of Human NK Cell Lines, Immature Monocyte-Derived Dendritic Cells and NK/NKTs

The naïve Vα24i NKT cells were separated using indirectly conjugated anti-Vα24iTCR microbeads (Miltenyl Biotec, USA). The isolated cells were incubated in the presence of 50 U/ml IL-2 (R&D system) and replenished with fresh media every 3 days. The generation of α-Galcer-pulsed or phenyl glycolipid-pulsed Vα24i NKT were done as follows. Anti-Vα24i TCR mAbs, and anti-CD14 mAbs, each coupled to magnetic beads (Miltenyi Biotec, Auburn, CA), were used sequentially to isolate Vα24i NKT cells and CDI4 cells from leukopaks. Immature dendritic cells were generated from the CD14 cells after a 2-day incubation in the presence of 300 U/ml GM-CSF (R & D Systems) and 100 U/ml IL-4 (R& D Systems). After irradiation with 2,000 rad, the immature dendritic cells were cocultured with syngeneic CD161 cells in the presence of 100 ng/ml α-GalCer or C11 and 50 U/ml IL-2 (Invitrogen) for 10-14 days. After stimulating the Vα24i NKT cells a second time with 100 nglml α-GalCer or C11-pulsed irradiated immature dendritic cells to generate α-GalCer pulsed or phenyl-glycolipid pulsed iNKT cells, respectively. All iNKT cell lines (naïve, α-GalCer pulsed or phenyl-glycolipid pulsed) were shown flow cytometrically to express Vα24i T cell antigen receptor (95% purity). NK and NKT cells were isolated from human leukopaks using anti-CD56 microbeads (Miltenyi Biotec, USA).

The generation of α-GalCer analog-pulsed human NKT cell lines was done according to the methods of Fujio et al., and these cells were used to assess cytokine response to the studied α-GalCer analogs (see Figures 5 and 6). Immature DCs were derived from CD14⁺ cells in leukopaks after a two-day incubation with 300 U/ml GM-CSF and 100 U/ml IL-4. After irradiation (3,000 rad), the iDCs were cultured together with autologous CD161⁺ cells in the presence of 100 ng/ml α-GalCer and 10 U/ml IL-2 for 10 days. After repeating this stimulation, NK cell lines were generated and shown to express CD161⁺/CD3₊/Vα24iTCR⁺ (99% purity). To generate immature human monocyte-derived DCs, CD14⁺ cells in leukopaks were cultured in the presence of 300 U/ml GM-CSF and 100 U/ml IL-4 for 6 days. These DCs had an immature phenotype (CD14⁻CD80⁺CD86⁺CD83^{weak} HLA⁻DR⁺) and exhibited higher CD1d expression than mature DCs. The iDCs were pulsed with various α-GalCer analogs at 3 µg /ml and their phenotype and morphology were examined 48 hours later.

The naïve NKTs (CD161⁺/CD3⁺) used forTCR activation experiments (see Figure 19) were isolated by using indirectly conjugated anti-CD161 multi-sort microbeads and were further separated by anti-CD3 microbeads. The isolated cells were incubated in the presence of 100 U/ml IL-2 and replenished with fresh media every 3 days.

### In vitro Human NKT Cell Cytokine Secretion Assay

Vα24i human NKT cells (1x10⁵) were cocultured with 5x10⁴ irradiated immature CD14⁺ DCs in the presence of the α-GalCer analogs at 10 µg/ml in a 96-well flat-bottom plate. Cytokines/chemokines in the supernatant collected at 18h were quantified with the Beadlyte® Human 22-plex Multi-Cytokine Detection System and determined by Luminex® 100TM system.

### In vitro Expansion of iNKTs.

Human CD56+ cells (NK/NKT mixtures) used for iNKT cell expansion experiments (see Figures 13 and 14) were isolated from human leukopaks by using anti-CD56 microbeads. Human CD56+ cells (NK/NKT mixtures) were cultured with 4 x 10s autologous immature CD14+ DCs pulsed with the indicated α-GalCer analogs at 3 µg /ml or 0.3% DMSO on day 2 for 18 hours (see Figures 13 and 14) or at 10 or ng/ml on day 2 for 18 hours (see Figure 15). On day 3, the suspension cells were transferred to a new dish, cultured in the presence of 100 U/ml IL-2, and replenished with fresh medium every 3 days. The population of CD161+/Vα24TCR+ cells in the NK/NKT mixtures were gated by flow cytometry on day 9, and the total number of Vα24i NKT were counted.

### Human NKT TCR Activation

HeLa, Hela-CD1d or autologous iDCs were incubated on 24 well-plate with C1, C11, C13 or C17 at 10 µg /ml or with DMSO for 2h, and then 3 x 10s naive CD161+/CD3+ NKTs were added (see Figure 19). In another exemplary implementation, HeLa or HeLa-CD1d cells were loaded with C1, C16, C23, C8-5, C8-6 or C26 at 100 ng/ml or with DMSO for 2 hours, and then 3 x 10s naive CD161+/CD3+ NKTs were added (see Figure 20). After 5-10 mm stimulation, cells in suspension were transferred to tubes, washed with PBS, and lysed with Beadlyte® Cell Signaling Universal Lysis Buffer at 4°C. The concentrations of phospho-CD3ε (Phospho-tyrosine), phospho-ERK1/2 (Thr185/Tyr187), phospho-CREB (Ser133), phospho-Syk (Phospho-tyrosine), phospho-p38 (THr180/Tyr 182), phospho-IκBα (Ser32), phospho-Lck, phospho-Lat, phospho-STAT3 (Ser727), phospho-STAT5 A/B (Tyr 694/699) and phospho-Dap-70 (Phospho-tyrosine) in lysates were assessed by Beadlyte® Phosphoprotein Detection System according to the assay protocol, and determined by a Luminex100 system. The value was normalized with the amount of total input protein.

### In vitro CD1d-tetramer assay

1 µg of soluble divalent mouse CD1d-IgG1 fusion protein (mouse CD1d-IgG1 tetramers, BD Pharmingen) was incubated overnight with 10 mole of each α-GalCer analog at 37°C and at neutral pH according to the manufacturer's protocol. The glycolipid-loaded CD1d-IgG1 tetramers were incubated with mouse NKTs at 4°C for 60 min, followed by incubation with FITC-coupled anti-mouse IgG1 mAb (A85-1). The cells were also surface-stained with a PE coupled anti-NK and APC coupled anti-CD3 mAb (BD Pharmingen).

### Preparation of mouse splenocytes

BALB/c mice treated with the indicated α-GalCer analogs or vehicle were sacrificed at 72 h after injection. The spleens were harvested. In brief, after pressing the spleen through 70 µm strainer and lysis of erythrocytes, the nucleated cells were resuspended in Hank's Balanced Salt Solution and centrifuged at 300 g for 5 min at 4°C, then subjected to FACS analysis.

### Determination of Mouse Splenocyte Subpopulations

BALB/c mice treated with the indicated α-GalCer analogs (2 ug mouse) or vehicle (1% DMSO in PBS) and were sacrificed at 72 h and the spleen was harvested. In brief, after pressing the spleen through 70 µm strainer and lysis of erythrocytes, the nucleated cells were resuspended in Hank's Balanced Salt Solution and centrifuged at 300 g for 5 min at 4°C, then subjected to FACS analysis. The anti-CD3e-allophycocyanin, anti-CD4-PE, anti-CD8α-allophycocyanin-cyanide-dye7, anti-CD11c-allophycocyanin, anti-CD23-PE, anti-45R-allophycocyanin, anti-CD69-FITC, anti-CD80-FITC, anti-CD86-PE, anti-Ly6G-PE, and U5A2-13Ag+ -PE were obtained from BD Bioscience-Pharmingen.

### Determination of Mouse Splenocyte NKT and NK Subpopulations

BALB/c mice treated with indicated α-GalCer analogs (0.1 ug/ mouse) or vehicle (0.1% DMSO in PBS) and were sacrificed at 72 h and the spleen was harvested. In brief, after pressing the spleen through 70 um strainer and lysis of erythrocytes, the nucleated cells were resuspended in Hank's Balanced Salt Solution and centrifuged at 300 g for 5 mm at 4°C, then subjected to FACS analysis. The anti-CD3e-allophycocyanin and NK marker U5A2-13Ag⁺ -PE were obtained from BD Bioscience-Pharmingen.

### Serum Cytokines/Chemokines

Mouse serum samples were collected at 0, 2, 18, 36, 48, and 72 h after administration of vehicle or synthetic α-GalCer analogs. The serum concentrations of various cytokines/chemokines were measured by Beadlyte® Mouse 21-plex Cytokine Detection System and read by a Luminex®100TM system.

### Lung Cancer Model in Mice

C57BL/6 mice (6-8 weeks, female) were injected IV with 2 X 10⁵ syngeneic lung cancer (TC1) cells suspended in 0.1 ml of PBS. At 1 hr, groups of C57BL/6 mice (n=5) were treated with the indicated α-GalCer analogs IV (2 µg per mouse) or vehicle twice per week for four weeks. The body weight was recorded for one month and survival was monitored for 50 days.

### Breast Cancer Model in Mice

BALB/C mice (6-8 weeks, female) were inoculated with 2 X 10⁵ syngeneic breast cancer (4T1) SubQ on the right lower back. Groups of BALB/c mice (n=6) were treated IV or SubQ with the indicated α-GalCer analogs or vehicle twice per week for four weeks 3 days after tumor inoculation. The α-GalCer analogs were injected at a site distal to the tumor inoculation site. The tumor volume was recorded every 3 days for one month by measuring with a caliper along the long axis (a), the short axis (b) and the height (c). Tumor volumes (mm³) were calculated by the formula: a x b x c, and survival was monitored for 70 days.

### Real Time Assessement of Tumor Growth in Mice

Mouse images were obtained and analyzed by Xenogen's IVIS® 200 Series and Living Image® Software (Xenogen, U.S.). In melanoma model, C57BL/6 mice (6-8 weeks, female) were injected intravenously with 2X10⁵ syngeneic melanoma (B16) cells suspended in 0.1 ml of PBS. After 3 days, groups of C57BL/6 mice (n=5) were treated intravenously with indicated glycolipids under the indicated therapeutic protocol. The tumor volume was recorded every three days for 24 days.

### Infiltration of Lymphocytes by Flow Cytometric Analysis

Tumors from control and glyclolipids treated mice were aseptically removed on days 21 after tumor implantation and manually cut into 2-3-mm pieces in a culture Petri dish. The small tissue fragments were then digested with 0.01% DNase, 0.01% hyaluranidase, and 0.1% collagenase (all from Sigma Chemical Co.) In RPMI 1640 for 2-3 h at 37°C with continuous stirring. The resulting single cell suspensions were then washed twice with 0.1% FCS in PBS and stained by standard flow cytometry methods. To detect subpopulations of lymphocytes infiltrating these tissues, the following conjugated antibodies were used for FACS: FITC-anti-CD3, PE-anti-NK, APCCy7-anti-CD8, (BD Biosciences PharMingen, San Diego, CA).

### Immunohistochemistry Staining

The lung nodules were taken from B6 mice i.v injected with 2X10⁵ TC1 tumor cells for 3 weeks then sacrificed to do paraffin-embedded sections. 3 µm thick sections were treated at 56°C oven overnight followed by deparaffinization & heat-mediated antigen retrieval (in pH 9 Tris-EDTA buffer at 121°C for 7.5 mins) and incubated with anti-CD45RA antibody (clone RA3-6B2; BD Biosciences PharMingen, San Diego, CA) as an indicative of common lymphocyte antigens at a titration of 1:100 at 4°C overnight. The bound primary antibody is detected by the addiction of secondary antibody conjugated with horseradish peroxidase and DAB substrate. All sections were counterstained with haematoxylin prior to mounting.

### Statistical analysis

Unpaired two-tailed Student's t test was used for data analysis with PRISM software. Graphs show mean values of triplicate experiments, and error bars represent the SD. Differences in tumor protection of each group were analyzed by using the log-rank test. P<0.05 was considered statistically significant.

### Antibacterial Efficacy Studies

### Glycolipid Analogs of α-GalCer

The structures of the α-GalCer analogs used in the antibacterial studies are shown in Figure 2, C3, C9, C11 and C14-C17. α-GalCer analogs stock solutions were prepared as 1 mg/ml DMSO solutions. α-GalCer analogs were diluted with phosphate buffered saline (PBS) to 10 µg/ml before use.

### Animals and Bacteria

Female C57U6 and BALB/c-Byl mice at 6-8 week old were used for studies. Mice were housed in plastic cages with free access to food and water and allowed to acclimate at least one week prior to the start of the experiments. The bacterial strain Spingomonas capsulate (ATCC 14666) was obtained from BCRC, Taiwan. The bacterial strain Klebsiella pneumoniae (NTUH-KP2044) was a gift from Dr. J. T. Wang, National Taiwan University Hospital, Taiwan.

### Antibacterial Efficacy Study Using Sphingomonas Capsulate Infected Mice

Six to eight week old female C57BU6 mice were injected IP with 5x10⁸ Sphingomonas capsulate cells. Mice were grouped into treatment and control groups with 4-6 mice per group. Four hours after the infection, mice in the treatment group were injected IP with testing α-GalCer analogs at 50 or 100 µg/kg, and the control group mice were injected with same volumes of PBS. Twenty-four hours after bacterial infection, mice from all groups were sacrificed. Livers were removed from mice and homogenized in 0.9% NaCl, 0.02% Tween 80 using tissue homogenizers. Colony formation units (CFU) of Sphingomonas capsulate in liver homogenates were determined by plating diluted samples on nutrient plates. Colonies were counted after incubation for 48 hours at 37°C.

### Antibacterial Efficacy Study Using K. Pneumoniae Infected Mice

BALB/c-Byl female mice (ten mice per group) were administered a single dose (10⁶ CFU) of live K. pneumoniae by oral gavage. Mice in the treatment groups were injected with testing α-GalCer analogs at 100 µg/kg twice at 4-hour and 8-hour after bacterial infection. Mice in the control group were injected with PBS at 4-and 8-hour. Twenty four hours after infection, all mice were sacrificed. Both livers and lungs were collected from each mouse, and homogenized. Bacterial counts were determined similarly as described above.

### Statistical analysis

Comparative efficacies of testing α-GalCer analogs were illustrated by comparison of the organ CFU values of treatment groups with those in control groups, and the significance of the efficacy was indicated in p-values of <0.05 or <0.01, respectively.

### Glycolipid testing agents

The structures of glycolipids and their code names used are shown in Figure 64 (A-B). Glycolipid stock solutions were prepared as 1 mg/mL DMSO solutions. Compounds were diluted with phosphate buffered saline (PBS) to 10-30 µg/mL and used for animal studies.

### Mice, viruses, and bacteria used

C57U6 and BALB/c mice at 6-8 week old were used for studies. Mice were housed in plastic cages with free access to food and water and allowed to acclimate at least one week prior to the start of the experiments. The viruses used in this study are influenza strain WSN (A/WSN/1933/H1N1), and Japanese Encephalitis Virus (JEV, RP-9 strain) as previously described. The bacterial strain *Sphingomonas capsulata* (ATCC 14666) was obtained from BCRC, Taiwan. The luminescence *Staphylococcus aureus* Xen29 was purchased from Xenogen Corporation (CA, USA). All animal studies were approved by the Academia Sinica Animal Study Committee and were conducted according to the guidelines. Immuno-deficient mice used in this study are as described previously.

### Antibacterial efficacy study using Sphingomonas capsulata infected mice

Six to eight week old female C57BU6 mice were injected intraperitoneally with 5x10⁸ Sphingomonas capsulata cells. Mice were grouped into treatment and control groups with 5-10 mice per group. Three to four hours after the infection, mice in the treatment group were injected intraperitoneally with testing glycolipids at 50 or 100 µg/kg, and the control group mice were injected with same volumes of PBS. Twenty-four hours after bacterial infection, mice from all groups were sacrificed. Livers were removed from mice and homogenized in 0.9% NaCl, 0.02% Tween 80 using tissue homogenizers. Colony formation units (CFU) of *Sphingomonas capsulata* in liver homogenates were determined by plating diluted samples on nutrient plates. Colonies were counted after incubation for 48 hours at 37°C. The antibacterial results were analyzed using one-way ANOVA to assess the significance of the difference in treatment group followed by Turkey post test.

### Antibacterial efficacy study using luminescence Staphylococcus aureus (X29) by image analyses

Balb/C female mice were weighed, grouped and injected in the posterior quadriceps muscle of the left thighs with 200 µL PBS containing 3 X 10⁸ CFU of S. aureus Xen29. At different times after Xen29 infection, mice were IP injected with vehicle or glycolipids as described. Mice were periodically imaged using IVIS Imaging System (Xenogen Corporation, CA, USA) as described. The imaging results, in relative luminescence were analyzed using one-way ANOVA to assess the significance of the difference in treatment group followed by Turkey post test.

### Japanese Encephalitis Virus (JEV) infection animal model

Seven week old C57B56 mice were grouped and injected with glycolipids at various times in the JEV infected mice. The infection was done by IP injection with 5X10⁵ PFU of the RP-9 strain JEV in 500 µL PBS and simultaneously injected intracerebrally with 50 mL PBS (the IP plus IC routes) as described previously. The survival of the mice was monitored daily. The survival curves of glycolipids treated groups were compared to the control group using Prism software (GraphPad Software, San Diego, CA).

### Anti-influenza animal model study

For the evaluation of the in vivo efficacy of the α-GalCer analogs, Balb/c mice were treated with C34 (C34 being of the present invention) at different times relative to the nasal infection using 10 LD₅₀ WSN virus. Mouse survivals in all groups were examined daily for 14 days post-infection. The survival curves of glycolipids treated groups were compared to the control group using Prism software (GraphPad Software, San Diego, CA).

### Immunoprotection against avian influenza virus infection by intramuscular injection of two dosages of pCHA5 + /-glycolipid vaccine.

Mice were vaccinated with pCHA5 (30 µg) + /-glycolipids (1 µg) through IM route and were boosted with pCHA5 only two weeks later. Two weeks after booster injection, sera were collected and tested for HA-specific antibody titers by ELISA (Fig. 73A) and mice were challenged with 200 LD₅₀ of NIBRG-14 viruses and monitored for survival (Fig. 73B).

### Single intramuscular vaccination of pCHA5 with and without glycolipids.

Immune responses and mice survival after single IM dose of pCHA5 were enhanced by using glycolipids as adjuvants. Mice were vaccinated with pCHA5 (50 µg) +/-glycolipids (2 µg) or alum through IM route. Three weeks after vaccination, sera were collected and tested HA-specific antibody titers by ELISA (Fig. 74A). At the same time, mice were sacrified and splenocytes were harvested for ELISPOT assay (Fig. 74B). Another group of mice were challenged with 200 LD₅₀ of NIBRG-14 virus and monitored for survival (Fig. 74C).

### Comparison of the adjuvant effects of glycolipids for pCHA5 and pCHA5-II vaccine.

**Mice** were vaccinated with pCHA5 or pCHA5-II (30 µg) +/-glycolipids (2 µg) through IM/EP route at week 0 and three. Two weeks after booster injection, sera were collected and tested anti-HA antibody titers by ELISA (Fig. 75A), and, mice which were vaccinated with pCHA5 (Fig. 75B) and pCHA5-II (Fig. 75C) were challenged with E319 virus and survivals were monitored.

### Dose effects of pCHA5-II and C34 on HA-specific antibody production and immune protection in mice.

Mice were vaccinated with single dose of pCHA5-II (100, 75, 50 µg) with or without C34 (0.5, 1, 2, 4 µg) as adjuvant through IM route. Three weeks after vaccination, sera were collected and HA-specific antibody titers were assayed by ELISA (Fig. 76A). At the same time, mice were sacrified and splenocytes were harvested for ELISPOT assay (Fig. 76B). Meanwhile, other groups of mice were challenged with NIBRG-14 virus and mice survivals were monitored. Fig. 76C shows survivals of mice treated with different doses of pCHA5-II and 2 µg C34 adjuvant. Fig. 76D shows survivals of mice treated with 100 tg pCHA5-II and C34 at various dosages.

### Adjuvant effect of C34 on neutralization antibody production against various HA-pseudotyped viruses.

Female BALB/c mice were IM/EP vaccinated with 0.2 µg of pCHA5 dissolved in PBS containing 2 µg of C34 on week 0 and 3. Sera were collected 2 weeks after the last vaccination and examined by HA-pseudotyped virus neutralization assay. Data were reported as serum dilutions giving 50% of HA-pseudotyped virus neutralization (ID₅₀). TK (Fig. 77A), VN1194 (Fig. 77B), ID05 (Fig. 77C), and Anhui05 (Fig. 77D) HA-pseudotyped viruses were used in this assay. Nonlinear regression analysis was used to examine the data fit. Value of p was the comparison of fits. * p < 0.05 and presented statistical significant when compare C34-adjuvanted group with pCHA5 only group.

### Serum cytokine expression profiles in mice receiving pCHA5 with or without C34 as adjuvant.

Female BALB/c mice were vaccinated with 0.2 µg pCHA5 with or without C34 through IM/EP route at week 0 and 3. Sera were collected before (0h) and 20h after second vaccination. Serum concentrations of cytokine were assayed by Luminex 200 system. * p < 0.05 by two-tailed unpaired t test when compare C34 adjuvanted group with pCHA5 only group. # p < 0.05 by two-tailed unpaired t test when compare the 0h with 20h. Results are shown with respect to IL-2 (Fig. 78A), IL-5 (Fig. 78B), IL-13 (Fig. 78C), RANTES (Fig. 78D), MIP-I α (Fig. 78E), MIP-1β (Fig. 78F), KC (Fig. 78G), IL-1β (Fig. 78H), IL-17 (Fig. 781), IL-12p40 (Fig. 78J), G-CSF (Fig. 78K), and IFN-y (Fig. 78L).

### The neutralization abilities of antisera with induction by C34 through single dose intramuscular injection.

Female BALB/c mice were IM vaccinated with 50 µg of pCHA5-II dissolved in PBS containing 2 µg of C34. Three weeks later, sera were collected and examined by HA-pseudotyped virus neutralization assay. HA-pseudotyped virus of TK, VN1194, ID5, and RG5 were used in this assay. Data were reported as ID₅₀. Nonlinear regression analysis was used to examine the data fit. Value of p was the comparison of fits. * p < 0.05 and presented statistical significant when compare C34-adjuvanted group with pCHA5-II only group. Results are shown with respect to Anhi05 (Fig. 79A), TK05 (Fig. 79B), ID05 (Fig. 79C), and VN1194 (Fig. 79D).

## Claims

1. A compound represented by the structure of formula 1: 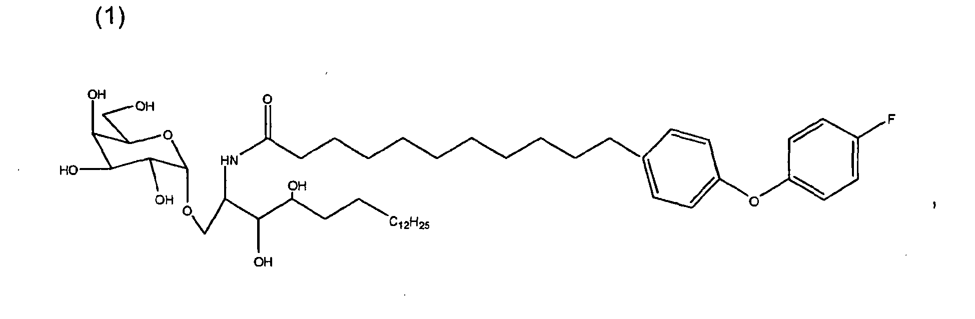 or a pharmaceutically acceptable salt thereof; for use in medicine.

2. A pharmaceutical composition comprising the compound of claim 1 and a pharmaceutically acceptable carrier.

3. The composition of claim 2, wherein the composition is in the form of a vaccine and the compound of formula 1 functions as an adjuvant.

4. The composition of claim 3, wherein the vaccine comprises a vaccine agent and wherein the vaccine agent is selected from the group consisting of: a killed microorganism; a live attenuated virus microorganism; a toxoid; a fragment of an inactivated or attenuated microorganism; DNA; a protein; a glycoprotein; a saccharide; a combination of the markers on cancer cells; and a combination of the markers on microorganisms.

5. The composition of claims 3 and 4, wherein the composition elicits a neutralization efficacy against H5N1 influenza virus, wherein the H5N1 influenza virus is preferably selected from at least one of: wild type H5N1 influenza virus (E319); TK05; VN1194; ID05; Anhui05; pCHA5; and pCHA5-II.

6. The composition of claim 2 for use in anti-tumour immunotherapy, wherein the composition is preferably in the form of a a cancer vaccine, and wherein the cancer is preferably selected from the group consisting of: lung cancer; breast cancer; hepatoma; leukemia; solid tumour and carcinoma.

7. The composition of claim 2 for use in the treatment or prophylaxis of a viral or bacterial infection, wherein the infection is preferably caused by one of the group selected from: Sphingomonas capsulate; Japanese Encephalitis Virus; Staphylococcus aureus; and Influenza.

8. The composition of any of claims 2 to 7, wherein the composition is formulated so as to be suitable for administration via a route selected from: subcutaneous; intravenous; intranasal; intramuscular; and oral.

9. A method of synthesizing a compound represented by the structure of formula 1: 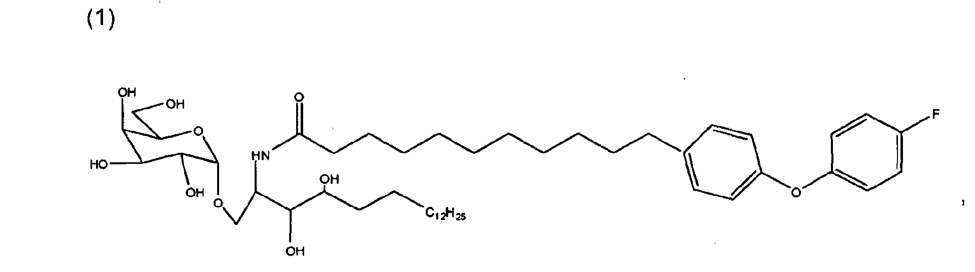 comprising:
selectively protecting 2,3-dihydroxy groups of D-lyxose by 2-methoxypropene in the presence of acid to give an actonide intermediate;
subjecting a primary hydroxyl group of the actonide intermediate to trityl chloride and base condition to give a trityl ether intermediate;
reacting the trityl ether intermediate with C₁₃H₂₇+PPh₃-Br by Wittig olefination in the presence of lithium hexamethyldisilazide (LHMDS), whereby an alkene intermediate with the E/Z ratio of 2:1 by ₁H NMR spectrometry characterization is yielded;
hydrogenating the alkene intermediate to give an alkane;
activating a hydroxy group of the alkane by triflate anhydride and 2,6-lutidine to give a triflate intermediate;
reacting the triflate intermediate with tetramethylguanidinium azide (TMGA) by S_{N}2 reaction to give an azido compound with inverted configuration;
removing a trityl group of the azido compound by using triflouroacetic acid (TFA) to give phytosphingosine;
effecting glycosylation of doner-galactose derivative and acceptor phytosphingosine using trifluoromethanesulfonic anhydride (Tf₂O) and dimethyl sulfide (Me₂S) as promoters to give a key intermediate having an azido group;
reducing the azido group of the key intermediate using a Staudinger reaction to give an amine intermediate; and
coupling the amine intermediate with fatty acid using EDC and HBTU.

10. A compound of formula 1: 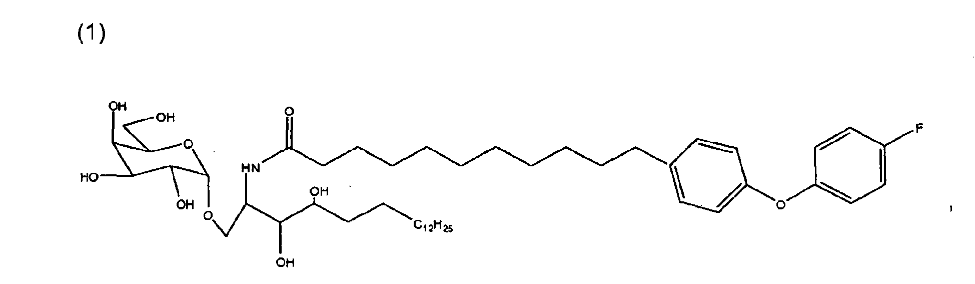 or a pharmaceutically acceptable salt thereof, for use in the treatment or prophylaxis of a viral or bacterial infection.

11. The compound of claim 10, wherein the infection is caused by one of the group selected from: Sphingomonas capsulate; Japanese Encephalitis Virus; Staphylococcus aureus; and Influenza.

12. A compound of formula 1: 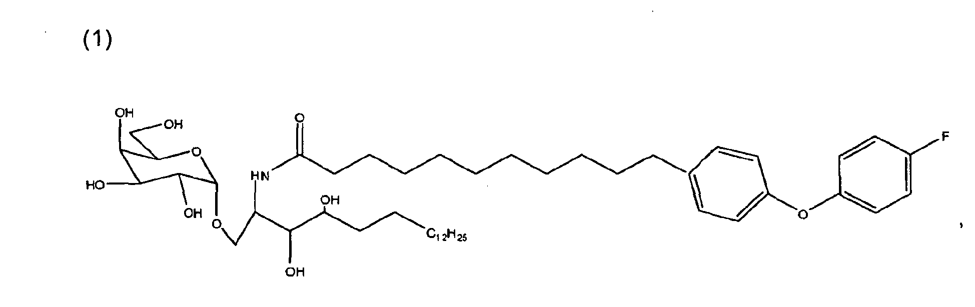 or a pharmaceutically acceptable salt thereof, for use in the treatment of cancer.

13. The compound of claim 12', wherein the cancer is selected from the group consisting of: lung cancer; breast cancer; hepatoma; leukemia; solid tumour and carcinoma.

14. A compound represented by the structure of formula 1: 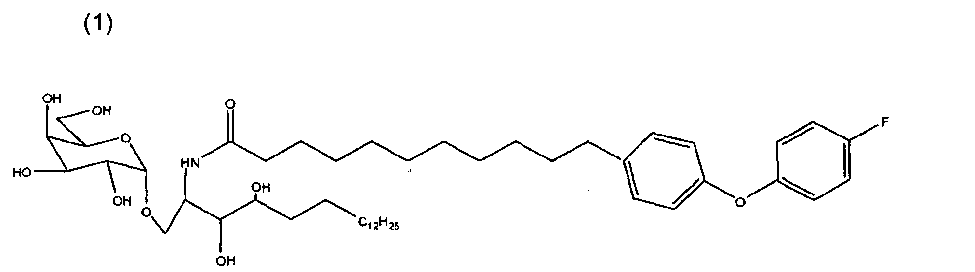 or a pharmaceutically acceptable salt thereof.

15. A compound according to claim 14 represented by the structure 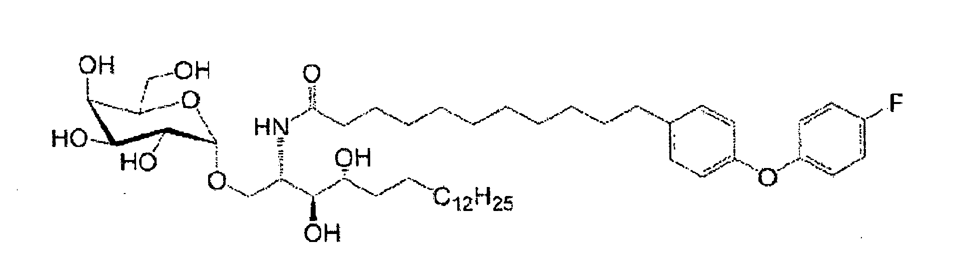 or a pharmaceutically acceptable salt thereof.

## Patentansprüche

1. Verbindung, die durch die Struktur der Formel 1 dargestellt wird: 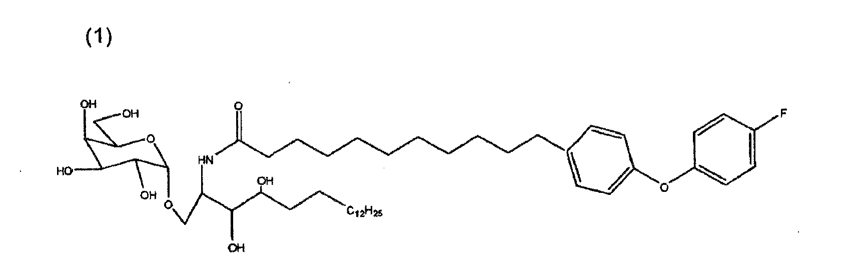 oder ein pharmazeutisch annehmbares Salz davon zur Verwendung in Medizin.

2. Pharmazeutische Zusammensetzung, umfassend die Verbindung gemäß Anspruch 1 und einen pharmazeutisch annehmbaren Träger.

3. Zusammensetzung gemäß Anspruch 2, wobei die Zusammensetzung in der Form eines Vakzins ist und die Verbindung der Formel 1 als Adjuvans wirkt.

4. Zusammensetzung gemäß Anspruch 3, wobei das Vakzin ein Vakzin-Agens umfasst und wobei das Vakzin-Agens aus der Gruppe, bestehend aus einem abgetöteten Mikroorganismus; einem lebenden attenuierten Virus-Mikroorganismus; einem Toxoid, einem Fragment eines inaktivierten oder attenuierten Mikroorganismus; DNA; einem Protein; einem Glycoprotein; einem Saccharid; einer Kombination der Marker an Krebszellen und einer Kombination der Marker an Mikroorganismen, ausgewählt ist.

5. Zusammensetzung gemäß Anspruch 3 und 4, wobei die Zusammensetzung eine Neutralisationswirksamkeit gegen H5N1-Influenzavirus auslöst, wobei das H5N1-Influenzavirus vorzugsweise aus wenigstens einem von Wildtyp-H5N1-Influenzavirus (E319); TK05; VN1194; ID05; Anhui05; pCHA5 und pCHA5-II ausgewählt ist.

6. Zusammensetzung gemäß Anspruch 2 zur Verwendung in der Antitumor-Immuntherapie, wobei die Zusammensetzung vorzugsweise in der Form eines Krebs-Vakzins ist und wobei der Krebs vorzugsweise ausgewählt ist aus der Gruppe, bestehend aus: Lungenkrebs; Brustkrebs; Hepatom; Leukämie; solidem Tumor und Karzinom.

7. Zusammensetzung gemäß Anspruch 2 zur Verwendung in der Behandlung oder Prophylaxe einer Virus- oder Bakterieninfektion, wobei die Infektion vorzugsweise durch eins aus der Gruppe, ausgewählt aus: Sphingomonas capsulata; dem japanischen Encephalitis-Virus; Staphylococcus aureus und Influenza, verursacht ist.

8. Zusammensetzung gemäß einem der Ansprüche 2 bis 7, wobei die Zusammensetzung so formuliert ist, dass sie zur Verabreichung über einen Weg, der ausgewählt ist aus subkutanem; intravenösem; intranasalem; intramuskulärem und oralem, geeignet ist.

9. Verfahren zur Synthetisierung einer Verbindung, die durch die Struktur der Formel 1 dargestellt wird: 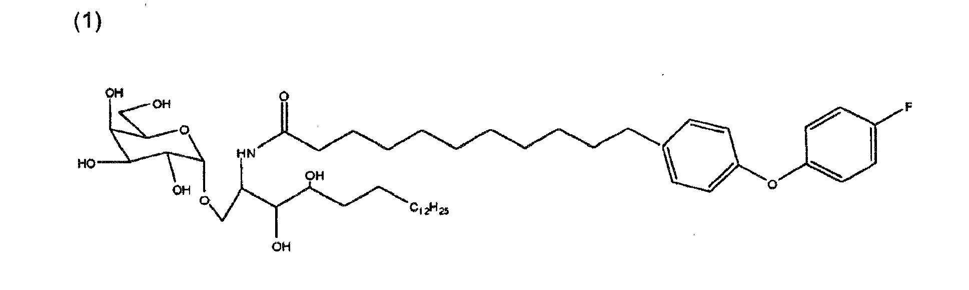 umfassend:
selektives Schützen von 2,3-Dihydroxygruppen von D-Lyxose durch 2-Methoxypropen in Gegenwart von Säure unter Erhalt eines Actonid-Intermediats;
Unterwerfen einer primären Hydroxylgruppe des Actonid-Intermediats Tritylchlorid und Basenbedingungen unter Erhalt eines Tritylether-Intermediats;
Umsetzen des Tritylether-Intermediats mit C₁₃H₂₇+PPh₃-Br durch Wittig-Olefinierung in Gegenwart von Lithiumhexamethyldisilazid (LHMDS), wodurch ein Alken-Intermediat mit dem E/Z-Verhältnis von 2:1 nach ¹H-NMR-Spektrometriecharakterisierung erhalten wird;
Hydrieren des Alken-Intermediats unter Erhalt eines Alkans;
Aktivieren einer Hydroxygruppe des Alkans durch Triflatanhydrid und 2,6-Lutidin unter Erhalt eines Triflat-Intermediats;
Umsetzen des Triflat-Intermediats mit Tetramethylguanidiniumazid (TMGA) durch S_{N}2-Reaktion unter Erhalten einer Azidoverbindung mit invertierter Konfiguration;
Entfernen einer Tritylgruppe der Azidoverbindung unter Verwendung von Trifluoressigsäure (TFA) unter Erhalt von Phytosphingosin;
Durchführen einer Glycosylierung von Donor-Galactosederivat und Akzeptor-Phytosphingosin unter Verwendung von Trifluormethansulfonsäureanhydrid (TF₂O) und Dimethylsulfid (Me₂S) als Promotoren unter Erhalt eines Schlüs-selintermediats, das eine Azidogruppe hat;
Reduzieren der Azidogruppe des Schlüsselintermediats unter Verwendung einer Staudinger-Reaktion unter Erhalt eines Amin-Intermediats und
Kuppeln des Amin-Intermediats mit Fettsäure unter Verwendung von EDC und HBTU.

10. Verbindung der Formel 1: 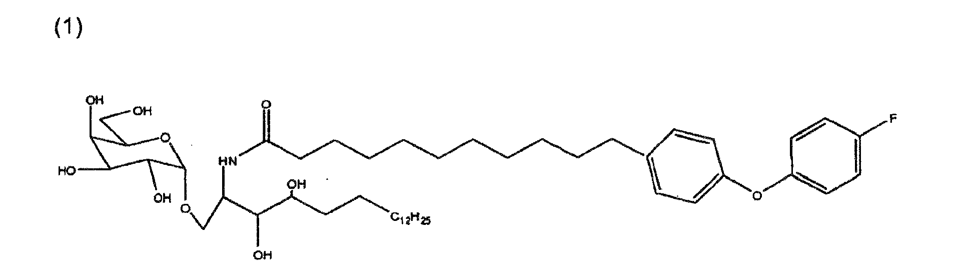 oder ein pharmazeutisch annehmbares Salz davon zur Verwendung in der Behandlung oder Prophylaxe einer Virus- oder Bakterieninfektion.

11. Verbindung gemäß Anspruch 10, wobei die Infektion durch eins aus der Gruppe, ausgewählt aus: Sphingomonas capsulata; japanischem Encephalitis-Virus; Staphylococcus aureus und Influenza, verursacht ist.

12. Verbindung der Formel 1: 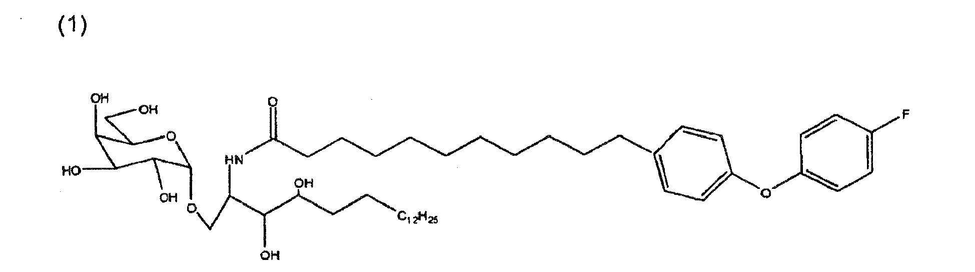 oder ein pharmazeutisch annehmbares Salz davon zur Verwendung in der Krebsbehandlung.

13. Verbindung gemäß Anspruch 12, wobei der Krebs aus der Gruppe, bestehend aus Lungenkrebs; Brustkrebs; Hepatom; Leukämie; solidem Tumor und Karzinom, ausgewählt ist.

14. Verbindung, dargestellt durch die Struktur der Formel 1: 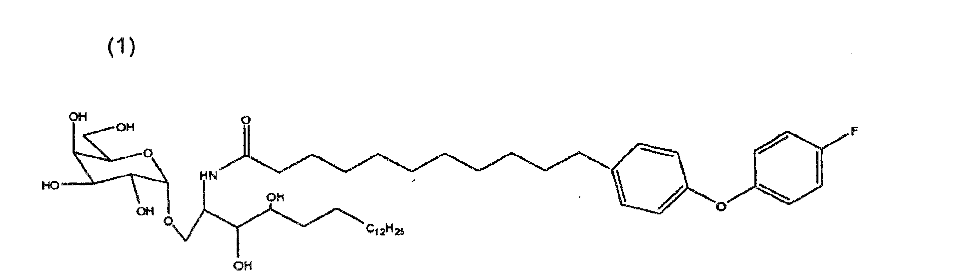 oder ein pharmazeutisch annehmbares Salz davon.

15. Verbindung gemäß Anspruch 14, dargestellt durch die Strutur 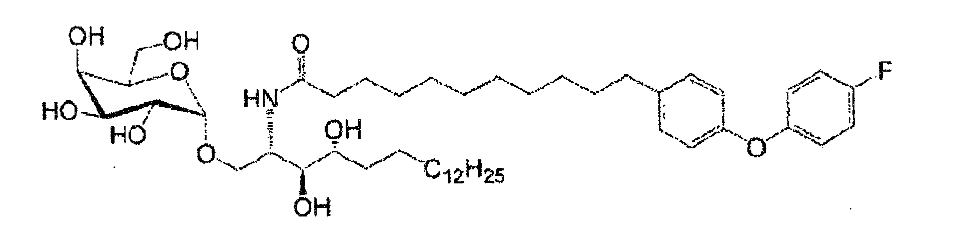 oder ein pharmazeutisch annehmbares Salz davon.

## Revendications

1. Composé représenté par la formule structurale 1 : 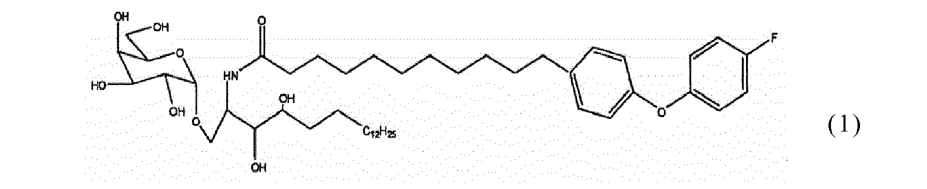 ou sel pharmacologiquement admissible de ce composé, pour utilisation en médecine.

2. Composition pharmaceutique comprenant du composé conforme à la revendication 1 et un véhicule pharmacologiquement admissible.

3. Composition conforme à la revendication 2, qui se présente sous forme d'un vaccin et dans laquelle le composé de formule 1 joue le rôle d'un adjuvant.

4. Composition conforme à la revendication 3, dans laquelle le vaccin comprend un agent vaccin, lequel agent vaccin est choisi dans l'ensemble formé par un microorganisme tué, un microorganisme virus vivant atténué, une anatoxine, un fragment d'un microorganisme inactivé ou atténué, un ADN, une protéine, une glycoprotéine, un saccharide, une combinaison de marqueurs de cellules cancéreuses, et une combinaison de marqueurs de microorganismes.

5. Composition conforme à la revendication 3 ou 4, laquelle composition exerce une activité efficace de neutralisation contre un virus grippal H5N1, lequel virus grippal H5N1 est de préférence choisi au nombre d'au moins un parmi les suivants : virus grippal H5N1 de type sauvage (E319), TK05, VN1 194, ID05, Anhui05, pCHA5 et pCHA5-II.

6. Composition conforme à la revendication 2 pour utilisation en immunothérapie anti-tumorale, laquelle composition se présente de préférence sous la forme d'un vaccin anti-cancer, le cancer étant de préférence choisi dans l'ensemble formé par un cancer pulmonaire, un cancer du sein, un hépatome, une leucémie, une tumeur solide et un carcinome.

7. Composition conforme à la revendication 2 pour utilisation dans le traitement ou la prophylaxie d'une infection virale ou bactérienne, laquelle infection est de préférence causée par un agent choisi dans l'ensemble constitué par les suivants : Sphingomonas capsulata, virus de l'encéphalite japonaise, staphylocoque doré, et agent grippal.

8. Composition conforme à l'une des revendications 2 à 7, laquelle composition est formulée de manière à être appropriée pour une administration par une voie choisie parmi les voies sous-cutanée, intraveineuse, intranasale, intramusculaire et orale.

9. Procédé de synthèse d'un composé représenté par la formule structurale 1 : 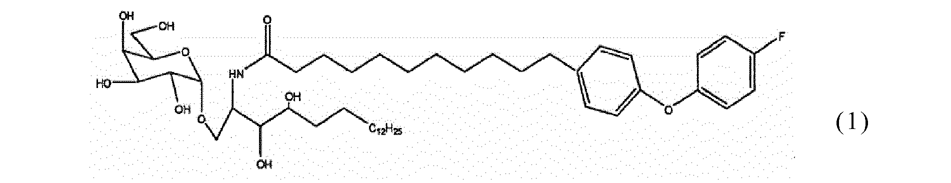 comportant les étapes suivantes :
- protéger sélectivement les groupes 2-hydroxyle et 3-hydroxyle du D-lyxose, avec du 2-méthoxy-propène, en présence d'un acide, ce qui donne un intermédiaire acétonide ;
- soumettre un groupe hydroxyle primaire de cet intermédiaire acétonide à l'action du chlorure de trityle, dans des conditions basiques, pour obtenir un intermédiaire éther de trityle ;
- faire réagir cet intermédiaire éther de trityle avec le composé de formule C₁₃H₂₇P⁺Ph₃,Br⁻, suivant une réaction de Wittig de formation d'oléfines, en présence de LHDMS (hexaméthyl-disilyl-amidure de lithium), ce qui donne un intermédiaire alcène dont le rapport E/Z, déterminé par spectrométrie de RMN du proton, vaut 2/1 ;
- hydrogéner l'intermédiaire alcène, pour obtenir un alcane ;
- activer un groupe hydroxyle de cet alcane, avec de l'anhydride trifluorométhanesulfonique et de la 2,6-lutidine, pour obtenir un intermédiaire triflate ;
- faire réagir cet intermédiaire triflate avec de l'azoture de tétraméthyl-guanidinium (TMGA), selon une réaction SN², pour obtenir un composé azoture à configuration inversée ;
- éliminer le groupe trityle de ce composé azoture au moyen d'acide trifluoroacétique (TFA) pour obtenir une phytosphingosine ;
- effectuer la glycosylation de cette phytosphingosine (accepteur) par un dérivé de galactose (donneur), en utilisant de l'anhydride trifluorométhanesulfonique (Tf₂O) et du sulfure de diméthyle (Me₂S) en tant que promoteurs, ce qui donne un intermédiaire clé doté d'un groupe azido ;
- réduire le groupe azido de cet intermédiaire clé, selon une réaction de Staudinger, pour obtenir un intermédiaire amine ;
- et coupler cet intermédiaire amine avec un acide gras, au moyen d'EDC et d'HBTU.

10. Composé de formule 1 : 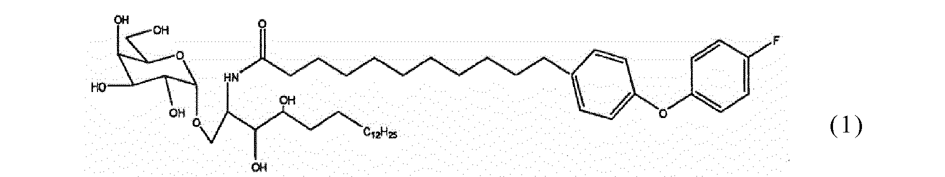 ou sel pharmacologiquement admissible de ce composé, pour utilisation dans le traitement ou la prophylaxie d'une infection virale ou bactérienne.

11. Composé conforme à la revendication 10, pour lequel l'infection est causée par un agent choisi dans l'ensemble formé par les suivants : Sphingomonas capsulata, virus de l'encéphalite japonaise, staphylocoque doré, et agent grippal.

12. Composé de formule 1 : 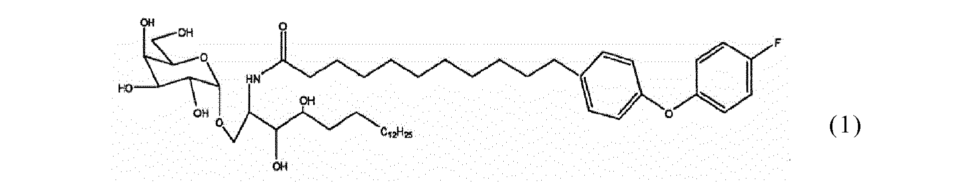 ou sel pharmacologiquement admissible de ce composé, pour utilisation dans le traitement d'un cancer.

13. Composé conforme à la revendication 12, pour lequel le cancer est choisi dans l'ensemble formé par un cancer pulmonaire, un cancer du sein, un hépatome, une leucémie, une tumeur solide et un carcinome

14. Composé représenté par la formule structurale 1 : 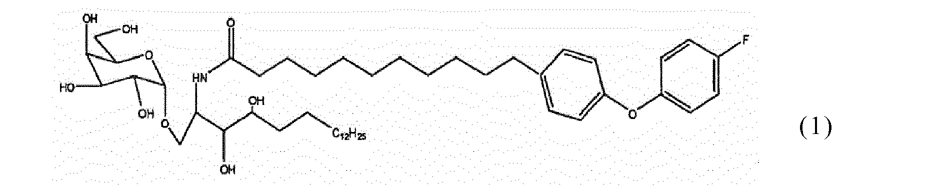 ou sel pharmacologiquement admissible de ce composé.

15. Composé conforme à la revendication 14, représenté par la formule structurale : 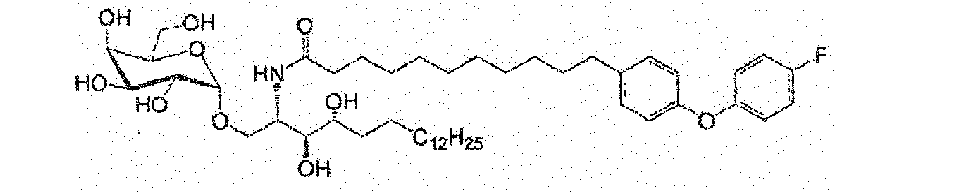 ou sel pharmacologiquement admissible de ce composé.
